(19) Europäisches Patentamt / European Patent Office / Office européen des brevets

(11) **EP 4 557 299 A1**

(12) **EUROPEAN PATENT APPLICATION**

(43) Date of publication:
**21.05.2025 Bulletin 2025/21**

(21) Application number: **23383180.9**

(22) Date of filing: **17.11.2023**

(51) International Patent Classification (IPC):
**G16B 20/10** (2019.01)    **C12Q 1/68** (2018.01)
**G16B 40/20** (2019.01)

(52) Cooperative Patent Classification (CPC):
(C-Sets available)
**G16B 20/10; C12Q 1/6869; C12Q 1/6886;**
**G16B 40/20;** C12Q 2600/106; C12Q 2600/118;
C12Q 2600/156                          (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicants:
• **Fundación Del Sector Público Estatalcentro**
**Nacional De Investigaciones Oncológicas Carlos**
**III**
**(F.S.P. CNIO)**
**28029 Madrid (ES)**

• **Fundación para la Investigación Biomédica**
**del Hospital Universitario 12 de Octubre**
**28041 Madrid (ES)**

(72) Inventor: **The designation of the inventor has not**
**yet been filed**

(74) Representative: **Mewburn Ellis LLP**
**Aurora Building**
**Counterslip**
**Bristol BS1 6BX (GB)**

(54) **PREDICTION OF GENE AMPLIFICATION**

(57)   Computer-implemented method of predicting whether a tumour is likely to acquire a gene amplification are provided, the method comprising obtaining a tumour copy number profile for the tumour; determining, for each of one or more signatures of chromosomal instability, a metric indicative of the presence of the signature in the tumour copy number profile, and determining whether the tumour is likely to acquire a gene amplification based on the output of a model trained to take as input the values of said metrics and produce as output a score indicative of the likelihood that the tumour will acquire a gene amplification. Methods of predicting treatment response and prognosis, and related systems and products are also described.

FIG. 1A

Obtain cohort of samples — 110

Obtain sequence data — 112

Obtain tumour copy number profile for sample — 114

Quantify set of copy number features of non-diploid segments (events) in copy number profile — 116

Determine event-specific associations with signature(s) of chromosomal instability — 118

Determine sample-level exposures to signature(s) of chromosomal instability — 120

Determine sample-normalised event specific associations for events including gene amplification event (if present) — 122

For each sample in cohort

Obtain signature weight for gene amplification — 124

Determine threshold for amplification score — 126

Provide results to user — 128

# FIG. 1B

(52) Cooperative Patent Classification (CPC): (Cont.)

C-Sets
**C12Q 1/6869, C12Q 2535/122, C12Q 2537/16,
C12Q 2537/165**

**Description**

FIELD OF THE DISCLOSURE

The present invention relates to methods of predicting whether a subject will acquire a gene amplification, and in particular to methods of predicting the likelihood of future gene amplification in a tumour genome based on copy number signature exposure in a sample comprising DNA from said tumour.

BACKGROUND

The most significant hurdle in precision medicine lies in the dynamic nature of tumours, evolving from benign to malignant, acquiring metastatic potential, and ultimately developing resistance to specific therapies. The amplification of oncogenes can play a role in all of these tumour progression steps.

Indeed, the detection of an oncogene amplification in a tumour can serve as a prognostic or diagnostic biomarker. Examples of oncogene-based interventions include increased surveillance in *MYCN* amplified neuroblastomas, treatment with Herceptin in HER2-amplified breast cancers, and treatment with MET inhibitors in non-small cell lung cancer with *MET* amplifications. Indeed, the presence of an oncogene amplification in a tumour often represents a critical point in tumour evolution where the tumour is more aggressive and difficult to treat.

Identification of tumours at an earlier evolutionary time point that don't yet have an amplification but are at high-risk of acquiring one in the future, offers the opportunity to realise the benefit of oncogene-based interventions earlier. However, there is no method that is able to do this accurately for a range of oncogenes at present.

SUMMARY

Activation of an oncogene via DNA amplification can underpin tumour initiation, progression and treatment resistance. The ability to predict future amplification using a genomic test offers new opportunities for improved disease management and treatment. The present inventors hypothesised that predicting oncogene amplification would be possible by developing a predictive model that includes both mutational processes - represented by a compendium of pan-cancer chromosomal instability (CIN) signatures that they previously published - and selection. They verified this hypothesis through extensive investigation of the mechanisms that underpin oncogene amplification and its relationship with signatures of chromosomal instability. Based on this, they developed a method to forecast oncogene amplification for individual patients. They trained predictive models for each oncogene using the different types of chromosomal instability found underlying oncogenic amplifications across 6,335 patients. They validated the approach using longitudinal pairs of glioma, lung cancer (lung adenocarcinoma and non-small cell carcinoma) and different tumour types (1 adrenal gland, 1 anus, 1 lymphoid tissue, 1 vagina, 2 penis, 2 thymus, 3 gallbladder, 3 gastroesophageal, 3 kidney, 3 small intestine, 4 bile duct, 5 head and neck, 6 pancreas, 7 ovary, 8 uterus, 9 unknown primary, 10 oesophagus, 14 urothelial tract, 16 bone/soft tissue, 28 skin, 42 colorectum, 45 lung, 59 prostate and 85 breast) from patients in the Netherlands with metastatic samples collected by the Hartwig Medical Foundation. To demonstrate clinical utility they forecasted poor prognosis in gliomas via *CDK4* amplification and osimertinib resistance in lung cancers via *MET* amplification. They additionally validated the approach using a cohort of samples with paired longitudinal data, where the latest sample from each patient is used to train predictive models and the resulting predictive models are applied to the earlier samples from the patients. They further provided guidelines and recommendations for clinical implementation paving the way for a new class of biomarker where the mutation generating processes in a tumour can be used to predict future genomic changes.

Thus, according to a first aspect, there is provided a computer-implemented method of predicting whether a tumour is likely to acquire a gene amplification, the method comprising: obtaining a tumour copy number profile for the tumour; determining, for each of one or more signatures of chromosomal instability, a metric indicative of the presence of the signature in the tumour copy number profile, wherein a signature of chromosomal instability comprises a set of weights for each of a plurality of components each associated with a copy number feature of a set of copy number features quantified for a plurality of segments in the copy number profile, wherein the weights are indicative of the probability that a mutational process associated with the signature generates a segment having characteristics defined by the component; and determining whether the tumour is likely to acquire a gene amplification based on the output of a model trained to take as input the values of said metrics and produce as output a score indicative of the likelihood that the tumour will acquire a gene amplification, wherein the model has been trained using copy number profiles for a plurality of tumours comprising tumours that have the gene amplification and tumours that do not have the gene amplification.

The method may have any one or more of the following optional features.

The gene may be an oncogene. The gene may be an oncogene selected from the genes listed in Tables 7 and 8. The model may be specific to a single gene. Alternatively, the model may be predictive for a plurality of genes. The plurality of genes may be oncogenes, such as oncogenes selected from the genes listed in Tables 7 and 8.

The model may be a model that has been trained to take as input the values of said metrics for each of a plurality of signatures of chromosomal instability and to produce as output a score indicative of the likelihood of acquiring a gene amplification. The model may have been trained to provide as output a score indicative of the likelihood of acquiring a gene amplification at a single specific gene. The model may have been trained to provide as output a score indicative of the likelihood of acquiring a gene amplification at any of a plurality of genes. The plurality of genes may each be oncogenes. The plurality of genes may comprise one or more or all oncogenes selected from Tables 7 and 8.

A gene amplification may refer to the presence of a segment encompassing the gene in a tumour copy number profile, where the segment has an absolute copy number >=6 or a copy number >=4 above tumour ploidy. A gene amplification may refer to the presence of a segment encompassing the gene in a tumour copy number profile, where the segment has an absolute copy number >=8.

The copy number features may comprise or consist of: segment size, copy number change-point, and breakpoint count in one or both flanking regions of a predetermined length. The breakpoint count may be a summarised value of the breakpoint count in the upstream and downstream flanking regions of a predetermined length around the segment. The summarised value may be the maximum. The predetermined length may be between 2 and 10 Mb, between 3 and 8 Mb, or about 5 Mb. Alternatively, the copy number features may comprise or consist of: segment size, copy number change-point, breakpoint count per predetermined length of sequence, breakpoint count per chromosome arm, and number of segments with oscillating copy number, optionally wherein the predetermined length of sequence is 10Mb.

Determining a metric indicative of the presence of a signature in the tumour copy number profile may comprise quantifying the value of each copy number feature for each of one or more segments in the copy number profile. Each of the one or more segments may be a non-diploid segment.

Quantifying the set of copy number features may comprise using unrounded copy number segments. Obtaining a copy number profile may comprise collapsing and merging near diploid segments to a diploid state, wherein near diploid segments are segments that have a copy number within a predetermined distance from 2. The predetermined distance may be 0.1.

Determining a metric indicative of the presence of a signature in the tumour copy number profile may comprises obtaining, for each of a plurality of segments in the copy number profile, a value for each of the plurality of components for the segment. Each component may define characteristics of a segment as a predetermined distribution of values of a copy number feature.

The value determined for each segment may be the probability of a quantified copy number feature of the segment belonging to each of a set of predetermined distribution associated with respective components of the plurality of components, for the copy number feature. Thus, the probability of the feature value from a segment belonging to the predetermined distribution associated with the component may be determined. This may be performed for each component, thereby obtaining a an "Event-by-component" probabilities vector for each segment, or an event-by-component probability matrix comprising respective vectors for each of a plurality of segments from the same copy number profile. These probabilities may be referred to as "posterior probabilities".

The components may define a respective distribution of values of the associated copy number feature. The components may be selected from those in Table 1 or those in Table 11, or corresponding distributions obtaining by fitting mixture models to values for the set of copy number features obtained from copy number profiles from a plurality of tumour samples. Each copy number feature may be associated with a plurality of components, and each component may define a characteristic of copy number events using a respective predetermined distribution, wherein the predetermined distributions for a copy number feature that is quasi-continuous (e.g. segment size, copy number changepoint) is a set of Gaussian distributions, and/or wherein the predetermined distributions for any count feature (e.g. breakpoint count in one or both flanking regions of a predetermined length) is a set of Poisson distributions. The predetermined distributions associated with the components may be the distributions defined by the parameters in Table 1, or corresponding distributions obtained by fitting mixture models to values for the set of copy number features obtained from copy number profiles from a plurality of tumour samples. The predetermined distributions may comprise between 15 and 25 Gaussian distributions for the segment size, between 5 and 15 Gaussian distributions for the copy number changepoint, and/or between 5 and 15 Poisson distributions for the breakpoint count in one or both flanking regions of a predetermined length.

The set of copy number features may comprise a copy number change-point, wherein the copy number changepoint of a current segment is determined by reference to the upstream segment of the current segment when the copy number changepoint relative to the upstream segment is at or above a predetermined threshold, by reference to the downstream segment of the current segment when the copy number changepoint relative to the upstream segment is below the predetermined threshold and the copy number changepoint relative to the downstream segment is at or above the predetermined threshold, and by reference to a reference copy number value when the copy number changepoints relative to the upstream and downstream segments are below the predetermined threshold. The reference copy number value may be 2. The predetermined threshold may be -3.

The metric indicative of the presence of a signature in the tumour copy number profile may bethe activity of the signature in the tumour copy number profile, optionally wherein the activity of one or more signatures in a tumour copy number profile is determined by:

> obtaining, for each component, the sum of probabilities associated with the respective component for each of a plurality of segments in the copy number profile; and
> determining a linear combination of one or more signatures comprising the signature that satisfies the equation

$$\text{the equation } PbC \approx E \times SbC \qquad \text{(Equation 1)}$$

> where E is a vector of size n comprising coefficients $E_{1,...,n}$ where $E_i$ is the activity of signature i; PbC is a vector of size c, each element in the vector representing a sum of probabilities associated with a respective component; and SbC is a matrix of size c by n, each value representing the weight of a component in a signature $l$, and the probability associated with a component for a segment is the probability of a segment with a copy number feature value equal to the value for the segment having been drawn from a distribution associated with the component.

The signatures may be selected from those in Table 3 or those in Table 12. The signatures may be selected from CX1, CX2, CX3, CX4, 5, CX8, CX9, CX10, and CX13 defined in Table 3 or Table 12 or corresponding signatures obtained by quantifying the set of copy number features in a plurality of tumour samples, and identifying one or more signatures likely to result in the copy number profiles of the plurality of tumour samples by nonnegative matrix factorisation.

The model may be specific to a tumour type that is the same tumour type as that of the tumour that is being analysed. Such a model may be obtained by training the model using a reference cohort of samples comprising samples of said tumour type.

The trained model may be a model that produces a score indicative of the likelihood of acquiring an oncogene amplification as a weighted combination of the values of the metrics indicative of the presence of the one or more signatures in the tumour copy number profile. The combination may be a linear combination. The model may produce a score according to the following equation:

$$score = \sum_{m=1}^{n} CX_m \omega_m$$

where $CX_m$ is the activity of signature m in the tumour copy number profile, $\omega_m$ is a signature weight for signature m for the gene, and n is the total number of signatures considered in the model.

The weights of the model may be determined in a tumour type-specific manner using a reference cohort of samples as, for each signature, a ratio of (i) an averaged signature score of all amplified segments spanning the gene in samples of the specific tumour type in the reference cohort and (ii) an averaged signature scores of all non-diploid segments found in the reference cohort, wherein the signature score for a segment and signature is a metric indicative of association between the signatures of chromosomal instability and the segment. Said metric may be the sum of the product of probabilities associated with each of the plurality of components of the signature for the segment by the corresponding weight in the signature, the probability for a segment being the probability that a segment with a copy number feature value equal to the value for the segment having been drawn from a distribution associated with the component, or an activity corrected and/or normalised version of said sum, wherein an activity corrected version of said sum is the value of the sum multiplied by the activity of the signature in the copy number profile and a normalised version of said sum or activity corrected sum is a value obtaining by dividing the value by the sum of corresponding values for each of the one or more signatures.

The weights of the model may be determined in a tumour type-agnostic manner using a reference cohort of samples as, for each signature, a ratio of (i) a weighted average signature score of all amplified segments spanning the gene in samples of a specific tumour type in the reference cohort, wherein the average signature scores in the specific tumour type are weighted by the normalised empirical frequency of the gene amplification in the respective tumour type in the reference cohort and (ii) an averaged signature scores of all non-diploid segments found in the reference cohort, wherein the signature score for a segment and signature is a metric indicative of association between the signatures of chromosomal instability and the segment. Said metric may be the sum of the product of probabilities associated with each of the plurality of components of the signature for the segment by the corresponding weight in the signature, the probability for a segment being the probability that a segment with a copy number feature value equal to the value for the segment having been drawn from a distribution associated with the component, or an activity corrected and/or normalised version of said sum, wherein an activity corrected version of said sum is the value of the sum multiplied by the activity of the signature in the copy number profile and a normalised version of said sum or activity corrected sum is a value obtaining by dividing the value by the sum of corresponding values for each of the one or more signatures.

In embodiments, the weights ($\omega_m$) are determined as $\omega_{OG,t,m} = \dfrac{q_{OG,t,m}}{q_m}$ where:

$\omega_{OG,t,m}$ is the weight for gene OG for signature m in samples of tumour type t in a reference cohort,

$q_{OG,t,m}$ is given by: $q_{OG,t,m} = \dfrac{\sum_{a=1}^{A_{t,OG}} e_{a,m}}{A_{t,OG}}$ where $e_{a,m}$ is the activity-corrected score signature (m) for a particular amplified event (a) encompassing gene OG in samples of tumour type t in the reference cohort; $A_{t,OG}$ is the number of amplified segments spanning gene OG in the samples of the tumour type (t) in the reference cohort, and

$q_m$ is given by $q_m = \dfrac{\sum_{p=1}^{P} e_{p,m}}{P}$ $e_{p,m}$ is the activity-corrected score of signature m for a particular copy number event ($p$) in a copy number profile of the reference cohort; and $P$ is the total number of copy number events observed in the copy number profiles of the reference cohort. Such a model may be referred to as a tumour-type specific model.

In embodiments, the weights ($\omega_m$) are determined as $\omega_{OG,m} = \dfrac{q_{OG,m}}{q_m}$ where:

$\omega_{OG,m}$ is the weight for gene OG for signature m in the reference cohort,

$q_{OG,m}$, is given by $q_{OG,m} = \sum_{t=1}^{T} \dfrac{f_{OG,t}}{\sum_{t=1}^{T} f_{OG,t}} \times q_{OG,t,m}$ where $q_{OG,t,m}$ is given by:

$q_{OG,t,m} = \dfrac{\sum_{a=1}^{A_{t,OG}} e_{a,m}}{A_{t,OG}}$ where $e_{a,m}$ is the activity-corrected score signature ($m$) for a particular amplified event ($a$) encompassing gene OG in samples of tumour type t in the reference cohort; $A_{t,OG}$ is the number of amplified segments spanning gene OG in the samples of the tumour type ($t$) in the reference cohort,

$q_m$ is given by $q_m = \dfrac{\sum_{p=1}^{P} e_{p,m}}{P}$ where $e_{p,m}$ is the activity-corrected score of signature $m$ for a particular copy number event ($p$) in a copy number profile of the reference cohort; and $P$ is the total number of copy number events observed in the copy number profiles of the reference cohort, and

$f_{OG,t}$ is the empirical frequency of the amplification of gene OG in a tumour type $t$ in the reference cohort; and $T$ is the number of tumour types in the reference cohort. Such a model may be referred to as a pancancer model.

The weights determined in a tumour type-specific manner may be used when the reference cohort comprises less than a threshold number of samples of the specific tumour type or the frequency of amplification of the gene in the specific tumour type in the reference cohort is lower than a threshold frequency. The threshold number of sample may be 5 and the threshold frequency may be 5%. The weights determined in a tumour type-agnostic manner may be used otherwise.

The tumour may be a primary and/or untreated tumour. The tumour may be a treated and/or advanced tumour and the weights of the model are determined in a tumour type-agnostic manner using for (i) only tumour types in which the gene is recurrently amplified in the reference cohort, optionally wherein a gene is recurrently amplified in a tumour type if it is amplified in more than 5% of the samples of the tumour type in the reference cohort.

$$score = \sum_{m=1}^{9} CX_m \cdot \omega_m$$

The output of model may be a score given by: , where m refers to one of CX1, CX2, CX3, CX4, 5, CX8, CX9, CX10, and CX13 defined in Table 3 or Table 12 or corresponding signatures obtained by quantifying the set of copy number features in a plurality of tumour samples, and identifying one or more signatures likely to result in the copy number profiles of the plurality of tumour samples by nonnegative matrix factorisation, CX represents the signature activities in the tumour copy number profile, and $\omega$ represents the signature weights computed for a given gene.

The method may further comprise classifying the tumour between a first class that has a high likelihood of acquiring the gene amplification and a second class that has a low likelihood of acquiring the gene amplification, wherein the tumour is classified in the first class when the output of the model is above a predetermined threshold. The predetermined threshold may have been determined by obtaining the output of the model for each of a plurality of samples in a reference cohort with a known expected % of samples with the gene amplification, and selecting the threshold that is such that a % of samples corresponding to the expected % of samples are classified in the first class. The predetermined threshold may have been determined by obtaining the output of the model for each of a plurality of samples in a reference cohort with known gene amplification status, and selecting the threshold that satisfies one or more predetermined criteria that apply to the sensitivity, selectivity or Youden's index when used to classify the samples between the first and second classes.

The gene may be selected form those in Table 9. The gene may be selected from MET, AR, EWSR1, JUN, RBFOX2, POLD1, MDM2, CDK4, HIST1H3B, and ERBB2. The gene may be selected from Table 9 and the model may be a corresponding linear model with weights specified in Table 7 or Table 8, or equivalent model trained using a different reference cohort.

The model may have been trained using a reference cohort comprising samples that are one or more of: the same tumour type, the tumour grade/stage, undergone the same treatment, have the same driver mutations, have been analysed with the same sequencing technology, as the tumour to be analysed.

The tumour may be selected from sarcoma, breast cancer, lung adenocarcinoma, ovarian cancer, bladder carcinoma, glioblastoma, head and neck squamous cell carcinoma, renal adenocarcinoma, lung squamous cell carcinoma, adenocarcinoma, stomach adenocarcinoma, esophagal carcinoma, liver hepatocellular carcinoma, Cervical Squamous Cell Carcinoma and Endocervical Adenocarcinoma, Uterine Corpus Endometrial Carcinoma, Colon Adenocarcinoma, Brain Lower Grade Glioma.

Also described herein according to a second aspect is a computer-implemented method of predicting whether a tumour is likely to respond to a therapy, the method comprising: predicting whether the tumour is likely to acquire a gene amplification using the method of any embodiment of the first aspect, wherein the gene amplification is associated with resistance to the therapy, and whether a tumour predicted as likely to acquire the gene amplification is unlikely to respond to the therapy.

The gene may be MET and the therapy may be an EGFR inhibitor. The method may further comprise recommending a subject with a tumour that is unlikely to respond to the therapy for upfront treatment with both EGFR and MET inhibitors. The gene may be BRAF and the therapy may be a MEK inhibitor. The gene may be androgen receptor (AR), the tumour may be a prostate tumour and the therapy may be androgen deprivation therapy.

Also described herein according to a third aspect is a computer-implemented method of providing a prognosis for a subject with a tumour, the method comprising: predicting whether the tumour is likely to acquire a gene amplification using the method of any embodiment of the first aspect, wherein the gene amplification is associated with poor prognosis. The gene may be CDK4 and the tumour may be a low grade glioma.

Also described herein according to a fourth aspect is a computer-implemented of providing a model for predicting whether a tumour is likely to acquire a gene amplification, the method comprising: obtaining, a plurality of tumour copy number profiles for a reference cohort of samples, the plurality of tumour copy number profiles comprising copy number profiles that comprise the gene amplification and copy number profiles that do not comprise the amplification; and determining, for each of one or more signatures of chromosomal instability, a weight that is a ratio of (i) an average signature score of all amplified segments spanning the gene in samples of a specific tumour type in the reference cohort, optionally wherein the average signature scores in the specific tumour type are weighted by the normalised empirical frequency of the gene amplification in the respective tumour type in the reference cohort and (ii) an average signature scores of all non-diploid segments found in the reference cohort, wherein the signature score for a segment and signature is a metric indicative of association between the signatures of chromosomal instability and the segment. Said metric may be the sum of the product of probabilities associated with each of the plurality of components of the signature for the segment by the corresponding weight in the signature, the probability for a segment being the probability that a segment with a copy number feature value equal to the value for the segment having been drawn from a distribution associated with the component, or an activity corrected and/or normalised version of said sum, wherein an activity corrected version of said sum the value of the sum multiplied by the activity of the signature in the copy number profile and a normalised version of said sum or activity corrected sum is a value obtaining by dividing the value by the sum of corresponding values for each of the one or more signatures.

The method according to the current aspect may have any of the features described in relation to the first aspect.

According to any embodiment of any aspect, a tumour copy number profile or sample may have been obtained from a subject who has been diagnosed as having cancer. A tumour copy number profile may have been obtained by analysing sequence data. The sequence data may have been obtained using sequencing. The sequencing may be selected from WGS, sWGS, single cell WGS, WES or panel sequencing, or a copy number array.

The method may further comprise determining that the copy number profile comprises a number of non-diploid segments above a predetermined threshold. The copy number profile may comprise at least 15 non-diploid segments.

Obtaining the tumour copy number profile may comprise determining the tumour copy number profile from sequence data. The sequence data may be next-generation sequencing data.

Determining the tumour copy number profile may comprise determining a copy number for each of a plurality of genomic bins of a predetermined size. The predetermined size may be at most 100kb, at most 50kb, or about 30kb. The method may comprise obtaining a tumour copy number profile from DNA sequence data comprising a plurality of sequence read, wherein the tumour copy number profile comprises a copy number estimate for each of a plurality of genomic bins comprising a number of mapped reads above a predetermined threshold. The predetermined threshold may be 15 reads.

Methods of the disclosure also encompass treating a subject who has been diagnosed with cancer, the method comprising determining whether the subject is likely to respond to a therapy as described herein and administering the therapy to a subject who has been determined as likely to respond to the therapy and/or administering a treatment that does not comprise the therapy or that comprises the therapy in addition to a further therapy to a subject who has been determined as unlikely to respond to the therapy.

According to a further aspect, there is provided one or more non-transitory computer readable media comprising instructions that, when executed by one or more processors, cause the one or more processors to perform the steps of any method described herein, such as a method according to any embodiment of any preceding aspect.

According to a further aspect, there is provided a computer program comprising code which, when the code is executed on a computer, causes the computer to perform the steps of any method described herein, such as a method according to any embodiment of any of the first to fourth aspects.

According to a further aspect, there is provided system comprising: a processor; and a computer readable medium comprising instructions that, when executed by the processor, cause the processor to perform the steps of any method described herein, such as a method according to any embodiment of any of the first to fourth aspects.

BRIEF DESCRIPTION OF THE FIGURES

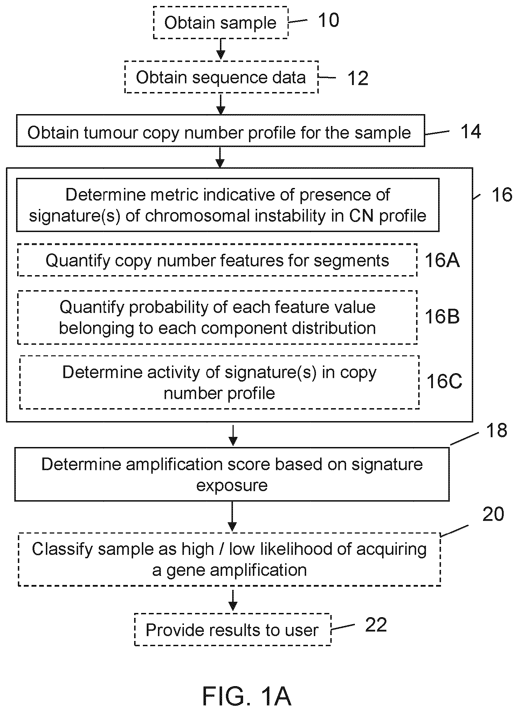

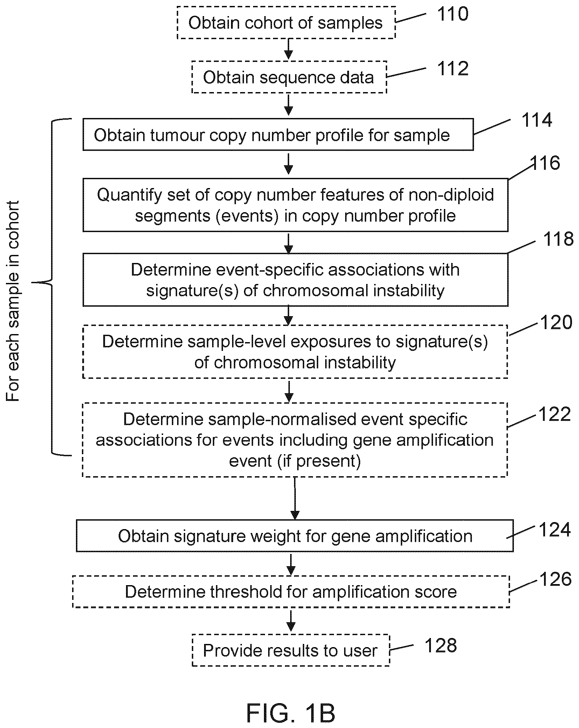

Figure 1 is a flowchart illustrating schematically a method of predicting whether a tumour in a subject is likely to acquire a gene amplification (**A**), and a method of obtaining a model for predicting whether a tumour in a subject is likely to acquire a gene amplification (**B**).

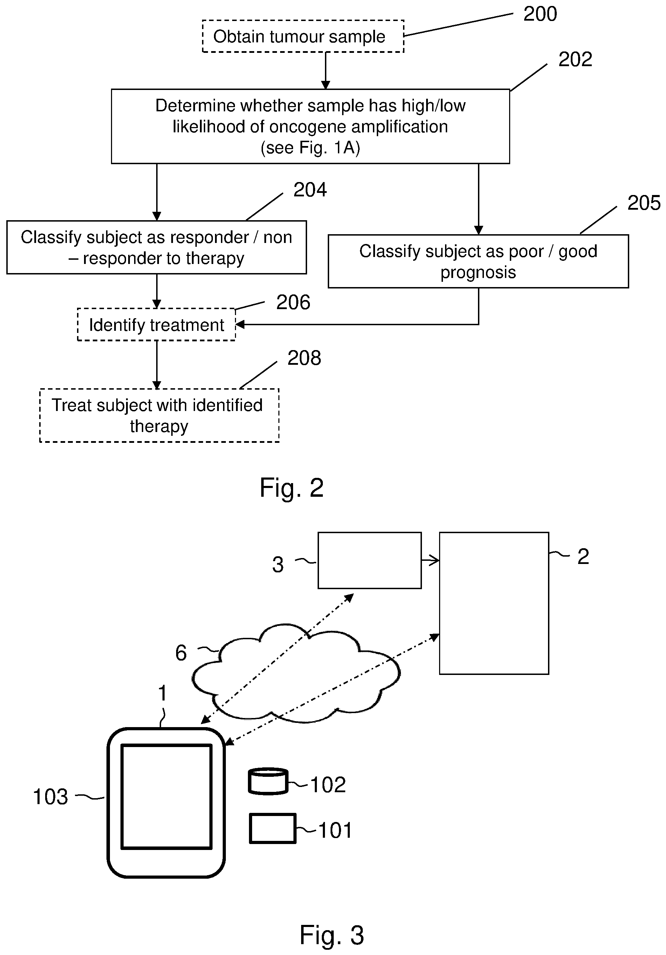

Figure 2 is a flowchart illustrating schematically methods of providing a prognosis, identifying a therapy or treating a subject.

Figure 3 shows an embodiment of a system for performing methods of the disclosure.

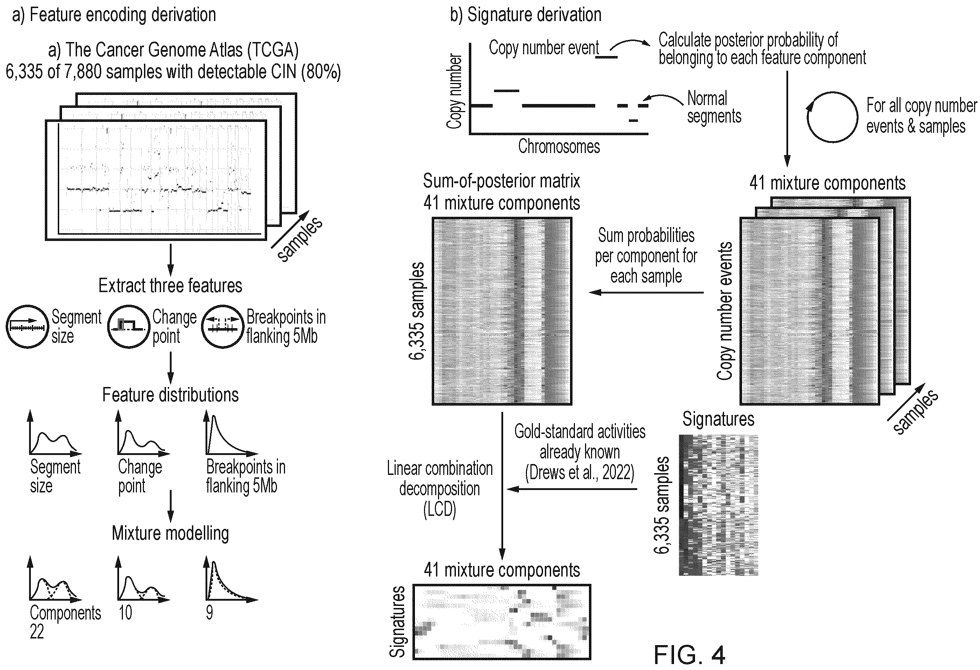

Figure 4 shows a schematic overview of copy number features and CIN signature identification according to embodiments of the disclosure, **a)** Schematic of feature encoding design and derivation of feature components. Mixture modelling was applied to split the 3 feature distributions into smaller components by either Variational Bayes Gaussian mixture models (for segment size and change point) or Finite Poisson mixture models (for breakpoints in flanking 5Mb). **b)** For all samples and features, the probability of each copy number event (no diploid segments) to belong to each mixture component is computed. Then, component probabilities of all events are summed to obtain a 41-dimensional feature vector per sample. In the illustrated examples, the activities of CIN signatures according to Drews et al. 2022 were already known for all 6,335 samples of the TCGA cohort (see Example 2 below). Therefore, linear combination decomposition was applied for deriving signature definitions in the new feature space.

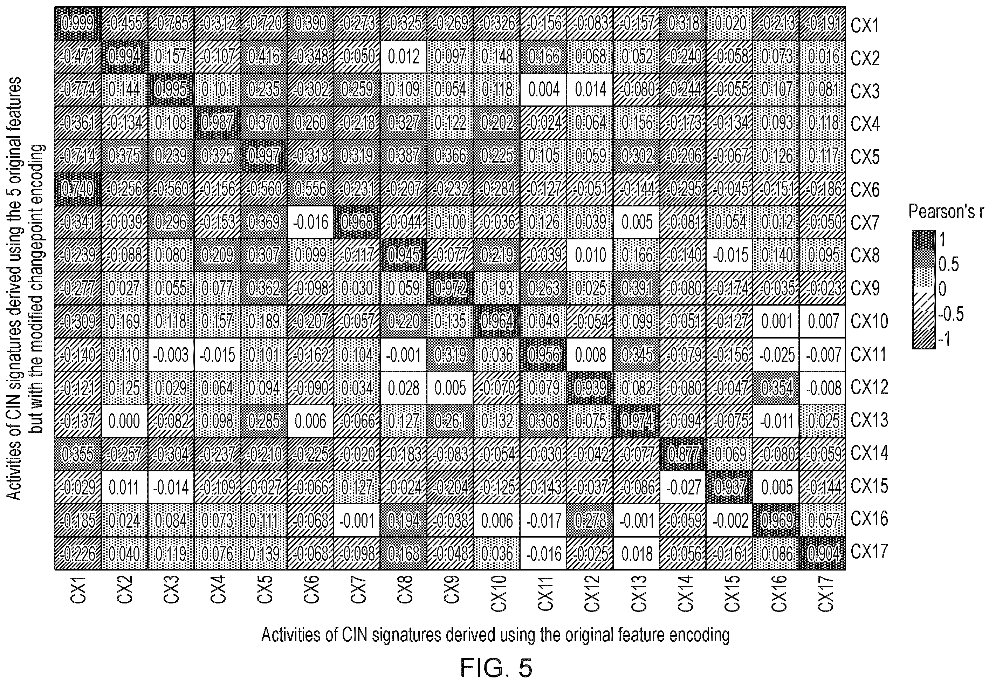

Figure 5 shows data investigating the impact of modifying the changepoint feature encoding in CIN signatures. The heatmap shows Pearson correlation coefficients between CIN signature activities derived by using the feature encoding (x-axis) described in Drews et al. 2022 and those derived by extracting the 5 original features but using the modified encoding version of the changepoint feature described in Example 1. Red colour denotes a high positive correlation between activities, while blue colour denotes a high negative correlation.

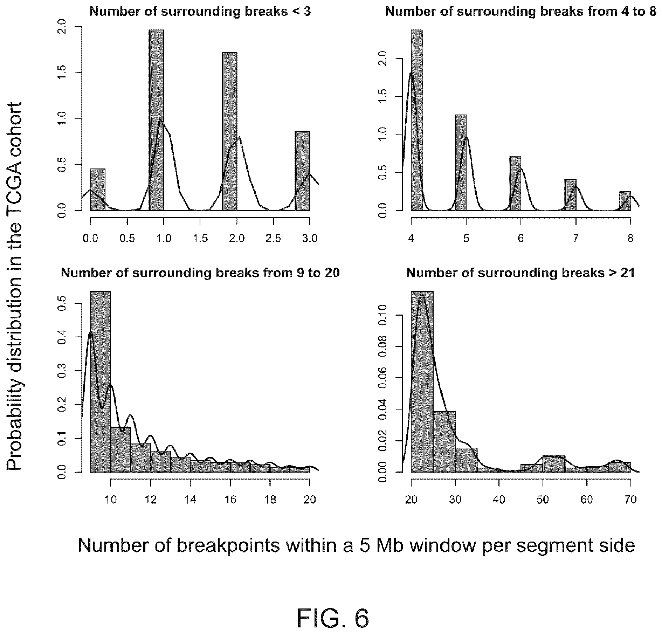

**Figure 6** displays histograms and fitted density curves showing the distribution of the breakpoints per side feature in the TCGA cohort (see Example 1). For visualisation purposes, the data are split in four subsets according to the number of breakpoints within a 5 Mb window per segment side. The combined distribution is used for deriving the different feature components capturing the signal of this novel feature.

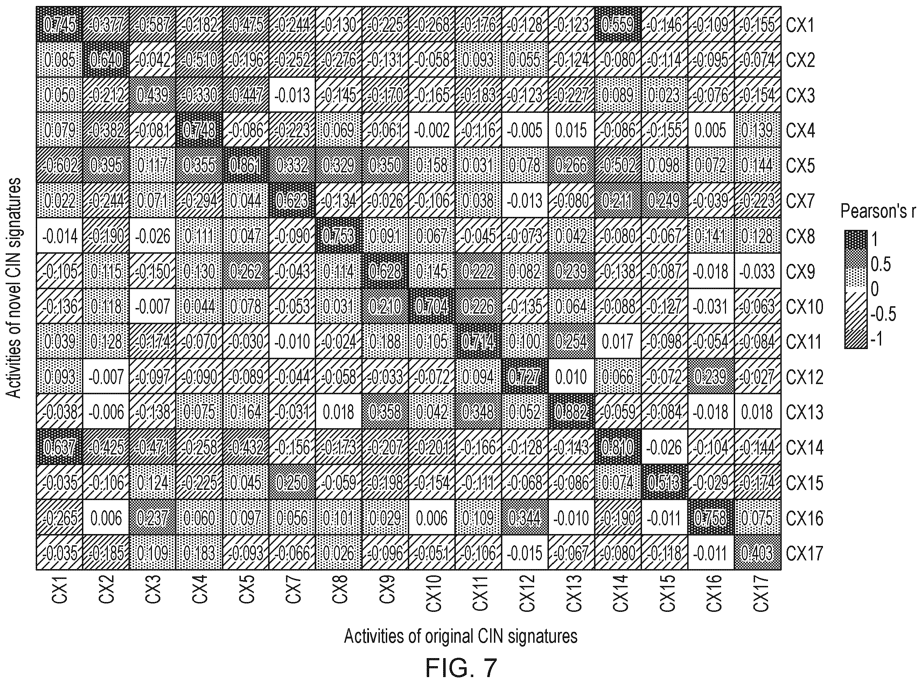

**Figure 7** shows a comparison of CIN signatures encoded using the original features (Drews et al. 2022) and the new features of the present disclosure. The heatmap shows Pearson's r correlation coefficients between activities of original (x-axis) and novel (y-axis) CIN signatures computed in 6,335 TCGA samples. Red colour denotes a high positive correlation between activities, while blue colour denotes a high negative correlation.

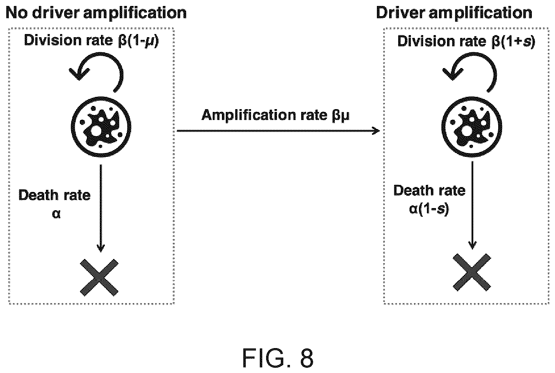

**Figure 8** shows an overview of simulations of oncogene amplification acquisition and selection. Schematics of birth-death simulations of tumour growth (fitness advantage: s, death rate: $\alpha$, birth rate: $\beta$, amplification rate: $\mu$). Tumour cells without amplification die with rate $\alpha$, divide yielding two cells without amplification with rate $\beta(1-\mu)$ and divide giving rise to one cell with amplification and another without with rate $\beta\mu$ (left box). Tumour cells with amplifications (right box) have a fitness advantage and as such die with rate $\alpha(1-s)$ and divide with rate $\beta(1+s)$, giving rise to two cells with amplification.

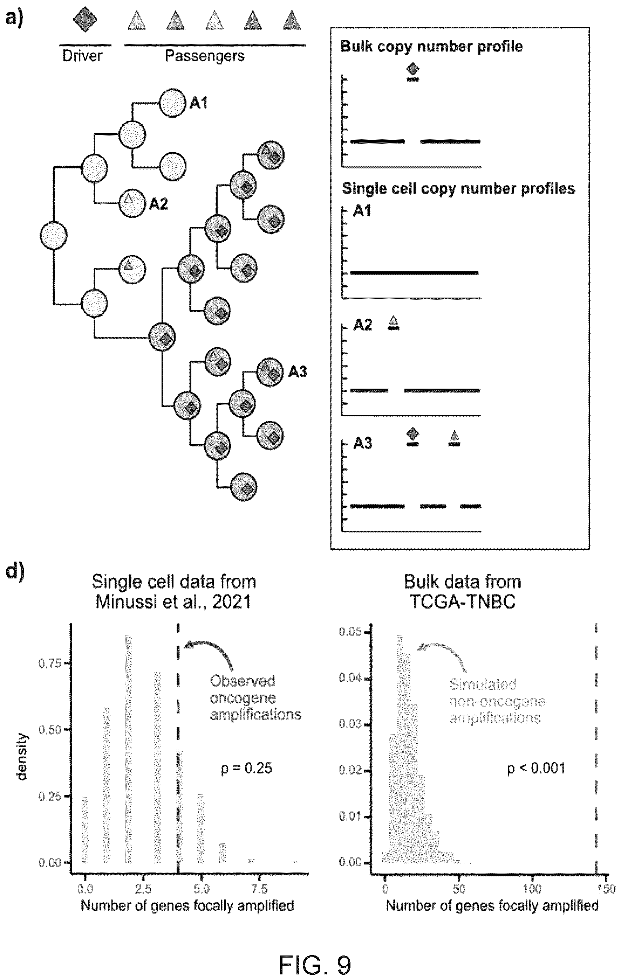

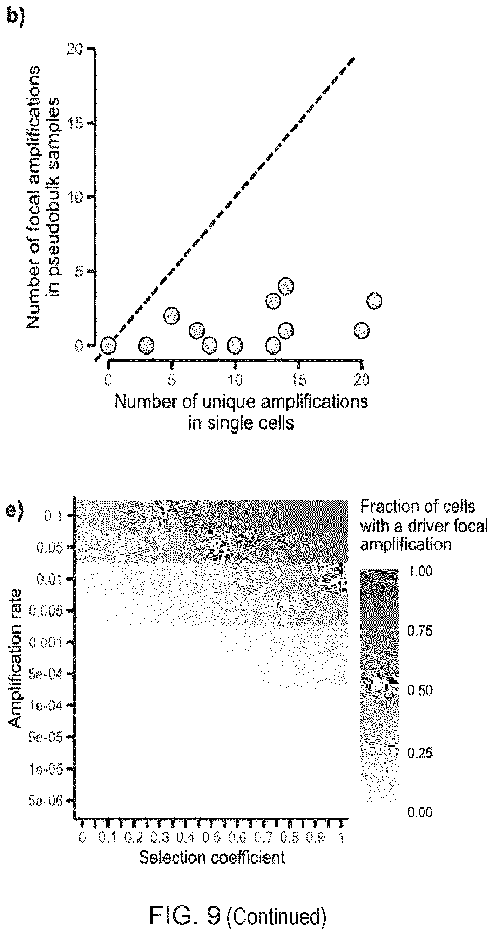

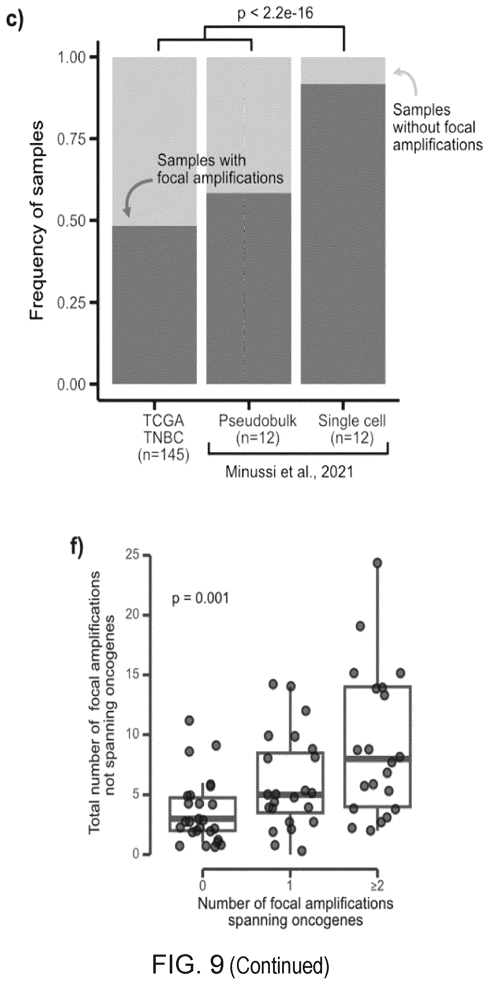

**Figure** 9 shows data investigating the acquisition, selection and expansion of focal amplifications in triple-negative breast cancer. **a)** Schematic showing the ability to identify unique and clonally expanded focal amplifications in single cell and bulk DNA sequencing data. **b)** Scatter plot comparing the number of unique focal amplifications detected across all single cells with the number of clonally expanded focal amplifications seen at pseudobulk resolution in 8 triple-negative breast cancer (TNBC) samples and 4 TNBC cell lines. **c)** Bar plot showing the frequency of samples with (red) and without (grey) focal amplifications in the TCGA-TNBC cohort (n=145), and in the TNBC tumours and cell lines from b) found at pseudobulk (clonally expanded amplifications) and single cell level (unique amplifications). P-value estimated by Binomial test. **d)** Histograms showing the expected number of oncogene amplifications in single cells (left) and in the TCGA-TNBC cohort (right) based on 1,000 iterations with lists of random genes. The red dashed line highlights the observed number of oncogenes amplified. Statistical significance was measured by calculating the empirical p-value. **e)** Mean fraction of cells harbouring an oncogene amplification at the end of 20 replicate birth-death tumour growth simulations using different combinations of selection coefficient (x-axis) and amplification rate (y-axis) (see Figure 8 for simulation scheme). **f)** Box plot comparing the number of focal amplifications in the TCGA-TNBC cohort spanning (x-axis) or not (y-axis) breast-cancer specific oncogenes. P-value estimated by Jonckheere's trend test.

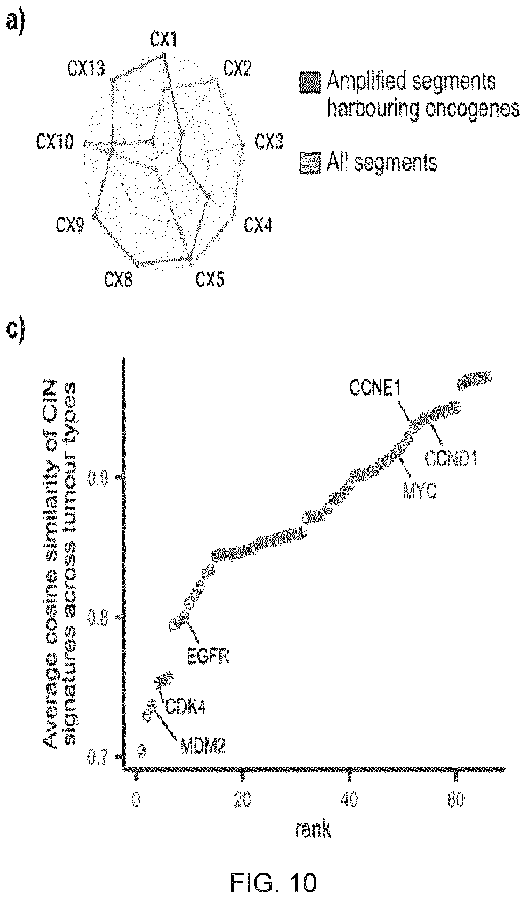

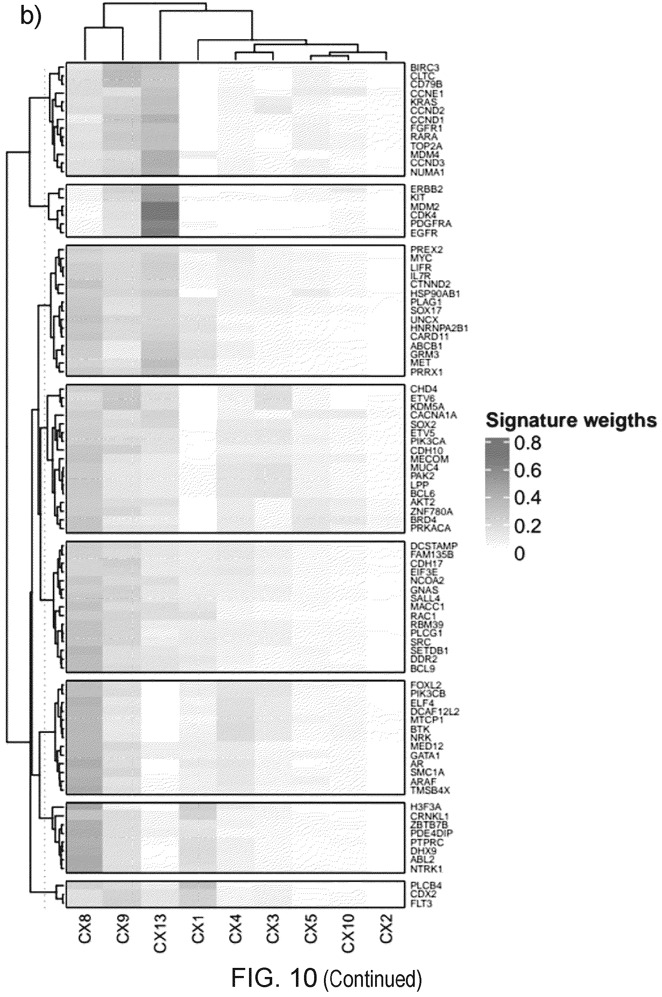

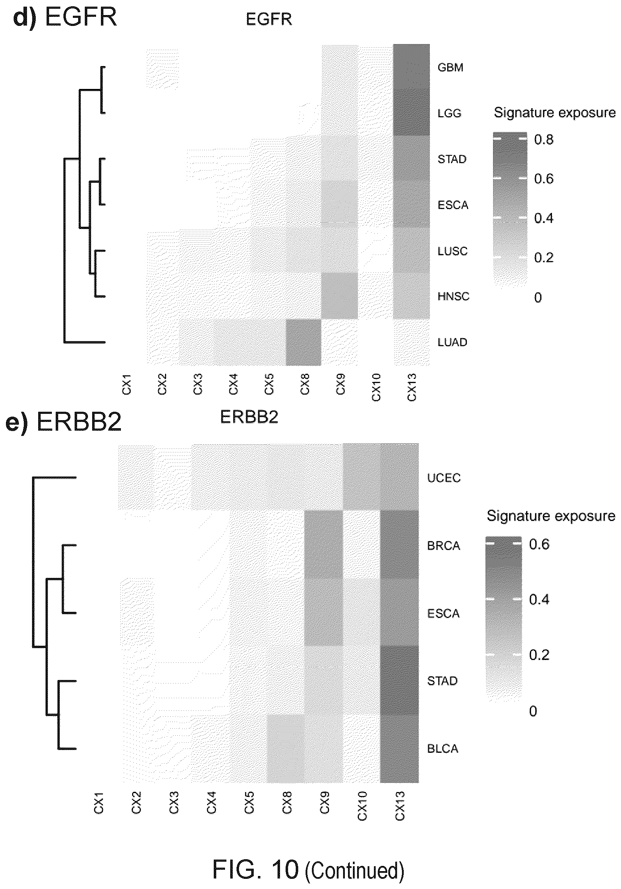

**Figure 10** shows data investigating the types of chromosomal instability underpinning oncogene amplification. **a)** Spider chart representing the relative scores of chromosomal instability (CIN) signatures in amplified segments harbouring an oncogene with respect to CIN signature scores of all copy number events across TCGA. **b)** Heatmap highlighting the average CIN signature weights of amplified segments harbouring a specific oncogene across TCGA. Signature aetiologies are: CX3, CX2, CX5: IHR, impaired homologous recombination; CX4: WGD, whole-genome duplication; CX10: impaired NHEJ, non-homologous end joining; CX8, CX13, CX9: replication stress; CX1: mitotic errors. **c)** Rank plot showing average cosine similarity across CIN signature weights assigned to amplifications of a given oncogene in the different TCGA tumour types. Oncogenes are ranked in increasing order of cosine similarities. **d-e)** Heatmap representation of the average CIN signature scores of amplified segments mapping **d)** EGFR and **e)** ERBB2 across samples per tumour type. Scores are scaled per tumour type to compare relative contribution of each signature across tumour types. Only tumour types with at least 1% of all samples and a minimum of 5 samples harbouring the oncogene amplification were included.

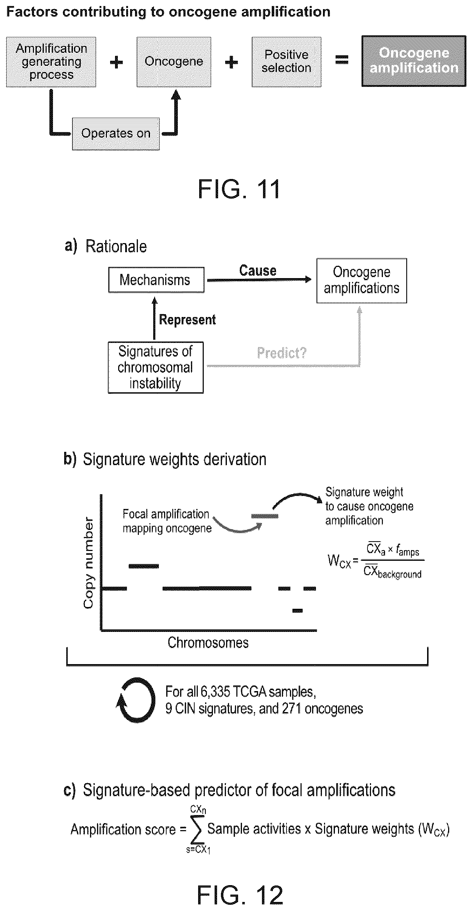

**Figure 11** illustrates schematically the different factors contributing to the amplification of a given oncogene.

**Figure 12** illustrates schematically a process of building a signature-based predictor of focal amplification according to embodiments of the disclosure, **a)** Rationale for building a chromosomal instability signature-based approach for predicting focal amplifications. **b)** Schematic for computing the weight of a CIN signature to cause the amplification of a given oncogene. Signature weights were derived by evaluating 271 oncogenes and using 6,335 TCGA tumours of 33 cancer types.

$$\overline{CX_a}$$

represents mean signature scores of amplified segments spanning a given oncogene. $f_{amps}$ represents the fraction of samples from a tumour type out of the total number of samples across tumour types harbouring the amplification of the specific oncogene; note that $f_{amps}$ is equal to 1 when calculating tumour-type specific weights. and $\overline{CX_{background}}$ represents mean signature scores of all copy number events (see Methods for further details). **c)** Schematic of the signature-based predictor application. The amplification score of a sample is computed by summing up the result of multiplying activities of each CIN signature by its relative weight to the acquisition of an oncogene amplification.

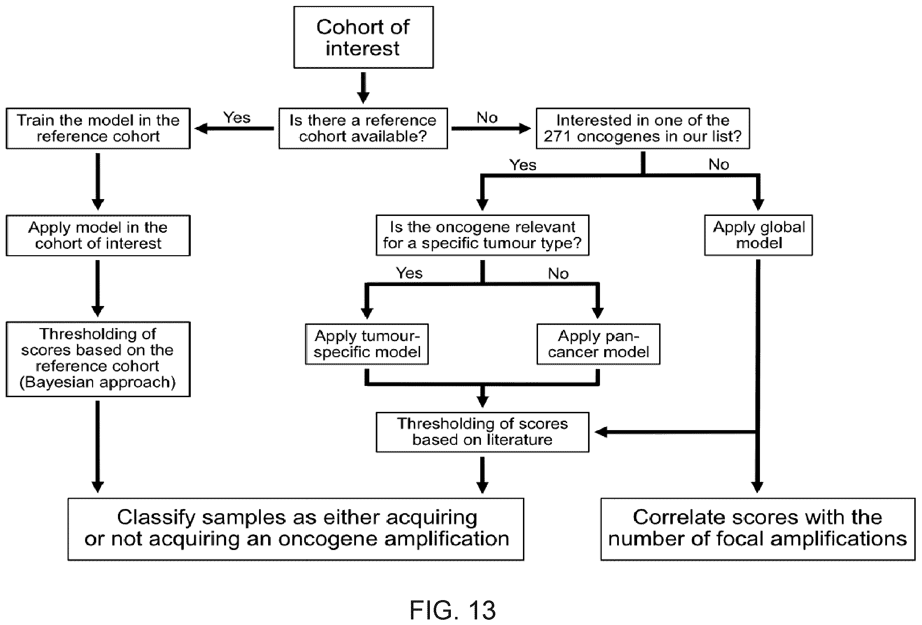

**Figure 13** illustrates schematically a workflow for applying a signature-based predictor according to the disclosure to a new clinical sample.

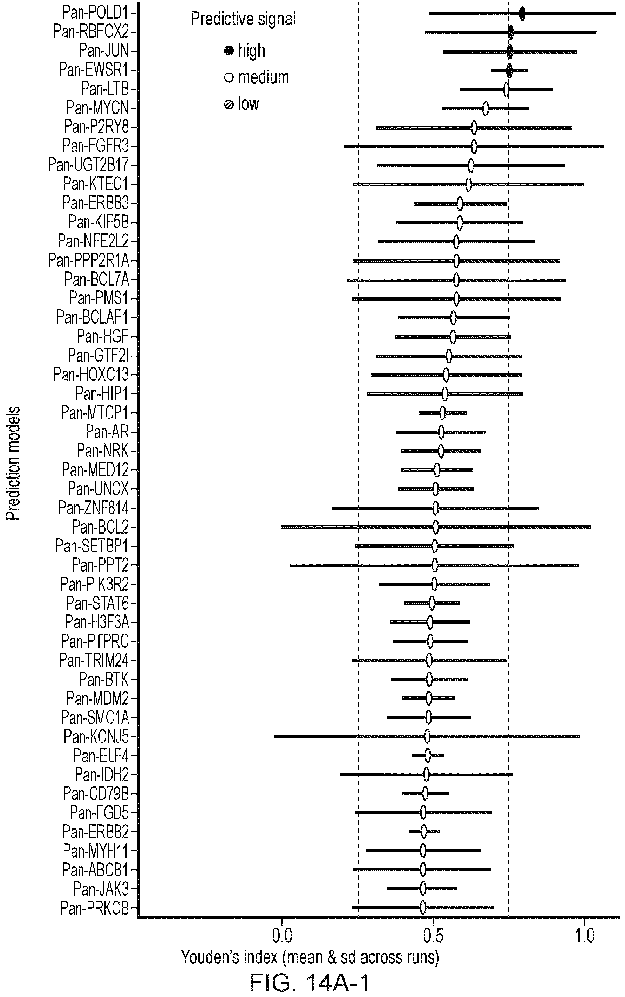

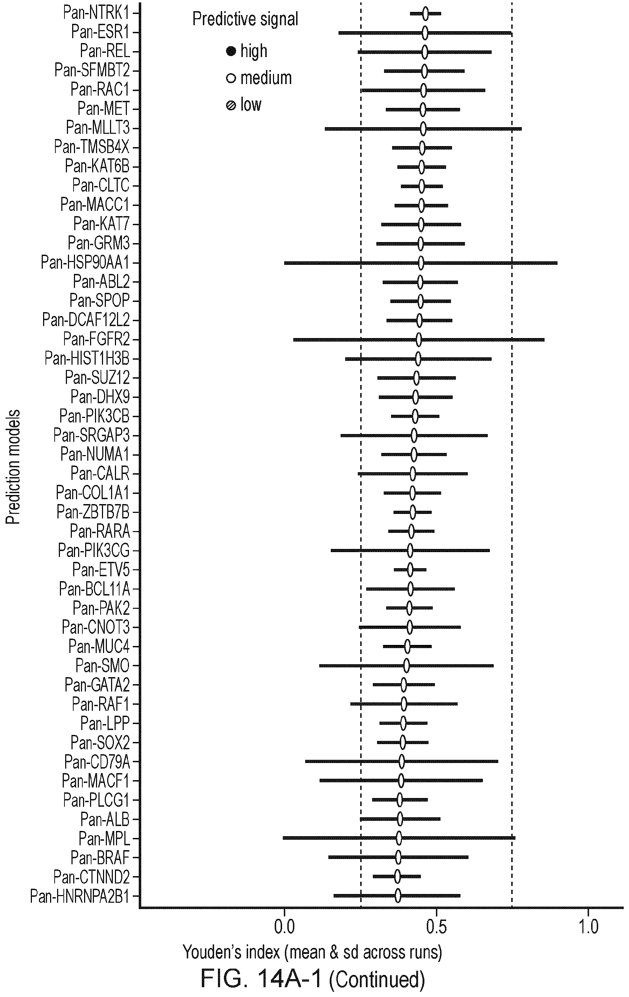

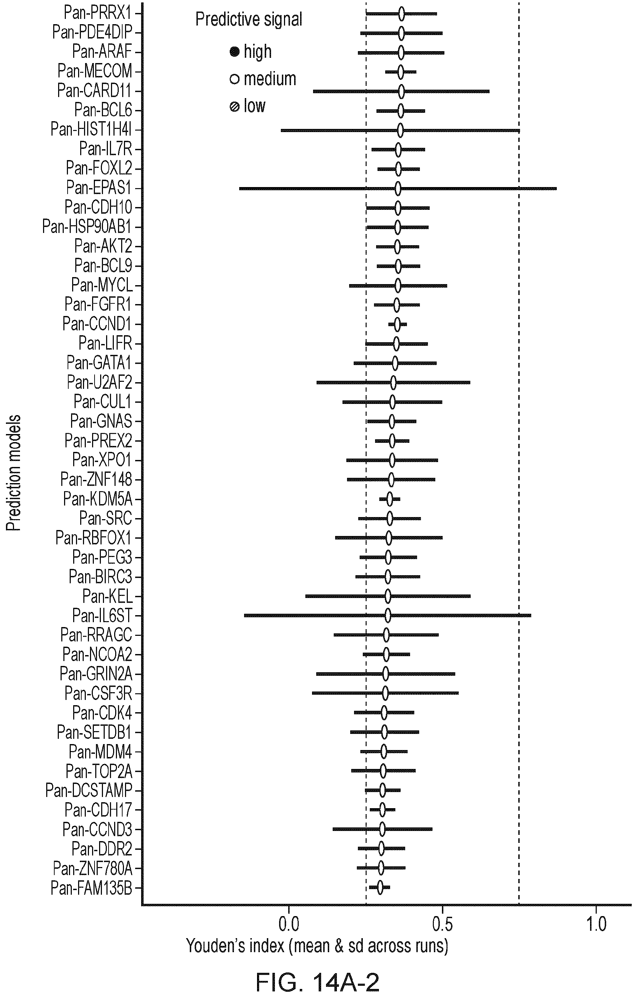

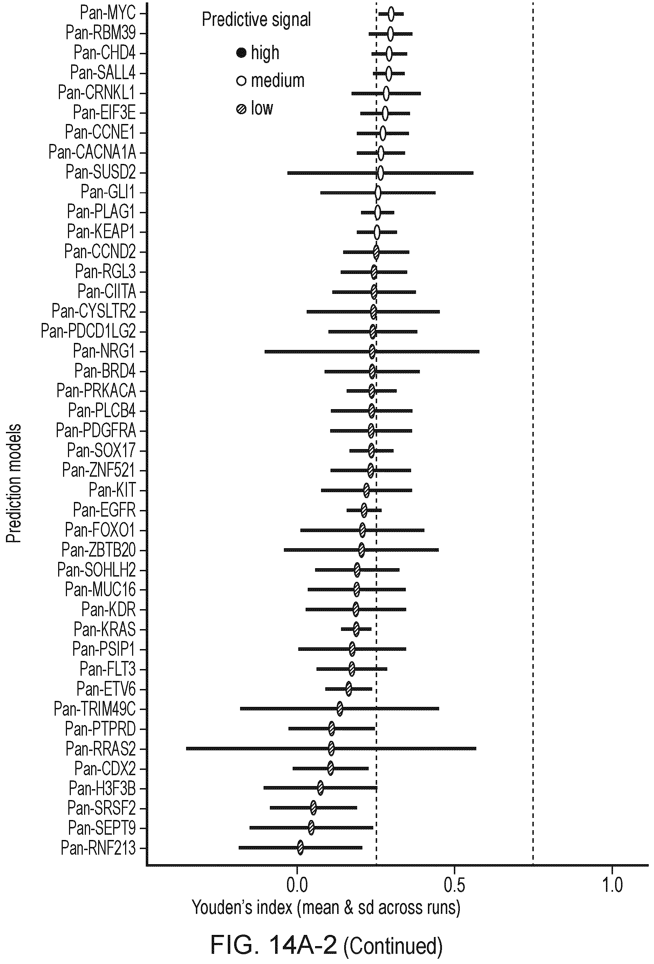

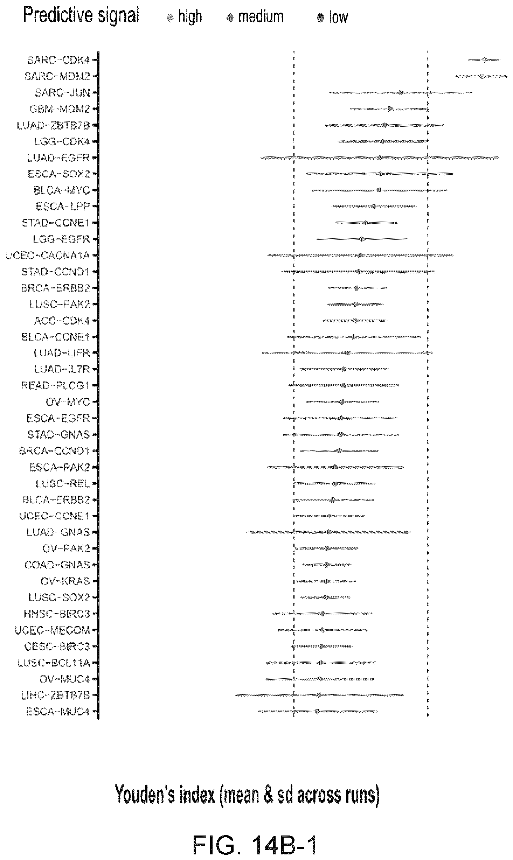

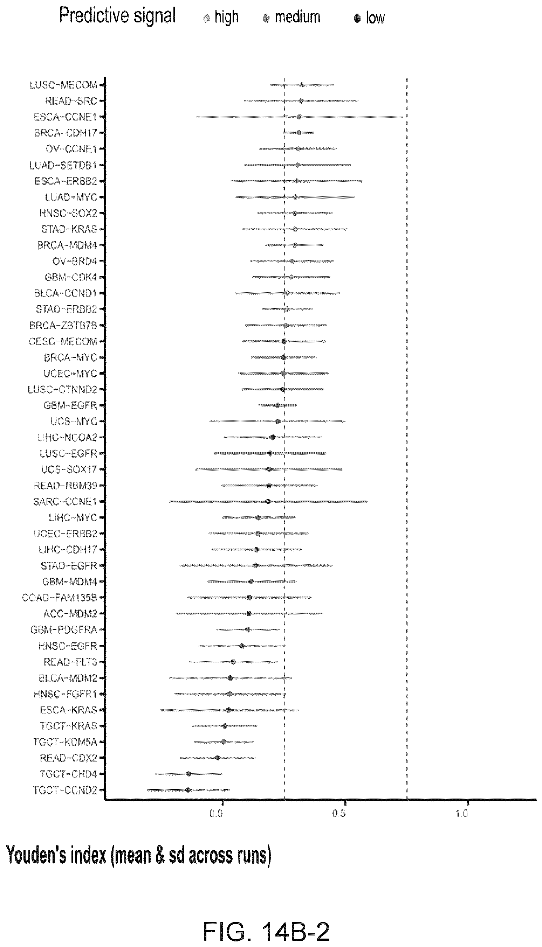

**Figure 14** shows results of an evaluation of the predictive signal of pan-cancer (A) and tumour-specific (A) oncogene amplification forecasting models of the disclosure assessed by computing the Youden's index using a 10-fold cross-validation strategy. For ease of reading each panel has been split onto two pages (A-1, A-2, and B-1, B-2, respectively).

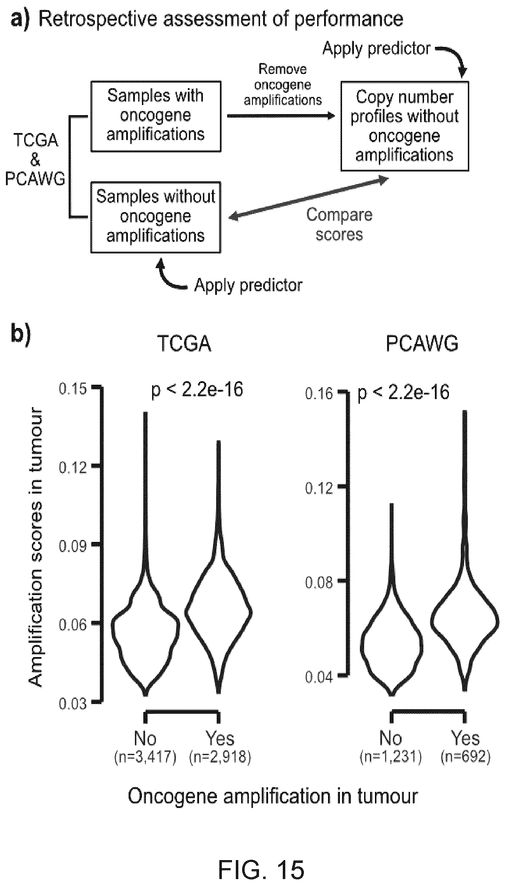

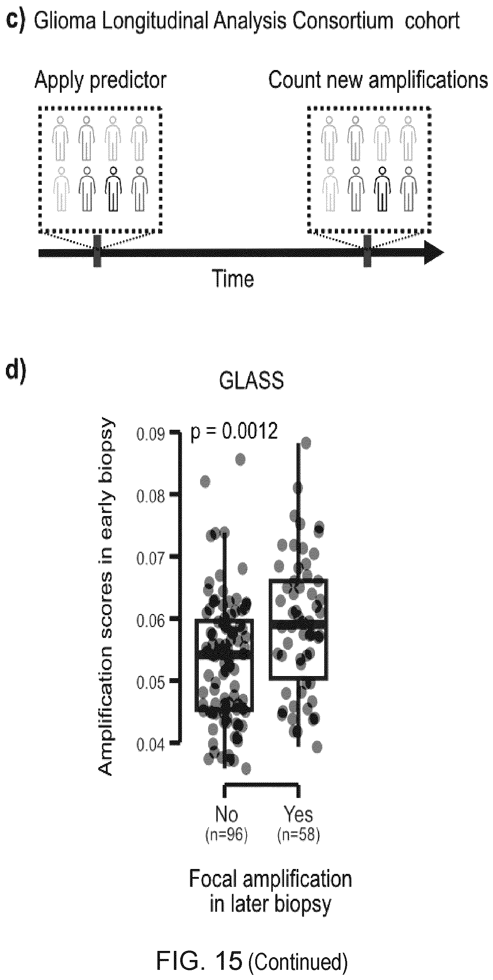

**Figure 15** shows principles and results of analyses testing signature-based predictors of focal amplification according to embodiments of the disclosure, **a-b)** Pan-cancer assessment of predictor performance in the TCGA (training cohort) and PCAWG (held-out cohort). Violin plots showing differences in amplification scores in tumours with and without an amplification in at least one oncogene across TCGA (left) and PCAWG (right). Only oncogenes amplified in a minimum of 10 samples were included. **c-d)** Testing the ability to predict future oncogene amplifications using paired longitudinal samples from the GLASS cohort. Signature-based scores of acquiring an oncogene amplification were computed in the early biopsy sample, and box plots in **d)** show comparison between late biopsy samples with and without oncogene amplification acquired. All p-values were estimated by Wilcoxon two-sided rank test.

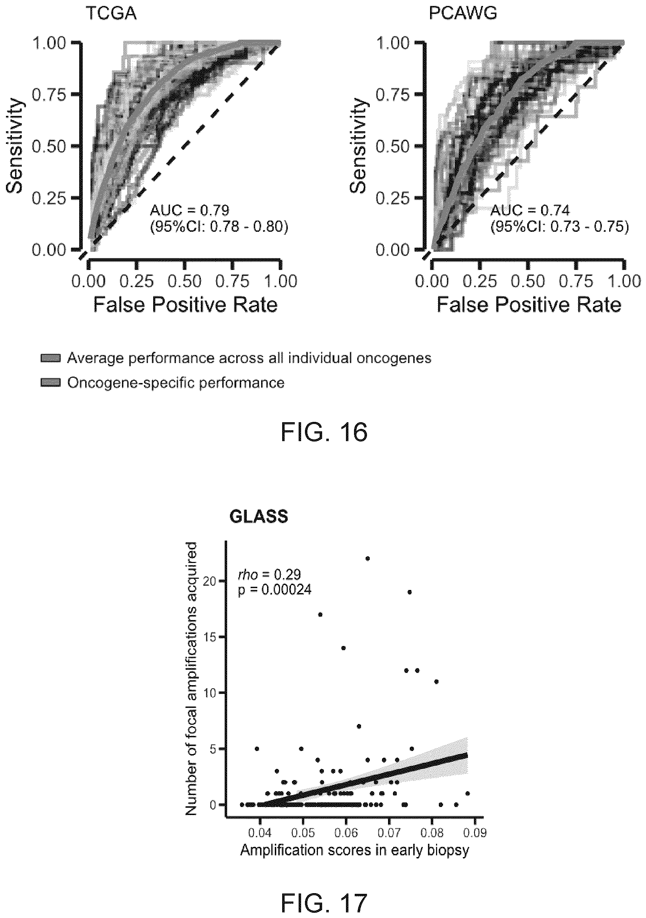

**Figure 16** shows ROC curves showing performance of the signature-based predictor in the TCGA and PCAWG cohorts. The ROC curve showing the average performance across all individual oncogenes is represented in purple. Only oncogenes amplified in a minimum of 10 samples (grey lines) were included. An oncogene was considered amplified when its absolute copy number was $\geq 8$.

**Figure 17** shows results of analyses assessing the ability to predict the number of focal amplifications acquired. Scatter plot showing amplification scores (x-axis) derived in early samples from GLASS and the total number of focal amplifications acquired in the second biopsy (y-axis). Spearman correlation test used for measuring the association degree between variables.

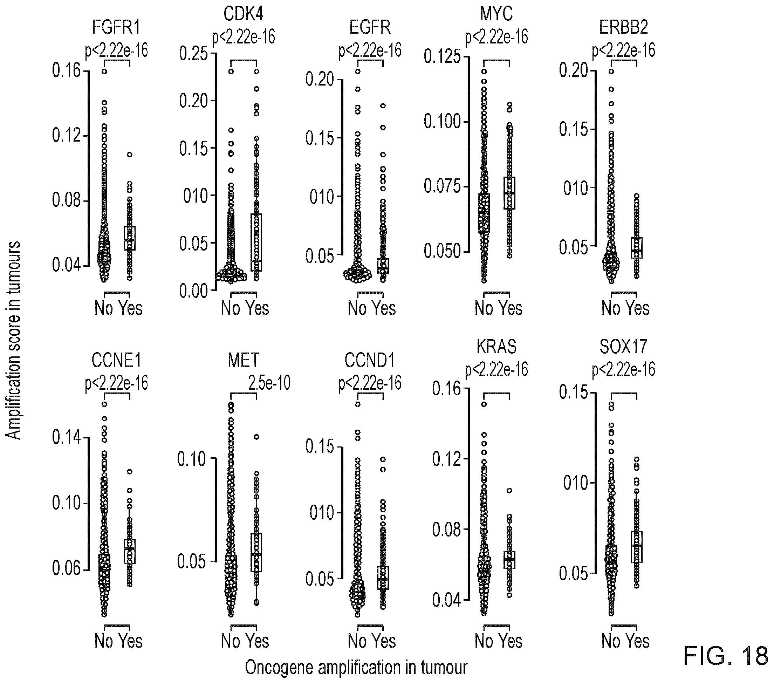

**Figure 18** shows results of gene-specific assessment of retrospective performance of predictors of the disclosure in the TCGA cohort. Box plots showing amplification scores (y-axis) in TCGA samples with and without amplification in a specific oncogene. A subset of 10 key oncogenes are illustrated. P-values estimated by Wilcoxon rank test.

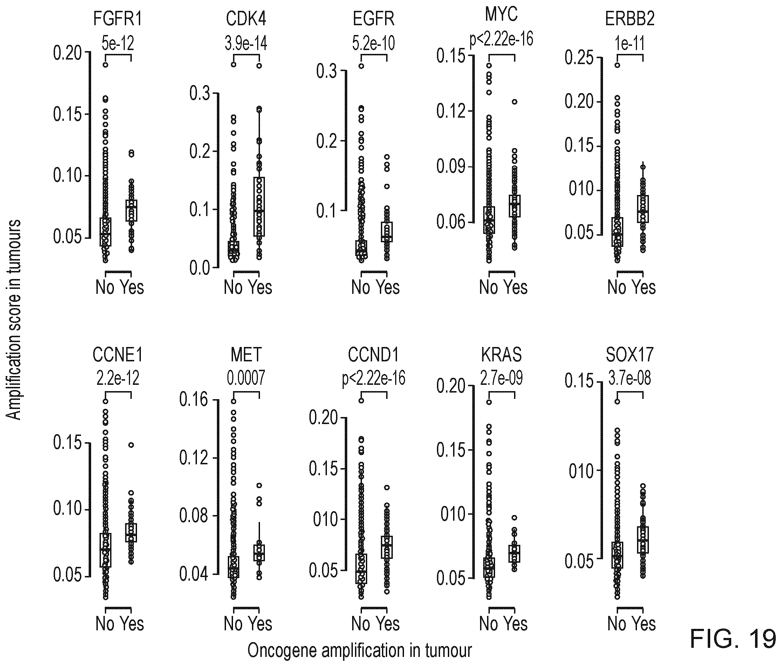

**Figure 19** shows results of gene-specific assessment of retrospective performance of predictors of the disclosure in the PCAWG cohort. Box plots showing amplification scores (y-axis) in PCAWG samples with and without amplification in a specific oncogene. A subset of 10 key oncogenes are illustrated. P-values estimated by Wilcoxon rank test.

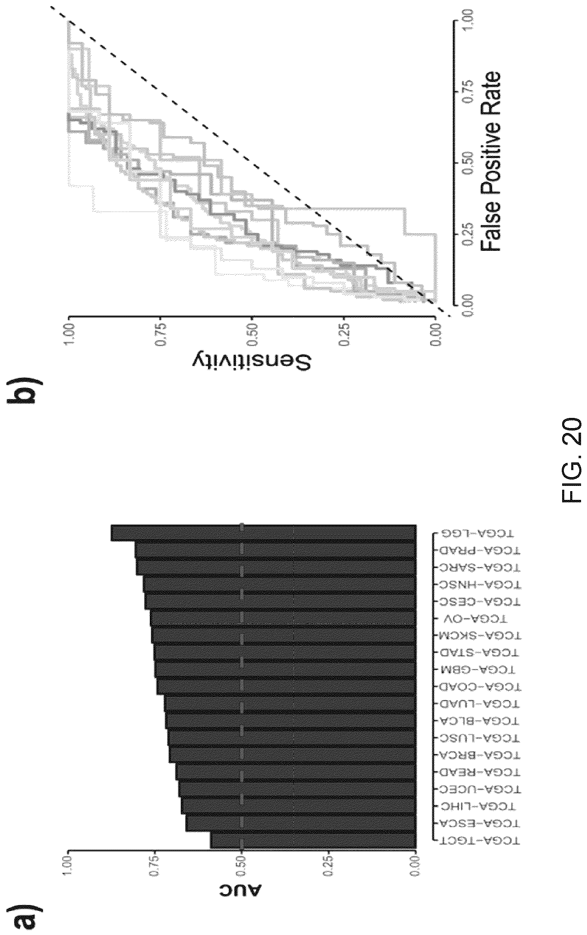

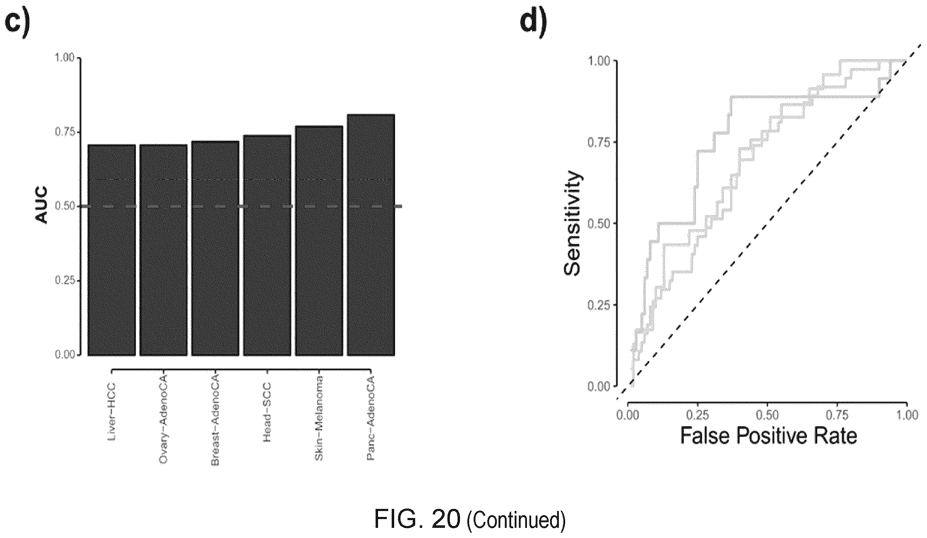

**Figure 20** shows results of a tumour-specific assessment of performance of predictors of the disclosure. **a,c)** Bar plots showing the average oncogene-specific performance (AUC values) by tumour type in **a)** the TCGA and **c)** the PCAWG cohorts. Only oncogenes amplified in a minimum of 10 samples per tumour type were included. **b,d)** ROC curves showing *MYC*-specific prediction performance across b) TCGA and d) PCAWG samples from tumour types with at least 10 *MYC* amplifications. Each colour represents a different tumour type.

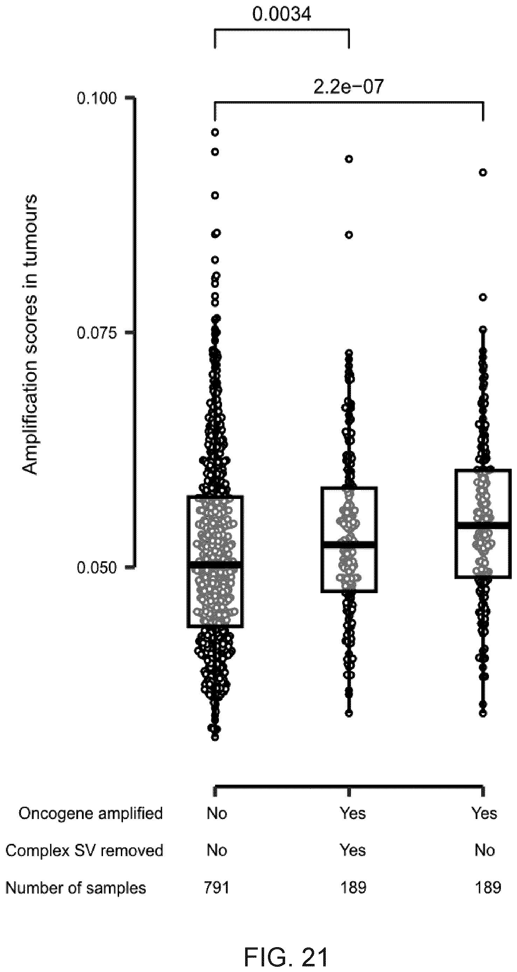

**Figure 21** shows results of an assessment of performance of predictors of the disclosure in the PCAWG cohort after removing segments connected to focal amplifications by complex/simple structural variants (SVs). Box plots showing amplification scores (y-axis) in PCAWG samples with and without focal amplifications. Scores of samples with amplifications were calculated after removing or not segments connected to amplifications by SVs.

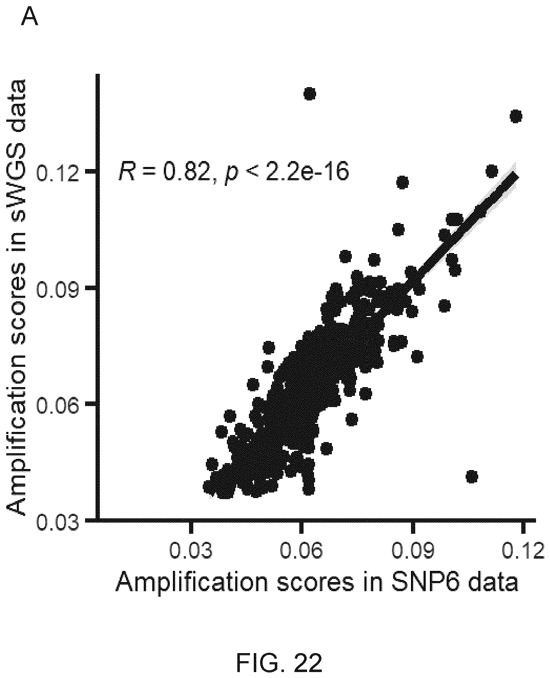

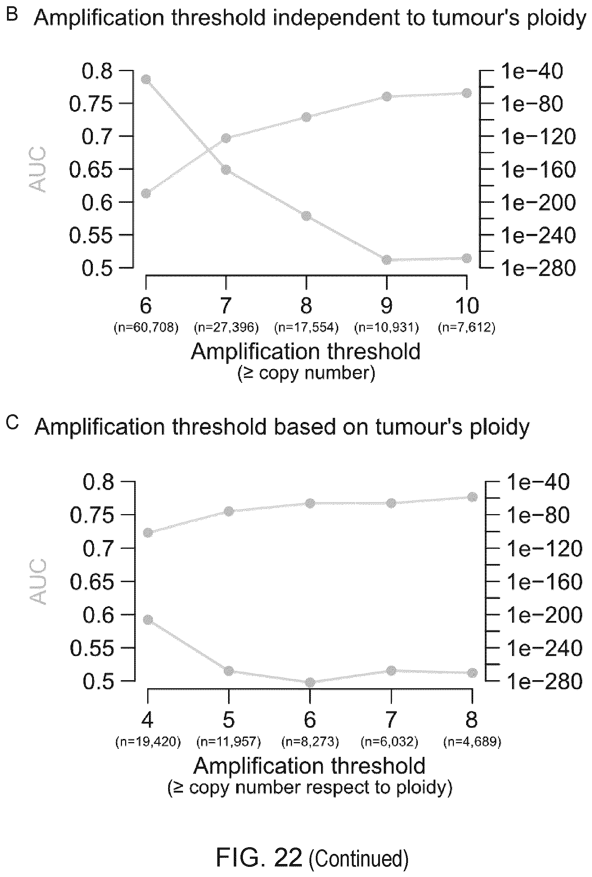

**Figure 22** shows (A) data investigating the stability of predictor tools as described herein across different copy number profiling technologies, and (B-C) data investigating the optimal threshold for defining focal amplifications. **(A)** Scatter plot showing amplification scores derived from SNP6 (x-axis) and sWGS (y-axis) data from the same set of 478 tumours. Pearson correlation test used for comparing amplification scores between technologies. (**B**) AUROC and p-value of a Wilcoxon two-sided test (comparing amplification scores between samples with and without oncogene amplifications) of a pan-cancer pan-oncogene model for forecasting oncogene amplifications for each of a plurality of a threshold values for calling focal amplifications, using absolute copy number values (ranging from $\geq 6$ to $\geq 10$ copies). (**C**) AUROC and p-value of a Wilcoxon two-sided test (comparing amplification scores between samples with and without oncogene amplifications) of a pan-cancer pan-oncogene model for forecasting oncogene amplifications for each of a plurality of a threshold values for calling focal amplifications, using copy number values relative to tumour ploidy (ranging from $\geq 4$ to $\geq 8$ copies respect to tumour's ploidy).

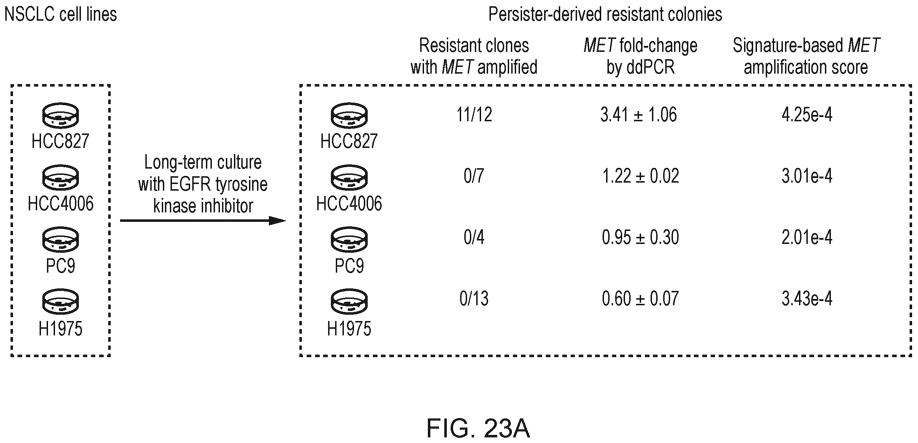

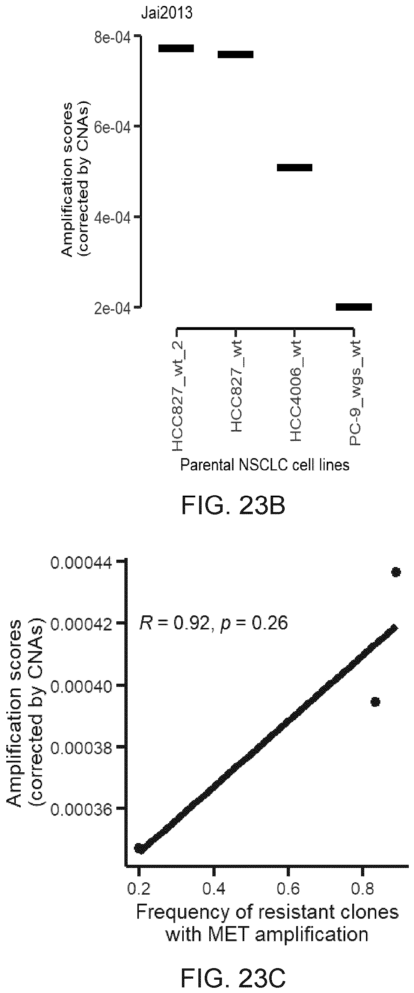

**Figure 23** shows results of methods of predicting EGFR tyrosine kinase resistance according to embodiments of the disclosure, **a)** Experimental validation of predicting EGFR-TKI resistance via *MET* amplification using a panel of NSCLC cell lines treated with EGFR-TKI until the emergence of treatment resistance. Signature-based MET amplification scores were computed in parental cell lines, and the presence of *MET* amplification in resistant clones was tested via ddPCR. Amplification scores were normalised by the total number of CNAs in each parental cell line. Red colour indicates resistance driven by *MET* amplification; **b)** Experimental validation of predicting EGFR-TKI resistance via MET amplification using a panel of NSCLC cell lines treated with EGFR-TKI until the emergence of treatment resistance by Jia et al, Genome Research, 2013. Signature-based MET amplification scores were computed in parental cell lines and displayed in the y axis. Amplification scores were normalised by the total number of CNAs in each parental cell line. Cell lines in the x axis are ordered by the frequency of emergence of MET amplification, as determined via WES. Cell lines in the x axis are ordered by the frequency of emergence of MET amplification. **c)** Experimental validation of prediction of EGFR-TKI resistance via MET amplification by associating signature-based MET amplification scores of 3 different parental HCC827 clones (y-axis) with the frequency of resistance clones acquiring MET amplification in culture (x-axis). *MET* amplification was determined via ddPCR.

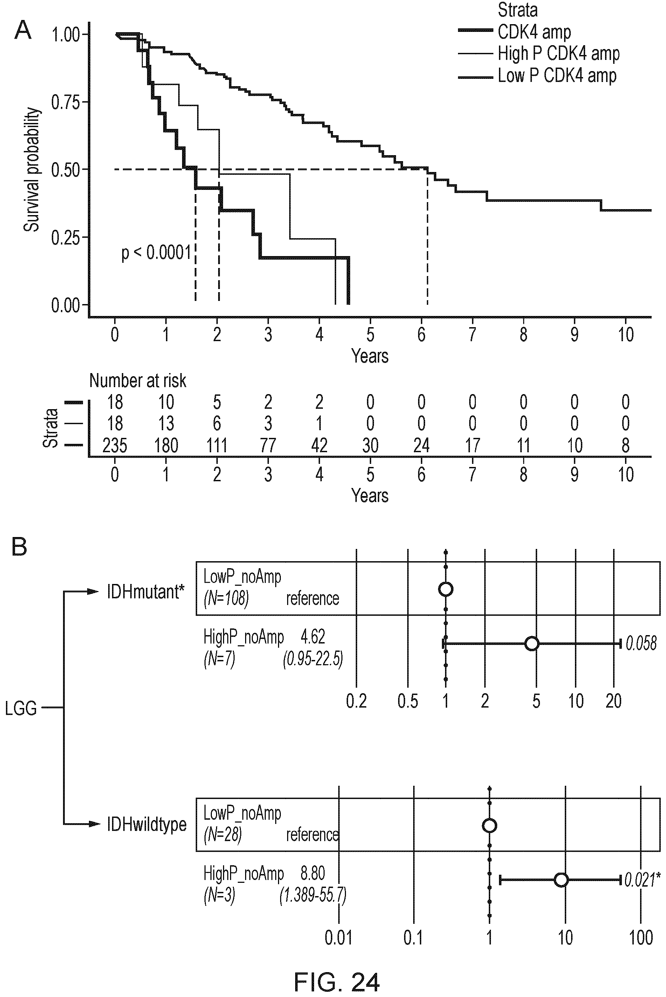

**Figure 24** shows results of methods of predicting prognosis in low grade gliomas according to embodiments of the disclosure, **a)** Kaplan-Meier curves showing overall survival for low grade glioma (LGG) patients from TCGA with CDK4 amplification (red), without CDK4 amplification but high probability to acquire it in the future (orange), and without CDK4 amplification and unlikely to acquire it (green). P-value was estimated by the log-rank test. **b)** Cox proportional hazards model for LGG-TCGA patients without *CDK4* amplification. Within each molecular subtype, patients are classified as high or low risk of acquiring *CDK4* amplification in the future by a signature-based classifier as described herein. Model was corrected for the fraction of genome altered and age at diagnosis.

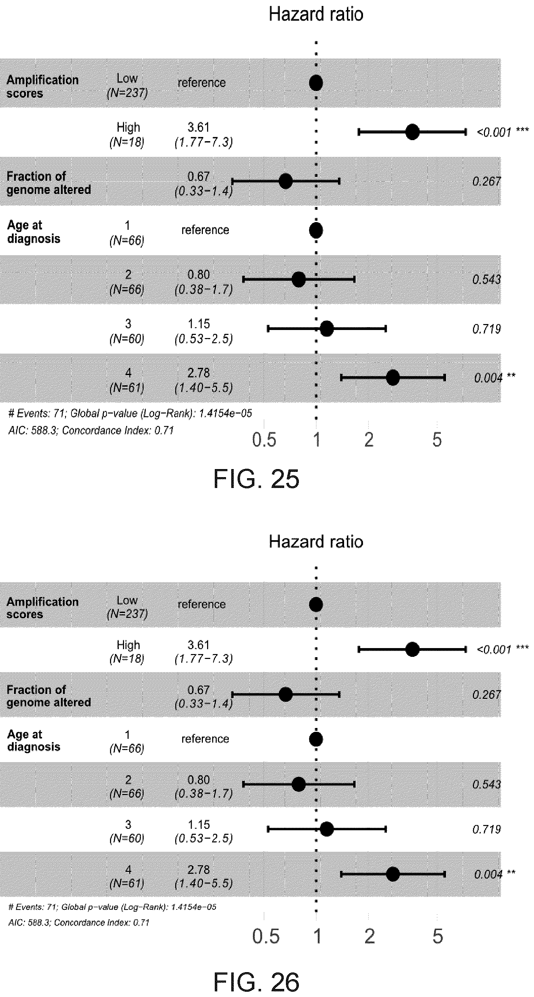

**Figure 25** shows results of a cox proportional hazards model for LGG-TCGA patients with and without CDK4 amplification. Model was corrected for the fraction of genome altered and age at diagnosis. Age at diagnosis was categorised into four groups by dividing cohort distribution into quantiles.

**Figure 26** shows results of a cox proportional hazards model for LGG-TCGA patients without CDK4 amplification. Patients are classified as high or low risk of acquiring CDK4 amplification in the future by a signature-based classifier as described herein. Model was corrected for the fraction of genome altered and age at diagnosis. Age at diagnosis was categorised into four groups by dividing cohort distribution into quantiles.

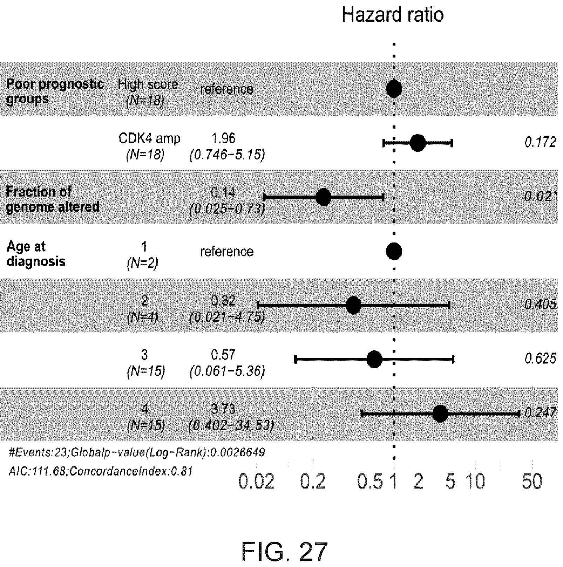

**Figure 27** shows results of a cox proportional hazards model for LGG-TCGA patients with and without CDK4 amplified but at risk of acquiring it in the future according to a signature-based classifier as described herein. Model was corrected for the fraction of genome altered and age at diagnosis. Age at diagnosis was categorised into four groups by dividing cohort distribution into quantiles.

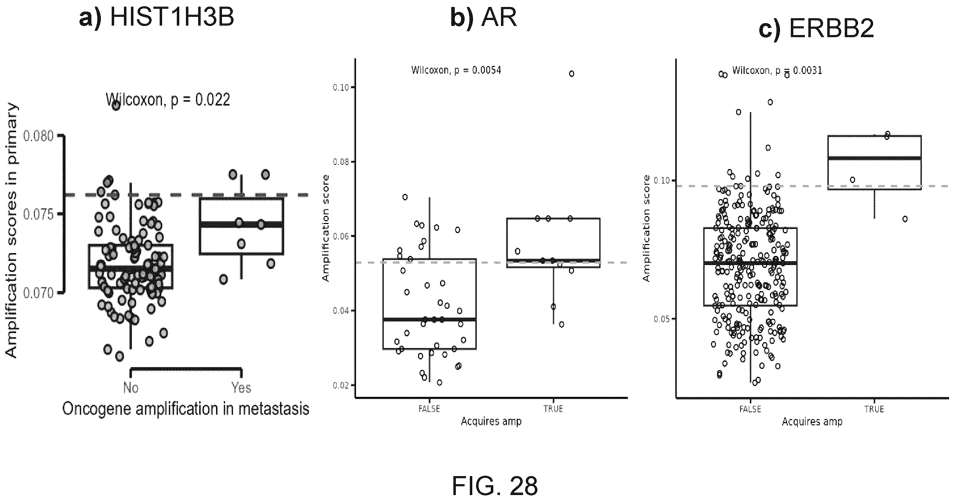

**Figure 28** shows results of analyses assessing the ability to predict the acquisition of amplifications of specific oncogenes in TRACERx Lung (126 pairs, lung adenocarcionma and non-small cell lung cancer) and the Hartwig Medical Foundation cohorts (227 pairs, different tumour types). Plots show the matched oncogene-specific model (i.e. HIST1H3B in **a),** AR in **b)** and ERBB2 in **c))** in the y-axis, the acquisition of the amplification in the x axis and a threshold to categorise patients based on the score into high risk and low risk of amplification groups. a) Acquisition of HIST1H3B amplification in TRACERx Lung, b) Acquisition of AR amplification in subset of prostate cancers with paired data in the Hartwig Medical Foundation cohort and c) Acquisition of ERBB2 amplification in all patients with paired data in the Hartwig Medical Foundation cohort.

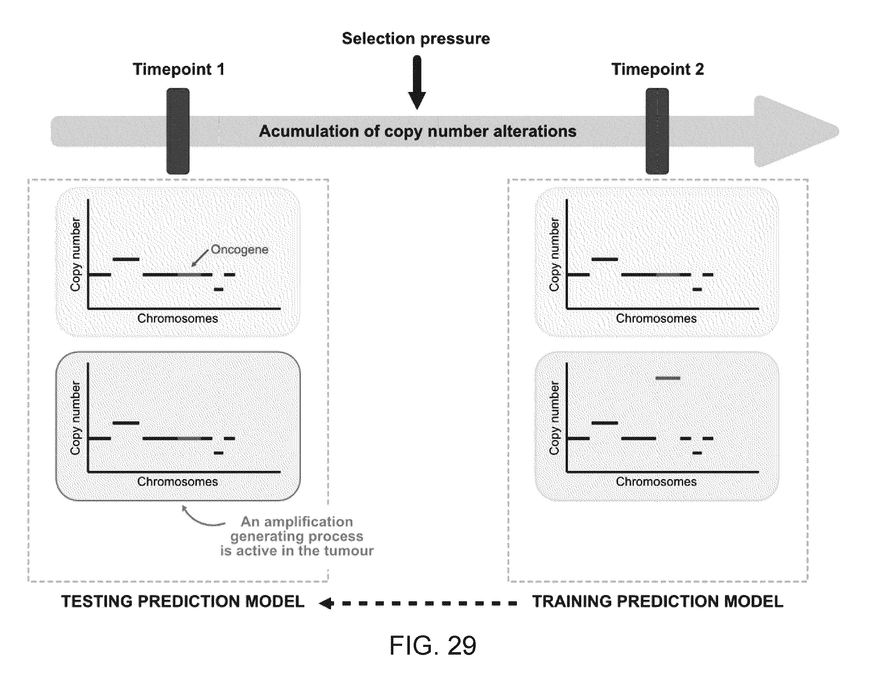

**Figure 29** shows the rationale for an alternative procedure to train the predictor of oncogene amplification using the latest biopsy from a cohort of patients with paired biopsies.

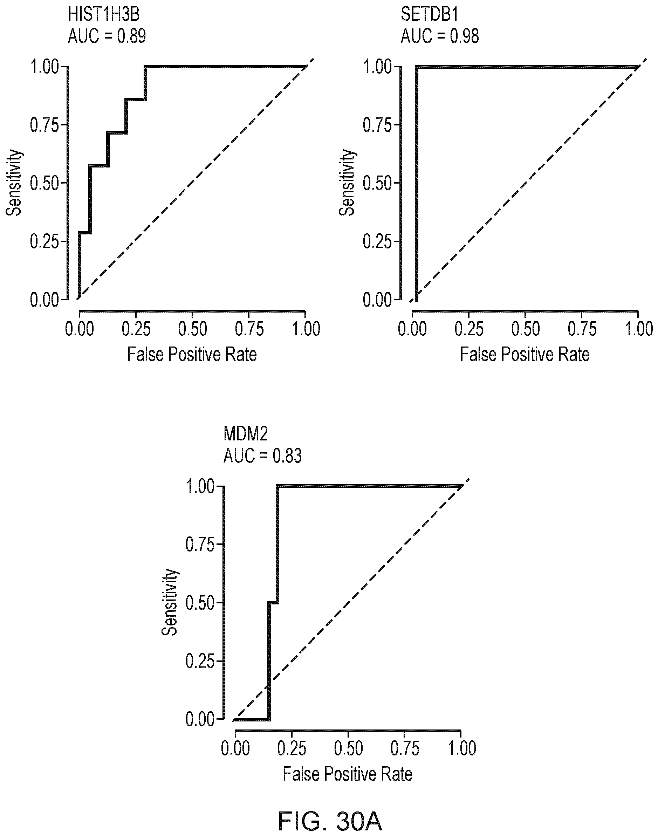

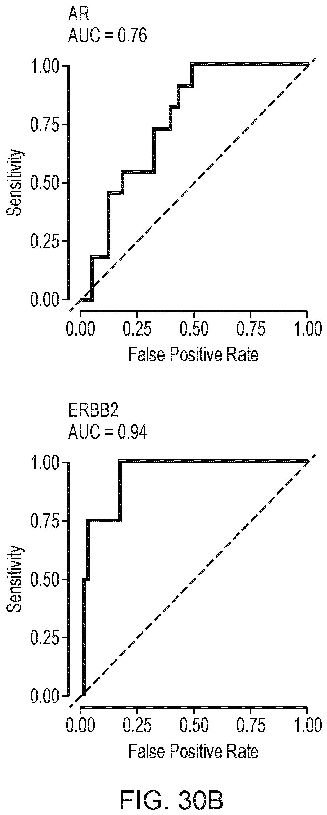

**Figure 30** shows the performance of oncogene-specific models trained either in TRACERx Lung (HIST1H3B, SETDB1 and MDM2) (**A**) or Hartwig Medical Foundation (AR, ERBB2) (**B**) cohorts using the latest biopsy available for each patient in these paired cohorts. ROC curves, with corresponding area under the curve (AUC) values are represented.

DETAILED DESCRIPTION

In the present disclosure, the following terms will be employed, and are intended to be defined as indicated below.

Different types of chromosomal instability (CIN) such as mitotic defects, replication stress or impaired homologous recombination leave distinct patterns of copy number change in the genome.

The inventors previously established a CIN signature framework that can identify these patterns and quantify the activity of each corresponding type of CIN in a genome (see Drews et al. 2022 and WO 2023/057392). In this work, the inventors used this framework to obtain models that are predictive of future oncogene amplification in a sample, using activity of signatures of chromosomal instability in the sample.

The present disclosure relates broadly to the characterisation of DNA samples, particularly tumour samples, in terms of their copy number profiles and signatures of chromosomal instability that are identified to be present in these samples.

A "copy number profile" refers to the quantification of the number of copies for each of a plurality of portions of a genomic sequence. In the context of the present disclosure, a copy number profile is preferably a genome-wide copy number profile. A copy number profile is obtained by analysing sequencing data. For example, a copy number profile may be obtained by obtaining sequence data from a sample of genomic DNA (or a DNA library derived therefrom, as explained further below, including a sample of DNA derived from genomic DNA by fragmentation, such as e.g. cell free DNA), and quantifying the number of copies per portion (e.g. per bin, where a bin is a region of fixed size typically between 20kb and 100kb, preferably between 30kb and 50kb, depending on the data available - for example for data comprising sequencing reads a predetermined minimum number of reads per bin is preferred such as e.g. 15 reads, which limits the bin size resolution that can be used) of the genomic sequence, as known in the art. A "tumour copy number profile" refers to a copy number profile that is associated with a tumour genome. A tumour copy number profile may be obtained by sequencing a sample comprising tumour genomic DNA, as explained further below. In embodiments, a tumour copy number profile is a copy number profile that has been obtained by sequencing a sample of genomic DNA derived from tumour cells. Thus, a copy number profile is typically in the form of an estimate, for each of a plurality of genomic segments, of the number of copies of the segment in the sample or a subset of the sample (e.g. in the case of samples comprising cells with different genotypes or genetic material derived therefrom, such as e.g. samples comprising a mixture of tumour and normal cells or tumour and normal DNA). In other words, for samples comprising tumour cells and non-tumour cells, a tumour copy number profile can be estimated for the tumour genome using methods known in the art. Unless specified otherwise, in the context of the present disclosure a copy number profile refers to a copy number profile for a tumour genome regardless of whether it is obtained from a sample also comprising genetic material from normal cells. Copy number profiles can be obtained by segmentation and copy number estimation from read count data using methods known in the art. For example, methods such as circular binary segmentation may be used for segmentation. Copy number fitting may be performed as described in the examples below. Alternatively, methods such as ASCAT (Raine et al. 2016), ichorCNA (Adalsteinsson et al. 2017) and others may be used to perform segmentation and copy number estimation (and also % tumour DNA estimation, in the case of ASCATt and ichorCNA). A copy number profile may be a genome-wide copy number profile. A genome-wide copy number profile may be obtained from a sample using WGS, sWGS or single cell WGS.

A "segment" in a copy number profile refers to a portion of a sequence represented in a copy number profile which is associated with a consistent absolute copy number. The consistent copy number is different from that associated with the sequence directly upstream (if such a sequence is present and associated with a copy number estimate) and the sequence directly downstream (if such a sequence is present and associated with a copy number) of said portion. In other words, a segment refers to a portion of sequence that has a copy number associated with it, where the copy number associated with the segment differs from the copy number associated with its immediate neighbouring segment(s). The copy number associated with a segment may differ from the copy number associated with its immediate neighbouring segment(s) because the segment(s) that surround the segment are associated with a different copy number, because the segment(s) that surround the segment are not associated with a copy number (e.g. because data for the segment(s) is missing, or of insufficient quality), or a combination of both (e.g. a segment may be surrounded by a segment that is associated with a different copy number on one side, and a segment that is not associated with a copy number on the other side). In other words, segments refer to the longest continuous portion of a copy number profile that are each associated with a single copy number. A segment may be associated with a set of coordinates, e.g. genomic coordinates, which define the boundaries of the segment. Each boundary may be associated with a copy number changepoint (also referred to as "changepointThe copy number of a segment may in practice be a copy number estimate obtained using a method for determining tumour copy number profiles, such as e.g. ASCAT [Van loo et al.,2010] or Sequenza [Favero et al., 2015].

According to the present disclosure a "copy number event" refers to a single segment in a copy number profile. In particular, a copy number event may refer to a segment with a copy number that deviates from the expected copy number in a normal genome (e.g. a non-diploid segment). Copy number events / segments are characterised by a plurality of copy number features, further defined below. Thus, the terms "copy number event" and "segment" may be used interchangeably. Note that this is not necessarily the case in the prior art, such as e.g. in WO 2023/057392 where copy number features are determined for copy number events that encompass multiple segments. In embodiments of the present disclosure, all features are determined in relation to a specific copy number event / segment, as will be described further below.

Chromosomal instability (CIN) is the process of accumulating numerical and structural changes in DNA. A signature of chromosomal instability (CIN) (also referred to herein as copy number signature or "signature") is a signature (typically in the form of a set of weights associated with respective categories of copy number features, also referred to as "components") representing the genome-wide imprint of distinct putative mutational processes (where a "mutational process" as used herein refers to any process that can cause chromosomal instability). CIN signatures are described in Macintyre et al. 2018, Drews et al. (2022) and in WO 2023/057392, which are incorporated herein by reference. The presence or absence of a CIN signature in a sample may be determined by calculating the exposure (also referred to as "activity") of the sample to the signature. A "CIN signature" is a set of weights associated with each of a plurality of components. The plurality of weights may sum to 1 and may each represent the probability that a mutational process associated with the signature generates a copy number event with a particular characteristic defined by the component. In other words, the plurality of weights of a signature may each represent the probability that a mutational process associated with the signature generates a particular type of copy number event. Each type of copy number event is referred to as a "component". Each component is defined by a predetermined distribution for possible values of a copy number feature.

The term "copy number (CN) feature" refers to properties of copy number events observable in a copy number profile. Copy number features typically include a plurality of features selected from: segment size (also referred to as "segment length" typically expressed in number of bases), changepoint copy number (the absolute difference in copy number between a segment and an adjacent/neighbouring segment in the copy number profile), number of breakpoints per flanking region (also referred to as "breakpoints per side"), breakpoint count per x MB (the number of changepoints appearing in a sliding windows across the copy number profile, where x can be e.g. 10 Mb), breakpoint count per chromosome arm (the number of changepoints occurring per chromosome arm), number of segments with oscillating copy number (sometimes referred to as "length of segments with oscillating copy number"; number of continuous segments alternating between two copy number states, rounded to the nearest integer copy-number state; also referred to as length of chain of oscillating copy number states), and absolute copy number. The signatures in Macintyre et al. 2018 use the following features: segment size, changepoint, breakpoint count per 10 Mb, breakpoint count per chromosome arm, number of segments with oscillating copy number and absolute copy number. The signatures in Drews et al. 2022 and WO 2023/057392 use the following features: segment size, changepoint, breakpoint count per 10 Mb, breakpoint count per chromosome arm, and number of segments with oscillating copy number (i.e. they exclude the "absolute copy number" feature from MacIntyre et al. 2018). The signatures used in the examples of the present disclosure use the following features: segment size, changepoint and number of breakpoints per flanking region (i.e. they exclude the breakpoint count per 10 Mb, breakpoint count per chromosome arm, and number of segments with oscillating copy number of Drews et al. 2022 and replace them with a single feature that can be quantified for individual segments). Any of these schemes may be used for determining an exposure to a signature for a sample. Only the latter can be used for determining an association between a signature and a single copy number event. The latter is used to identify models that can be used to predict the likelihood of gene amplification from sample level exposures in embodiments of the disclosure, and will therefore be described in further detail below. However, note that the other sets of features and corresponding signatures may also be used in other embodiments, where a machine learning model is fitted to predict a likelihood of amplification of a gene from sample level exposures to signatures, using training data comprising a plurality of copy number profiles (or quantified features and/or quantified exposures) and gene amplification ground truth labels (i.e. known presence or absence of a gene amplification). Further, the signatures used in the Examples herein (see Table 3) are derived from those in Drews et al. 2022 (see Table 7 of WO 2023/057392) and therefore exposure to these signatures at the sample level is interchangeable with exposure to the corresponding signatures in Drews et al. 2022.

Thus, in embodiments of the present disclosure, copy number features include: segment size (also referred to as "segment length" typically expressed in number of bases), change-point copy number (the absolute difference in copy number between a segment and an adjacent/neighbouring segment in the copy number profile, which may be defined relative to the upstream segment, the downstream neighbouring segment, or a reference copy number state, typically 2 when analysing normally diploid genomes), and number of breakpoints per flanking region (also referred to as "breakpoints per side", the number of breakpoints, i.e. changes in copy number / junction between individual segments in a flanking region of the segment, which can be upstream or downstream of the segment). The flanking region used to determine the "breakpoints per side" feature may have a predetermined length between 2 and 10 Megabases (Mb), preferably between 3 and 8Mb, or about 5 Mb. The number of breakpoints per flanking region may be determined for one or both flanking regions, and a summarised metric may be obtained when both flanking regions are used. The summarised metric may be the maximum or average number of breakpoints observed in the upstream and downstream flanking regions. In embodiments, the breakpoint per side feature for a segment is the maximum number of breakpoints observed in the upstream or downstream flanking region of the segment. The copy number features according to such embodiments of the disclosure do not include any feature that is not uniquely associated with individual events. In other words, the copy number features according to these embodiments do not include any copy number feature that is quantified over a genomic region that is not defined by reference to a segment. In particular, the copy number features according to these embodiments of the disclosure do not use any of: breakpoint count per x MB (the number of changepoints appearing in a sliding window of a predetermined size x across the copy number profile), breakpoint count per chromosome arm (the number of changepoints occurring per chromosome arm), and number of segments with oscillating copy number (sometimes referred to as "length of segments with oscillating copy number"; number of continuous segments alternating between two copy number states, rounded to the nearest integer copy-number state; also referred to as length of chain of oscillating copy number states). Such a set of copy number features can be used to associate signatures with individual copy number events, and are used in the Examples of the present disclosure to identify parameters of models used to predict the likelihood of gene amplification in a sample (by comparing association between respective signatures for gene amplification events and other events in a cohort of samples), as will be described further below.

In embodiments, the copy number (CN) features used according to the present disclosure do not include the segment copy number (the observed absolute copy number state of each segment, also referred to herein as "copy number" or "absolute copy number").

Each CN feature is associated with one or more components. Each component is defined by a distribution. CN features are observed for individual copy number events. Typically, CN features are observed for each event on a genome-wide basis for a sample or collection of samples, these are used to quantify a metric for each component that is the probability that the observed value of the feature has been drawn from the distribution defined by the component. Summarised metrics can then be obtained across events in a sample for each component, such as by summing the probabilities obtained. These summarised metrics are in turn used to determine signature exposures for the sample. According to the present disclosure, signature exposures may be determined for individual events, as well as for a sample (i.e. for a whole copy number profile).

Methods for determining the exposure to a signature, from a copy number profile (i.e. at a sample level), as well as identifying signatures from a cohort of samples are known in the art (see e.g. Macintyre et al., 2018, Drews et al. (2022)). In particular, the determination of the exposure of a sample to one or more CN signatures (i.e. activity of the one or more signatures in the sample) may be performed by identifying the matrix $E$ that satisfies $C \approx PE$ where $C$ is a mutational catalogue for one or more samples for which exposure is to be determined (also referred to as patient-by-component, PbC matrix), $P$ is a signature matrix comprising the one or more CN signatures for which exposure is to be determined (also referred to as signature-by-component, SbC matrix), and $E$ is an exposure matrix (also referred to as patient-by-signature, PbS). For example, exposure to a signature may be determined by performing matrix decomposition to identify the value (or vector of values) that satisfies the equation: PbC ≈ PbS x SbC (or, in practice PbC = PbS x SbC + $\varepsilon$, where $\varepsilon$ is a residual term to be minimised), where SbC is the row of the signature-by-component matrix that corresponds to a particular signature of a set of signatures, and PbS is the patient-by-signature value (or vector of values). Exposure to multiple copy number signatures can be calculated using the corresponding rows of the signature-by-component matrix. In embodiments, exposure ($E$) to a copy number signature $i$ as described herein (from a set of $n$ signatures, where n can be e.g. 16 or 17, where the signatures can include any or all of the signatures disclosed herein or corresponding signatures) is the value $E_i$ that satisfies the equation:

$$\text{PbC} \approx E \times \text{SbC} \qquad \text{(Equation 1)}$$

where: E is a vector of size $n$ comprising coefficients $E_{1,...,n}$ where $E_i$ is the exposure to signature $i$; PbC is a vector of size c, each value representing the sum-of-posterior probabilities of each copy number event in the copy number profile belonging to a component C, where each component C is a distribution of values for a copy number feature; and SbC is a matrix of size c by n, each value representing the weight of a component C in a copy number signature $i$. This is equivalent to solving the minimisation problem $\min(\|E*SbC-PbC\|)$ with constraints on nonnegativity of $E$, with $SbC$ and $PbC$ being known. For example, linear combination decomposition as implemented in R package YAPSA (Hübschmann, D. et al. 2021) may be used for this purpose. A similar approach can be used to identify signatures using a cohort of samples and nonnegative matrix factorisation (see e.g. Drews et al. 2022) to identify both an exposure matrix E and a signature matrix $SbC$. The present disclosure provides, in addition to the determination of exposure of samples to signatures, the determination of associations between signatures and individual segments (in particular, copy number events) in a copy number profile. This will be explained further below.

In the context of the present disclosure, each component represents a type of copy number abnormality defined by an individual component of a mixture model fitted to the distribution of values obtained for each of 3 copy number features described herein over a cohort of samples from the Cancer Genome Atlas (TCGA). Note that any other cohort of cancer samples can be used for this purpose and the disclosure is not limited in any way in relation to the cohort of samples from which the components (and corresponding signatures) are extracted. This follows a process as described in Drews et al. 2022, except that in embodiments of the disclosure three features in Drews et al. 2022 (breakpoints per chromosome arm, breakpoints per 10 Mb, number of segments with oscillating copy number) were removed and replaced by a new feature as described herein (breakpoints per side). The components in Drews et al. 2022 (see Table 6 of WO 2023/057392) were used for the segment size and changepoint features, and new components were identified for the new feature. Copy number signatures (i.e. signatures of copy number alteration processes) are obtained using these, which capture the probability of copy number alteration processes causing copy number events that are distributed according to each of the copy number feature components. Thus, the term "exposure" (also referred to as "signature activity" or "activity") in this context captures the strength of evidence for the presence of copy number alteration events attributable to the signature. As previously mentioned, in embodiments, the components and associated signatures in Drews et al. 2022 and WO 2023/057392 may be used (see Tables 6 and 7 of WO 2023/057392 which provide, respectively, component definitions and signature definitions identified using a cohort of samples comprising a plurality of tumour types including the TCGA cohort), or corresponding components and signatures identified using a different cohort of samples.

The term "genome-wide" refers to data that covers a whole genome or substantial parts thereof such as e.g. one or more entire chromosomes. As the skilled person understands, sequencing data or a copy number profile derived therefrom may be referred to as "genome wide" even though it may not contain information (i.e. reads or a copy number estimate) for each and every single position in the genome. Indeed, even in the context of sWGS there may be regions of the genome that have no mapping reads due to e.g. low mappability, sequencing difficulty, or the sampling nature of next generation sequencing. Thus, data may be referred to as "genome-wide" if it contains data for at least 70%, at least 80% or at least 90%, preferably at least 90%, of one or more chromosomes or all chromosomes present in a reference genome.

A "sample" as used herein may be a cell or tissue sample, a biological fluid, an extract (e.g. a DNA extract obtained from a cell, tissue or fluid sample), from which genomic material can be obtained for genomic analysis, such as genomic sequencing (e.g. whole genome sequencing, or targeted sequencing such as whole exome sequencing or targeted panel sequencing). The sample may be a cell, tissue or biological fluid sample obtained from a subject (e.g. a biopsy). Such samples may be referred to as "subject samples". The sample may be any sample comprising genomic DNA or cell free DNA. In particular, the sample may be a tumour sample, a biological fluid sample containing DNA or cells, a blood sample (including plasma or serum sample), a urine sample, a cervical smear, an ascites fluid sample, or a sample derived therefrom (e.g. after DNA purification).

It has been found that urine, ascites fluid and cervical smears contains cells, and so may provide a suitable sample for use in accordance with the present invention. Other sample types suitable for use in accordance with the present invention include fine needle aspirates, lymph nodes samples (e.g. aspirates or biopsies), surgical margins, bone marrow or other tissue from a tumour microenvironment, where traces of tumour DNA may be found or expected to be found. The sample may be one which has been freshly obtained from a subject or may be one which has been processed and/or stored prior to genomic/transcriptomic analysis (e.g. frozen, fixed or subjected to one or more purification, enrichment or extraction steps). The sample may be a cell or tissue culture sample. As such, a sample as described herein may refer to any type of sample comprising cells or genomic material derived therefrom, whether from a biological sample obtained from a subject, or from a sample obtained from e.g. a cell line. In embodiments, the sample is a sample obtained from a subject, such as a human subject. The sample is preferably from a mammalian (such as e.g. a mammalian cell sample or a sample from a mammalian subject, such as a cat, dog, horse, donkey, sheep, pig, goat, cow, mouse, rat, rabbit or guinea pig), preferably from a human (such as e.g. a human cell sample or a sample from a human subject). Further, the sample may be transported and/or stored, and collection may take place at a location remote from the sequence data acquisition (e.g. sequencing) location, and/or any computer-implemented method steps described herein may take place at a location remote from the sample collection location and/or remote from the sequence data acquisition (e.g. sequencing) location (e.g. the computer-implemented method steps may be performed by means of a networked computer, such as by means of a "cloud" provider). A sample may be a tissue biopsy, such as a sample of fresh frozen tissue or a sample of formalin-fixed paraffin embedded (FFPE) tissue, or a sample comprising circulating tumour DNA (ctDNA) such as e.g. a sample of biological fluid (sometimes referred to as liquid biopsy). The samples used in methods of the present disclosure are typically samples comprising tumour cells (e.g. a tumour sample or sample comprising circulating tumour cells) or genetic material derived from tumour cells (such as e.g. cell free DNA, or DNA extracted from a sample comprising tumour cells - e.g. from a cell line or tissue sample - or circulating tumour cells). A sample may be a "mixed" sample comprising cells with different genotypes or genetic material derived therefrom. For example, a sample may be a sample comprising tumour cells and normal cells, or DNA derived therefrom, such as e.g. in the context of cell free DNA (cfDNA) samples comprising circulating tumour DNA (ctDNA). Copy number profiles may be determined separately for cells that have a genotype comprising somatic copy number alterations, in a sample comprising both cells that have the genotype comprising somatic copy number alterations (e.g. tumour cells) and cells that do not contain such alterations (e.g. normal cells), using methods known in the art. For example, sequence analysis processes that deconvolute tumour and germline genomes from copy number profiles include ASCAT (Raine et al., 2016), ABSOLUTE (Carter et al., 2012), or, in the context of cfDNA, ichorCNA (Adalsteinsson et al., 2017). A "tumour sample" refers to a sample that contains tumour cells or genetic material derived therefrom. The tumour sample may be a cell or tissue sample (e.g. a biopsy) obtained directly from a tumour. A tumour sample may be a sample that comprises tumour cell or genetic material derived therefrom, that has not been obtained directly from a tumour. For example, a tumour sample may be a sample comprising circulating tumour cells or circulating tumour DNA. Thus, a tumour sample may also be a biological fluid (e.g. a liquid biopsy such as a blood, urine, or cerebrospinal fluid biopsy). A sample comprising a mixture of tumour cells and other cells (or material genetic derived therefrom) may be subject to one or more processing steps, whether prior to or subsequent to the acquisition of sequence data, in order to identify sequence data that is representative of the genetic material from the tumour. For example, a sample comprising cells may be subject to one or more cell purification steps which selectively enrich the sample for tumour cells. As another example, a sample of genetic material may be subject to one or more capture and/or size selection steps to selectively enrich the sample for tumour-derived genetic material. Protocols for doing this are known in the art. As another example, sequence data may be subject to one or more filtering steps (e.g. based on fragment length in the context of cell-free DNA samples) to enrich the data for information that relates to tumour-derived genetic material. Protocols for doing this are known in the art. In embodiments, the sample is a sample comprising tumour cells. Preferably, such a sample has a tumour purity (where tumour purity can be quantified as the proportion of cells in the sample that are tumour cells) of at least 30%, at least 35%, at least 40%, at least 45%, or at least 50%. Advantageously, the sample has a tumour purity of at least 40%. Without wishing to be bound by theory, it is believed that the copy number profiles generated from samples that have lower tumour purity may be less suitable as the signal corresponding to the tumour genome may be lost amongst the signal from the genomes of other cells. A "normal sample" (also referred to as "germline sample") refers to a sample that contains non-tumour or non-modified cells or genetic material derived therefrom. A normal sample may be matched to a particular tumour or modified sample in the sense that it is obtained from the same biological source (subject or cell line) as the tumour or modified sample. A matched normal sample is a sample that is expected to be representative of the genome of a tumour sample in the absence of somatic abnormalities. This is typically a sample obtained from the same subject as the tumour sample. A normal sample may be used in the context of the present invention as a control to analyse a tumour sample. For example, a tumour sample and a (typically matched) normal sample may be analysed together in order to obtain a copy number profile for the tumour. Methods to obtain tumour copy number profile from a pair of normal and tumour samples are known in the art. For example, these include ASCAT [Van Loo et al. 2010] and Sequenza [Favero et al. 2015]. Such methods may provide a tumour copy number profile as well as an estimate of the purity (proportion of tumour cells) of the tumour sample. The purity estimate may be used to exclude tumour samples that have low purity and hence could lead to low quality copy number estimates.

The term "sequence data" refers to information that is indicative of the presence of genetic material in a sample that has a particular sequence. Such information may be obtained using sequencing technologies, such as e.g. next generation sequencing (NGS), for example whole exome sequencing (WES), whole genome sequencing (WGS), or sequencing of captured genomic loci (targeted or panel sequencing), or using array technologies, such as e.g. SNP arrays, or other molecular counting assays. In embodiments, the sequence data is obtained by DNA sequencing, and particularly next generation sequencing. In such embodiments, the sequence data comprises sequencing reads, or information derived therefrom such as the count of the number of sequencing reads that have a particular sequence. When non-digital technologies are used such as array technology, the sequence data may comprise a signal (e.g. an intensity value) that is indicative of the number of sequences in the sample that have a particular sequence, for example by comparison to an appropriate control. Information such as the count of sequencing reads that have a particular sequence can be derived from sequencing reads by mapping the sequence data to a reference sequence, for example a reference genome, using methods known in the art (such as e.g. Bowtie (Langmead et al., 2009) or BWA (Li and Durbin 2009)). This may result in aligned sequencing reads, for example in the form of a SAM or BAM file. Thus, sequence data may be associated with a particular genomic location (where the "genomic location" refers to a location in the reference genome to which the sequence data was mapped). Sequence data may be filtered as part of the methods described herein or may have been filtered prior to application of the methods described herein, for example to remove low quality reads or remove reads that map to regions that have "low mappability". Methods for filtering low quality reads are known in the art, and include e.g. removing duplicate reads, removing supplementary alignments, removing reads with low sequencing quality scores (reads below Q20 or Q30), removing reads with low mapping quality score (e.g. MAPQ<37), etc. Methods to filter reads that map to regions that have low mappability are known in the art and include e.g. Umap (Karimzadeh et al. 2018), and GenMap (Pockrandt et al. 2020).

A composition as described herein may be a pharmaceutical composition which additionally comprises a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

As used herein "treatment" refers to reducing, alleviating or eliminating one or more symptoms of the disease which is being treated, relative to the symptoms prior to treatment.

"Patient" as used herein in accordance with any aspect of the present invention is intended to be equivalent to "subject" and specifically includes both healthy individuals and individuals having a disease or disorder (*e.g.* a proliferative disorder such as a cancer). Preferably, the patient is a human patient. In some cases, the patient is a human patient who has been diagnosed with, is suspected of having or has been classified as at risk of developing, a cancer.

As used herein, the terms "computer system" includes the hardware, software and data storage devices for embodying a system or carrying out a method according to the above described embodiments. For example, a computer system may comprise a central processing unit (CPU) and/or a graphic processing unit (GPU), input means, output means and data storage, which may be embodied as one or more connected computing devices. A computer system may comprise a display or comprises a computing device that has a display to provide a visual output display. The data storage may comprise RAM, disk drives or other non-transitory computer readable media. The computer system may include a plurality of computing devices connected by a network and able to communicate with each other over that network. It is explicitly envisaged that computer system may consist of or comprise a cloud computer.

As used herein, the term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

Predicting gene amplification

The present disclosure relates at least in part to the prediction of future gene amplification. In other words, the present disclosure provides methods of determining whether a tumour is likely to acquire one or more gene amplifications (i.e. amplification events that are not currently present in the tumour genome but likely to emerge in the future). The gene amplifications according to the disclosure are preferably amplification events that encompass all or part of the coding region of an oncogene. An oncogene may also be referred to herein as "driver". An oncogene or driver gene is a gene that is known to cause or support abnormal cellular proliferation and/or resistance to cytotoxic therapy. A list of known oncogenes is provided in **Tables 7 and 8** (column "oncogene"). The present disclosure encompasses prediction of the likelihood of acquisition of an amplification of any of the genes in **Tables 7 and 8,** but is not limited to this specific list of genes as new oncogenes are still being discovered and the same methodology described herein can be applied to any gene with oncogenic potential.

An amplification, also referred to herein as "amplification event" (segment with at least 8 copies), is the presence of an abnormal elevated copy number at a genomic region. In embodiments, any region with a copy number higher than 2 (diploid genome) may be considered an amplification event. Preferably, an amplification event refers to a genomic region with at least 6 copies. In embodiments, any region with an absolute copy number value $\geq 6$ may be considered an amplification event. In embodiments, any region with a copy number value relative to tumour ploidy $\geq 4$ may be considered an amplification event. For example, when the tumour ploidy is 2 (i.e. no whole genome duplication), any region with at least 6 copies may be considered amplified, whereas when the tumour ploidy is 4 (i.e. the tumour genome has undergone a whole genome duplication), any region with at least 8 copies may be considered amplified.

**Figure 1A** is a flow diagram showing, in schematic form, a method of predicting whether a tumour is likely to acquire a gene amplification using methods described herein.

At optional step 10, a DNA sample is obtained from a tumour of a subject. Optionally, a matched normal sample may also be obtained from the subject. At optional step 12, sequence data is obtained from the tumour (and optionally the matched normal) DNA sample(s), for example using sequencing or array technologies. At step 14, a tumour copy number profile for the sample is obtained, for example by analysing the sequence data obtained at step 12 or by receiving the copy number profile from a user, database etc. Thus, steps 10-12 are optional because methods described herein may start from a previously obtained tumour copy number profile for the sample. The data may be bulk sequence data (from a bulk DNA sequencing technology or an array technology) or single cell sequencing data (from a single cell DNA sequencing technology). When single cell sequencing data is used, the process described by reference to steps 12-20 may be performed individually for each of a plurality of single cells. Step 14 of obtaining a tumour CN profile may comprise one or more of: aligning a plurality of sequence reads to a reference genome, determining that the copy number profile comprises a number of non-diploid segments above a predetermined threshold (e.g. at least 15 or at least 20 events in the copy number profile), determining a copy number for each of a plurality of genomic bins of a predetermined size, excluding genomic bins comprising a number of mapped reads below a predetermined threshold, and/or performing absolute copy number fitting. The predetermined bin size may be at most 100kb, at most 50kb, or about 30kb. The predetermined threshold on number of mapped reads may be 15 reads.

Step 16 comprises determining, for each of one or more signatures of chromosomal instability, a metric indicative of the presence of the signature in the tumour copy number profile. As explained above, a signature of chromosomal instability comprises a set of weights for each of a plurality of components each associated with a copy number feature of a set of copy number features quantified for a plurality of segments in the copy number profile, wherein the weights are indicative of the probability that a mutational process associated with the signature generates a segment having characteristics defined by the component. For example, in the illustrated embodiment a sample level exposure to one or more signatures of chromosomal instability is determined (also referred to as activity of a signature in the sample). Step 16 may comprise step 16A of quantifying a set of copy number features, for each of one or more segments in the copy number profile. This step may use unrounded copy number segments. This step may comprise collapsing and merging near diploid segments to a diploid state, wherein near diploid segments are segments that have a copy number within a predetermined distance from 2. The predetermined distance may be 0.1. Thus, quantifying the set of copy number features may comprise assigning a copy number of 2 (i.e. collapsing) to any segment that has a copy number within predetermined boundaries (such as e.g. above 1.9 and below 2.1), and merging any contiguous segments that have the same copy number as a result of the assigning. This may advantageously avoid including signal from segments that are likely normal diploid segment and which could act as noise in the process. Unrounded copy number segments may be segments whose copy number has not been rounded to the near integer. Thus, the method preferably uses copy number profiles that have not been rounded and/or wherein the only rounding that is performed relates to the collapsing or near diploid segments. This uses more of the information present in copy number profiles (in terms of number of segments that would otherwise be merged but also in terms of the copy number information that is contained in said profile for each segment). Rounding of copy numbers for segments may be performed in order to remove noise in a copy number profile, at the cost of loss of information. It has been previously shown that the additional noise that is associated with the use of unrounded copy number segments could be compensated at least in part, with minimal loss of information relevant to CIN by merging segments with minor deviations from the "normal" copy number state, and the present inventors have shown that this also applies to the new methods described herein. The features may not be quantified for diploid segments. In other words, the methods of the present disclosure may comprise quantifying a plurality of copy number features for one or more non-diploid segment, such as e.g. each non-diploid segment in a copy number profile.

Step 16 may further comprise step 16B, where a metric indicative of association between one or more signatures of chromosomal instability and each segment is determined using the quantified features for the segment. A signature of chromosomal instability comprises a set of weights for each of a plurality of components each associated with a copy number feature of the set of copy number features, wherein the weights are indicative of the probability that a mutational process associated with the signature generates a segment having characteristics defined by the component. Thus, step 16B may comprise obtaining, for each segment, a value for each of the plurality of components for the segment. Each component defines characteristics of a segment as a predetermined distribution of values of a copy number feature. Determining a metric indicative of association between a signature of chromosomal instability and a segment may comprise determining the probability of the copy number features of the segment belonging to each of a set of predetermined distribution associated with respective components of the plurality of components. Thus, the probability of the feature value from a segment belonging to the predetermined distribution associated with the component may be determined. This may be performed for each component, thereby obtaining an "Event-by-component" probabilities vector for each segment, or an event-by-component probability matrix comprising respective vectors for each of a plurality of segments from the same copy number profile. These probabilities may be referred to as "posterior probabilities". Thus, step 16B may comprise quantifying the posterior probabilities of each feature value belonging to each of a set of predetermined distributions, also referred to as "components". The components may have been identified empirically by quantifying the copy number features in a plurality of tumour samples. Each copy number feature may be associated with a plurality of components. A posterior probability may be obtained for each component and for each event. Further, a summarised measure across the copy number profile may be obtained for each such component, such as e.g. by summing the posterior probabilities obtained for each event for the component. The use of empirically identified components means that the features are truly reflective of biological processes rather than arbitrary categories that while convenient to manipulate, may not be biologically relevant. For example, a set of predetermined distributions may be identified by applying mixture modelling on a data set comprises the quantified set of copy number features for a plurality of tumour samples. This enables the identification of the true states of the copy number features present in tumours, and hence the quantification of the evidence for the presence of these states in a new sample to be analysed. The use of posterior probabilities of each feature value belonging to each of a set of predetermined distributions advantageously enables the method to deal with uncertainty in the assignment of a segment to a state (i.e. uncertainty in whether the segment provides evidence for the presence of a particular category of CIN events characterised by one of the distributions for a copy number feature). This means that the method is by design able to deal with inevitable noise in the data, resulting in a more accurate characterisation of the sample. The set of predetermined distributions for a copy number feature may be a set of Gaussian distributions for any feature that is quasi-continuous, such as e.g. the segment size or changepoint feature. The set of predetermined distributions may be a set of Poisson distributions for any count feature, such as e.g. the breakpoint per side feature. A set of predetermined distributions may have been obtained using a mixture modelling technique. A quasi-continuous feature may be any feature that is not a count feature, such as e.g. segment size and copy number changepoint. For example, the segment size feature may be quantified for an event by obtaining posterior probabilities (or for a copy number profile as sum-of-posterior probabilities summed across copy number events in the copy number profile) for each of a plurality of Gaussian distributions, such as e.g. between 20 and 25 Gaussian distributions (e.g. 22 Gaussian distributions). As another example, the copy number changepoint feature may be quantified by obtaining posterior probabilities (or for a copy number profile as sum-of-posterior probabilities summed across copy number events in the copy number profile) for each of a plurality of Gaussian distributions, such as e.g. between 5 and 15 Gaussian distributions (e.g. 10 Gaussian distributions). As another example, the breakpoint per side feature may be quantified by obtaining posterior probabilities (or for a copy number profile as sum-of-posterior probabilities summed across copy number events in the copy number profile) for each of a plurality of Poisson distributions, such as e.g. between 5 and 15 Poisson distributions (e.g. 9 Poisson distributions). The precise number of distributions used may depend at least in part on the number and diversity of samples used to obtain the signatures. In the examples provided herein, the inventors used a very large number of samples from a wide variety of tumour types, enabling them to provide a nuanced picture of the behaviours of copy number features in cancer, resulting in a higher number of components identified than e.g. in Macintyre et al., 2018. The parameters of each of the predetermined distributions (such as e.g. mean and variance for a Gaussian distribution, $\lambda$ for a Poisson distribution) may have been determined as part of the process of obtaining the signatures of chromosomal instability. The parameters of each of the predetermined distributions (such as e.g. mean and variance for a Gaussian distribution, $\lambda$ for a Poisson distribution) may have been determined by fitting mixture models to the quantified set of copy number features in the plurality of tumour samples from which the signatures of chromosomal instability have been obtained. In the examples below, these parameters were obtained by analysis of over 6000 samples from the Cancer Genome Atlas (TCGA). As the skilled person understands, slightly different parameters may be obtained when using different data. The parameters of each of a set of Gaussian distributions may have been obtained using a Variational Bayes Gaussian mixture model. In particular, the parameters of Gaussian distributions (for example for features such as segment size and changepoint) may have been obtained by fitting Dirichlet-Process Gaussian mixture models using variational inference. The parameters of each of a set of Poisson distributions (for examples the breakpoint per side feature) may have been obtained using a Poisson mixture model and/or using predetermined parameters. The parameters of the components used in the examples described herein are provided in **Table 1.** Thus, in embodiments, step 16B uses the components defined in **Table 1** or corresponding components defined using a different cohort of samples. Corresponding components refer to components defined by distributions that correspond to the distributions of the components in **Table 1,** but where the exact parameters of each distribution may vary. Alternatively, the signatures in Drews et al. 2022 and WO 2023/057392 may be used, with components defined in Table 6 of WO 2023/057392 and signatures defined in Table 7 of WO 2023/057392. The component parameters for the changepoint and segment size features in **Table 1** are as in Drews et al. 2022 and WO 2023/057392. The breakpoints per side feature is new to the present disclosure. In embodiments, the set of signatures used herein include one or more of the signatures in Drews et al. 2022 (e.g. signatures selected from CX1 to CX17, optionally excluding CX6). Aetiologies associated with these signatures are provided in **Table 2.** The full definitions of these signatures are provided in Drews et al. 2022 and WO 2023/057392 (see Table 7 of WO 2023/057392, reproduced herein as **Table 12,** with components specified in **Table 11**). In embodiments, signatures corresponding to those in WO 2023/057392 (see Table 7 of WO 2023/057392) in the new feature space described herein are obtained by applying linear combination decomposition using a cohort of samples with known exposures to the signatures. In other words, signature definitions can be identified as those that solve the minimisation problem $\min(\|E^*SbC\text{-}PbC\|)$ with constraints on nonnegativity of $SbC,$ with $E$ and $PbC$ being known. For example, linear combination decomposition as implemented in R package YAPSA (Hübschmann, D. et al. 2021) may be used for this purpose. Corresponding signature definitions according to the present disclosure are provided in **Table 3.** As mentioned above, the process of defining the signatures in Drews et al. 2022 in the new feature space may be performed using one or more or all of CX1 to CX17, excluding CX6. In embodiments, a subset of the signatures in Drews et al. 2022 and/or their equivalent in the feature space described herein **(Table 3)** are used. For example, a subset of the signatures that is found across a plurality of technologies (i.e. a plurality of methods for obtaining sequence data suitable for the generation of a copy number profile) may be used. These may be referred to as "universal signatures". For example, CX1-5, CX8-10, and CX13 were found by the present inventors to show robust signals across technologies (including at least array data such as SNP6 arrays and shallow whole genome sequencing data, sWGS), and were classified as universal signatures. Other signatures in Table 3 were classified as SNP6-specific signatures (CX7, CX11-12, and CX14-17). CX14 was not considered universal because its signal was captured by CX1 in sWGS data.

Step 16 may further comprise step 16C where a sample level exposure to one or more of the signatures is determined (i.e. the activity of the signatures in the tumour copy number profile is determined). This may comprise obtaining, for each component, the sum of probabilities associated with the respective component for each of a plurality of segments in the copy number profile; and determining a linear combination of one or more signatures comprising the signature that satisfies the equation:

$$PbC \approx E \times SbC \qquad\qquad \text{(Equation 1)}$$

where E is a vector of size n comprising coefficients $E_{1,...,n}$ where $E_i$ is the exposure to signature i; PbC is a vector of size c, each element in the vector representing a sum of probabilities associated with a respective component; and SbC is a matrix of size c by n, each value representing the weight of a component in a signature *i*. The resulting exposures may further be normalised by sample such that they sum to 1 (i.e. dividing the exposure for each signature in a sample by the sum of exposures for all signatures in the sample).

At step 18, the metrics obtained at step 16 (e.g. activity of the one or more signatures in the profile) are used as input to a model trained to take these metrics as inputs and produce as output a score indicative of the likelihood that the tumour will acquire a gene amplification. The model may be a linear model that obtains a score as a weighted combination of the metrics obtained at step 16 (e.g. signature activities). The weights may have been determined as will be described below by reference to Figure 1B.

At step 20, the sample / tumour may be classified between a first class that has a high likelihood of acquiring the gene amplification and a second class that has a low likelihood of acquiring the gene amplification, wherein the tumour is classified in the first class when the output of the model is above a predetermined threshold.

At step 22, the results of any of the preceding steps may be provided to a user, for example through a user interface, in the form of a report, etc., or to a computing device or memory.

Identifying signatures associated with oncogene amplification

The present disclosure also provides methods of identifying signatures associated with oncogene amplification. Such methods can be used to define models that are used to determine the likelihood of future amplification in a sample as described by reference to Figure 1A. In embodiments, these make use of methods of identifying signatures associated with individual copy number events (such as oncogene amplifications). A method of identifying signatures of chromosomal instability associated with individual copy number alterations in a sample will now be described by reference to **Figure 1B.**

In particular, **Figure 1B** is a flow diagram showing, in schematic form, a method of identifying a model for predicting the likelihood of acquisition of a gene amplification event according to embodiments of the disclosure. At optional step 110, a plurality of reference tumour samples are obtained which together form a reference cohort. At optional step 112, sequence data is obtained from the samples, for example using sequencing or array technologies. At step 114, a tumour copy number profile is obtained for each sample in the reference cohort, for example by analysing the sequence data obtained at step 112 or by receiving the copy number profile from a user, database etc. Thus, steps 10-12 are optional because methods described herein may start from a previously obtained tumour copy number profile for each sample in the cohort of samples or from previously acquired sequence data for each sample. Steps 110-114 may have any of the features described above by reference to steps 10-14 of **Figure 1A.**

At step 116, a set of copy number features are quantified, for each of one or more segments in the copy number profile. This step may have any of the features described above by reference to step 16A of **Figure 1A.** The set of copy number features consists of copy number features that are associated with respective segments. In other words, the set of copy number features may not include any copy number feature that is defined for a section of the genome that is not a segment or a region defined by reference to a segment. As such, each copy number feature is defined for a specific segment, and quantifies a property of the segment and/or a region defined by reference to a segment (such as e.g. a flanking region of a predetermined length, a region of a predetermined length centred on the segment, etc.). The set of copy number features quantified for each segment at step 16 may comprise a segment size feature, a copy number changepoint feature, and a breakpoint per side feature (breakpoint count in one or both flanking regions of a predetermined length). The copy number changepoint feature may be quantified for the first segment of any chromosome by as the copy number change relative to the diploid state (i.e. 2 is subtracted from the copy number value of the segment). In other words, the changepoint feature may be quantified by subtracting 2 from the absolute copy number of the first segment of any chromosome if said segment is not a normal segment. This assumes that the (non-existing) previous segment was a normal segment (copy number 2) if the segment is not a normal segment. The copy-number changepoint feature may be quantified for a current segment relative to the upstream or downstream segment of the current segment. In particular, the copy-number changepoint may be quantified relative to the upstream (left) neighbouring segment when the changepoint value relative to the upstream segment is at or higher than a predetermined threshold (e.g. -3 or more, such as e.g. -3, -2, -1, 1, 2, 3, etc.). The copy-number changepoint may be quantified relative to the downstream (right) neighbouring segment when the changepoint value relative to the upstream segment is lower than the predetermined threshold (e.g. less than -3, such as e.g. -4, -5, etc.). When the copy number changepoint relative to the downstream neighbouring segment is also lower than -3, the copy number changepoint may be calculated by removing two from the copy number value of the current segment (i.e. calculating the difference between (current segment copy number) and (diploid state)). In any embodiment, the copy number changepoint may be quantified as an absolute value. In other words, a relative copy number changepoint between a current segment and a neighbouring segment may be calculated and the absolute value of this may then be taken as the copy number changepoint for the current segment. This approach differs from the approach in Drews et al. 2022 in which the changepoint captured the difference in absolute copy number compared to the left neighbouring segment without taking into account whether the relative change was a positive or a negative value. The approach proposed herein advantageously avoids linking an event next to a focal amplification to a replication stress signature, as was the case in the previous approach since segments with low copy number value next to a focal amplification were defined by having high copy number change. In other words, the left segment may be used by default unless it is determined that the left segment is likely a segment with focal amplification. In this case, the right segment may be used unless it is determined that the right segment is also likely a segment with focal amplification. In such cases the copy number changepoint may be calculated relative to a diploid state.

At step 118, a metric indicative of association between one or more signatures of chromosomal instability and each segment is determined using the quantified features for the segment. Step 18 may have any of the features described above by reference to step 16B of Figure 1A.

Step 118 (determining a metric indicative of association between a signature of chromosomal instability and a segment using the quantified features for the segment) may comprise multiplying probabilities associated with each of the plurality of components for the segment by the corresponding weight in the signature, and summing up the resulting values. As explained above, the probability associated with a component for the segment are typically the probability of a segment with the quantified feature having been drawn from the distribution associated with the component. The resulting summed up values may be referred to as an "exposure" of the event to the respective signatures, or "signature score".

At step 120, a sample level exposure to one or more of the signatures may be determined. This may be performed as explained above by reference to step 16C of **Figure 1A.** At step 122, a sample-corrected signature score (also referred to herein as activity corrected signature score) is obtained for each segment, by multiplying the signature score obtained at step 118 by the sample level exposure obtained at step 120. Instead or in addition to this, step 122 may further comprise normalising the signature exposures for a segment by dividing each exposure (optionally sample-corrected) by the sum of exposures (optionally sample-corrected) across the plurality of signatures for the segment.

Note that at this point, , the results of steps 118 and optionally 120 may be used to determine the activity of signatures (and correspondingly, the activity of CIN causing processes associated with the signatures). This is not part of the process of generating a model as described herein. This may comprise the step of assigning a signature of chromosomal instability to a segment of the one or more segments as the signature of the one or more signatures of chromosomal instability with the highest value of the metric for the segment. For example, the metric for a signature may be the exposure of the segment to the signature and a segment may be associated with the signature with the highest exposure for the segment. Alternatively, the metric for a signature may be a distance between the vector of probabilities associated with the plurality of components for the segment and the corresponding weights in the signature, and a segment may be associated with the signature with the lowest value of the metric for the segment (when the distance is a true distance metric such as Euclidian distance) or the highest value of the of the metric for the segment (when the distance is a similarity metric such as cosine similarity). Alternatively when the metric for a signature is a probability that the segment is associated with the signature (exposure), an event may be associated with a plurality of signatures, each with respective probabilities. As explained above, the one or more signatures of chromosomal instability may be selected from those defined in Table 3 or corresponding signatures obtained by: (i) quantifying the set of copy number features in a plurality of tumour samples, and (ii) identifying one or more signatures likely to result in the copy number profiles of the plurality of tumour samples by nonnegative matrix factorisation. Examples of signature and their associations are provided in Table 2. Thus, determining the presence or absence of a signature may be equivalent to determining whether a corresponding CIN process is or has been active in the sample, or in other words determining whether the copy number alterations in the copy number profile have been generated at least in part by the CIN process. Thus, the one or more signatures comprise one or more signatures selected from: one or more signatures associated with chromosome missegregation, optionally chromosome missegregation via defective mitosis and/or telomere dysfunction, one or more signatures associated with impaired homologous recombination, one or more signatures associated with tolerance of whole genome duplication, one or more signatures associated with impaired non-homologous end joining, one or more signatures associated with replication stress, and one or more signature associated with impaired DNA damage sensing. Further, the presence / absence of a signature or corresponding process may also be determined at the sample level, by comparing the exposures obtained at step 120 to a respective predetermined threshold. The predetermined thresholds may have been obtained by performing simulations as the threshold such that a predetermined proportion (e.g. 95%) of simulated samples in which a signature is truly not present (but where the signature exposure may nonetheless be non-zero due to the presence of noise in the simulated data) are below the threshold. Examples thresholds for the signatures in Table 3 are provided in Table 4. Other thresholds may be used depending on the desired stringency in identifying the presence of a signature.

Turning back to a method of providing a model as described herein, at step 124, a weight may be obtained for each signature and for one or more gene amplifications (individually or collectively). In embodiments, weights (and therefore models comprising said weights) are obtained for individual genes. This will be described in further detail below, although the same principles can be applied when obtaining a model for a plurality of genes jointly. The weights may be determined in a tumour type specific manner, or in a tumour type-agnostic manner.

When the weights of the model are determined in a tumour type-specific manner, a weight may be obtained for each signature as a ratio of (i) an averaged signature score of all amplified segments spanning the gene in samples of the specific tumour type in the reference cohort and (ii) an averaged signature scores of all non-diploid segments found in the reference cohort. As explained above, the signature score for a segment and signature is a metric indicative of association between the signatures of chromosomal instability and the segment, such as the sum of the product of probabilities associated with each of the plurality of components of the signature for the segment by the corresponding weight in the signature (obtained at step 118), the probability for a segment being the probability that a segment with a copy number feature value equal to the value for the segment having been drawn from a distribution associated with the component, or an activity corrected (i.e. multiplied by the value obtained at step 120) and/or normalised version of said sum (obtained at step 122). As explained above, an activity corrected version of said sum may be the value of the sum multiplied by the activity of the signature in the copy number profile and a normalised version of said sum or activity corrected sum is a value obtaining by dividing the value by the sum of corresponding values for each of the one or more signatures.

Alternatively, the weights of the model may be determined in a tumour type-agnostic manner for each signature, a ratio of (i) a weighted average signature score of all amplified segments spanning the gene in samples of a specific tumour type in the reference cohort, wherein the average signature scores in the specific tumour type are weighted by the normalised empirical frequency of the gene amplification in the respective tumour type in the reference cohort and (ii) an averaged signature scores of all non-diploid segments found in the reference cohort. All tumour types present in the reference cohort may be used. Alternatively, only tumour types where the gene is recurrently amplified may be considered. The former may be particularly suitable for primary and/or untreated tumours. The latter may be particularly suitable for advanced and/or treated tumours.

At step 126, a predetermined threshold is identified applicable to the output of the model comprising the weights obtained at step 124. The predetermined threshold may be such that samples with a score from the model above the threshold are likely to acquire the gene amplification, and samples with a score from the model below the threshold are likely to acquire the gene amplification. Thus, the score and predetermined thresholds may be used to obtain a binary classifier that can classify samples between a first class that has a high likelihood of acquiring the gene amplification and a second class that has a low likelihood of acquiring the gene amplification, wherein the tumour is classified in the first class when the output of the model is above a predetermined threshold. The threshold amy be identified using prior knowledge. For example, when there is a known expected % of samples with the gene amplification in the cohort or a subset of the cohort (e.g. a specific tumour type), the threshold may be selected such that a % of samples corresponding to the expected % of samples in the cohort or subset are classified in the first class (i.e. have scores above the threshold). Alternatively, the threshold may be selected using one or more criteria applying to the sensitivity, selectivity or Youden's index of the binary classification (where a "positive" sample is one classified in the first class). For example, a threshold that maximises the Youden's index may be used.

It is advantageous (although not necessary) for the reference cohort to be selected to match one or more characteristics of a patient sample to be analysed. For example, the reference cohort may comprise or consist of samples that are one or more of: the same tumour type, the tumour grade/stage, undergone the same treatment, have the same driver mutations, have been analysed with the same sequencing technology, as the sample to be analysed.

At optional step 128, the results of one or more of the preceding steps (e.g. one or more of the tumour copy number profile, quantified features for segments, quantified metrics for segments, parameters of the components used, sample exposure, weights, etc) are provided to a user, for example through a user interface, or to a computing system memory or database. In particular, the weights identified at step 124 may be provided for use in a method as described in relation to **Figure 1A.**

**Table 1.** Definition of the 43 mixture components used. Feature=One of the five fundamental features of copy number aberrations; Mean = If SD is present, then this is the mean of the Gaussian component, otherwise mean of the Poisson component; SD = If present, standard deviation of the Gaussian component; Number = Number of component used as identifiers.

| Feature | Mean | SD | Number |
|---|---|---|---|
| segsize | 38420.7697 | 15438.8759 | 1 |
| segsize | 86319.107 | 30243.0733 | 2 |
| segsize | 171234.256 | 55096.0468 | 3 |
| segsize | 427454 | 174237 | 4 |
| segsize | 918939.408 | 260126.652 | 5 |
| segsize | 1494667.9 | 404499.004 | 6 |
| segsize | 2496039.63 | 608098.846 | 7 |

(continued)

| Feature | Mean | SD | Number |
|---|---|---|---|
| segsize | 3898217.97 | 583841.597 | 8 |
| segsize | 4908317.82 | 398896.482 | 9 |
| segsize | 6371179 | 985163 | 10 |
| segsize | 8780945 | 1086234 | 11 |
| segsize | 13435586 | 1439442 | 12 |
| segsize | 18665397 | 1664701 | 13 |
| segsize | 22913723.2 | 1028090.2 | 14 |
| segsize | 30753807 | 2370747.45 | 15 |
| segsize | 36812267.4 | 1799660.32 | 16 |
| segsize | 44866788 | 4250506.18 | 17 |
| segsize | 57831002.6 | 3574456.9 | 18 |
| segsize | 68086495.8 | 6023604.95 | 19 |
| segsize | 81015695.9 | 3149494.22 | 20 |
| segsize | 93268268 | 10492824.6 | 21 |
| segsize | 154120037 | 27901372.7 | 22 |
| changepoint | 0.10594582 | 0.06312023 | 1 |
| changepoint | 0.34283504 | 0.1572276 | 2 |
| changepoint | 1.00818588 | 0.2528096 | 3 |
| changepoint | 1.40754134 | 0.3988254 | 4 |
| changepoint | 2.20266882 | 0.4656683 | 5 |
| changepoint | 3.20155742 | 0.32865165 | 6 |
| changepoint | 4.11442601 | 0.49429328 | 7 |
| changepoint | 5.13850887 | 1.16026304 | 8 |
| changepoint | 6.93357762 | 1.76527488 | 9 |
| changepoint | 9.96341765 | 2.61954362 | 10 |
| bpwindow | 0 | NA | 1 |
| bpwindow | 1 | NA | 2 |
| bpwindow | 2 | NA | 3 |
| bpwindow | 3 | NA | 4 |
| bpwindow | 4 | NA | 5 |
| bpwindow | 5 | NA | 6 |
| bpwindow | 6 | NA | 7 |
| bpwindow | 8.912199 | NA | 8 |
| bpwindow | 26.982550 | NA | 9 |

**Table 2.** Aetiologies of CIN signatures in Drews et al. 2022. Sign=signature. Note CX6 is not used in the present work.

| Sign | Putative cause | Pattern of Change |
|---|---|---|
| CX1 | Chromosome missegregation via defective mitosis and/or telomere dysfunction | Whole arm/chromosome changes |
| CX2 | Impaired homologous recombination (HR) | Clustered, short to medium sized segments |

(continued)

| Sign | Putative cause | Pattern of Change |
|------|----------------|-------------------|
| CX3 | Impaired HR with replication stress and impaired damage sensing | Long segments with one copy change |
| CX4 | PI3K/AKT-mediated toleration of whole-genome duplication | Short and long segments with two copies changed |
| CX5 | Impaired HR with replication stress | Clustered, medium to medium-long sized segments in oscillating chains |
| CX6 | Chromosome missegregation via defective mitosis | Whole arm/chromosome changes |
| CX7 | Unknown | Segments with small changepoints |
| CX8 | Replication stress | Low level amplifications |
| CX9 | Replication stress | Mid-level focal amplifications |
| CX10 | Impaired NHEJ with replication stress | Clustered short to medium sized segments |
| CX11 | Replication stress | Clustered short segments |
| CX12 | Unknown | Short segments in long oscillating chains |
| CX13 | Replication stress | High level clustered amplifications |
| CX14 | Chromosome missegregation via defective mitosis | Whole arm/chromosome changes |
| CX15 | Unknown | Clustered medium sized segments |
| CX16 | Unknown (Impaired DNA repair?) | Clustered short segments of two to three copies |
| CX17 | Unknown (Replication stress?) | Clustered medium to medium-long sized segments of one to two copies |

**Table 3.** Signature by component definition matrix. CIN signatures are defined in the new feature space, which is composed by 41 components from 3 copy number features.

| | CX1 | CX2 | CX3 | CX4 | CX5 | CX7 |
|--|-----|-----|-----|-----|-----|-----|
| segsize1 | 0 | 0 | 0 | 0 | 0.003008 | 0 |
| segsize2 | 0 | 0 | 0 | 0.000258 | 0.006984 | 0 |
| segsize3 | 0 | 0.01485 | 0 | 0.009062 | 0.012509 | 0 |
| segsize4 | 0 | 0.046256 | 0 | 0.024486 | 0.038855 | 0 |
| segsize5 | 0 | 0.046792 | 0 | 0.024972 | 0.049311 | 0 |
| segsize6 | 0 | 0.042981 | 0 | 0.024842 | 0.054903 | 0 |
| segsize7 | 0 | 0.03803 | 0 | 0.024146 | 0.056993 | 0 |
| segsize8 | 0 | 0.017155 | 0.011221 | 0.010445 | 0.036519 | 0 |
| segsize9 | 0 | 0.006448 | 0.01542 | 0.002691 | 0.022418 | 0 |
| segsize10 | 0 | 0.006183 | 0.035946 | 0.003389 | 0.032475 | 0 |
| segsize11 | 0 | 0.002782 | 0.053594 | 0.001443 | 0.032137 | 0 |
| segsize12 | 0 | 0.000859 | 0.064384 | 0.002082 | 0.024896 | 0.002473 |
| segsize13 | 0 | 0.001325 | 0.048199 | 0.003212 | 0.013212 | 0.013641 |
| segsize14 | 0 | 0.002722 | 0.031708 | 0.005693 | 0.005202 | 0.014667 |
| segsize15 | 0 | 0.005238 | 0.049833 | 0.016119 | 0.002042 | 0.027837 |
| segsize16 | 0 | 0.001718 | 0.021927 | 0.013338 | 0 | 0.011535 |
| segsize17 | 0 | 0.001562 | 0.033293 | 0.015699 | 0 | 0.010037 |
| segsize18 | 0.038826 | 0 | 0.015552 | 0.009292 | 0 | 0.009259 |

(continued)

|  | CX1 | CX2 | CX3 | CX4 | CX5 | CX7 |
|---|---|---|---|---|---|---|
| segsize19 | 0.01016 4 | 0 | 0.01245 1 | 0.00982 4 | 0 | 0.00600 3 |
| segsize20 | 0.03804 3 | 0 | 0.00267 6 | 0.00998 9 | 0 | 0.00406 9 |
| segsize21 | 0.17135 3 | 0 | 0.00737 9 | 0.01391 4 | 0 | 0.00387 2 |
| segsize22 | 0.08557 1 | 0 | 0 | 0.01954 | 0 | 0 |
| changepoint1 | 0 | 0 | 0.00826 | 0 | 0.00742 2 | 0.23452 3 |
| changepoint2 | 0 | 0.01046 5 | 0.03166 1 | 0.00191 6 | 0.03594 6 | 0.48350 7 |
| changepoint3 | 0 | 0.29153 | 0.10156 7 | 0 | 0.06160 2 | 0 |
| changepoint4 | 0 | 0.15175 1 | 0.05381 | 0.09350 1 | 0.05796 4 | 0 |
| changepoint5 | 0 | 0 | 0.00477 2 | 0.30287 5 | 0.04912 6 | 0 |
| changepoint6 | 0 | 0 | 0 | 0.02110 3 | 0.01463 1 | 0 |
| changepoint7 | 0 | 0 | 0 | 0.00804 1 | 0.00558 | 0 |
| changepoint8 | 0 | 0 | 0 | 0.00876 1 | 0.00709 7 | 0 |
| changepoint9 | 0 | 0 | 0 | 0.00252 1 | 0.00522 6 | 0 |
| changepoint1 0 | 0 | 0 | 0 | 0 | 0.00330 8 | 0 |
| bpwindow1 | 0.65604 4 | 0 | 0 | 0.01178 1 | 0 | 0 |
| bpwindow2 | 0 | 0.05048 4 | 0.17277 | 0.06291 8 | 0.03788 1 | 0.09266 6 |
| bpwindow3 | 0 | 0.06447 1 | 0.13959 1 | 0.06603 4 | 0.06734 7 | 0.06071 9 |
| bpwindow4 | 0 | 0.06631 4 | 0.06912 9 | 0.06106 6 | 0.07397 6 | 0.02502 2 |
| bpwindow5 | 0 | 0.05942 4 | 0.01485 8 | 0.05242 8 | 0.06836 | 0.00017 |
| bpwindow6 | 0 | 0.04410 9 | 0 | 0.03927 1 | 0.05636 2 | 0 |
| bpwindow7 | 0 | 0.02654 8 | 0 | 0.02334 3 | 0.04223 4 | 0 |
| bpwindow8 | 0 | 0 | 0 | 0 | 0.01447 3 | 0 |
| bpwindow9 | 0 | 0 | 0 | 0 | 0 | 0 |

**Table 3 (continued).** Signature by component definition matrix. CIN signatures are defined in the new feature space, which is composed by 41 components from 3 copy number features.

|  | CX8 | CX9 | CX10 | CX11 | CX12 | CX13 |
|---|---|---|---|---|---|---|
| segsize1 | 0 | 0.01456 9 | 0.01658 7 | 0.12110 8 | 0.43320 8 | 0.01637 |
| segsize2 | 0 | 0 | 0.03310 6 | 0.14459 6 | 0.23770 3 | 0.01149 3 |
| segsize3 | 0 | 0 | 0.08134 3 | 0.18779 7 | 0.09070 4 | 0 |
| segsize4 | 0 | 0.02821 5 | 0.15967 4 | 0.13662 5 | 0 | 0 |
| segsize5 | 0 | 0.01241 7 | 0.06374 9 | 0 | 0 | 0 |
| segsize6 | 0 | 0 | 0.00683 4 | 0 | 0 | 0 |
| segsize7 | 0.00103 4 | 0 | 0 | 0 | 0 | 0 |
| segsize8 | 0 | 0 | 0 | 0 | 0 | 0 |
| segsize9 | 0 | 0 | 0 | 0 | 0 | 0 |
| segsize10 | 0 | 0 | 0 | 0 | 0 | 0 |
| segsize11 | 0 | 0 | 0.00392 4 | 0 | 0 | 0 |
| segsize12 | 0 | 0 | 0.00712 4 | 0 | 0 | 0 |

(continued)

|  | CX8 | CX9 | CX10 | CX11 | CX12 | CX13 |
|---|---|---|---|---|---|---|
| segsize13 | 0.00281 8 | 0 | 0.00667 7 | 0 | 0 | 0 |
| segsize14 | 0.01327 4 | 0.00331 8 | 0.00288 5 | 0 | 0 | 0 |
| segsize15 | 0.03011 4 | 0.03296 3 | 0.00445 1 | 0 | 0 | 0 |
| segsize16 | 0.02020 3 | 0.02627 3 | 0 | 0 | 0 | 0 |
| segsize17 | 0.03190 2 | 0.05072 1 | 0 | 0 | 0 | 0 |
| segsize18 | 0.01648 | 0.03182 4 | 0 | 0 | 0 | 0.002 |
| segsize19 | 0.01297 7 | 0.02501 3 | 0 | 0 | 0 | 0.00071 1 |
| segsize20 | 0.00907 9 | 0.00984 3 | 0 | 0 | 0 | 0.00109 6 |
| segsize21 | 0.01981 4 | 0.02760 8 | 0 | 0 | 0 | 0.00505 |
| segsize22 | 0.01225 8 | 0 | 0 | 0 | 0 | 0.00822 3 |
| changepoint1 | 0 | 0.02385 1 | 0.04722 6 | 0.02786 1 | 0.01833 9 | 0 |
| changepoint2 | 0 | 0 | 0 | 0.03056 1 | 0.02091 3 | 0 |
| changepoint3 | 0 | 0 | 0 | 0 | 0 | 0 |
| changepoint4 | 0 | 0 | 0.05171 4 | 0 | 0 | 0 |
| changepoint5 | 0 | 0 | 0.14806 9 | 0 | 0 | 0 |
| changepoint6 | 0.26506 2 | 0.07978 | 0.02575 | 0 | 0 | 0 |
| changepoint7 | 0.19741 | 0.09444 9 | 0.00666 3 | 0 | 0 | 0 |
| changepoint8 | 0.14005 8 | 0.10255 1 | 0.00579 4 | 0 | 0 | 0.02437 9 |
| changepoint9 | 0.08313 4 | 0.10612 4 | 0.00186 5 | 0 | 0.00230 2 | 0.07558 7 |
| changepoint1 0 | 0.02314 | 0.15412 8 | 0 | 0 | 0 | 0.53435 3 |
| bpwindow1 | 0 | 0 | 0 | 0 | 0 | 0.00485 2 |
| bpwindow2 | 0.06310 9 | 0 | 0.02525 5 | 0 | 0 | 0 |
| bpwindow3 | 0.03530 8 | 0 | 0.04776 3 | 0 | 0 | 0 |
| bpwindow4 | 0.01563 9 | 0 | 0.05639 9 | 0 | 0 | 0 |
| bpwindow5 | 0.00606 9 | 0 | 0.05775 3 | 0.01849 9 | 0 | 0 |
| bpwindow6 | 0.00111 7 | 0 | 0.05367 9 | 0.04886 4 | 0 | 0 |
| bpwindow7 | 0 | 0.04609 6 | 0.04864 6 | 0.06279 4 | 0.03900 5 | 0.05104 2 |
| bpwindow8 | 0 | 0.13025 7 | 0.03707 | 0.11987 2 | 0.08691 8 | 0.24513 3 |
| bpwindow9 | 0 | 0 | 0 | 0.10142 2 | 0.07090 8 | 0.01971 3 |

**Table 3 (continued).** Signature by component definition matrix. CIN signatures are defined in the new feature space, which is composed by 41 components from 3 copy number features.

|  | CX14 | CX15 | CX16 | CX17 |
|---|---|---|---|---|
| segsize1 | 0 | 0 | 0.13831 3 | 0 |
| segsize2 | 0 | 0 | 0.03019 | 0.00184 8 |
| segsize3 | 0 | 0 | 0.00909 | 0.00267 |
| segsize4 | 0 | 0 | 0.00308 8 | 0 |
| segsize5 | 0 | 0 | 0 | 0 |
| segsize6 | 0 | 0.02074 2 | 0.00432 5 | 0 |

(continued)

|  | CX14 | CX15 | CX16 | CX17 |
|---|---|---|---|---|
| segsize7 | 0 | 0.07228 4 | 0.01454 3 | 0.00988 9 |
| segsize8 | 0 | 0.05304 2 | 0.01835 1 | 0.01924 6 |
| segsize9 | 0 | 0.02931 5 | 0.01453 4 | 0.01070 3 |
| segsize10 | 0 | 0.04514 3 | 0.02442 8 | 0.01473 3 |
| segsize11 | 0 | 0.04096 9 | 0.02451 4 | 0.02577 6 |
| segsize12 | 0 | 0.03126 8 | 0.01899 | 0.03742 4 |
| segsize13 | 0 | 0.01462 3 | 0.01047 9 | 0.04257 4 |
| segsize14 | 0 | 0.00833 9 | 0.00535 4 | 0.02155 9 |
| segsize15 | 0 | 0.01434 8 | 0.00323 7 | 0.02572 2 |
| segsize16 | 0.04817 5 | 0.00242 2 | 0 | 0.02082 4 |
| segsize17 | 0.05332 2 | 0.00371 7 | 0 | 0.02495 1 |
| segsize18 | 0.03529 9 | 0.00208 8 | 0 | 0.02350 8 |
| segsize19 | 0.04476 5 | 0.00100 8 | 0 | 0.01788 7 |
| segsize20 | 0.05721 7 | 0 | 0 | 0.00512 2 |
| segsize21 | 0.05847 4 | 0.00257 1 | 0 | 0.00561 7 |
| segsize22 | 0.18922 4 | 0 | 0 | 0.00686 1 |
| changepoint1 | 0.25740 8 | 0.04066 2 | 0.01010 8 | 0.00301 7 |
| changepoint2 | 0.10930 1 | 0.14691 5 | 0.01258 7 | 0 |
| changepoint3 | 0 | 0.10511 7 | 0.03538 3 | 0 |
| changepoint4 | 0 | 0.03176 1 | 0.06506 6 | 0.10074 7 |
| changepoint5 | 0 | 0 | 0.13936 7 | 0.14213 2 |
| changepoint6 | 0 | 0 | 0.05640 8 | 0.03853 8 |
| changepoint7 | 0 | 0 | 0.00598 8 | 0.02669 8 |
| changepoint8 | 0 | 0 | 0.00800 3 | 0.01870 6 |
| changepoint9 | 0 | 0 | 0.00347 3 | 0.00870 1 |
| changepoint1 0 | 0 | 0 | 0.00041 3 | 0 |
| bpwindow1 | 0.11839 1 | 0 | 0 | 0.00522 9 |
| bpwindow2 | 0.02842 5 | 0.11329 7 | 0.05518 4 | 0.12114 3 |
| bpwindow3 | 0 | 0.10770 4 | 0.08058 7 | 0.11034 9 |
| bpwindow4 | 0 | 0.07103 2 | 0.07813 6 | 0.07099 4 |
| bpwindow5 | 0 | 0.03708 8 | 0.06292 1 | 0.0342 |
| bpwindow6 | 0 | 0.00454 7 | 0.04308 2 | 0.00263 2 |
| bpwindow7 | 0 | 0 | 0.02385 9 | 0 |
| bpwindow8 | 0 | 0 | 0 | 0 |
| bpwindow9 | 0 | 0 | 0 | 0 |

Clinical Applications

The above methods find applications in the context of predicting whether a subject's tumour is likely to acquire a gene amplification, such as an oncogene amplification. Oncogene amplification is associated with treatment response and prognosis. Thus, the disclosure also provides a method of determining a prognosis and/or predicting a treatment response for a subject. Further, the disclosure also provides a method of treating cancer in a subject, wherein the method comprises administering or recommending a subject for administration of a particular therapy, depending on the signature(s) of chromosomal instability that have been found to be active in the sample (and in particular depending on whether the subject is predicted as likely to acquire an oncogene amplification associated with response to a therapy and/or prognosis).

**Figure 2** illustrates a method of providing a prognosis, identifying a therapy or treating a subject according to an embodiment of the present disclosure.

At step 200, one or more tumour samples may be obtained. These may have been previously obtained from a subject. At step 202, a tumour copy number profile derived from the sample is analysed using a method as described herein, such as e.g. by reference to Figure 1A, to determine whether the tumour is likely to acquire an oncogene amplification.

At step 204, the subject may be identified as likely to respond to a therapy or to acquire resistance to the therapy (i.e. fail to respond to the therapy), based on the results of step 202. In particular the oncogene amplification may be one that is known to be associated with resistance to the therapy. Instead or in addition to this, at step 205, the subject may be identified as having a poor or good prognosis based on the results of step 202. In particular the oncogene amplification may be one that is known to be associated with poor prognosis. At step 206, a treatment may be identified based on te results of step 204 and/or step 205. For example, a subject identified as likely to acquire resistance to a therapy through acquisition of the gene amplification may be selected for treatment with an alternative or additional therapy. Conversely, a subject identified as unlikely to acquire resistance to the therapy may be selected for treatment with the therapy as a first line of therapy. As another example, a subject identified as having poor prognosis may be treated with more aggressive treatment and/or with a treatment suitable for advanced tumours. At optional step 208, the subject may be treated with the therapy(ies) identified at step 206.

Oncogene amplification events have been shown to be associated with different response to therapy, and in particular to acquisition of resistance to therapy. Thus, also described herein are methods of determining whether a subject that has been diagnosed as having a cancer is likely to acquire resistance to a therapy, the method comprising determining whether the subject is likely to acquire a gene amplification (e.g. oncogene amplification) using a method as described herein. The method may further comprise classifying the subject between a group that is likely to acquire resistance to the therapy (i.e. unlikely to respond to the therapy), and a group that is unlikely to acquire resistance to the therapy (i.e. likely to respond to the therapy). For example, the method may comprise determining whether a sample from a tumour of the subject has a high or low likelihood of acquiring a gene amplification that has been identified to be associated with resistance to therapy (as explained above). A subject may then be classified in the group that is likely to acquire resistance to the therapy if the sample is determined to have a high likelihood of acquiring the gene amplification, and in a group that is unlikely to acquire resistance to the therapy otherwise.

Any treatment described herein may be used alone or in combination with another treatment. For example, any treatment with a drug may be used in combination with one or more chemotherapies, one or more courses of radiation therapy, and/or one or more surgical interventions. In particular, any treatment described herein may be used in combination with a treatment for which the subject has been identified as likely to be responsive.

Additionally, oncogene amplification events have been shown to be associated with different prognosis. Thus, also described herein are methods of providing a prognosis for a subject that has been diagnosed as having a cancer, the method comprising determining whether the subject is likely to acquire a gene amplification (e.g. oncogene amplification) using a method as described herein. The method may further comprise classifying the subject between a group that has good prognosis, and a group that has poor prognosis. For example, the method may comprise determining whether a sample from a tumour of the subject has a high or low likelihood of acquiring a gene amplification that has been identified to be associated with prognosis (as explained above). A subject may then be classified in the group that has poor prognosis if the sample is determined to have a high likelihood of acquiring the gene amplification, and in a group that has good prognosis otherwise.

Whether a prognosis is considered good or poor may vary between cancers and stage of disease. In general terms a good prognosis is one where the overall survival (OS), disease free survival (DFS) and/or progression-free survival (PFS) is longer than that of a comparative group or value, such as e.g. the average for that stage and cancer type. A prognosis may be considered poor if OS, DFS and/or PFS is lower than that of a comparative group or value, such as e.g. the average for that stage and type of cancer. Thus, in general terms, a "good prognosis" is one where survival (OS, DFS and/or PFS) and/or disease stage of an individual patient can be favourably compared to what is expected in a population of patients within a comparable disease setting. Similarly, a "poor prognosis" is one where survival (OS, DFS and/or PFS) of an individual patient is lower (or disease stage worse) than what is expected in a population of patients within a comparable disease setting.

The subject is preferably a human patient. The subject may be a patient with a cancer selected from any cancer type present in the TCGA and/or PCAWG data. The cancer may be selected from: ovarian cancer, breast cancer, endometrial cancer, kidney cancer, lung cancer, pancreatic cancer, liver cancer, oesophagus cancer, stomach cancer, head and neck cancer, brain cancer, colon cancer, pancreatic cancer, prostate cancer, bladder cancer, cervical cancer, leukemia, lymphoma, testicular cancer, thyroid cancer, melanoma, adrenal cancer, bowel cancer, sarcoma, thymoma, neuroendocrine tumour, and bile duct cancer.

Systems

**Figure 3** shows an embodiment of a system for implementing any method according to the present disclosure. The system comprises a computing device 1, which comprises a processor 101 and computer readable memory 102. In the embodiment shown, the computing device 1 also comprises a user interface 103, which is illustrated as a screen but may include any other means of conveying information to a user such as e.g. through audible or visual signals. The computing device 1 is communicably connected, such as e.g. through a network 6, to sequence data acquisition means 3, such as a sequencing machine, and/or to one or more databases 2 storing sequence data. The one or more databases may additionally store other types of information that may be used by the computing device 1, such as e.g. reference sequences, parameters, etc. The computing device may be a smartphone, tablet, personal computer or other computing device. The computing device is configured to implement a method as described herein. In alternative embodiments, the computing device 1 is configured to communicate with a remote computing device (not shown), which is itself configured to implement a method as described herein. In such cases, the remote computing device may also be configured to send the result of the method to the computing device. Communication between the computing device 1 and the remote computing device may be through a wired or wireless connection, and may occur over a local or public network such as e.g. over the public internet or over WiFi. The sequence data acquisition 3 means may be in wired connection with the computing device 1 and/or the database 2, or may be able to communicate through a wireless connection, such as e.g. through a network 6, as illustrated. The connection between the computing device 1 and the sequence data acquisition means 3 may be direct or indirect (such as e.g. through a remote computer). The sequence data acquisition means 3 are configured to acquire sequence data from nucleic acid samples, for example genomic DNA samples extracted from cells and/or tissue samples. The sequence data acquisition means is preferably a next generation sequencer, but can also be an array reader. The sequence data acquisition means 3 may be in direct or indirect connection with one or more databases 2, on which sequence data (raw or partially processed) may be stored.

The following is presented by way of example and is not to be construed as a limitation to the scope of the claims.

EXAMPLES

Introduction

Mutation, selection and genetic drift ultimately determine the structure of a tumour genome. Therefore, to accurately predict future somatic copy number alterations (CNAs), we need an understanding of how tumours acquire new CNAs and the selective pressures that act on them.

The inventors have recently described that tumours acquire somatic CNAs through different mechanisms, reflected in a compendium of pan-cancer chromosomal instability (CIN) signatures (Drews et al. 2022). These signatures provide a framework to read out the different types of CIN present in a tumour. Given that the probability of fixation of a CNA in a tumour can be linked to the rate at which the CNA emerges, the inventors hypothesised that the mutational mechanisms within a tumour influence the next most likely step in its progression. For example, tumours with breakage-fusion-bridge cycle errors tend to have higher numbers of focal amplifications. Therefore, the inventors hypothesised that it should be possible to use CIN signatures to identify the mutational processes active in a tumour and use their presence to forecast amplification.

Example 1 describes a new approach to CIN signature feature encoding to enable CIN signature quantification for individual copy number events.

Example 2 provides an investigation of the mutation generating processes and selection in cancer demonstrating the theoretical feasibility of associating signatures of chromosomal instability with oncogene amplification.

Example 3 explains a method of identifying CIN processes underlying oncogene amplification using the framework in Example 1, in particular associating signature specific weights to oncogene amplifications.

Example 4 demonstrates the use of the weights obtained in Example 4 to predict future oncogene amplification.

Example 5 extensively evaluates the performance of the predictor developed in Examples 3 and 4.

Example 6 provides an adapted version of the process described in Example 3, which uses weights identified as described in Example 3 but using a cohort of samples selected as later time points from longitudinal data.

Example 7 evaluates the robustness of the predictor developed in Examples 3 and 9.

Example 8 demonstrates the clinical utility of the predictor to predict future treatment resistance and prognosis.

Example 9 describes a variant of the approach in examples 3 and 4, using a pan-oncogene model.

Data & Methods

*Data Sources.* All data used in the present examples are publicly accessible, except for the Hartwig Medical Foundation data (WGS). The access to the Hartwig data can be obtained by a Data Access Request through the Hartwig Medical Foundation https://www.hartwigmedicalfoundation.nl/en/data/data-access-request/. The TCGA copy number profiles used here were from Drews et al., 2022 (files hosted on github.com/VanLoo-lab/ascat). TCGA clinical data is available at TCGA Research Network: www.cancer.gov/tcga. PCAWG copy number profiles were from Gerstung, et al. 2020; Dentro, et al. 2021 (Files hosted on dcc.icgc.org/releases/PCAWG). Clinical annotation for PCAWG data is available from Pan-Cancer Analysis of Whole Genomes Consortium (2020). Files hosted on dcc.icgc.org/releases/PCAWG/. Triple-negative status for TCGA breast cancer samples was from Lehmann, et al., 2016. PCAWG SV amplicons annotation was from Kim et al., 2020.. GLASS copy number profiles were from Barthel et al., 2019 (files hosted on synapse.org/glass under the project ID syn17038081). GLASS clinical data was from Barthel et al., 2019 (files hosted on synapse.org/glass under the project ID syn17038081). TRACERx Lung copy number profiles and clinical data from Bakir et al, 2023 are publically available at zenodo.org/records/7649257. Raw single cell DNA sequencing data from TNBC tumours and cell lines was from Minussi et al., 2021 (files hosted on the NCBI Sequence Read Archive under the accession number PRJNA629885 www.ncbi.nlm.nih.gov/sra?term=PRJNA629885). Driver gene classification was from Bailey et al., 2018; Jassal et al., 2020; Martínez-Jiménez et al., 2020; Sondka et al., 2018. List of non-cancer genes was from Ensembl GRCh37 genome browser: grch37.ensembl.org/index.html. Genomic data of parental and resistant lung cancer cell lines was generated in this project (see section "Experimental data").

*Software.* If not otherwise stated, all analyses were performed using the free statistical software R (v>4.0). If functions outside the standard packages were used, they are mentioned and cited throughout the text. The following packages were used: ggradar v.0.2 (Bion, R. ggradar: Create radar charts using ggplot2. (2023)); showtext v.0.9-6 (Qiu, Y. & details., A. of T. I. S. S. F. A. F. showtext: Using Fonts More Easily in R Graphs. Preprint at CRAN.R-project.org/package=showtext (2023)); showtextdb v.3.0 (Qiu, Y. & details., A. of T. I. F. S. F. A. F. showtextdb: Font Files for the 'showtext' Package. CRAN.R-project.org/package=showtextdb (2020)); sysfonts v.0.8.8 (Qiu, Y. & details., A. of T. I. F. S. F. A. F. sysfonts: Loading Fonts into R. CRAN.R-project.org/package=sysfonts (2022)); QDNAseqmod v.1.21.0 (github.com/gmaci/QDNAseqmod); randomcoloR v.1.1.0.1 (Ammar, R. randomcoloR: Generate Attractive Random Colors. CRAN.R-project.org/package=randomcoloR (2019)); ROCit v.2.1.1

(Khan, M. R. A. & Brandenburger, T. ROCit: Performance Assessment of Binary Classifier with Visualization. CRAN.R-project.org/package=ROCit (2020)); CNpare v.0.99.0 (Chaves-Urbano, B., Hernando, B., Garcia, M. J. & Macintyre, G. CNpare: matching DNA copy number profiles. Bioinformatics 38, 3638-3641 (2022)); forcats v.1.0.0(Wickham, H. forcats: Tools for Working with Categorical Variables (Factors). Preprint at https://CRAN.R-project.org/package=forcats (2023)); purrr v.1.0.1 (Wickham, H. & Henry, L. purrr: Functional Programming Tools. Preprint at CRAN.R-project.org/package=purrr (2023)); tidyr v.1.3.0 (Wickham, H., Vaughan, D. & Girlich, M. tidyr: Tidy Messy Data. Preprint at CRAN.R-project.org/package=tidyr (2023)); tidyverse v.2.0.0 (Wickham, H. et al. Welcome to the tidyverse. Journal of Open Source Software vol. 4 1686 doi.org/10.21105/joss.01686 (2019)); knitr v.1.42 (Xie, Y. Dynamic Documents with R and Knitr, 2nd Edition. (2015)); cowplot v.1.1.1 (Wilke, C. O. cowplot: Streamlined Plot Theme and Plot Annotations for 'ggplot2'. CRAN.R-project.org/package=cowplot (2020)); lattice v.0.20-45 (Sarkar, D. Lattice: Multivariate Data Visualization with R. Preprint at lmdvr.r-forge.r-project.org (2008)); patchwork v.1.1.2 (Pedersen, T. L. patchwork: The Composer of Plots. Preprint at CRAN.R-project.org/package=patchwork (2022)); mclust v.6.0.0 (mclust: Gaussian Mixture Modelling for Model-Based Clustering, Classification, and Density Estimation; cran.r-project.org/web/packages/mclust/index.html); lsa v.0.73.3 (Wild, F. lsa: Latent Semantic Analysis. CRAN.R-project.org/package=lsa (2022); qgraph v.1.9.4 (Epskamp, S., Cramer, A. O. J., Waldorp, L. J., Schmittmann, V. D. & Borsboom, D. qgraph: Network Visualizations of Relationships in Psychometric Data. Journal of Statistical Software vol. 48 1-18 (2012)); RColorBrewer v.1.1-3 (Neuwirth, E. RColorBrewer: ColorBrewer Palettes. Preprint at CRAN.R-project.org/package=RColorBrewer (2022)); ggthemes v.4.2.4 (Arnold, J. B. ggthemes: Extra Themes, Scales and Geoms for 'ggplot2'. CRAN.R-project.org/package=ggthemes (2021)); ComplexHeatmap v.2.12.1 (Gu, Z., Eils, R. & Schlesner, M. Complex heatmaps reveal patterns and correlations in multidimensional genomic data. Bioinformatics 32, 2847-2849 (2016)); ggplot2 v.3.4.2 (Wickham, H. ggplot2: Elegant Graphics for Data Analysis. Preprint at ggplot2.tidyverse.org (2016)); iterators v.1.0.14 (Analytics, R. & Weston, S. iterators: Provides Iterator Construct. CRAN.R-project.org/package=iterators (2022)); GenomicRanges v.1.50.0 (Lawrence, M. et al. Software for Computing and Annotating Genomic Ranges. PLoS Computational Biology vol. 9 doi.org/10.1371/journal.pcbi.1003118 (2013)); IRanges v.2.32.0 (Lawrence, M. et al. Software for Computing and Annotating Genomic Ranges. PLoS Computational Biology vol. 9 doi.org/10.1371/journal.pcbi.1003118 (2013)); S4Vectors v.0.36.0 (Pagès, H., Lawrence, M. & Aboyoun, P. S4Vectors: Foundation of vector-like and list-like containers in Bioconductor. bioconductor.org/packages/S4Vectors (2022)); data.table v.1.14.8 (Dowle, M. & Srinivasan, A. data.table: Extension of 'data.frame'. CRAN.R-project.org/package=data.table (2023));

Biobase v.2.58.0 (Huber, W. et al. Orchestrating high-throughput genomic analysis with Bioconductor. Nature Methods vol. 12 115-121 nature.com/nmeth/journal/v12/n2/full/nmeth.3252.html (2015)); MEDICC2 v.1.0.0 (Kaufmann, T. L. et al. MEDICC2: whole-genome doubling aware copy-number phylogenies for cancer evolution. Genome Biol. 23, 241 (2022)); survminer v.0.4.9 (Kassambara, A., Kosinski, M., Biecek, P. & Fabian, S. Drawing Survival Curves using 'ggplot2'. (2021)); survival v.3.5-5 (Therneau, T. M. A package for survival analysis in R. (2023)); PRROC v.1.3.1 (Keilwagen, J., Grosse, I. & Grau, J. Area under precision-recall curves for weighted and unweighted data. PLoS One 9, e92209 (2014)); gridExtra v.2.3 (Auguie, B. gridExtra: Miscellaneous Functions for 'Grid' Graphics. Preprint at CRAN.R-project.org/package=gridExtra (2017)); ggbeeswarm v.0.7.1 (Clarke, E., Sherrill-Mix, S. & Dawson, C. ggbeeswarm: Categorical Scatter (Violin Point) Plots. CRAN.R-project.org/package=ggbeeswarm (2022)); pROC v.1.18.0 (Robin, X. et al. pROC: an opensource package for R and S+ to analyze and compare ROC curves. BMC Bioinformatics vol. 12 77 (2011)); biomaRt v.2.52.0 (Durinck, S., Spellman, P. T., Birney, E. & Huber, W. Mapping identifiers for the integration of genomic datasets with the R/Bioconductor package biomaRt. Nat. Protoc. 4, 1184-1191 (2009)); lubridate v.1.9.2 (Grolemund, G. & Wickham, H. Dates and Times Made Easy with lubridate. Journal of Statistical Software vol. 40 1-25 www.jstatsoft.org/v40/i03/ (2011)); stringr v.1.5.0 (Wickham, H. stringr: Simple, Consistent Wrappers for Common String Operations. CRAN.R-project.org/package=stringr (2022)); readr v.2.1.4 (Wickham, H., Hester, J. & Bryan, J. readr: Read Rectangular Text Data. CRAN.R-project.org/package=readr (2023)); tibble v.3.2.1 (Müller, K. & Wickham, H. tibble: Simple Data Frames. CRAN.R-project.org/package=tibble (2023)); rstudioapi v.0.14 (Ushey, K., Allaire, J. J., Wickham, H. & Ritchie, G. rstudioapi: Safely Access the RStudio API. CRAN.R-project.org/package=rstudioapi (2022)); circlize v.0.4.15 (Gu, Z., Gu, L., Eils, R., Schlesner, M. & Brors, B. circlize implements and enhances circular visualization in R. Bioinformatics vol. 30 2811-2812 (2014)); flexmix v.2.3-19 (Grün, B. & Leisch, F., 2008); ggrepel v.0.9.3 (Sarkar, D. Lattice: Multivariate Data Visualization with R. lmdvr.r-forge.r-project.org (2008)); reshape2 v.1.4.4 (Wickham, H. Reshaping Data with the reshape Package. Journal of Statistical Software vol. 21 1-20 www.jstatsoft.org/v21/i12/ (2007)); dplyr 1.1.1 (Wickham, H., François, R., Henry, L., Müller, K. & Vaughan, D. dplyr: A Grammar of Data Manipulation. CRAN.R-project.org/package=dplyr (2023)); SnowballC v. 0.7.0

(Bouchet-Valat, M. SnowballC: Snowball Stemmers Based on the C 'libstemmer' UTF-8 Library. CRAN.R-project.org/package=SnowballC (2020)); igraph v. 1.4.1 (Csardi, G. & Nepusz, T. The igraph software package for complex network research. InterJournal vol. Complex Systems 1695 igraph.org (2006)); lemon v. 0.4.6(Edwards, S. M. lemon: Freshing Up your 'ggplot2' Plots. Preprint at CRAN.R-project.org/package=lemon (2022)); ggpubr v.0.6.0 (Kassambara, A. ggpubr: 'ggplot2' Based Publication Ready Plots. Preprint at CRAN.R-project.org/package=ggpubr (2023)); YAPSA v1.22.0 (Hübschmann, D. et al. 2021); doMC v. 1.3.8(Analytics, R. & Weston, S. doMC: Foreach Parallel Adaptor for 'parallel'. CRAN.R-project.org/package=doMC (2022)); foreach v.1.5.2 (foreach: Provides Foreach Looping Construct, cran.r-project.org/web/packages/foreach/index.html); GenomeInfoDb v.1.34.8 (Arora, S., Morgan, M., Carlson, M. & Pagès, H. GenomeInfoDb: Utilities for manipulating chromosome names, including modifying them to follow a particular naming style. bioconductor.org/packages/GenomeInfoDb (2023).); DescTools v.0.99.49(DescTools: Tools for Descriptive Statistics; cran.r-project.org/package=DescTools); QDNAseq v.1.21.0 (Scheinin, I. et al. DNA copy number analysis of fresh and formalin-fixed specimens by shallow whole-genome sequencing with identification and exclusion of problematic regions in the genome assembly. Genome Res. 24, 2022-2032 (2014)); this.path v.1.2.0 (Simmons, A. this.path: Get Executing Script's Path, from 'Rgui', 'RStudio', 'VSCode', 'source()', and 'Rscript' (Shells Including Windows Command Line / / Unix Terminal). CRAN.R-project.org/package=this.path (2023)); BiocGenerics v.0.44.0 (Huber, W. et al. Orchestrating high-throughput genomic analysis with Bioconductor. Nature Methods vol. 12 115-121 Preprint at www.nature.com/nmeth/journal/v12/n2/full/nmeth.3252.html (2015)).

*Experimental data*

*Human cancer cell lines.* The 4 EGFR-mutant NSCLC cell lines (HCC827, HCC4006, PC9 and H1975) were obtained from the American Type Culture Collection (Manassas, VA). All cell lines were maintained in Roswell Park Memorial Institute (RPMI) 1640 medium (GIBCO, Carlsbad, CA) with 10% fetal bovine serum (FBS), penicillin (100 U/mL), and streptomycin (50 mg/mL) (Complete Medium) in a humidified $CO^2$ incubator at 37°C. All cells were passaged for less than 3 months before being renewed with frozen, early-passage stocks. Cells were regularly screened for mycoplasma using a MycoAlert Mycoplasma Detection Kit (Lonza). Before performing the experiments, cell line identities were confirmed by STR profiling and *EGFR* mutation was confirmed by droplet digital PCR (ddPCR) using the following ddPCR™ Mutation Detection Assays (Bio-Rad Laboratories): ddPCR *EGFR* Exon 19 Deletions Screening Kit (12002392), *EGFR* p.L858R (dHsaMDS463105111), and *EGFR* p.T790M (dHsaMDS759157834).

Resistant clones were generated by long-term culture of individual cell lines with high concentration of a EGFR tyrosine kinase inhibitor (EGFR-TKI), as previously described (Ramiraz et al. 2016). Briefly, a total of $1 \times 10^6$ cells were seeded into 10-cm dishes and allowed to adhere overnight. The following day, the medium was replaced with fresh complete medium containing 2 mM erlotinib for HCC827, 10 mM erlotinib for HCC4006 and PC9, or 0.7 mM osimertinib for H1975. After approximately 9 days, most cells perished, leaving behind a small number of isolated survivor cells. Clearly separated colonies were isolated and transferred to 96-well plates between 8 and 12 weeks of drug treatment. From there, colonies were expanded successively from 96-well plates to 24-well plates, then to 6-well plates, until finally reaching confluency in a 10-cm plate, thereby establishing individual resistant clones. During the whole process and after generation of resistant clones, medium was replaced every 2-3 days with fresh drug at the same concentration used initially. Cell lines were periodically examined and found negative for mycoplasma contamination during the course of this work. Canonical resistant mutations, such as T790M (dHsaMDS759157834) and C797S (dHsaMDS759157834), were assessed by ddPCR and tested negative for all resistant clones.

*DNA extraction.* Formalin-fixed, paraffin-embedded (FFPE) tissue blocks were cut as 5µm sections. Microdissection was not used for recovering tumour-enriched regions. DNA was extracted from 10-25 sections using QIAamp DNA FFPE Tissue Kit from Qiagen (Cat. No. 56404) following manufacturer's instructions. DNA extraction from cell pellets of about five million cells per sample was performed using the DNeasy® Blood & Tissue Kit from Qiagen (Cat. No. 69504). Cell pellets were equilibrated at room temperature for 15 minutes and then resuspended in PBS as recommended by the manufacturer. DNA was finally eluted in Tris-HCl (pH=8, 10 mM) instead of using Qiagen's Buffer AE. This buffer was not used for elution because it contains EDTA, which can inhibit some needed enzymatic reactions during Library Preparation. DNA was quantified using the ADNds Quant-iT™ PicoGreen™ kit from Invitrogen™ (Cat. No. P7589).

*DNA sequencing - Shallow whole genome sequencing (sWGS).* For sample preparation, DNA at 5 ng/µL was required. 50 µL of sample was dispensed into a Covaris microTUBE AFA Fiber Pre-Slit Snap-Cap 6x16mm (Cat. No. 520045) and DNA was mechanically fragmented using the E220evolution focused-ultrasonicator from Covaris. 150 seconds of shearing for cell pellets and 300 seconds for FFPE samples was needed for obtaining fragments of about 240 bp. DNA profile was checked with the LabChip DNA High Sensitivity Reagent kit from PerkinElmer (Cat. No. CLS760672) using the LabChip GX Touch Nucleic Acid Analyzer (Cat. No. CLS137031) from PerkinElmer. For library preparation, ThruPLEX® DNA-Seq Kit (Cat. No. R400674) was used following manufacturer's instructions for 50 ng of input DNA sample, but performing 5 cycles for sample amplification. We used indexes from the DNA HT Dual Index Kit - 24 N from Takara (Cat. No. R400664) for cell pellet DNA samples, and from the Unique Dual Index Kit - 48U from Takara (Cat. No. R400744) for FFPE DNA samples. Library cleanup was performed with Agencourt AMPure XP Reagent (Cat. No. A63881) following manufacturer's instructions. Final quality control of libraries was assessed via sample quantification using the ADNds Quant-iT™ PicoGreen™ kit, and via library profiles using the LabChip DNA High Sensitivity Reagent kit. Libraries were sequenced by the Genomics Unit of the Spanish National Cancer Research Centre (CNIO) with the NextSeq™ 550 system from Illumina.

*DNA sequencing - Whole exome sequencing (WES).* For sample preparation, a total of 200 ng of DNA was required. WES was conducted by a commercial service (Macrogen Inc., Seoul, Korea). Libraries were constructed using the SureSelect Human All Exon v6 (Agilent Technologies, Santa Clara, CA, USA). Sequencing of pair-end reads of 151 bp length was performed with the Novaseq system from Illumina, obtaining an average total reads across samples of 350 million reads (range 98-510 million reads).

*Sequence read alignment.* Reads were aligned as single-end against the human genome assembly GRCh37 using BWA-MEM (v.0.7.17)(Li and Durbin, 2009). Duplicate reads were then identified and marked using samtools-markdup (v1 .15)(Li et al. 2009).

*Generating copy number profiles.* After alignment, absolute copy number profiles were fitted from sequencing data generated across different high-throughput technologies (method applied for each dataset is detailed below). Following absolute copy number fitting, samples were rated using a star system as previously described (Macintyre, G. et al. 2018; Madrid, L. et al. 2023). Briefly, samples with noisy or flat copy number profiles where absolute copy number cannot be estimated are given 1-star, samples with fittable absolute copy number but with some noise are given 2-star, well fit samples without noise are given 3-star. Those with 1-star were discarded for downstream analyses. In addition, samples without the appropriate read counts (15 number of reads per bin per chromosome copy) for supporting the fitting were also discarded.

*Absolute copy number fitting from sVVGS.* We used the QDNAseq R package (Scheinin, I. et al. 2014) to count reads within 30 kb bins (resolution threshold determined to accurately call copy number signatures in Drews et al. 2022). Bins mapped to centromeres and regions of undefined sequence in the reference genome hg19 were excluded. Read counts were then corrected for sequence mappability and GC content, followed by copy number segmentation. After the profile was segmented, we inferred absolute copy numbers for combinations of purity and ploidy values (0.05<purity<1, and 1.5<ploidy<8) as follows:

$$cn = \frac{1}{purity} \times \left( \frac{r}{d} - 2 * (1 - purity) \right)$$

where *purity* is the fraction of tumour cells in the sample, *r* is the read counts, and *d* is a constant proportional to the read depth and the average absolute copy number of tumour cells in the sample (*ploidy*):

$$d = \frac{r}{(ploidy \times purity + 2 \times (1 - purity))}$$

The optimal mathematical solution was considered the one with the lowest root mean squared deviation (RMSD) between the non-rounded and the rounded copy numbers for all bins. All solutions were manually inspected to either confirm or correct for more appropriate fits. For pure cancer models (i.e. cell lines, single cells), a range of purities from 0.9 to 1 for fitting absolute copy numbers can be used instead.

*Absolute copy number fitting from VVES.* Paired-end raw reads were aligned to the human hg19 reference genome. The resulting alignments were split into equally-sized bins of 50 kb in size. Bins were annotated with replication timing and a set of metadata inherited from QDNAseq: gc, mappability, residuals, bases and blacklist. The annotated bins were then interrogated for overlaps with target regions (bed file) and divided in two groups: 1) off-target bins, with a zero overlap with targeted regions. In this case, reads were counted normally in the entire bin. 2) on-target bins, those with at least 1 overlap with a target region. In this case every overlap was deleted and the remaining off-target regions were pasted together forming a new, smaller bin, ready for read counting. GC correction was performed using LOESS. To correct for artificially high off-target read counts in parts of the genome with high sequence similarity to the target regions we generated a score per-bin that quantified the magnitude of this bias and used it for a single LOESS fit and correction. We segmented these data using a modified version of the circular binary segmentation (CBS) from the DNAcopy R package. Absolute copy numbers were computed as described above in the "Inferring absolute copy number from sWGS" section.

*Absolute copy number fitting from single cell WGS.* To infer copy number profiles from single cell DNA sequencing data, the inventors defined 100 kb windows along the genome and applied the same methodology described in the "Inferring absolute copy number from sWGS" section.

*Absolute copy number fitting from pseudobulk WGS.* The inventors reconstructed bulk copy number profiles by concatenating reads of all single cells sequenced from a specific sample. They then downsampled pseudobulk bams to appropriate read counts based on the bin size (30 kb), purity and ploidy. Absolute copy number fitting can then be performed as described in the "Inferring absolute copy number from sWGS" section.

*Detection of focal amplifications - bulk samples.* Focal amplifications in unpaired WGS, WES and SNP6 bulk data were defined as genomic regions with absolute copy number higher or equal to 8. This threshold was selected after an extensive analysis using a range of threshold values (see section "Assessing the threshold for defining focal amplifications"). The same threshold was applied for detecting clonal amplifications in pseudobulk profiles. In longitudinal analyses, the acquisition of a new focal amplification in the late/relapse/metastasis biopsies was defined as the gain of at least 6 copies of a genomic region with respect to the early time point sample.

*Detection of focal amplifications single cells.* Unique focal amplifications were defined as those genomic regions with absolute copy numbers higher or equal to 8 present in a specific cell. If those amplifications were present in more than one cell, the inventors defined them as shared focal amplifications. Given the uncertainty in the location of the breakpoints during segmentation of single cell copy number profiles, the inventors took a heuristic approach to determine whether an amplification was truly present in only one or more cells. In brief, they extended each of the putative unique amplifications by a 1 Mb window per segment side, and looked for overlaps with the amplifications present in other cells. If they found overlaps, the amplification was classified as shared, and otherwise as unique.

*Exploring acquisition of focal amplifications.* The inventors first explored how amplifications arise in the absence of selection pressures. In this regard, they used publicly available single cell sequencing data from 8 triple negative breast cancer (TNBC) patients and 4 TNBC cell lines (Minussi, D. C. et al. 2021). Focal amplifications present in one single cell (unique amplifications) are randomly caused by a mutational process and their acquisition is therefore not subjected to selection pressures. Unique amplifications cannot be detected at bulk level. On the contrary, only positively selected amplifications (which are therefore shared across a high number of cells in the tumour, i.e. clonal amplifications) can be detected at bulk level. The inventors used a binomial test (function *binom.test* from the R package *stats*) to compare the fraction of cells carrying at least one unique focal amplification with the fraction of clonal amplifications observed across the 12 matched pseudobulk samples and the 145 TCGA-TNBC samples. In addition, they tested if having higher amplification rates is required for acquiring amplifications targeting oncogenes. Jonckheere's trend test (function *JonckheereTerpstraTest* from the *DescTools* package in R) was used to test an association between the number of amplified segments that span and that do not span TCGA-BRCA oncogenes.

*Exploring selection of focal amplifications.* The inventors tested the role of selection in sculpting the landscape of focal amplifications in a tumour. Briefly, they compared the number of focal amplifications spanning oncogenes with a background distribution derived by using a Monte Carlo approach. First, they empirically selected the 10 oncogenes most frequently amplified in the TCGA-TNBC cohort and counted the number of focal amplifications mapping to those 10 cancer-specific oncogenes. A background distribution was then derived by counting the number of focal amplifications mapping 10 non-cancer genes which were randomly selected from a list of 53,989 total non-cancer genes annotated in ensembl GRCh37 genome assembly. In total, the inventors ran this procedure 1,000 times. Statistical significance was measured by calculating the empirical p-value. Empirical p-values were computed as the fraction of iterations where the number of amplifications within the 10 non-cancer genes (background) was greater than the number of amplifications observed within the 10 TNBC-specific oncogenes.

*Exploring acquisition and selection of focal amplifications - simulations.* The inventors performed simulations to model the acquisition of an oncogenic amplification with varied amplification rates and selection coefficients. They modelled cancer evolution as a stochastic system of birth (occurring at rate $\beta$) and death (occurring at rate $\alpha$) events. In this process, cells without oncogene amplification can acquire it in the process of cell division at rate $\mu$ **(Figure 8).** This means that the acquisition of oncogene amplification occurs at overall rate $\beta\mu$. In our simulations, we conferred to cells with oncogene amplification a fitness advantage (s), which increases the division rate by (1 +*s*) and decreases death rate by (1-s). Overall, this means that tumour cells without amplification die with rate $\alpha$, divide yielding two cells without amplification with rate $\beta$(1-$\mu$) and divide giving rise to one cell with amplification and another without with rate $\beta\mu$. Meanwhile, tumour cells with amplifications die with rate $\alpha$(*1-s*) and divide with rate $\beta$(1+*s*), giving rise to two cells with amplification.

The inventors used the Gillespie algorithm (López, S. et al. Interplay between whole-genome doubling and the accumulation of deleterious alterations in cancer evolution. Nat. Genet. 52, 283-293 (2020)) to implement the simulations in practice. For all simulations, we used the following fixed parameters: starting population of 1,000 cells, cell division rate of 0.3, cell death rate of 0.15, and maximum number of amplified drivers equal to 1. In the different simulations, we tested a range of amplification rates (from 5e-6 to 0.1) and selection coefficients (from 0 (neutral selection) to 1). Simulations of tumour growth were run until the population size reached 200,000 cells or until 20,000 generations. In total, the inventors ran 20 different simulations for each of the combinations of amplification rate and selection coefficient. For each simulation, they calculated the fraction of cells that acquired the oncogene amplification and computed the average across simulations with the same parameters.

## Example 1- Mapping signatures to individual copy number events

An approach to evaluate the origin and extent of chromosomal instability at a genome-wide level has been previously described in Drews, R. M. et al. 2022. This approach was used to identify a compendium of 17 copy number signatures. As part of this method, the copy number changes observed in a tumour were embedded into a feature space consisting of 5 fundamental copy number features (the segment size, the difference in copy number between adjacent segments, the lengths of oscillating copy number segment chains, the breakpoint count per 10 Mb, and the breakpoint count per chromosome arm). These 5 fundamental features were capable of capturing well-known copy number patterns and thus were able to distinguish different CIN types at genome-wide level.

However, this feature encoding cannot be directly applied to analyse *individual* copy number changes in a genome. In the original feature encoding the 3 copy number features used to model the distribution of copy number alterations across the genome (the lengths of oscillating copy number segment chains, the breakpoint count per 10 Mb, and the breakpoint count per chromosome arm) were encoded *per genomic region* rather than per individual copy number event. Thus, they cannot be used to map CIN signatures to each individual copy number event in a tumour.

To overcome this limitation, in the present example, the inventors designed a single new feature to replace these 3 features (Figure 4a). This new feature, called "the breakpoint per side" feature, is described below. This feature, alongside the segment size and changepoint features, can be used to recapitulate the original CIN signatures and facilitates mapping of signatures to individual copy number events. In addition to this new feature, the inventors also made slight modifications to the changepoint feature to enable single event mapping.

## New "breakpoints per side" feature

When defining this new feature, the inventors focused on an encoding that not only captured the key properties of the 3 features that it was replacing, but also facilitated recovery of the CIN signatures with high weights for these features. Furthermore, they aimed to maintain a distinction between mutational processes leading to both clustered (i.e. replication stress) and isolated (i.e. mitotic errors) copy number alterations. In order to construct an event-based encoding, one must start with a specific copy number event in the genome. For ease in explanation, we call this event the currentCN.

To encode clustered copy number changes as previously represented by the breakpoint counts per 10 Mb and chromosome arm features, the inventors devised an encoding that took a window around the currentCN and counted the number of breaks occurring either side. The side with the maximum number of breaks was considered the count of the feature. The inventors took the maximum to account for the case where the currentCN is located at the telomere or centromere. For example, for segments located within e.g. 5Mb of the telomere or centromere, the counts would be expected to be underrepresented in the flanking region proximal to the centromere/telomere. This encoding has a high count if other copy number events are clustered with the currentCN and 0 if there are no neighbouring copy number events.

The inventors opted for a 5 Mb window flanking each side of the currentCN. While this doesn't completely represent the breakpoint per chromosome arm feature, they reasoned that it was a good compromise between the two original breakpoint features (the breakpoint count per 10 Mb, and the breakpoint count per chromosome arm). This feature is able to capture more information than just the breakpoint count per 10Mb feature (although it does also capture the information capture by the latter) because if the currentCN is large then the counts effectively span a much larger region than 10Mb. For example, if the currentCN is 40MB, taking +5MB flanking regions means that the total region being interrogated is 50MB (close to the size of a chr arm).

Fortunately, this encoding also managed to capture information encoded by the previous length of oscillating chains feature: neighbouring copy number events of the same size that are part of an oscillating chain will have the same number of breaks in the 5 Mb flanking region, and will therefore cause an overrepresentation of a specific count value in the new feature. Hence, the encoding of the breakpoints per size feature also captures oscillating chains.

As was also done in Drews et al. 2022, diploid segments were not considered and thus the breakpoints per size feature was only extracted for non-diploid copy number events.

To test the robustness of this new feature encoding, the inventors rederived signatures and compared their activities to those obtained by using the original encoding (see below).

*Modifications to the changepoint feature*

This feature was also included in the feature space in Drews et al. 2022, however the feature encoding has been modified in order to avoid linking an event next to a focal amplification to a replication stress signature. In the original feature encoding, the changepoint captured the difference in absolute copy number compared to the left neighbouring segment without taking into account whether the relative change was a positive or a negative value. Therefore, segments with low copy number value next to a focal amplification were defined by having high copy number change, and therefore were assigned to one of the amplification-related signatures.

For non-diploid segments, the relative changepoint value is now calculated with respect to the segments on both sides (even if the neighbouring segments are diploid). The changepoint value is assigned with respect to the left neighbouring segment when this value is equal or higher than - 3. In case this condition is not fulfilled, the changepoint value is assigned with respect to the right neighbouring segment. Then, for those segments with relative changepoint values lower than -3 (i.e. segments flanked by focal amplifications on both sides), the changepoint value is recalculated by subtracting 2 (normal copy number) from the copy number value of this segment. In other words, this subtracts the normal state from the current copy number (as if the segment was flanked by normal segments), to avoid assigning segments that are between two focal amplifications to a replication stress signature. Finally, relative changepoint values are transformed to absolute changepoint values. Making these changes improves the precise mapping of each event to a specific signature. This is not as important when signatures are evaluated at the genome wide level, but improves the accuracy at the single event level.

As in Drews et al. 2022, no changepoint value is extracted for diploid segments. For non-diploid segments located at the beginning of the chromosome, the changepoint value is calculated with respect to the normal copy number value (i.e. 2 is subtracted from the copy number value).

This change had overall little impact on the signature activities quantified for the TCGA samples using the original feature encoding **(Figure 5,** see Example 2 for methods of TCGA data analysis). The major difference was a merge of mitotic signatures CX1 and CX6 (the activities of these signatures are highly correlated and it is therefore possible that these two signatures are in fact the same signature artificially split during the NMF process due to limited data input). For that reason, signature CX6 is excluded from any downstream analyses.

*Absolute copy number feature*

As explained in Drews et al. 2022, the inventors made a deliberate decision to exclude the absolute copy number of a segment as a feature for decoding chromosomal instability. This choice was motivated by the need to ensure robustness in the presence of whole-genome doubling (WGD). Through an extensive examination of the ovarian copy number signatures (Macintyre *et al.* 2018), the inventors discovered that the inclusion of the copy number feature could artificially split a signature based on ploidy status, even when the underlying mutational process was the same. For instance, a single copy loss on a whole genome duplication (WGD) background would result in a copy number state of 3, while the same deletion on a non-WGD background would yield a copy number state of 1. Consequently, a segment loss could be encoded by two distinct signatures, despite originating from the same mutational process that generates deletion patterns. As in Drews et al. 2022, the new feature encoding described herein exclusively incorporates the changepoint feature, which quantifies changes in copy number relative to adjacent segments. By using simulated genomes, the inventors explicitly confirmed that the new feature encoding remains resilient to the influence of WGD effects (see section "Assessing feature encoding performance"). Furthermore, the approach to assign CIN signatures to individual copy number events was also robust to the WGD effects.

*Feature distribution and modelling*

Each feature was split into categories or components by applying mixture modelling to the feature distributions (see Figure 4a). For the segment size and changepoint feature, the same mixture components used in Drews et al. 2022 were used (see **Table 1**). For the new feature (bpwindow), the inventors estimated Poisson mixture models with the flexmix package in R (Grün, B. & Leisch, F. 2008), and used the Bayesian Information Criterion (BIC) to decide on the number of components. The algorithm was initially run using all data with the number of components ranging from 1 to 10. This only yielded 2 components which was not sufficient to represent range of values observed **(Figure 6).** Therefore, given that 96% of the data presented less than 6 breakpoints at 5 Mb window per segment side, the inventors decided to manually define 7 components representing from 0 to 6 counts (see **Table 1**). Then, they applied mixture modelling to the remaining 4% of the data and derived 2 additional components, resulting in a total of 9 components for the breakpoint counts at 5 Mb window per segment size feature (see **Table 1**). The Lambda/Mean values of each of these distributions are provided in Table 1. The bpwindow components with lambda values from 0 to 6 were manually predetermined. For the last two components, the inventors run flemix for getting lambda values with the following parameters: MINCOMP=1, MAXCOMP=10, FITDIST="pois", PRIOR=0.001, NITER=1000, DECISION="BIC", SEED=77777. Note that other parameters may be used to identify mixtures of Poisson distributions for events that present more than 6 breakpoints per 5Mb, as well as different cohorts of samples, which would result in Poisson distributions with slightly different lambda values.

In total, this resulted in a feature space defined by 41 mixture components (22 from the segment size feature, 10 from the changepoint feature, and 9 from the breakpoints per side feature).

*Event-by-component probabilities matrix*

Once components were derived, the inventors computed the probability for each copy number event to belong to each component per feature, resulting in a 1x41 dimensional vector of posterior probabilities. For a given sample, all probability vectors were combined in an event-by-component probabilities matrix, which served as the input matrix for mapping each individual copy number event to a specific CIN type.

*Event-by-signature scores matrix*

For each signature and copy number event, the inventors multiplied the component probabilities vector by the signature definition (signature definition in the new feature space, see Table 3 - obtained by linear combination decomposition given a sample level input matrix in the new feature space and the signature definitions in Drews et al. 2022 in the original feature space). Then, they summed up the resulting vector to calculate a signature score per event. For the purpose of oncogene amplification forecasting (se Examples 2 onwards), the resulting event-by-signature matrix was multiplied by the sample-level signature activities (see section "Sample-level signature quantification") to compute activity-corrected signature scores for each copy number event. This is not performed when computing signature scores for the purpose of assigning signatures to individual events.

Signature scores per event were finally normalised per signature. This is done per event so that the signature scores sum to 1, i.e. the score for each signature for a specific event is divided by the sum of scores for all signatures for the event.

*Deriving novel signatures*

The inventors aimed at defining a compendium of CIN signatures in the new feature space (see Figure 4b). In the present examples, the inventors used the signatures in Drews et al. 2022 in the new feature encoding. However, it is also possible to use the new feature encoding to derive signatures from scratch (e.g. using NMF) as described in Drews et al. 2022. To derive signature definitions, they used the 6,335 TCGA samples with sufficient evidence of chromosomal instability ($\geq$20 CNAs) according to the threshold estimated in Drews et al. 2022. In the context that the activities of the original copy number signatures are already known for all 6,335 samples, the inventors used the LCD function from YAPSA (Hübschmann, D. et al. 2021) for deriving signature definitions given the new feature space input matrix (6,335 patients by 41 components sum-of-posterior matrix).

However, the novel feature encoding introduces changes in the input matrix that need to be taken into account for following this procedure. As stated before (see section "Copy number feature encoding: "Modifications to the changepoint feature"), the inventors have modified the original encoding for the changepoint feature, which limits their ability to distinguish between mitotic signatures CX1 and CX6. This precludes the use of the original signature activities in Drews et al. 2022 as the gold-standard activities for deriving event-level signatures.

After excluding CX6 from the signature definition matrix, the final signature activities for all 6,335 TCGA samples with detectable chromosomal instability were computed using the linear combination decomposition (LCD) function from YAPSA on the input matrix. This input matrix included the sum-of-posterior probabilities for components of the 5 original features but applying the new changepoint encoding.

As described in Drews et al. 2022, the inventors computed and applied signature-specific thresholds to clarify small signature activities. The activity matrix was finally normalised per sample to obtain the new gold-standard activities for all TCGA samples. This gold-standard sample-by-activities matrix was used for deriving the definitions of the novel signatures, as explained above, using linear combination decomposition. Novel signatures (defined in **Table 3)** captured the same signal of different CIN types as the original compendium of copy number signatures in Drews et al. 2022 **(Figure 7).**

*Sample-level signature quantification*

After defining CIN signatures that can be mapped to individual events, the final signature activities for all 6,335 TCGA samples were computed using the linear combination decomposition function from YAPSA on the input matrix (sample-by-component sum-of-posterior matrix). This input matrix was derived by summing up the event probability vectors per sample (i.e. this is a matrix comprising, for each sample, a vector that has activities that are the sum of a row of the event-by-signature matrix (i.e. sum of all event specific activities for a single signature) prior to any adjusting based on sample-level signature and normalising by signature). Indeed, as the linear decomposition is not exact, the sample level activities are re-computed given the signatures defined in the new feature space. For new samples, the component vector (new encoding) is computed, then the new signature definitions (Table 3 or any equivalent signature definitions using the new feature encoding) are used to estimate the activity using linear value decomposition (e.g. as implemented in YAPSA).

Following the same procedure as in our Drews et al. 2022, the inventors identified signature-specific thresholds to be confident in small signature activities. Briefly, the inventors performed 1000 simulations whereby noise was added to the input components. For signatures with a true zero value, the non-zero simulated values were fitted with a single Gaussian distribution using Mclust from the mclust R package, v5.4.6. A threshold was estimated at 95% of this distribution and activities to zero if they were below the signature-specific threshold **(Table 4)**. The sample-by-activity matrix was finally normalised per sample (sample-level activity matrix from here) - dividing by the sum of all signatures for that sample so the signature activity vector sums to one.

**Table 4.** Signature-specific thresholds for setting zero activity values.

| Signature | Threshold |
|-----------|-----------|
| CX1 | 0.003483 |
| CX2 | 0.002938 |
| No, CX3 | 0.008615 |
| CX4 | 0.004013 |
| CX5 | 0.007166 |
| CX7 | 0.001442 |
| CX8 | 0.00198 |
| CX9 | 0.002412 |
| CX10 | 0.00235 |
| CX11 | 0.00128 |
| CX12 | 0.001194 |
| CX13 | 0.0006052 |
| CX14 | 0.0009475 |
| CX15 | 0.005096 |
| CX16 | 0.004579 |
| CX17 | 0.01085 |

The inventors evaluated the performance of the new signature mapping method described above in terms of its ability to capture types of CIN samples in simulated data, correctly assign these types of CIN to individual events, and perform well across a range of technologies and extent of CIN.

*Assessing feature encoding performance*

To evaluate the robustness of the new feature encoding described in Example 1, the inventors used a dataset of 240 simulated genomes which were generated in Drews et al. 2022. Briefly, they simulated the activity of five CIN types by introducing copy number changes into diploid genomes. These CIN types included: chromosome missegregation via mitotic errors (CHR), large-scale state transitions via homologous recombination deficiency (LST), focal amplification via ecDNA circularisation and amplification (ecDNA), early whole-genome duplication via cytokinesis failure (WGD early), and late whole-genome duplication via endoreduplication (WGD late). For any given sample, three of the five mutational processes were active. Half of the samples had one dominant signature and the other half had two. Each mutational process was designed to be dominant in a specific subset of samples (N=100 for CHR, ecDNA and LST; N=96 for WGD early; N=24 for WGD late).

The inventors applied the new feature encoding described above to the simulated genomes to generate a sample-by-component sum-of-posteriors matrix. Then, the NMFpackage in R (with the Brunet algorithm specification) was used to deconvolute the sample-by-component sum-of-posteriors matrix into a sample-level activity matrix and a signature definition matrix. A signature search range of 3 to 6 was applied, running the NMF algorithm 1,000 times with different random seeds. The optimal number of signatures was based on achieving stability in cophenetic, dispersion, and silhouette coefficients. These evaluation metrics were computed for the definition matrix (basis), the activity matrix (coefficients), and the connectivity matrix (consensus). This consensus matrix represented sample clusters based on their predominant signature across the 1,000 runs. The optimal solution was defined as the smallest number of signatures that achieved stability in the cophenetic, dispersion, and silhouette coefficients; but also maximised sparsity without surpassing the sparsity observed in the randomly permuted matrices. This process yielded a total of 5 signatures.

Next, the inventors assessed whether the identified signatures effectively captured the simulated CIN types. They observed a strong alignment between the definitions of the identified signatures and the expected definitions of the simulated CIN types within the feature space (average cosine similarity of 0.66; **Table 5).** The expected definitions were generated by adding weight to those components that reflect the mutational process (adding 1 to those components that define a simulated CIN type, and 0 to those components that do not define it, then normalising the definition vector to sum to 1). The signature activities largely agreed with the simulated distributions across samples, resulting in an overall accuracy of 70.5% (296/420) in identifying samples with a dominant signature. The inventors also compared activities of identified signatures by using the original and the new feature encoding. This analysis revealed that both feature spaces were equally proficient in extracting CIN signals, as indicated by an average Pearson's r of 0.71. Overall, these results demonstrate that the new feature encoding described herein effectively accomplishes both identifying signatures of mutational processes and quantifying their corresponding activities.

**Table 5**. Similarity between expected and observed definitions of identified signatures.

| Signature | Cosine similarity |
|---|---|
| HRD/LST | 0.80 |
| ecDNA | 0.58 |
| CHR | 0.65 |
| Early WGD | 0.67 |
| Late WGD | 0.61 |
| **Average** | **0.66** |

*Assessing signature assignment to individual events*

The new approach described above for mapping CIN signatures to individual copy number events outputs a score that reflects the likelihood of an event being attributed to each signature. This flexibility accommodates situations where copy number events exhibit features resembling more than one signature, and also avoids assigning a specific CIN type to a copy number event which has been produced by an uncertain or unrepresented mutational process. Note that there are multiple options to map signatures to events, including: 1) assign to the event a probability of being caused by a signature (as described in example 1), and 2) assign a specific signature to the event by selecting the signature with the highest probability. The process of computing the signature probability or score of an event is by multiplying "event by component" vector to the "signature by component" vector. These scores can be but do not need to be corrected by the sample-level activity. Another possibility to map signatures to events would be to assign to an event the signature with the most similar definition compared to the "event by component" vector (e.g. using cosine similarity, Euclidean distance, etc).

In an attempt to assess the reliability of the proposed mapping approach, the inventors allocated each copy number event to the signature with the highest associated probability. Subsequently, they assessed the accuracy of these assignments by calculating the fraction of events displaying similar features to the definition of the signature assigned. The signature with the highest cosine similarity in comparing the event-by-component vector and the signature-by-component vector was considered as the most similar. This analysis revealed an overall accuracy of 72.2% (479,565 out of 647,630 events), denoting a substantial alignment between the mapped signature and features defining an individual copy number event. **Table** 6 summarises mapping accuracy for each CIN signature.

**Table 6.** Fraction of events correctly assigned to the CIN signature with the most similar definition.

| CIN signature | Events correctly assigned (%) |
| --- | --- |
| CX1 | 52.3 |
| CX2 | 75.8 |
| CX3 | 76.8 |
| CX4 | 78.1 |
| CX5 | 100 |
| CX8 | 59.3 |
| CX9 | 60.5 |
| CX10 | 89.0 |
| CX13 | 57.9 |

*Assessing number of individual events required for sample-level signature quantification*

The inventors performed an analysis to determine the minimum number of copy number events required within a sample to accurately replicate its signature composition. To achieve this, they progressively increased the number of events of a sample profile via random selection. They then quantified sample-level signature activities by using the linear combination decomposition function from YAPSA on the input matrix (sample-by-component sum-of-posterior matrix). In each iteration of this decomposition, they expanded the information within the input matrix by progressively introducing an increasing number of events, ranging from 1 to 100. They then evaluated whether a sample's signature composition (defined by its three dominant signatures) was correctly captured depending on the number of events. This procedure was repeated 1000 times with 1000 TCGA samples being randomly selected for each run. As little as 15 copy number events were sufficient to correctly decompose >90% cases. This procedure was also expanded to encompass individual signatures. For each specific signature, the inventors performed random selection of copy number events from the subset of 200 TCGA samples exhibiting the highest signature activity. Then, they quantified sample-level signature activities and evaluated the success rate of detecting the signature. This procedure was done 100 times per signature. The results indicated that an average of 7 copy number events was sufficient for calling an individual signature with >90% of accuracy.

*Sample-level signature stability across technologies*

CIN signatures were derived using TCGA SNP 6.0 array data because this represents the largest pan-cancer collection of high-resolution copy number profiled tumours to date. In Drews et al. 2022, the inventors evaluated the robustness of the approach across different high-throughput sequencing technologies by comparing signature definitions and activities. As expected, they saw a drop in similarity scores for the activities and signature definitions between SNP6 and sWGS data (Drews et al. 2022).

Here, the inventors extended this analysis by means of testing the ability to capture the compendium of signatures in sWGS. For this analysis, the inventors used 478 TCGA samples with detectable levels of CIN that were also part of the PCAWG project. WGS data from the 478 TCGA/PCAWG samples was downsampled to 15 number of reads per bin per chromosome copy (according to the ploidy, purity and bin size) for mimicking sWGS. The inventors fitted absolute copy number profiles (see section "Absolute copy number fittings from sWGS"), extracted the 5 original copy number features but using the new changepoint encoding (see section "Copy number feature encoding: Changepoint"), quantified activities for the 16 compendium signatures (CX6 excluded) using YAPSA, and compared them to the SNP6-derived signature activities using Kendall's tau. This looks at the effect of the changes to the changepoint feature.

This analysis showed some deterioration of some signatures across technologies. These deteriorations were not due to methodology but rather due to differences in how the copy number is measured by these technologies. All signatures would be detectable given a sufficient number of training samples for that technology. Fortunately, this deterioration was only observed for tumour type-enriched signatures, with the remaining signatures showing robust signals across technologies. Signatures showing robust signals across technologies were classified as universal signatures (CX1-5, CX8-10, and CX13) whereas others were classified as SNP6-specific signatures (CX7, CX11-12, and CX14-17). CX14 was not considered universal because its signal was captured by CX1 in sWGS data. It is important to note here, that SNP6-specific signatures can also be detected in sWGS, it is just that the copy number changes captured by the signatures manifest differently across the two technologies.

*Sample-level signature stability across bin resolutions*

Given that the gold-standard bin size for quantifying the compendium of CIN signatures in Drews et al. 2022 is 30 kb, and the bin size used for deriving copy number profiles from WES data is 50 kb, the inventors explored the robustness of the novel signatures across bin resolutions by comparing activities.

Here, they segmented copy number profiles from the 478 TCGA/PCAWG samples using 8 different bin sizes (30, 40, 50, 60, 70, 80, 90 and 100 kb). Once absolute copy number profiles were fitted, the inventors quantified the novel signatures developed in this work per sample. They then computed cosine similarities of SNP6-derived signature activities with those obtained at each bin resolution (all of which had very similar distributions with a single peak around cosine similarity ~0.9). In addition, they compared signature activities derived at 30 kb (gold-standard) and 50 kb (resolution used for WES data) using Kendall's tau (resulting in Kendall's tau between the same signature at the two resolutions between 0.710 and 0.910 for all signature apart from CX10 which had a slightly lower tau of 0.610). These comparisons indicated that the novel signatures are stable across bin resolutions.

Example 2 - Mutation generating processes and selection

The ultimate fixation of an amplification is dependent on selective pressures, however selection is challenging to model directly given the diversity of pressures found within any given patient. Across a population of tumours, the frequency of a CNA observed can act as a proxy for selection (Zack, T. I. et al., 2013) and thus can be used to forecast. However, in a single tumour, there needs to be sufficient passenger CNAs present to detect the signature of the mutational process which will ultimately give rise to the future CNA. Contrary to single-base substitutions, where passenger mutations are abundant and have limited functional consequences (Stratton et al. 2009), it is unclear how frequent passenger CNAs are outside the context of whole-genome duplication (López, S. et al. 2020). This potentially creates a challenge for forecasting. As gains are less likely to be negatively selected than losses, DNA amplifications represent an appealing opportunity for forecasting, which is why the inventors focussed on these types of events in these examples.

To establish the feasibility of forecasting oncogene amplifications, the inventors first sought evidence that the accumulation of amplifications could be attributed to a random generative process. In this scenario, tumour samples should exhibit a notably higher number of unique amplifications, which are exclusive to individual cells, compared to the amplifications identified in bulk sequencing. The latter are likely a result of clonal expansion, meaning they are present in multiple cells **(Figure 9a)**. Using single cell DNA sequencing of 8 human triple-negative breast cancers and 4 triple-negative breast cancer cell lines, the inventors observed that the number of unique amplifications detected at single cell resolution was consistently higher than the number of clonally expanded amplifications at bulk resolution **(Figure 9b)**. In addition, the fraction of samples with at least one detected amplification at the single cell level was significantly higher than the fraction seen across 12 matched pseudobulk samples and 145 unmatched triple-negative breast cancer samples as part of The Cancer Genome Atlas (TCGA) project (p-value < 2.2e-16, Binomial test; **Figure 9c**). One sample did not show any unique amplifications, suggesting that mechanisms generating amplifications were less active in this tumour, or there was a sampling bias across the single cells. Additionally, 4 samples did not show a clonally expanded amplification despite showing a moderate number of unique amplifications, suggesting that the amplifications had not yet been positively selected. To test this idea, the inventors designed a positive selection test for amplifications (see Methods). They reasoned that positively selected amplifications should predominantly amplify oncogenes, contrary to passenger amplifications. They applied this test to unique amplifications, finding that they did not enrich breast cancer oncogenes **(Figure 9d)**. To the contrary, clonal amplifications were enriched in breast cancer oncogenes, suggesting that they were the product of positive selection **(Figure 9d).** This indicates that CIN generates a substantial number of random amplifications, predominantly composed of passenger alterations.

To further understand the interplay between the blindfolded ongoing acquisition of amplification and selection, the inventors simulated the evolution of a tumour (starting with 1,000 cells) with varying rates of amplification and resulting fitness advantage **(Figure 8).** They observed that driver amplifications were only detectable in tumours with high amplification rates **(Figure 9e).** This implies that tumours with driver amplifications should have higher numbers of passenger amplifications. Indeed, TCGA triple-negative breast cancers with a greater number of amplified oncogenes showed a higher number of passenger amplifications (p-value = 0.001, Jonckheere's trend test; **Figure 9f).**

Taken together, these results support a model whereby certain types of CIN can generate amplifications at random across the genome and only when one of these amplifications spans an oncogene does it become fixed in the population. Thus, the data indicates that it should be feasible to forecast the likely acquisition of an oncogene using the CIN types found in a tumour.

Example 3 - Determining the CIN processes underlying oncogene amplification

The inventors computed the contribution of each CIN signature to the amplification of each oncogene by comparing the signature scores assigned to the amplified segment containing the oncogene to the signature scores for all other segments (described in detail below). This, in effect, provides a way to determine which CIN types likely caused the amplification of a given oncogene. This was performed using 6335 genome-wide copy number profiles from 33 cancer types derived from SNP6 array data from the TCGA. To create a list of oncogenes, the inventors integrated and curated the annotations provided by Bailey *et al.,* 2018; Jassal *et al.,* 2020; Martínez-Jiménez *et al.,* 2020; and Sondka *et al.,* 2018. This was performed for single tumour types when sufficient data was available, and across multiple tumour types otherwise.

Within a tumour-type: an oncogene was considered a putatively amplified oncogene in a tumour type when 1) it was amplified in $\geq 5$ samples in the tumour-specific cohort, 2) its amplification frequency in the tumour-specific cohort was $\geq$ 5%, and 3) it was previously identified as significantly amplified in the tumour type by GISTIC (Mermel, C. H. et al. 2011). Given an amplified oncogene, the inventors calculated the relative contribution of a CIN signature to the oncogene amplification for a given tumour type ($q_{OG,t,m}$) by averaging the activity-corrected CIN signature score of all amplified events spanning a given oncogene in a specific tumour type as follows:

$$q_{OG,t,m} = \frac{\sum_{a=1}^{A_{t,OG}} e_{a,m}}{A_{t,OG}}$$

Equation (2)

where $e_{a,m}$ represents the activity-corrected score of one of the 9 universal CIN signatures (m) for a particular amplified event (a) (see section "Event-by-signature scores matrix"); and $A_{t,OG}$ represents the number of amplified segments spanning a particular oncogene (OG) in a given tumour type (t). Next, the inventors computed the relative contribution of a CIN signature to the acquisition of any copy number event in any tumour type ($q_m$) by averaging the activity-corrected CIN signature scores of all copy number events found in the entire TCGA cohort as follows:

$$q_m = \frac{\sum_{p=1}^{P} e_{p,m}}{P}$$

Equation (3)

where $e_{p,m}$ represents the activity-corrected score of one of the 9 universal CIN signatures (m) for a particular copy number event ($p$) (see section "Event-by-signature scores matrix"); and $P$ represents the total number of copy number events observed in TCGA. Finally, the inventors computed the tumour-specific oncogene-specific CIN signature weights ($\omega_{OG,t,m}$) by normalising the relative contribution of a CIN signature to the oncogene amplification in a given tumour type ($q_{OG,t,m}$) to the relative contribution of a CIN signature to the acquisition of any copy number event in any tumour type ($q_m$). Note that he normalisation term $q_m$ is obtained across all tumour types in TCGA whereas the denominator $q_{OG,t,m}$ is tumour type specific. The inventors found that this approach better captures the expected contribution of each signature to a copy-number alteration, hence being a better representation of the "background" to use for correction. These weights represent the relative contribution of each CIN signature to an oncogene amplification.

$$\omega_{OG,t,m} = \frac{q_{OG,t,m}}{q_m}$$

Equation (4)

In total, the inventors carried out this process across 19 tumour types and 40 different oncogenes resulting in 86 tumour-specific oncogene-specific weight vectors. These are listed in **Table 7**.

**Table 7.** Tumour type specific signature weights.

| Tumour type | oncogene | CX1_weights | CX2_weights | CX3_weights | CX4_weights | CX5_weights |
|---|---|---|---|---|---|---|
| GBM | MDM4 | 0.01541 | 0.0237 | 0.02679 | 0.033327 | 0.017423 |
| GBM | CDK4 | 0.00849 | 0.013688 | 0.011292 | 0.012243 | 0.011148 |
| GBM | MDM2 | 8.16E-11 | 0.007306 | 0.003738 | 0.003921 | 0.007095 |
| GBM | EGFR | 0.019147 | 0.039622 | 0.018679 | 0.01625 | 0.018456 |
| GBM | PDGFRA | 0.016397 | 0.023186 | 0.022974 | 0.022636 | 0.019114 |
| OV | KRAS | 1.95E-08 | 0.048666 | 0.061983 | 0.054056 | 0.092889 |
| OV | CCNE1 | 1.64E-10 | 0.081347 | 0.055628 | 0.064983 | 0.130389 |
| OV | MYC | 0.005461 | 0.064883 | 0.123278 | 0.150595 | 0.101995 |
| OV | BRD4 | 3.99E-10 | 0.058007 | 0.052205 | 0.087756 | 0.133715 |
| OV | PAK2 | 6.28E-05 | 0.043275 | 0.098864 | 0.131527 | 0.075227 |
| OV | MUC4 | 6.30E-05 | 0.050445 | 0.096731 | 0.122808 | 0.076915 |
| LUAD | SETDB1 | 0.223333 | 0.016117 | 0.062638 | 0.063007 | 0.054074 |
| LUAD | IL7R | 0.099014 | 0.015562 | 0.065894 | 0.09701 | 0.062221 |
| LUAD | EGFR | 4.20E-09 | 0.039484 | 0.08989 | 0.132477 | 0.130275 |
| LUAD | MYC | 0.003958 | 0.022645 | 0.040345 | 0.041183 | 0.059504 |
| LUAD | GNAS | 4.55E-08 | 0.014881 | 0.047651 | 0.056782 | 0.059697 |
| LUAD | LIFR | 0.104416 | 0.011436 | 0.072444 | 0.091635 | 0.052418 |
| LUAD | ZBTB7B | 0.227288 | 0.014192 | 0.057024 | 0.068093 | 0.045682 |
| LUSC | MECOM | 0.002078 | 0.026242 | 0.108396 | 0.106889 | 0.082876 |
| LUSC | EGFR | 7.25E-08 | 0.038907 | 0.06822 | 0.060816 | 0.102272 |
| LUSC | BCL11A | 1.42E-04 | 0.032821 | 0.071949 | 0.079488 | 0.118535 |
| LUSC | PAK2 | 0.002714 | 0.032298 | 0.155686 | 0.147644 | 0.086188 |
| LUSC | CTNND2 | 3.25E-04 | 0.023202 | 0.122321 | 0.083862 | 0.070967 |
| LUSC | REL | 1.40E-04 | 0.033623 | 0.072322 | 0.08766 | 0.124491 |
| LUSC | SOX2 | 0.001658 | 0.03256 | 0.108482 | 0.099742 | 0.084953 |
| UCEC | ERBB2 | 4.19E-11 | 0.057895 | 0.033906 | 0.071401 | 0.082131 |
| UCEC | CACNA1 A | 7.06E-10 | 0.035559 | 0.057698 | 0.115631 | 0.082043 |
| UCEC | CCNE1 | 4.40E-11 | 0.042725 | 0.058108 | 0.09438 | 0.117156 |
| UCEC | MECOM | 2.69E-04 | 0.031627 | 0.067106 | 0.101544 | 0.102421 |
| UCEC | MYC | 0.079339 | 0.042245 | 0.055943 | 0.098786 | 0.098654 |
| BLCA | CCND1 | 9.34E-11 | 0.031613 | 0.027019 | 0.031936 | 0.042017 |
| BLCA | MDM2 | 1.03E-10 | 0.025384 | 0.022527 | 0.025498 | 0.054268 |
| BLCA | ERBB2 | 0.002016 | 0.021869 | 0.024802 | 0.040054 | 0.065913 |
| BLCA | CCNE1 | 2.08E-09 | 0.038622 | 0.021447 | 0.044377 | 0.113864 |
| BLCA | MYC | 0.031897 | 0.019494 | 0.045454 | 0.092081 | 0.081832 |
| TGCT | KDM5A | 1.71E-06 | 0.03756 | 0.238222 | 0.066669 | 0.043608 |
| TGCT | CCND2 | 1.68E-06 | 0.03834 | 0.236256 | 0.067371 | 0.038129 |
| TGCT | CHD4 | 1.66E-06 | 0.041849 | 0.239914 | 0.069815 | 0.041598 |
| TGCT | KRAS | 1.71E-06 | 0.046829 | 0.213181 | 0.087956 | 0.044564 |

(continued)

| Tumour type | oncogene | CX1_weights | CX2_weights | CX3_weights | CX4_weights | CX5_weights |
|---|---|---|---|---|---|---|
| ESCA | KRAS | 2.15E-07 | 0.041082 | 0.046453 | 0.042897 | 0.063544 |
| ESCA | ERBB2 | 8.02E-11 | 0.036803 | 0.012116 | 0.021335 | 0.063786 |
| ESCA | CCNE1 | 1.15E-11 | 0.082228 | 0.025272 | 0.053151 | 0.104979 |
| ESCA | EGFR | 5.34E-11 | 0.02319 | 0.021753 | 0.031545 | 0.06355 |
| ESCA | LPP | 3.66E-04 | 0.024672 | 0.137202 | 0.131591 | 0.099342 |
| ESCA | PAK2 | 3.58E-04 | 0.035599 | 0.152687 | 0.125994 | 0.10275 |
| ESCA | MUC4 | 3.58E-04 | 0.035538 | 0.15589 | 0.125037 | 0.100468 |
| ESCA | SOX2 | 3.84E-04 | 0.029854 | 0.122304 | 0.089754 | 0.091818 |
| LIHC | NCOA2 | 0.228176 | 0.012063 | 0.076948 | 0.088807 | 0.034072 |
| LIHC | MYC | 0.221072 | 0.016184 | 0.095609 | 0.122448 | 0.041695 |
| LIHC | ZBTB7B | 0.099944 | 0.008826 | 0.070795 | 0.096112 | 0.030258 |
| LIHC | CDH17 | 0.236524 | 0.015971 | 0.084138 | 0.097074 | 0.031542 |
| SARC | CDK4 | 1.13E-09 | 0.002829 | 7.01E-04 | 0.001354 | 0.009025 |
| SARC | MDM2 | 9.10E-10 | 0.00197 | 4.87E-04 | 6.58E-04 | 0.006991 |
| SARC | CCNE1 | 6.26E-10 | 0.007578 | 0.017311 | 0.048488 | 0.050285 |
| SARC | JUN | 1.08E-11 | 0.005738 | 0.001009 | 0.004563 | 0.017494 |
| BRCA | MDM4 | 0.267139 | 0.012342 | 0.043395 | 0.087478 | 0.045894 |
| BRCA | CCND1 | 6.32E-10 | 0.017739 | 0.008439 | 0.032733 | 0.052944 |
| BRCA | ERBB2 | 8.41E-09 | 0.02007 | 0.00588 | 0.018354 | 0.048648 |
| BRCA | MYC | 0.044231 | 0.031776 | 0.051338 | 0.078533 | 0.076035 |
| BRCA | ZBTB7B | 0.246382 | 0.014854 | 0.04083 | 0.055783 | 0.046024 |
| BRCA | CDH17 | 0.05114 | 0.027952 | 0.046657 | 0.066736 | 0.068252 |
| STAD | CCND1 | 1.82E-10 | 0.01744 | 0.017127 | 0.024725 | 0.038242 |
| STAD | KRAS | 3.81E-09 | 0.02554 | 0.024411 | 0.023531 | 0.052648 |
| STAD | ERBB2 | 5.65E-11 | 0.022387 | 0.018565 | 0.021042 | 0.054131 |
| STAD | CCNE1 | 3.61E-11 | 0.028795 | 0.02237 | 0.033596 | 0.077062 |
| STAD | EGFR | 0.001981 | 0.020137 | 0.029251 | 0.035545 | 0.055902 |
| STAD | GNAS | 0.092155 | 0.017635 | 0.06414 | 0.073568 | 0.057838 |
| CESC | MECOM | 0.057824 | 0.030316 | 0.156596 | 0.121226 | 0.058101 |
| CESC | BIRC3 | 4.17E-10 | 0.026407 | 0.067104 | 0.04809 | 0.066278 |
| COAD | FAM135B | 0.174405 | 0.018408 | 0.096596 | 0.093218 | 0.037576 |
| COAD | GNAS | 0.134274 | 0.022068 | 0.122466 | 0.12329 | 0.032026 |
| READ | SRC | 0.121779 | 0.033966 | 0.095607 | 0.091167 | 0.029181 |
| READ | CDX2 | 0.237967 | 0.024566 | 0.082843 | 0.080451 | 0.036883 |
| READ | FLT3 | 0.237967 | 0.024566 | 0.082843 | 0.080451 | 0.036883 |
| READ | PLCG1 | 0.121843 | 0.031164 | 0.088613 | 0.087988 | 0.025888 |
| READ | RBM39 | 0.12204 | 0.033629 | 0.093741 | 0.090439 | 0.030889 |
| HNSC | EGFR | 0.003293 | 0.04791 | 0.05961 | 0.046456 | 0.068499 |
| HNSC | FGFR1 | 2.65E-10 | 0.04286 | 0.053286 | 0.065744 | 0.072476 |

(continued)

| Tumour type | oncogene | CX1_weights | CX2_weights | CX3_weights | CX4_weights | CX5_weights |
|---|---|---|---|---|---|---|
| HNSC | BIRC3 | 1.90E-10 | 0.022337 | 0.040059 | 0.033895 | 0.059294 |
| HNSC | SOX2 | 0.068274 | 0.033739 | 0.124962 | 0.141593 | 0.052078 |
| LGG | CDK4 | 0.01248 | 0.015655 | 0.016962 | 0.018731 | 0.018296 |
| LGG | EGFR | 2.43E-10 | 0.02003 | 0.013891 | 0.018716 | 0.015025 |
| UCS | SOX17 | 8.14E-04 | 0.031071 | 0.089733 | 0.062441 | 0.054001 |
| UCS | MYC | 0.002694 | 0.039381 | 0.074362 | 0.087138 | 0.068873 |
| ACC | CDK4 | 0.230581 | 0.014673 | 0.016755 | 0.064383 | 0.037852 |
| ACC | MDM2 | 0.246715 | 0.01503 | 0.018754 | 0.065011 | 0.060364 |

**Table 7 (continued).** Tumour type specific signature weights.

| Tumour type | oncogene | CX8_weights | CX9_weights | CX10_weights | CX13_weights |
|---|---|---|---|---|---|
| GBM | MDM4 | 0.026844 | 0.110872 | 0.049937 | 0.695697 |
| GBM | CDK4 | 0.013744 | 0.102335 | 0.047037 | 0.780023 |
| GBM | MDM2 | 0.005106 | 0.083446 | 0.032422 | 0.856966 |
| GBM | EGFR | 0.021241 | 0.115217 | 0.059558 | 0.691831 |
| GBM | PDGFRA | 0.032583 | 0.102479 | 0.044161 | 0.716471 |
| OV | KRAS | 0.226695 | 0.174205 | 0.028882 | 0.312625 |
| OV | CCNE1 | 0.191111 | 0.245773 | 0.076349 | 0.15442 |
| OV | MYC | 0.338708 | 0.106734 | 0.032246 | 0.0761 |
| OV | BRD4 | 0.235766 | 0.184294 | 0.070896 | 0.177361 |
| OV | PAK2 | 0.558818 | 0.009903 | 0.058108 | 0.024215 |
| OV | MUC4 | 0.56053 | 0.009933 | 0.058286 | 0.024289 |
| LUAD | SETDB1 | 0.424078 | 0.062289 | 0.008068 | 0.086396 |
| LUAD | IL7R | 0.352388 | 0.087199 | 0.018192 | 0.20252 |
| LUAD | EGFR | 0.488259 | 0.039664 | 0.018886 | 0.061065 |
| LUAD | MYC | 0.143231 | 0.128733 | 0.011396 | 0.549003 |
| LUAD | GNAS | 0.28902 | 0.124932 | 0.021306 | 0.385732 |
| LUAD | LIFR | 0.346386 | 0.090343 | 0.011291 | 0.219632 |
| LUAD | ZBTB7B | 0.472792 | 0.033118 | 0.014454 | 0.067356 |
| LUSC | MECOM | 0.291515 | 0.127052 | 0.043025 | 0.211927 |
| LUSC | EGFR | 0.151431 | 0.191404 | 0.025448 | 0.361503 |
| LUSC | BCL11A | 0.277438 | 0.230125 | 0.029512 | 0.15999 |
| LUSC | PAK2 | 0.242693 | 0.170759 | 0.049876 | 0.112142 |
| LUSC | CTNND2 | 0.254582 | 0.265852 | 0.03379 | 0.145099 |
| LUSC | REL | 0.284312 | 0.206108 | 0.03013 | 0.161214 |
| LUSC | SOX2 | 0.242584 | 0.16806 | 0.039027 | 0.222933 |
| UCEC | ERBB2 | 0.099431 | 0.090849 | 0.252677 | 0.31171 |
| UCEC | CACNA1A | 0.459329 | 0.030127 | 0.167944 | 0.051671 |
| UCEC | CCNE1 | 0.248106 | 0.119559 | 0.100515 | 0.219451 |

(continued)

| Tumour type | oncogene | CX8_weights | CX9_weights | CX10_weights | CX13_weights |
|---|---|---|---|---|---|
| UCEC | MECOM | 0.339549 | 0.07629 | 0.177388 | 0.103806 |
| UCEC | MYC | 0.177975 | 0.070456 | 0.108476 | 0.268125 |
| BLCA | CCND1 | 0.069216 | 0.212599 | 0.058138 | 0.527462 |
| BLCA | MDM2 | 0.101808 | 0.218728 | 0.038165 | 0.513621 |
| BLCA | ERBB2 | 0.179327 | 0.137038 | 0.041161 | 0.487821 |
| BLCA | CCNE1 | 0.314359 | 0.184027 | 0.043066 | 0.240239 |
| BLCA | MYC | 0.261766 | 0.063532 | 0.024082 | 0.379861 |
| TGCT | KDM5A | 0.225198 | 0.329803 | 0.015857 | 0.043083 |
| TGCT | CCND2 | 0.225056 | 0.336938 | 0.01558 | 0.042329 |
| TGCT | CHD4 | 0.215137 | 0.336334 | 0.014508 | 0.040843 |
| TGCT | KRAS | 0.179045 | 0.321503 | 0.015308 | 0.091613 |
| ESCA | KRAS | 0.070497 | 0.183236 | 0.060788 | 0.491502 |
| ESCA | ERBB2 | 0.0571 | 0.285076 | 0.110092 | 0.413691 |
| ESCA | CCNE1 | 0.059365 | 0.114458 | 0.270438 | 0.29011 |
| ESCA | EGFR | 0.103314 | 0.234456 | 0.045906 | 0.476286 |
| ESCA | LPP | 0.177061 | 0.064587 | 0.087443 | 0.277737 |
| ESCA | PAK2 | 0.210395 | 0.076126 | 0.06985 | 0.226241 |
| ESCA | MUC4 | 0.21043 | 0.076139 | 0.069862 | 0.226279 |
| ESCA | SOX2 | 0.175382 | 0.121825 | 0.060625 | 0.308054 |
| LIHC | NCOA2 | 0.258131 | 0.18367 | 0.031816 | 0.086317 |
| LIHC | MYC | 0.244023 | 0.162352 | 0.038112 | 0.058505 |
| LIHC | ZBTB7B | 0.554982 | 0.082577 | 0.045766 | 0.010741 |
| LIHC | CDH17 | 0.242095 | 0.176648 | 0.028577 | 0.08743 |
| SARC | CDK4 | 0.001233 | 0.155239 | 0.034687 | 0.794932 |
| SARC | MDM2 | 9.89E-04 | 0.156411 | 0.021593 | 0.810902 |
| SARC | CCNE1 | 0.10814 | 0.124433 | 0.039392 | 0.604373 |
| SARC | JUN | 0.007261 | 0.203957 | 0.03859 | 0.721389 |
| BRCA | MDM4 | 0.240773 | 0.214314 | 0.028261 | 0.060403 |
| BRCA | CCND1 | 0.089683 | 0.311268 | 0.036378 | 0.450816 |
| BRCA | ERBB2 | 0.028783 | 0.344652 | 0.051866 | 0.481746 |
| BRCA | MYC | 0.213964 | 0.237999 | 0.050255 | 0.215869 |
| BRCA | ZBTB7B | 0.253057 | 0.230777 | 0.027336 | 0.084956 |
| BRCA | CDH17 | 0.212694 | 0.23681 | 0.035663 | 0.254096 |
| STAD | CCND1 | 0.084672 | 0.229641 | 0.028605 | 0.559549 |
| STAD | KRAS | 0.057999 | 0.145504 | 0.039493 | 0.630873 |
| STAD | ERBB2 | 0.067079 | 0.156227 | 0.086556 | 0.574013 |
| STAD | CCNE1 | 0.109396 | 0.074038 | 0.134904 | 0.519839 |
| STAD | EGFR | 0.083418 | 0.164726 | 0.074944 | 0.534098 |
| STAD | GNAS | 0.233145 | 0.220939 | 0.04324 | 0.197341 |

(continued)

| Tumour type | oncogene | CX8_weights | CX9_weights | CX10_weights | CX13_weights |
|---|---|---|---|---|---|
| CESC | MECOM | 0.141943 | 0.28946 | 0.061049 | 0.083484 |
| CESC | BIRC3 | 0.053985 | 0.423691 | 0.05138 | 0.263064 |
| COAD | FAM135B | 0.168597 | 0.328307 | 0.014511 | 0.068381 |
| COAD | GNAS | 0.317256 | 0.190151 | 0.030574 | 0.027895 |
| READ | SRC | 0.292795 | 0.212875 | 0.040202 | 0.082429 |
| READ | CDX2 | 0.132077 | 0.234147 | 0.041817 | 0.129249 |
| READ | FLT3 | 0.132077 | 0.234147 | 0.041817 | 0.129249 |
| READ | PLCG1 | 0.307392 | 0.218099 | 0.038962 | 0.080052 |
| READ | RBM39 | 0.298579 | 0.20125 | 0.044811 | 0.084622 |
| HNSC | EGFR | 0.078239 | 0.354012 | 0.048911 | 0.293069 |
| HNSC | FGFR1 | 0.164611 | 0.233903 | 0.03906 | 0.328059 |
| HNSC | BIRC3 | 0.062926 | 0.310089 | 0.032995 | 0.438403 |
| HNSC | SOX2 | 0.275548 | 0.247684 | 0.037079 | 0.019044 |
| LGG | CDK4 | 0.012583 | 0.186181 | 0.077056 | 0.642056 |
| LGG | EGFR | 0.027856 | 0.108859 | 0.044537 | 0.751087 |
| UCS | SOX17 | 0.374551 | 0.072775 | 0.077795 | 0.236818 |
| UCS | MYC | 0.357703 | 0.017003 | 0.070076 | 0.28277 |
| ACC | CDK4 | 0.230302 | 0.278048 | 0.041749 | 0.085657 |
| ACC | MDM2 | 0.253619 | 0.225491 | 0.075345 | 0.039671 |

Across tumour types: if there are insufficient numbers of samples with an oncogene amplification for a given tumour type (i.e. less than 5 samples with the oncogene amplification in that tumour type or a frequency of oncogene amplification lower than 5% in that tumour type), it is possible to determine the CIN signatures contributing to an oncogene amplification across tumour types. Here, the inventors followed a similar procedure as above but the training dataset consisted of samples from different tumour types. Only tumour types in which the oncogene demonstrated recurrent amplification (≥5 samples with the oncogene amplification) were included to calculate oncogene-specific weights. Unlike in the context of a specific tumour, estimating pan-cancer CIN signature weights must capture features impacting on the acquisition (i.e. genomic architecture) and expansion (i.e. selective advantage) of oncogene amplifications across tumour types. Hence, the inventors included a factor to assign greater weight to the tumour types where the amplification is more frequently observed. The relative contribution of a CIN signature to the oncogene amplification pan-cancer ($q_{OG,m}$) was therefore computed as follow:

$$q_{OG,m} = \sum_{t=1}^{T} \frac{f_{OG,t}}{\sum_{t=1}^{T} f_{OG,t}} \times q_{OG,t,m}$$

Equation (5)

where $q_{OG,t,m}$ represents the relative contribution of a CIN signature (m) to the amplification of a given oncogene (OG) in a tumour type (t) (equation (2)); $f_{OG,t}$ represents the empirical frequency of the oncogene amplification (OG) in each tumour type (t); and T represents all tumour types used for building the pan-cancer model. Thus, $q_{OG,m}$ is a weighted sum of the tumour type specific factors $q_{OG,t,m}$, where the weight is proportional to the frequency at which the oncogene amplification is observed in the cohort in the tumour type. Finally, the pan-cancer CIN signature weight per oncogene ($\omega_{OG,m}$) was computed by normalising the pan-cancer relative CIN signature contribution to the oncogene amplification ($q_{OG,m}$) by the relative contribution of a CIN signature to the acquisition of any copy number event in any tumour type ($q_m$, equation (3)):

$$\omega_{OG,m} = \frac{q_{OG,m}}{q_m}$$

Equation (6)

The inventors estimated pan-cancer weights for a total of 187 oncogenes. The oncogenes and signature weights are provided below in **Table 8.**

**Table 8.** Pancancer signature weights.

| oncogene | CX1_weights | CX2_weights | CX3_weights | CX4_weights | CX5_weights |
|----------|-------------|-------------|-------------|-------------|-------------|
| MDM2 | 0.014261 | 0.00931 | 0.008587 | 0.011738 | 0.0208 |
| ABCB1 | 0.155114 | 0.018879 | 0.046174 | 0.081035 | 0.037427 |
| ABL2 | 1.80E-01 | 0.009726 | 0.048083 | 0.077806 | 0.031318 |
| AKT2 | 9.48E-10 | 0.043658 | 0.051836 | 0.102958 | 0.114084 |
| ALB | 2.91E-10 | 0.033671 | 0.047947 | 0.061943 | 0.113165 |
| AR | 1.16E-01 | 0.008886 | 0.066482 | 0.135947 | 0.025232 |
| ARAF | 8.18E-02 | 0.021788 | 0.065527 | 0.12614 | 0.07284 |
| BCL11A | 2.54E-05 | 0.005869 | 0.012866 | 0.014214 | 0.021196 |
| BCL2 | 2.40E-08 | 7.49E-04 | 4.59E-04 | 9.81E-04 | 0.00325 |
| BCL6 | 3.70E-02 | 0.02918 | 0.143313 | 0.13708 | 0.07492 |
| BCL7A | 2.22E-10 | 0.001987 | 0.003175 | 0.001113 | 0.012036 |
| BCL9 | 0.097254 | 0.018458 | 0.044246 | 0.058207 | 0.054067 |
| BCLAF1 | 1.65E-09 | 0.006949 | 0.0032 | 0.014649 | 0.027859 |
| BIRC3 | 1.48E-02 | 0.024151 | 0.052843 | 0.046371 | 0.064509 |
| BRAF | 0.169535 | 0.005905 | 0.03666 | 0.071053 | 0.020706 |
| BRD4 | 6.43E-10 | 0.057792 | 0.049029 | 0.117227 | 0.128301 |
| BTK | 0.06689 | 0.026232 | 0.110846 | 0.184812 | 0.047753 |
| CACNA1A | 4.41E-10 | 0.035442 | 0.044316 | 0.079058 | 0.09856 |
| CALR | 6.94E-10 | 0.035201 | 0.058802 | 0.107739 | 0.101335 |
| CARD11 | 1.34E-01 | 0.012326 | 0.039948 | 0.044199 | 0.03194 |
| CCND1 | 3.66E-03 | 0.03194 | 0.036208 | 0.04594 | 0.063366 |
| CCND2 | 6.17E-05 | 0.024183 | 0.11424 | 0.05904 | 0.045293 |
| CCND3 | 8.10E-05 | 0.023842 | 0.03553 | 0.060616 | 0.071501 |
| CCNE1 | 8.66E-09 | 0.042975 | 0.040487 | 0.065559 | 0.096238 |
| CD79A | 2.09E-10 | 0.021088 | 0.021631 | 0.050868 | 0.075056 |
| CD79B | 1.62E-03 | 0.019006 | 0.018868 | 0.050328 | 0.072609 |
| CDH10 | 2.60E-02 | 0.023747 | 0.086808 | 0.079589 | 0.06148 |
| CDH17 | 1.03E-01 | 0.025338 | 0.074791 | 0.111404 | 0.054374 |
| CDK4 | 1.87E-02 | 0.010047 | 0.008765 | 0.013888 | 0.016018 |
| CDX2 | 0.231625 | 0.022338 | 0.078495 | 0.067596 | 0.031492 |
| CHD4 | 1.15E-06 | 0.028317 | 0.151429 | 0.075118 | 0.054471 |
| CIITA | 1.12E-01 | 0.031004 | 0.045828 | 0.055877 | 0.077385 |
| CLTC | 0.001283 | 0.024719 | 0.018063 | 0.038679 | 0.077462 |
| CNOT3 | 4.24E-11 | 0.001553 | 5.78E-04 | 0.004153 | 0.00597 |

(continued)

| oncogene | CX1_weights | CX2_weights | CX3_weights | CX4_weights | CX5_weights |
|---|---|---|---|---|---|
| COL1A1 | 2.36E-08 | 0.009669 | 0.003563 | 0.013143 | 0.025334 |
| CRNKL1 | 2.24E-01 | 0.015729 | 0.062982 | 0.064485 | 0.038345 |
| CSF3R | 5.35E-12 | 0.005818 | 0.005075 | 0.00698 | 0.012575 |
| CTNND2 | 5.26E-02 | 0.019015 | 0.070799 | 0.061003 | 0.051979 |
| CUL1 | 1.87E-01 | 0.008045 | 0.036207 | 0.078091 | 0.024266 |
| CYSLTR2 | 3.05E-01 | 0.012765 | 0.074998 | 0.061397 | 0.024953 |
| DCAF12L2 | 7.53E-02 | 0.026313 | 0.104992 | 0.162674 | 0.045593 |
| DCSTAMP | 9.65E-02 | 0.029027 | 0.07874 | 0.116833 | 0.055095 |
| DDR2 | 1.22E-01 | 0.018784 | 0.045752 | 0.073224 | 0.052792 |
| DHX9 | 1.89E-01 | 0.009832 | 0.047714 | 0.085996 | 0.027279 |
| EGFR | 1.48E-02 | 0.032202 | 0.022193 | 0.024778 | 0.026388 |
| EIF3E | 1.12E-01 | 0.030367 | 0.08432 | 0.126311 | 0.05279 |
| ELF4 | 6.99E-02 | 0.025936 | 0.106828 | 0.164886 | 0.045986 |
| EPAS1 | 2.53E-05 | 0.001292 | 0.005076 | 0.007251 | 0.005867 |
| ERBB2 | 0.003859 | 0.030641 | 0.020494 | 0.032691 | 0.05542 |
| ERBB3 | 0.323942 | 0.012431 | 0.029122 | 0.077374 | 0.040896 |
| ESR1 | 5.19E-10 | 0.002542 | 0.005777 | 0.018773 | 0.021664 |
| ETV5 | 3.12E-02 | 0.027752 | 1.28E-01 | 0.120651 | 0.069169 |
| ETV6 | 1.02E-06 | 0.034136 | 1.71E-01 | 7.75E-02 | 0.053618 |
| EWSR1 | 1.13E-11 | 0.001868 | 0.003044 | 0.007866 | 0.008382 |
| FAM135B | 1.04E-01 | 0.033613 | 0.089228 | 0.123779 | 0.054666 |
| FGD5 | 5.05E-10 | 0.024329 | 0.014707 | 0.051228 | 0.083787 |
| FGFR1 | 2.17E-02 | 0.030268 | 0.042361 | 0.053104 | 0.077266 |
| FGFR2 | 1.91E-12 | 0.001165 | 7.42E-04 | 0.00174 | 0.004243 |
| FGFR3 | 7.91E-10 | 0.002325 | 0.002028 | 0.002358 | 0.004462 |
| FLT3 | 0.23292 | 0.021717 | 0.079431 | 0.067942 | 0.03276 |
| FOXL2 | 0.085507 | 0.018013 | 0.127775 | 0.132809 | 0.059625 |
| FOXO1 | 2.72E-01 | 0.015579 | 0.082278 | 0.066052 | 0.03021 |
| FOXP1 | 2.73E-09 | 0.007846 | 0.008308 | 0.007285 | 0.017262 |
| GATA1 | 7.93E-02 | 0.019834 | 0.057907 | 0.114331 | 0.06442 |
| GATA2 | 1.46E-01 | 0.009048 | 0.120669 | 0.130046 | 0.039842 |
| GLI1 | 0.051756 | 0.014374 | 0.014677 | 0.024261 | 0.024068 |
| GNAS | 0.097091 | 0.025407 | 0.083853 | 0.089859 | 0.044186 |
| GRIN2A | 0.111122 | 0.0323 | 0.056305 | 0.059627 | 0.074973 |
| GRM3 | 1.69E-01 | 0.014155 | 0.046429 | 0.079637 | 0.032859 |
| GTF2I | 0.182288 | 0.004294 | 0.029971 | 0.063693 | 0.017399 |
| H3F3A | 0.251039 | 0.009975 | 0.060682 | 0.096807 | 0.019855 |
| H3F3B | 0.366248 | 0.026106 | 0.063425 | 0.154429 | 0.029686 |
| HGF | 0.197115 | 0.008967 | 0.039309 | 0.076426 | 0.023968 |

(continued)

| oncogene | CX1_weights | CX2_weights | CX3_weights | CX4_weights | CX5_weights |
|---|---|---|---|---|---|
| HIP1 | 0.182288 | 0.004294 | 0.029971 | 0.063693 | 0.017399 |
| HIST1H3B | 3.69E-04 | 0.029837 | 0.063128 | 0.08169 | 0.074656 |
| HIST1H4I | 4.34E-04 | 0.033484 | 0.064774 | 0.077725 | 0.074072 |
| HNRNPA2B1 | 0.155136 | 0.009729 | 0.036312 | 0.060371 | 0.027723 |
| HOXC13 | 1.30E-01 | 0.003301 | 0.012219 | 0.031715 | 0.019923 |
| HSP90AA1 | 2.76E-10 | 7.25E-04 | 3.00E-04 | 0.001353 | 0.003725 |
| HSP90AB1 | 9.86E-05 | 0.029487 | 0.043711 | 0.074232 | 0.093036 |
| IDH2 | 0.002234 | 0.015079 | 0.035152 | 0.075963 | 0.073948 |
| IL6ST | 1.46E-01 | 0.0066 | 0.00952 | 0.03419 | 0.017506 |
| IL7R | 5.81E-02 | 0.019277 | 0.062891 | 0.074332 | 0.057422 |
| JAK3 | 8.92E-10 | 0.031 | 0.04308 | 0.143809 | 0.105781 |
| JUN | 1.25E-11 | 0.006678 | 0.001174 | 0.005311 | 0.020361 |
| KAT6B | 1.14E-10 | 0.030626 | 0.023059 | 0.057725 | 0.114539 |
| KAT7 | 2.38E-08 | 0.008723 | 0.00343 | 0.013663 | 0.022137 |
| KCNJ5 | 2.30E-09 | 7.95E-04 | 0.002298 | 0.002915 | 0.003916 |
| KDM5A | 8.72E-07 | 0.027292 | 0.157691 | 0.066894 | 0.052774 |
| KDR | 0.06768 | 0.032048 | 0.054514 | 0.054484 | 0.069344 |
| KEAP1 | 7.40E-10 | 0.046277 | 0.04392 | 0.069335 | 0.115326 |
| KEL | 0.190747 | 0.005056 | 0.036849 | 0.071665 | 0.018078 |
| KIF5B | 7.50E-11 | 0.03238 | 0.048709 | 0.052963 | 0.084472 |
| KIFC1 | 5.15E-04 | 0.021153 | 0.069785 | 0.065549 | 0.054886 |
| KIT | 0.046331 | 0.026351 | 0.039019 | 0.037493 | 0.049489 |
| KRAS | 5.16E-07 | 0.033798 | 0.094846 | 0.062454 | 0.059928 |
| LIFR | 0.060856 | 0.020676 | 0.065398 | 0.075043 | 0.056223 |
| LPP | 0.036543 | 0.027662 | 0.141993 | 0.136737 | 0.073916 |
| LTB | 3.57E-04 | 0.026031 | 0.064416 | 0.068602 | 0.057815 |
| MACC1 | 0.13404 | 0.014466 | 0.039244 | 0.064917 | 0.045883 |
| MACF1 | 1.73E-10 | 0.061037 | 0.056518 | 0.080429 | 0.1518 |
| MDM2 | 0.014261 | 0.00931 | 0.008587 | 0.011738 | 0.0208 |
| MDM4 | 0.086667 | 0.0169 | 0.037373 | 0.053961 | 0.024933 |
| MECOM | 0.024863 | 0.031454 | 0.107813 | 0.115754 | 0.079454 |
| MED12 | 0.094539 | 0.010112 | 0.055991 | 0.118081 | 0.032755 |
| MET | 0.128093 | 0.014176 | 0.034806 | 0.052717 | 0.028346 |
| MITF | 2.73E-09 | 0.011382 | 0.011935 | 0.01425 | 0.031009 |
| MLLT3 | 0.005265 | 0.001001 | 0.001976 | 0.001492 | 0.003022 |
| MPL | 3.94E-12 | 0.004081 | 0.003013 | 0.007131 | 0.010301 |
| MTCP1 | 0.055793 | 0.032639 | 0.118041 | 0.169106 | 0.068976 |
| MUC16 | 1.21E-09 | 0.032911 | 0.049586 | 0.082732 | 0.100586 |
| MUC4 | 0.03824 | 0.034515 | 0.153734 | 0.138668 | 0.078892 |

(continued)

| oncogene | CX1_weights | CX2_weights | CX3_weights | CX4_weights | CX5_weights |
|---|---|---|---|---|---|
| MYC | 0.056909 | 0.033473 | 0.067508 | 0.093225 | 0.065894 |
| MYCL | 1.40E-10 | 0.056838 | 0.056258 | 0.064462 | 0.127556 |
| MYCN | 3.43E-10 | 0.008268 | 0.008126 | 0.004697 | 0.008911 |
| MYH11 | 0.253765 | 0.022321 | 0.043633 | 0.079812 | 0.041461 |
| NCOA2 | 0.094764 | 0.018864 | 0.068224 | 0.096204 | 0.046132 |
| NFE2L2 | 6.82E-09 | 0.043676 | 0.078227 | 0.100504 | 0.10408 |
| NRG1 | 0.001521 | 0.001779 | 0.00316 | 0.003647 | 0.004712 |
| NRK | 0.081106 | 0.026694 | 0.109142 | 0.174878 | 0.048159 |
| NTRK1 | 0.152938 | 0.012523 | 0.055038 | 0.075646 | 0.039743 |
| NUMA1 | 1.26E-09 | 0.018628 | 0.037835 | 0.063711 | 0.068651 |
| P2RY8 | 1.24E-10 | 0.002579 | 0.008248 | 0.0171 | 0.007928 |
| PAK2 | 0.036779 | 0.033013 | 0.149441 | 0.137532 | 0.078372 |
| PDCD1LG2 | 0.006284 | 0.022997 | 0.073794 | 0.051044 | 0.055176 |
| PDE4DIP | 0.154519 | 0.011368 | 0.048923 | 0.047738 | 0.038749 |
| PDGFRA | 0.049579 | 0.022079 | 0.028559 | 0.024449 | 0.031959 |
| PEG3 | 2.86E-10 | 0.049875 | 0.027307 | 0.093738 | 0.124999 |
| PIK3CA | 0.025155 | 0.030407 | 0.115576 | 0.115578 | 0.073068 |
| PIK3CB | 0.094553 | 0.014505 | 0.133337 | 0.138686 | 0.053416 |
| PIK3CG | 0.169284 | 0.011112 | 0.028062 | 0.054043 | 0.028739 |
| PIK3R2 | 1.09E-09 | 0.031832 | 0.048889 | 0.149021 | 0.093223 |
| PLAG1 | 0.126451 | 0.020912 | 0.068323 | 0.077019 | 0.045145 |
| PLCB4 | 0.30593 | 0.012562 | 0.069737 | 0.046153 | 0.035676 |
| PLCG1 | 0.101191 | 0.024098 | 0.084777 | 0.092254 | 0.042223 |
| PMS1 | 3.83E-11 | 0.047481 | 0.061124 | 0.090094 | 0.116808 |
| POLD1 | 3.76E-11 | 6.62E-04 | 2.08E-04 | 0.00117 | 0.003042 |
| PPP2R1A | 3.99E-11 | 0.001337 | 5.94E-04 | 0.002459 | 0.005073 |
| PPT2 | 5.66E-04 | 0.037746 | 0.074289 | 0.069345 | 0.063438 |
| PREX2 | 0.102895 | 0.018204 | 0.066006 | 0.097273 | 0.044035 |
| PRKACA | 7.19E-10 | 0.049983 | 0.061129 | 0.110598 | 0.122663 |
| PRKCB | 0.039377 | 8.43E-04 | 0.004964 | 0.00716 | 0.002256 |
| PRRX1 | 0.102655 | 0.011029 | 0.031466 | 0.050367 | 0.030779 |
| PSIP1 | 0.018587 | 0.029895 | 0.068918 | 0.057031 | 0.067896 |
| PTPRC | 0.175819 | 0.010888 | 0.04685 | 0.086582 | 0.028152 |
| PTPRD | 0.021031 | 0.024332 | 0.059722 | 0.040587 | 0.05254 |
| RAC1 | 0.159889 | 0.012436 | 0.043897 | 0.058412 | 0.033598 |
| RAF1 | 3.02E-11 | 0.01318 | 0.010926 | 0.018596 | 0.042026 |
| RARA | 5.80E-04 | 0.032289 | 0.021963 | 0.046917 | 0.080155 |
| RBFOX1 | 0.039895 | 0.001526 | 0.005998 | 0.006885 | 0.003031 |
| RBFOX2 | 7.10E-12 | 0.001477 | 0.001429 | 0.002726 | 0.003724 |

(continued)

| oncogene | CX1_weights | CX2_weights | CX3_weights | CX4_weights | CX5_weights |
|---|---|---|---|---|---|
| RBM39 | 0.091111 | 0.022522 | 0.080093 | 0.086559 | 0.045383 |
| REL | 2.56E-05 | 0.006141 | 0.01321 | 0.016011 | 0.022738 |
| RGL3 | 1.16E-09 | 0.050276 | 0.065346 | 0.1184 | 0.112002 |
| RNF213 | 0.330271 | 0.027086 | 0.077618 | 0.153206 | 0.032435 |
| RRAGC | 2.07E-10 | 0.064418 | 0.061411 | 0.087128 | 0.146755 |
| RRAS2 | 1.84E-11 | 0.001942 | 0.002911 | 0.005419 | 0.00471 |
| SALL4 | 0.099091 | 0.023707 | 0.073695 | 0.08372 | 0.048883 |
| Sep-09 | 0.365736 | 0.024242 | 0.062906 | 0.153532 | 0.029998 |
| SETBP1 | 2.41E-10 | 0.034486 | 0.056977 | 0.082079 | 0.150705 |
| SETDB1 | 0.096871 | 0.017093 | 0.052218 | 0.072635 | 0.058651 |
| SFMBT2 | 1.60E-09 | 0.059789 | 0.084896 | 0.102666 | 0.169232 |
| SMC1A | 0.08439 | 0.016468 | 0.060669 | 0.123685 | 0.05175 |
| SMO | 0.181124 | 0.005211 | 0.031667 | 0.06751 | 0.02054 |
| SOHLH2 | 0.268218 | 0.014001 | 0.080756 | 0.063734 | 0.030492 |
| SOX17 | 0.116198 | 0.021381 | 0.065997 | 0.070552 | 0.044527 |
| SOX2 | 0.022644 | 0.027966 | 0.105188 | 0.10469 | 0.067281 |
| SPOP | 2.38E-08 | 0.007026 | 0.003464 | 0.01365 | 0.022798 |
| SRC | 0.109757 | 0.022972 | 0.086565 | 0.087332 | 0.037976 |
| SRGAP3 | 1.55E-11 | 0.005626 | 0.005491 | 0.010558 | 0.018034 |
| SRSF2 | 0.389833 | 0.026879 | 0.067201 | 0.162682 | 0.028119 |
| STAT6 | 0.217547 | 0.01518 | 0.016479 | 0.055443 | 0.042616 |
| SUSD2 | 2.47E-10 | 0.028824 | 0.038104 | 0.085385 | 0.117206 |
| SUZ12 | 0.003818 | 0.0193 | 0.032082 | 0.05234 | 0.079488 |
| TMSB4X | 0.087259 | 0.016696 | 0.054697 | 0.106379 | 0.051386 |
| TOP2A | 6.73E-04 | 0.031553 | 0.024156 | 0.046658 | 0.082444 |
| TRIM24 | 0.166148 | 0.007467 | 0.038269 | 0.071282 | 0.02433 |
| TRIM49C | 1.11E-08 | 0.035769 | 0.05283 | 0.092702 | 0.08622 |
| U2AF2 | 3.62E-11 | 0.00159 | 6.11E-04 | 0.004612 | 0.006627 |
| UGT2B17 | 4.46E-11 | 0.002001 | 0.002038 | 0.002888 | 0.004538 |
| UNCX | 0.141115 | 0.01287 | 0.040434 | 0.04301 | 0.03068 |
| XPO1 | 2.55E-05 | 0.005638 | 0.012202 | 0.015735 | 0.019702 |
| ZBTB20 | 0.161125 | 0.021799 | 0.135149 | 0.117736 | 0.068271 |
| ZBTB7B | 0.134603 | 0.013344 | 0.052693 | 0.072532 | 0.042771 |
| ZNF148 | 0.156939 | 0.011437 | 0.125915 | 0.130148 | 0.044859 |
| ZNF521 | 1.09E-07 | 0.040752 | 0.0486 | 0.051264 | 0.101915 |
| ZNF780A | 1.41E-09 | 0.045392 | 0.054398 | 0.101492 | 0.116456 |
| ZNF814 | 3.05E-11 | 0.001595 | 7.06E-04 | 0.004508 | 0.005944 |

**Table 8 (continued).** Pancancer signature weights.

| oncogene | CX8_weights | CX9_weights | CX10_weights | CX13_weights |
|---|---|---|---|---|
| MDM2 | 0.037346 | 0.158492 | 0.030769 | 0.708697 |
| ABCB1 | 0.254346 | 0.099614 | 0.033354 | 0.274058 |
| ABL2 | 0.440848 | 0.152406 | 0.031012 | 0.029076 |
| AKT2 | 0.2617 | 0.172045 | 0.090919 | 0.162799 |
| ALB | 0.292743 | 0.312088 | 0.023532 | 0.11491 |
| AR | 0.422191 | 0.173273 | 0.040864 | 0.011279 |
| ARAF | 0.405452 | 0.146532 | 0.040146 | 0.039771 |
| BCL11A | 0.049611 | 0.04115 | 0.005277 | 0.028609 |
| BCL2 | 0.017005 | 0.018713 | 0.003768 | 0.001162 |
| BCL6 | 0.270919 | 0.137447 | 0.059664 | 0.110476 |
| BCL7A | 9.27E-04 | 0.091593 | 0.021045 | 0.26829 |
| BCL9 | 0.34238 | 0.189956 | 0.049281 | 0.146152 |
| BCLAF1 | 0.022152 | 0.096897 | 0.02695 | 0.801345 |
| BIRC3 | 0.119472 | 0.331172 | 0.038372 | 0.308288 |
| BRAF | 0.304057 | 0.188786 | 0.026177 | 0.177121 |
| BRD4 | 0.304103 | 0.120694 | 0.120279 | 0.102574 |
| BTK | 0.369324 | 0.116558 | 0.059348 | 0.018237 |
| CACNA1A | 0.256985 | 0.162047 | 0.096208 | 0.227382 |
| CALR | 0.392316 | 0.103257 | 0.098835 | 0.102514 |
| CARD11 | 0.296058 | 0.181065 | 0.021046 | 0.23897 |
| CCND1 | 0.091905 | 0.280204 | 0.049031 | 0.397742 |
| CCND2 | 0.17379 | 0.233371 | 0.029685 | 0.320337 |
| CCND3 | 0.161985 | 0.19696 | 0.032689 | 0.416795 |
| CCNE1 | 0.185134 | 0.16178 | 0.098703 | 0.309124 |
| CD79A | 0.131671 | 0.335836 | 0.031345 | 0.332504 |
| CD79B | 0.178955 | 0.337641 | 0.045611 | 0.275363 |
| CDH10 | 0.291504 | 0.239646 | 0.035604 | 0.155656 |
| CDH17 | 0.225094 | 0.243854 | 0.043837 | 0.118689 |
| CDK4 | 0.029353 | 0.162568 | 0.047719 | 0.692894 |
| CDX2 | 0.163496 | 0.22561 | 0.041829 | 0.137519 |
| CHD4 | 0.239373 | 0.266064 | 0.025859 | 0.159368 |
| CIITA | 0.221539 | 0.259628 | 0.031645 | 0.164732 |
| CLTC | 0.165352 | 0.352156 | 0.035972 | 0.286313 |
| CNOT3 | 0.011299 | 0.032974 | 0.005666 | 0.014098 |
| COL1A1 | 0.02192 | 0.140965 | 0.018258 | 0.099428 |
| CRNKL1 | 0.317559 | 0.184171 | 0.027176 | 0.06566 |
| CSF3R | 0.011572 | 0.017968 | 0.004164 | 0.01238 |
| CTNND2 | 0.29896 | 0.194711 | 0.037639 | 0.213258 |
| CUL1 | 0.305633 | 0.13784 | 0.023695 | 0.199258 |

(continued)

| oncogene | CX8_weights | CX9_weights | CX10_weights | CX13_weights |
|---|---|---|---|---|
| CYSLTR2 | 0.186551 | 0.136145 | 0.031354 | 0.166936 |
| DCAF12L2 | 0.370157 | 0.144816 | 0.055674 | 0.014465 |
| DCSTAMP | 0.249934 | 0.205191 | 0.040309 | 0.128355 |
| DDR2 | 0.34167 | 0.170774 | 0.034536 | 0.140482 |
| DHX9 | 0.418963 | 0.162626 | 0.033122 | 0.025816 |
| EGFR | 0.050551 | 0.132867 | 0.052273 | 0.643971 |
| EIF3E | 0.221946 | 0.220506 | 0.041327 | 0.110421 |
| ELF4 | 0.390084 | 0.121717 | 0.053964 | 0.020665 |
| EPAS1 | 0.031682 | 0.017462 | 7.99E-04 | 0.010592 |
| ERBB2 | 0.065693 | 0.221405 | 0.092665 | 0.477133 |
| ERBB3 | 0.358943 | 0.111617 | 0.044228 | 0.001447 |
| ESR1 | 0.036466 | 0.138334 | 0.022632 | 0.753812 |
| ETV5 | 0.253981 | 0.159129 | 0.052116 | 0.158302 |
| ETV6 | 1.89E-01 | 0.274576 | 0.019038 | 0.18182 |
| EWSR1 | 0.030843 | 0.004264 | 0.003067 | 0.006948 |
| FAM135B | 0.239044 | 0.183927 | 0.040607 | 0.131589 |
| FGD5 | 0.216083 | 0.257613 | 0.034997 | 0.317257 |
| FGFR1 | 0.156654 | 0.252653 | 0.038371 | 0.327578 |
| FGFR2 | 0.002964 | 0.014009 | 0.00272 | 0.037576 |
| FGFR3 | 0.015197 | 0.008543 | 0.001674 | 0.012587 |
| FLT3 | 0.167063 | 0.231978 | 0.038455 | 0.127734 |
| FOXL2 | 0.339702 | 0.163469 | 0.054686 | 0.018413 |
| FOXO1 | 0.210976 | 0.154627 | 0.032141 | 0.136172 |
| FOXP1 | 0.012776 | 0.031401 | 0.009816 | 0.015763 |
| GATA1 | 0.375524 | 0.165719 | 0.040287 | 0.08265 |
| GATA2 | 0.307583 | 0.200161 | 0.026437 | 0.019862 |
| GLI1 | 0.082961 | 0.218743 | 0.049605 | 0.519555 |
| GNAS | 0.252914 | 0.236043 | 0.036633 | 0.134015 |
| GRIN2A | 0.201748 | 0.293749 | 0.045299 | 0.124877 |
| GRM3 | 0.247276 | 0.090557 | 0.029275 | 0.291042 |
| GTF2I | 0.24643 | 0.131148 | 0.027735 | 0.297044 |
| H3F3A | 0.450702 | 0.067519 | 0.027796 | 0.015626 |
| H3F3B | 0.16524 | 0.125722 | 0.043691 | 0.025453 |
| HGF | 0.259258 | 0.09943 | 0.028045 | 0.267482 |
| HIP1 | 0.24643 | 0.131148 | 0.027735 | 0.297044 |
| HIST1H3B | 0.398668 | 0.244711 | 0.027164 | 0.079777 |
| HIST1H4I | 0.358637 | 0.25745 | 0.034888 | 0.098536 |
| HNRNPA2B1 | 0.281503 | 0.184417 | 0.025182 | 0.219628 |
| HOXC13 | 0.129497 | 0.134966 | 0.033235 | 0.505255 |

(continued)

| oncogene | CX8_weights | CX9_weights | CX10_weights | CX13_weights |
|---|---|---|---|---|
| HSP90AA1 | 0.005296 | 0.013561 | 7.50E-04 | 0.00334 |
| HSP90AB1 | 0.252756 | 0.205271 | 0.051433 | 0.249976 |
| IDH2 | 0.270419 | 0.206795 | 0.057464 | 0.262947 |
| IL6ST | 0.262586 | 0.020658 | 0.031056 | 8.21E-04 |
| IL7R | 0.249616 | 0.195467 | 0.044772 | 0.238156 |
| JAK3 | 0.319957 | 0.16454 | 0.090385 | 0.101448 |
| JUN | 0.008451 | 0.23739 | 0.044915 | 0.83964 |
| KAT6B | 0.234102 | 0.201818 | 0.054534 | 0.283596 |
| KAT7 | 0.024249 | 0.127671 | 0.016533 | 0.108887 |
| KCNJ5 | 0.006495 | 0.007182 | 0.001149 | 1.41E-04 |
| KDM5A | 0.209957 | 0.292498 | 0.022237 | 0.170656 |
| KDR | 0.101009 | 0.228156 | 0.047871 | 0.344893 |
| KEAP1 | 0.195677 | 0.238423 | 0.063421 | 0.22762 |
| KEL | 0.315373 | 0.140656 | 0.023677 | 0.197899 |
| KIF5B | 0.382901 | 0.176899 | 0.047859 | 0.173816 |
| KIFC1 | 0.397621 | 0.233533 | 0.027444 | 0.129514 |
| KIT | 0.066687 | 0.194715 | 0.041335 | 0.49858 |
| KRAS | 0.16417 | 0.22353 | 0.033249 | 0.328026 |
| LIFR | 0.244565 | 0.195592 | 0.037541 | 0.244106 |
| LPP | 0.281541 | 0.137408 | 0.056259 | 0.10794 |
| LTB | 0.272741 | 0.223425 | 0.058101 | 0.228512 |
| MACC1 | 0.314408 | 0.18293 | 0.027915 | 0.176198 |
| MACF1 | 0.213799 | 0.228802 | 0.036722 | 0.170893 |
| MDM2 | 0.037346 | 0.158492 | 0.030769 | 0.708697 |
| MDM4 | 0.14016 | 0.183998 | 0.044692 | 0.411316 |
| MECOM | 0.300353 | 0.149269 | 0.078992 | 0.112049 |
| MED12 | 0.368114 | 0.197395 | 0.037249 | 0.085764 |
| MET | 0.227574 | 0.144456 | 0.033083 | 0.336749 |
| MITF | 0.025579 | 0.101732 | 0.032064 | 0.091412 |
| MLLT3 | 0.019377 | 0.017507 | 0.001419 | 0.016537 |
| MPL | 0.013694 | 0.010489 | 0.003298 | 0.006761 |
| MTCP1 | 0.359929 | 0.118218 | 0.055112 | 0.022187 |
| MUC16 | 0.201167 | 0.233498 | 0.109216 | 0.190302 |
| MUC4 | 0.27015 | 0.128187 | 0.048119 | 0.109495 |
| MYC | 0.212053 | 0.18528 | 0.048474 | 0.237183 |
| MYCL | 0.175923 | 0.255541 | 0.040994 | 0.222428 |
| MYCN | 0.026392 | 0.021788 | 0.02618 | 0.25846 |
| MYH11 | 0.216948 | 0.196145 | 0.02005 | 0.125865 |
| NCOA2 | 0.28136 | 0.19269 | 0.032874 | 0.168888 |

(continued)

| oncogene | CX8_weights | CX9_weights | CX10_weights | CX13_weights |
|---|---|---|---|---|
| NFE2L2 | 0.526019 | 0.041472 | 0.02093 | 0.085092 |
| NRG1 | 0.02073 | 0.024313 | 0.002472 | 0.043585 |
| NRK | 0.361892 | 0.123464 | 0.05807 | 0.016594 |
| NTRK1 | 0.427311 | 0.169658 | 0.030986 | 0.036156 |
| NUMA1 | 0.16102 | 0.216759 | 0.043047 | 0.390348 |
| P2RY8 | 0.037998 | 0.008693 | 5.33E-04 | 0.004713 |
| PAK2 | 0.268123 | 0.130906 | 0.04882 | 0.117015 |
| PDCD1LG2 | 0.185626 | 0.209184 | 0.038127 | 0.357766 |
| PDE4DIP | 0.422083 | 0.171408 | 0.029889 | 0.075322 |
| PDGFRA | 0.046531 | 0.143421 | 0.036173 | 0.61725 |
| PEG3 | 0.189006 | 0.354714 | 0.10795 | 0.052412 |
| PIK3CA | 0.24952 | 0.183146 | 0.049884 | 0.157667 |
| PIK3CB | 0.333489 | 0.174154 | 0.037209 | 0.020651 |
| PIK3CG | 0.27049 | 0.161888 | 0.03598 | 0.240402 |
| PIK3R2 | 0.368099 | 0.158223 | 0.084988 | 0.065726 |
| PLAG1 | 0.274294 | 0.162795 | 0.026114 | 0.198947 |
| PLCB4 | 0.234165 | 0.186675 | 0.018718 | 0.090385 |
| PLCG1 | 0.31537 | 0.217787 | 0.044215 | 0.078085 |
| PMS1 | 0.419009 | 0.02902 | 0.0322 | 0.204264 |
| POLD1 | 0 | 0.021816 | 0.002481 | 0.018313 |
| PPP2R1A | 0.004927 | 0.032902 | 0.004479 | 0.006092 |
| PPT2 | 0.310149 | 0.296763 | 0.044386 | 0.103318 |
| PREX2 | 0.24848 | 0.197859 | 0.033434 | 0.191816 |
| PRKACA | 0.302825 | 0.143338 | 0.079009 | 0.130455 |
| PRKCB | 0.025143 | 0.027551 | 0.001032 | 0.008927 |
| PRRX1 | 0.265011 | 0.183417 | 0.031089 | 0.294186 |
| PSIP1 | 0.379687 | 0.270499 | 0.057878 | 0.049608 |
| PTPRC | 0.405754 | 0.166027 | 0.039512 | 0.040416 |
| PTPRD | 0.209254 | 0.261128 | 0.022908 | 0.308497 |
| RAC1 | 0.269872 | 0.224718 | 0.028671 | 0.168506 |
| RAF1 | 0.089351 | 0.043818 | 0.017673 | 0.152197 |
| RARA | 0.141165 | 0.275742 | 0.064579 | 0.336609 |
| RBFOX1 | 0.028577 | 0.035653 | 0.001103 | 0.013086 |
| RBFOX2 | 0.015107 | 0.006822 | 8.48E-04 | 0.015009 |
| RBM39 | 0.320485 | 0.215211 | 0.040244 | 0.098391 |
| REL | 0.05193 | 0.037646 | 0.005503 | 0.029446 |
| RGL3 | 0.302975 | 0.179009 | 0.08202 | 0.08997 |
| RNF213 | 0.234544 | 0.076665 | 0.058386 | 0.009788 |
| RRAGC | 0.163388 | 0.238572 | 0.030254 | 0.208074 |

(continued)

| oncogene | CX8_weights | CX9_weights | CX10_weights | CX13_weights |
|----------|-------------|-------------|--------------|--------------|
| RRAS2 | 0.01875 | 0.02384 | 0.003051 | 0.052013 |
| SALL4 | 0.287157 | 0.221321 | 0.039949 | 0.122477 |
| Sep-09 | 0.158481 | 0.144241 | 0.04246 | 0.018403 |
| SETBP1 | 0.318388 | 0.084526 | 0.044074 | 0.228765 |
| SETDB1 | 0.369423 | 0.166233 | 0.038819 | 0.128057 |
| SFMBT2 | 0.318017 | 0.149276 | 0.05438 | 0.061744 |
| SMC1A | 0.384348 | 0.205278 | 0.043119 | 0.030293 |
| SMO | 0.296296 | 0.131976 | 0.025953 | 0.239723 |
| SOHLH2 | 0.200583 | 0.170167 | 0.029437 | 0.142611 |
| SOX17 | 0.290516 | 0.148475 | 0.036865 | 0.205488 |
| SOX2 | 0.23812 | 0.183258 | 0.043583 | 0.207271 |
| SPOP | 0.022053 | 0.143169 | 0.01652 | 0.116502 |
| SRC | 0.30562 | 0.21732 | 0.038719 | 0.09374 |
| SRGAP3 | 0.021405 | 0.041697 | 0.006439 | 0.044647 |
| SRSF2 | 0.167703 | 0.105693 | 0.043597 | 0.008293 |
| STAT6 | 0.227146 | 0.362082 | 0.04401 | 0.019496 |
| SUSD2 | 0.37789 | 0.143748 | 0.041955 | 0.166887 |
| SUZ12 | 0.362383 | 0.150939 | 0.016873 | 0.282777 |
| TMSB4X | 0.429094 | 0.166525 | 0.043141 | 0.044823 |
| TOP2A | 0.160254 | 0.263053 | 0.063719 | 0.32749 |
| TRIM24 | 0.32104 | 0.150461 | 0.025595 | 0.195408 |
| TRIM49C | 0.120608 | 0.247872 | 0.046802 | 0.317198 |
| U2AF2 | 0.010189 | 0.027916 | 0.006674 | 0.008765 |
| UGT2B17 | 0.024189 | 0.005426 | 8.03E-04 | 0.008741 |
| UNCX | 0.3053 | 0.198798 | 0.026368 | 0.201424 |
| XPO1 | 0.05449 | 0.023778 | 0.00515 | 0.013996 |
| ZBTB20 | 0.277309 | 0.186945 | 0.023691 | 0.007975 |
| ZBTB7B | 0.413713 | 0.171773 | 0.035155 | 0.063415 |
| ZNF148 | 0.297418 | 0.183617 | 0.025139 | 0.024527 |
| ZNF521 | 0.351435 | 0.195637 | 0.025868 | 0.184529 |
| ZNF780A | 0.265775 | 0.20559 | 0.083779 | 0.127118 |
| ZNF814 | 0.004936 | 0.036049 | 0.008253 | 0.013527 |

For visualisation purposes, hierarchical clustering (using the hclust function from the stats package in R) was applied to the signature weights $\omega_{OG}$ to identify groups of oncogenes with amplifications putatively caused by similar mutational processes. The number of clusters was set to 8 , and only those oncogenes amplified in at least 1% of the TCGA cohort and with a corresponding pan-cancer model of amplification were included in this analysis **(Figure 10b).**

The inventors also explored tumour-specific associations between CIN signatures and oncogene amplifications. First, they computed, as above defined, signature weights for each oncogene and tumour-type. After that, they computed all pairwise cosine similarities (using the *cosine* function from the *lsa* package in R) between the signature weights vectors in different tumour types with at least 1% of all samples and a minimum of 5 samples harbouring the oncogene amplification. The average of all pairwise cosine similarities were taken as an overall metric reflecting how consistent/inconsistent CIN signature scores are across tumour types. The inventors then used the *Heatmap* function from *ComplexHeatmap* to illustrate the signature weights for the amplification of two oncogenes (ERBB2 and *EGFR*) across tumour types **(Figures 10d**, **e**). Hierarchical clustering was used for grouping tumour types without forcing the number of clusters.

By observing the frequency at which each CIN signature was assigned to all oncogene amplifications across 6,335 TCGA samples, the inventors could determine the relative contributions of each CIN signature to oncogene amplification (**Figure 10a**). In general, replication stress signatures (via CX8, CX9 and CX13) showed the strongest association with oncogene amplifications. In particular, amplifications had significantly higher CX13, CX8 and CX9 weights compared to other events across TCGA (CX13: 6.21-fold higher, CX8: 19.39-fold higher, CX9: 26.37-fold higher, p-value < 0.001). In contrast, the inventors observed a significant reduction of the activity of impaired homologous recombination (IHR)-associated signatures CX2 and CX3 by a factor of 0.27 and 0.10, respectively (p-value < 0.001), consistent with their tendency to associate with genomic losses.

The inventors identified context- and tumour-specific associations between CIN signatures and the amplification of certain oncogenes (**Figure 10b**). For example, *CDK4, ERBB2* and *EGFR,* oncogenes commonly amplified as extrachromosomal DNA (Kim et al. 2020), were clustered together in a group with the highest CX13 weight, a signature the inventors have previously linked to extrachromosomal DNA amplification (Drews et al. 2022). While some oncogene amplifications showed similar CIN signature scores pan-cancer, others showed more diverse patterns (**Figure 10c**), likely due to cell-of-origin differences in replicative, transcriptional, repair and chromatin dynamics (Polak et al. 2015): EGFR was predominantly associated with CX13 in 6 different tumour types, but showed a much stronger association with CX8 in lung adenocarcinomas (**Figure 10d**).

On the contrary, ERBB2 amplifications were predominantly associated with CX13 across all 5 different tumour types were its presence was recurrent (**Figure 10e**)

Example 4 - Forecasting focal amplifications

The most significant hurdle in precision medicine lies in the dynamic nature of tumours, evolving from benign to malignant, acquiring metastatic potential, and ultimately developing resistance to specific therapies. The amplification of oncogenes can play a role in all of these tumour progression steps and therefore forecasting the amplification of a relevant oncogene in an individual tumour can allow us to design tailored intervention strategies.

Classical evolutionary theory, together with the findings presented in this work (see Example 2), suggest that in many instances the acquisition of an amplification in a particular oncogene requires that a single cell acquires the oncogene amplification, which, if positively selected or hitchhiking a clonal expansion, will eventually dominate the tumour population. By dissecting the evolutionary forces of mutation and selection, the inventors identified three different major factors usually required for the amplification of an oncogene (**Figure 11).** First, an amplification generating process must be active in a tumour (process specific factor); second, this amplification generating process must be operating on the genomic region containing the oncogene of interest; finally, the amplification of the oncogene of interest must confer a selective advantage (oncogene amplification specific factors). Thus, by jointly modelling these 3 different factors, the inventors hypothesised that it should be feasible to forecast an oncogene amplification.

The inventors developed a model to predict the risk of acquiring a future amplification in a tumour sample (referred to herein as FOCAS - Forecasting OnCogenic AmplificationS). This model is made up of two major components, which capture the process and oncogene amplification specific factors discussed above **(Figure 12):**

1) the CIN signature activities for a given tumour (computed as described in Example 1, section "Sample-level signature quantification"), which represents the different CIN types operating in a tumour;

2) the oncogene amplification CIN signature weights (computed as described in Example 3), which represents the amplification generating process that is operating on the genomic region containing the oncogene of interest. (Note; these weights also encode a degree of positive selection pressure which is discussed further below).

In this model, the inventors only use the 9 universal signatures (CX1-5, CX8-10 and CX13) in order to be able to apply the model across all sample types and technologies.

Specifically, the signature-based predictor combines the two types of factors captured by the activities and oncogene amplification CIN signature weights within a linear model to output a score reflecting the likelihood of acquiring the amplification of the given oncogene in the future:

$$score = \sum_{m=1}^{9} CX_m \cdot \omega_m$$

Equation (7)

where m refers to one of the 9 universal CIN signatures, *CX* represents the sample-level signature activities (i.e. $CX_m$ is the sample-level activity of signature *m* in a sample), and $\omega_m$ represents the signature weight for signature m computed for a given oncogene in a pan-cancer or tumour-type context (see section "Determining the CIN processes underlying oncogene amplification"). When tumour specific weights available for a particular sample type and oncogene, these are used for prediction. A pan-cancer model is used when no tumour-specific model is available.

By applying a predetermined threshold, this score can be transformed into a binary classifier to explicitly forecast whether a tumour sample is at high risk or low risk of acquiring the amplification of the oncogene of interest. Given that each oncogene has a different prior probability of being amplified and selected depending on the disease context, each oncogene and cohort is associated with a respective appropriate threshold matched to the clinical and/or biological context. The procedure for setting the optimal threshold for a given oncogene in a specific context will also depend on the availability of a reference cohort with similar features than the sample to be forecasted. This is discussed further below.

While this prediction model does not explicitly contain a term representing selection pressures, the approach for determining the oncogene signature weights (see Example 3) indirectly captures selection pressure signals: by using only examples of recurrently amplified oncogenes within tumour types for estimating the $\omega$ term in the model, the resulting weights encapsulated only amplification properties which have already been subjected to positive selection.

To further ensure that the forecasting model for an oncogene is only used in a context where a similar selective pressure is present, the inventors further designed a set of guidelines for setting the optimal predetermined threshold and setting the clinical context of interest. Indeed, accurate forecasting of an oncogene is dependent on applying the forecasting model to the correct patient cohort. Ideally, the patient cohort should be homogenous with respect to selection pressures applied to the oncogene. Selecting the optimal threshold for the amplification score is dependent on the prior probability of acquiring an amplification, which is typically cohort specific. Therefore the inventors developed a set of guidelines for applying our oncogene forecasting models to patient samples, summarised in **Figure 13.**

To apply a signature-based model for forecasting oncogene amplification to a new tumour sample when a reference cohort is available, the inventors recommended to adhere to the following guidelines:

1. Define a matched reference cohort: An adequately matched reference cohort is ideal for accurately estimating oncogene signature weights $\omega_{OG}$ and defining the optimal prediction threshold. The new sample preferably has similar clinical and pathological traits to the reference cohort (such as e.g. a cohort of samples with the same tumour type, similar tumour grade/stage, treated with the same treatment, having the same driver mutations, etc.). The sequencing technology employed for the new sample preferably matches that used for the reference cohort, and the copy number profiles preferably have been derived using the same algorithm and resolution.

2. Estimate oncogene signature weights $\omega_{OG}$: The reference cohort is used to estimate the oncogene signature weights.

3. Define an optimal threshold: The threshold on the amplification score for determining whether a sample has a high or low risk of future amplification is dependent on the clinical context. If prior knowledge is available, this is advantageously used to prioritise sensitivity or specificity and an appropriate threshold selected. For example, if the % of samples that ultimately acquire the amplification in this population is known, then the threshold ca be set such that the top x% of samples with highest score (where x is the % of samples that are expected to acquire the amplification in the population) are classified as positives (likely to acquire the amplification). If no prior knowledge is available, then the Youden's index in the reference cohort can be used as a threshold. In particular, the Youden's index can be calculated to identify a threshold that has good sensitivity and specificity in identifying samples from the reference cohort that have an amplification (score above the threshold) vs those that do not (score below the threshold). The Youden's index is J=(TP/(TP+FN))+(TN/(TN+FP))-1, where TP=true positives, FN=false negatives, TN=true negatives, FP=false positives, where positives are samples identified as likely to have the amplification, and negatives are samples identified as not having the amplification. Note that other approaches such as maximising sensitivity at a chosen specificity or vice-versa, may be used depending on the circumstances.

4. Calculate amplification score and threshold for new sample: After acquiring the CIN signature activities of the new sample of interest, calculate the amplification score, and then apply the optimal threshold.

When a comparable reference cohort is not available, the recommendations for forecasting oncogene amplification can depend on the clinical context (i.e. whether the sample is a primary untreated tumour or a treated tumour).

For primary, untreated tumours:

1. Estimate oncogene signature weights $\omega_{OG}$: Use a suitable default cohort (e.g. TCGA/PCAWG) data to estimate the oncogene signature weights. Compute these oncogene amplification CIN signature weights for the specific tumour type only. As these weights are established using TCGA/PCAWG data, which primarily encompasses primary tumours, the prediction models are best suited to forecast oncogene amplification linked to the initiation and progression of a specific tumour type.

2. Define an optimal threshold: The threshold on the amplification score for determining whether a sample has a high or low risk of future amplification is dependent on the clinical context. If prior knowledge is available, this should be used to prioritise sensitivity or specificity and an appropriate threshold selected. For example, if it is known that approximately 30% of patients in the cohort will likely acquire an amplification, the threshold can be set to classify the 30% of samples with the higher scores as "positives" (i.e. samples likely to acquire the amplification). If no prior knowledge is available, then the frequency at which the amplification is found in the default cohort (e.g. TCGA/PCAWG cohort) should be used as the threshold. In other words, if the % of samples likely to acquire the amplification is not known for the particular population to which the sample belongs, the reference cohort is used to set the threshold, in a similar way as explained above.

3. Calculate amplification score and threshold for new sample: After acquiring the CIN signature activities of the new sample of interest, calculate the amplification score, and then apply the optimal threshold.

For treated and/or advanced tumours:

1. Estimate oncogene signature weights $\omega_{OG}$: In this scenario, the oncogene of interest is not expected to be recurrently amplified in the TCGA/PCAWG tumour-specific cohort as it may not be a critical event during the initial stages of tumour development. Ideally, for training oncogene-specific models, the training dataset would consist of samples at the same tumour stage and/or treated with the same therapy. The absence of such a comparable cohort can potentially limit the ability of the model to capture the specific selection pressure present in the samples. To address this limitation, an alternative approach is to compute oncogene amplification CIN signature weights on a pan-cancer level, but only considering those tumour types in which this oncogene demonstrates recurrent amplification. A default cohort (e.g. TCGA/PCAWG data) can be used to estimate the oncogene signature weights, in particular for all tumour types where an amplification is observed.

2. Define an optimal threshold: The threshold on the amplification score for determining whether a sample has a high or low risk of future amplification is dependent on the clinical context. If prior knowledge is available, this can be used to prioritise sensitivity or specificity and an appropriate threshold selected. If no prior knowledge is available, then the frequency at which the amplification is found in the TCGA/PCAWG cohort can be used as the threshold.

3. Calculate amplification score and threshold for new sample: After acquiring the CIN signature activities of the new sample of interest, calculate the amplification score, and then apply the optimal threshold.

This process is the same as that explained above for primary untreated tumours except that the oncogene signature weights are estimated from the default reference cohort (e.g. TCGA/PCAWG) using a plurality of tumour types in which the oncogene is recurrently amplified (rather than a single tumour type matched to the patient). This is because the default reference cohort used here is composed primarily of untreated samples in which the amplification frequency is likely to be lower than in a treated / advanced tumour population.

Example 5 - Predictor Performance Assessment

*Testing the predictive capacity of each oncogene forecasting model*

The inventors assessed the predictive capacity of all 271 forecasting models (86 tumour-specific and 185 pan-cancer) by applying them back to the training cohort after removing the oncogene amplifications and adjacent segments prior to computing the sample-level CIN signature activities from each copy number profile. They used a 10-fold cross-validation strategy with stratified sampling, selecting 80% of samples to determine the optimal threshold for transforming the amplification score in a binary classifier, then applying that threshold to forecast amplifications in the remaining 20%. The optimal threshold was selected using the Youden's index which is defined as the cut point on the receiver operating characteristic (ROC) curve which maximises the sensitivity and specificity. After applying the classifier to the remaining 20%, the Youden's index was also computed for assessing the predictive capacity of the models across each fold. The inventors ranked all models according to the average of Youden's index across runs **(Figure 14).** Out of all prediction models, 60 (29 tumour-specific and 31 pan-cancer) showed a low prediction capacity (Youden's index < 0.25) and therefore they were excluded from our collection of signature-based prediction models. These tended to be cases where there are few examples of tumours with amplifications. Further, it is possible that some amplifications simply may not arise due a specific mutational process. rather they are more stochastic or arise via mechanisms not captured by the CIN signatures. It is therefore possible that predictive models for any of these oncogenes could be obtained using the methods described herein in specific and/or larger cohorts of samples. Nevertheless, the data shows that even with these limitations it was possible to obtain a model with medium or high predictive capacity for the vast majority of oncogenes (211 models of the 271 models tested had medium or high predictive capacity. These are listed in **Table 9.** The parameters of the models are listed in **Tables 7 and 8**, although notes that specific parameters to be applied to a sample may vary since as explained above, a reference cohort specifically matched to the sample is preferably used. Indeed, among the remaining 211 models with predictive signal, 6 of them (4 pan-cancer - POLD1, RBFOX2, JUN and EWSR1 - and 2 tumour-specific - CDK4 and MDM2 amplifications in sarcoma) exhibited an excellent prediction capacity, with Youden's indexes higher than 0.75.

**Table 9.** Models with medium and high predictive capacity (high predictive capacity indicated with *). Pan=pan cancer. SARC=sarcoma, BRCA=breast cancer, LUAD=lung adenocarcinoma, OV=ovarian cancer, BLCA=bladder carcinoma, GBM=glioblastoma, HNSC=head and neck squamous cell carcinoma, READ=renal adenocarcinoma, LUSC=lung squamous cell carcinoma, ACC=adenocarcinoma, STAD=stomach adenocarcinoma, ESCA=esophagal carcinoma, LIHC=liver hepatocellular cariconma, CESC= Cervical Squamous Cell Carcinoma and Endocervical Adenocarcinoma, UCEC= Uterine Corpus Endometrial Carcinoma, COAD= Colon Adenocarcinoma, LGG= Brain Lower Grade Glioma.

| model | cohort | oncogene | youden.mean | youden.sd |
|---|---|---|---|---|
| Pan-KEAP1 | Pan | KEAP1 | 0.252455 | 0.061459 |
| Pan-PLAG1 | Pan | PLAG1 | 0.253998 | 0.053784 |
| Pan-GLI1 | Pan | GLI1 | 0.256147 | 0.183858 |
| BRCA-ZBTB7B | BRCA | ZBTB7B | 0.256691 | 0.163487 |
| STAD-ERBB2 | STAD | ERBB2 | 0.2625 | 0.099645 |
| Pan-SUSD2 | Pan | SUSD2 | 0.263776 | 0.295704 |
| BLCA-CCND1 | BLCA | CCND1 | 0.264255 | 0.210613 |
| Pan-CACNA1A | Pan | CACNA1A | 0.264622 | 0.07861 |
| Pan-CCNE1 | Pan | CCNE1 | 0.271689 | 0.084521 |
| Pan-EIF3E | Pan | EIF3E | 0.278917 | 0.072267 |
| GBM-CDK4 | GBM | CDK4 | 0.279452 | 0.154433 |
| Pan-CRNKL1 | Pan | CRNKL1 | 0.28053 | 0.110047 |
| OV-BRD4 | OV | BRD4 | 0.282633 | 0.168941 |
| Pan-SALL4 | Pan | SALL4 | 0.288263 | 0.046789 |
| Pan-CHD4 | Pan | CHD4 | 0.290102 | 0.057503 |
| Pan-RBM39 | Pan | RBM39 | 0.292035 | 0.075255 |
| BRCA-MDM4 | BRCA | MDM4 | 0.292782 | 0.114918 |
| STAD-KRAS | STAD | KRAS | 0.294286 | 0.210709 |
| HNSC-SOX2 | HNSC | SOX2 | 0.294444 | 0.15107 |
| LUAD-MYC | LUAD | MYC | 0.295349 | 0.239399 |
| Pan-MYC | Pan | MYC | 0.296198 | 0.040474 |
| Pan-FAM135B | Pan | FAM135B | 0.296438 | 0.031869 |
| Pan-DDR2 | Pan | DDR2 | 0.298368 | 0.075089 |
| ESCA-ERBB2 | ESCA | ERBB2 | 0.3 | 0.265565 |

| model | cohort | oncogene | youden.mean | youden.sd |
|---|---|---|---|---|
| Pan-CCND3 | Pan | CCND3 | 0.302928 | 0.161492 |
| LUAD-SETDB1 | LUAD | SETDB1 | 0.304444 | 0.213932 |
| Pan-CDH17 | Pan | CDH17 | 0.304503 | 0.038914 |
| Pan-DCSTAMP | Pan | DCSTAMP | 0.304775 | 0.055991 |
| Pan-TOP2A | Pan | TOP2A | 0.306351 | 0.099721 |
| OV-CCNE1 | OV | CCNE1 | 0.306617 | 0.153472 |
| Pan-MDM4 | Pan | MDM4 | 0.307289 | 0.077097 |
| Pan-SETDB1 | Pan | SETDB1 | 0.30733 | 0.11415 |
| BRCA-CDH17 | BRCA | CDH17 | 0.309105 | 0.060495 |
| Pan-CDK4 | Pan | CDK4 | 0.309396 | 0.093227 |
| ESCA-CCNE1 | ESCA | CCNE1 | 0.312 | 0.419015 |
| Pan-CSF3R | Pan | CSF3R | 0.314151 | 0.235462 |
| Pan-GRIN2A | Pan | GRIN2A | 0.315359 | 0.227589 |
| Pan-NCOA2 | Pan | NCOA2 | 0.316326 | 0.075611 |
| Pan-RRAGC | Pan | RRAGC | 0.318077 | 0.169266 |
| READ-SRC | READ | SRC | 0.319286 | 0.229785 |
| Pan-IL6ST | Pan | IL6ST | 0.32 | 0.465925 |
| Pan-KEL | Pan | KEL | 0.32004 | 0.271138 |
| Pan-BIRC3 | Pan | BIRC3 | 0.321166 | 0.101144 |
| Pan-PEG3 | Pan | PEG3 | 0.321649 | 0.093692 |
| LUSC-MECOM | LUSC | MECOM | 0.322222 | 0.124942 |
| Pan-RBFOX1 | Pan | RBFOX1 | 0.323077 | 0.178434 |
| Pan-SRC | Pan | SRC | 0.325804 | 0.10057 |
| Pan-KDM5A | Pan | KDM5A | 0.326272 | 0.036012 |
| Pan-ZNF148 | Pan | ZNF148 | 0.331846 | 0.137002 |
| Pan-XPO1 | Pan | XPO1 | 0.334649 | 0.144123 |
| Pan-PREX2 | Pan | PREX2 | 0.335337 | 0.05605 |
| Pan-GNAS | Pan | GNAS | 0.335893 | 0.077861 |
| Pan-CUL1 | Pan | CUL1 | 0.336504 | 0.164225 |
| ESCA-MUC4 | ESCA | MUC4 | 0.3375 | 0.220873 |
| Pan-U2AF2 | Pan | U2AF2 | 0.339583 | 0.2479 |
| Pan-GATA1 | Pan | GATA1 | 0.344962 | 0.133822 |
| LIHC-ZBTB7B | LIHC | ZBTB7B | 0.345755 | 0.31167 |
| OV-MUC4 | OV | MUC4 | 0.346602 | 0.199421 |
| Pan-LIFR | Pan | LIFR | 0.348583 | 0.103513 |
| Pan-CCND1 | Pan | CCND1 | 0.350812 | 0.028166 |
| Pan-FGFR1 | Pan | FGFR1 | 0.351447 | 0.074912 |
| LUSC-BCL11A | LUSC | BCL11A | 0.351786 | 0.205689 |
| CESC-BIRC3 | CESC | BIRC3 | 0.3525 | 0.113315 |

(continued)

| model | cohort | oncogene | youden.mean | youden.sd |
|---|---|---|---|---|
| Pan-MYCL | Pan | MYCL | 0.353169 | 0.157548 |
| Pan-BCL9 | Pan | BCL9 | 0.353361 | 0.071117 |
| Pan-AKT2 | Pan | AKT2 | 0.353361 | 0.067348 |
| Pan-HSP90AB1 | Pan | HSP90AB1 | 0.353561 | 0.097631 |
| Pan-CDH10 | Pan | CDH10 | 0.353952 | 0.103419 |
| Pan-EPAS1 | Pan | EPAS1 | 0.354237 | 0.517956 |
| Pan-FOXL2 | Pan | FOXL2 | 0.355673 | 0.07115 |
| Pan-IL7R | Pan | IL7R | 0.355939 | 0.088814 |
| UCEC-MECOM | UCEC | MECOM | 0.356923 | 0.165189 |
| HNSC-BIRC3 | HNSC | BIRC3 | 0.357456 | 0.186577 |
| Pan-HIST1H4I | Pan | HIST1H4I | 0.359434 | 0.389607 |
| Pan-BCL6 | Pan | BCL6 | 0.362164 | 0.080277 |
| Pan-CARD11 | Pan | CARD11 | 0.363846 | 0.286934 |
| Pan-MECOM | Pan | MECOM | 0.363933 | 0.048708 |
| Pan-ARAF | Pan | ARAF | 0.36398 | 0.141785 |
| Pan-PDE4DIP | Pan | PDE4DIP | 0.365636 | 0.132499 |
| Pan-PRRX1 | Pan | PRRX1 | 0.365939 | 0.116178 |
| Pan-PIK3CA | Pan | PIK3CA | 0.366731 | 0.048516 |
| LUSC-SOX2 | LUSC | SOX2 | 0.36932 | 0.091079 |
| Pan-HNRNPA2B1 | Pan | HNRNPA2B1 | 0.370413 | 0.20657 |
| OV-KRAS | OV | KRAS | 0.370505 | 0.108553 |
| Pan-CTNND2 | Pan | CTNND2 | 0.370552 | 0.076253 |
| COAD-GNAS | COAD | GNAS | 0.371986 | 0.08928 |
| OV-PAK2 | OV | PAK2 | 0.373625 | 0.116324 |
| Pan-BRAF | Pan | BRAF | 0.373785 | 0.230863 |
| Pan-MPL | Pan | MPL | 0.375234 | 0.379187 |
| Pan-ALB | Pan | ALB | 0.37844 | 0.133985 |
| Pan-PLCG1 | Pan | PLCG1 | 0.378823 | 0.087945 |
| LUAD-GNAS | LUAD | GNAS | 0.38 | 0.303695 |
| Pan-MACF1 | Pan | MACF1 | 0.380753 | 0.270824 |
| UCEC-CCNE1 | UCEC | CCNE1 | 0.383333 | 0.126837 |
| Pan-CD79A | Pan | CD79A | 0.384932 | 0.318095 |
| Pan-SOX2 | Pan | SOX2 | 0.387421 | 0.085182 |
| Pan-LPP | Pan | LPP | 0.389214 | 0.076251 |
| Pan-RAF1 | Pan | RAF1 | 0.391667 | 0.174801 |
| Pan-GATA2 | Pan | GATA2 | 0.392719 | 0.099367 |
| BLCA-ERBB2 | BLCA | ERBB2 | 0.395238 | 0.149641 |
| Pan-SMO | Pan | SMO | 0.40048 | 0.287799 |
| LUSC-REL | LUSC | REL | 0.401786 | 0.150172 |

(continued)

| model | cohort | oncogene | youden.mean | youden.sd |
|---|---|---|---|---|
| Pan-MUC4 | Pan | MUC4 | 0.403321 | 0.07625 |
| ESCA-PAK2 | ESCA | PAK2 | 0.404167 | 0.250809 |
| Pan-CNOT3 | Pan | CNOT3 | 0.410417 | 0.163959 |
| Pan-PAK2 | Pan | PAK2 | 0.410635 | 0.075648 |
| Pan-BCL11A | Pan | BCL11A | 0.4125 | 0.146639 |
| Pan-ETV5 | Pan | ETV5 | 0.413013 | 0.05135 |
| Pan-PIK3CG | Pan | PIK3CG | 0.413542 | 0.2635 |
| Pan-RARA | Pan | RARA | 0.417266 | 0.074912 |
| Pan-ZBTB7B | Pan | ZBTB7B | 0.419213 | 0.064229 |
| BRCA-CCND1 | BRCA | CCND1 | 0.419428 | 0.142337 |
| Pan-COL1A1 | Pan | COL1A1 | 0.420681 | 0.094082 |
| Pan-CALR | Pan | CALR | 0.42069 | 0.180612 |
| STAD-GNAS | STAD | GNAS | 0.424762 | 0.213098 |
| ESCA-EGFR | ESCA | EGFR | 0.425 | 0.210452 |
| Pan-NUMA1 | Pan | NUMA1 | 0.425889 | 0.107092 |
| Pan-SRGAP3 | Pan | SRGAP3 | 0.426 | 0.241854 |
| OV-MYC | OV | MYC | 0.429476 | 0.13444 |
| Pan-PIK3CB | Pan | PIK3CB | 0.429487 | 0.079465 |
| Pan-DHX9 | Pan | DHX9 | 0.430595 | 0.121721 |
| Pan-SUZ12 | Pan | SUZ12 | 0.433333 | 0.12634 |
| READ-PLCG1 | READ | PLCG1 | 0.435714 | 0.203429 |
| LUAD-IL7R | LUAD | IL7R | 0.436364 | 0.164169 |
| Pan-HIST1H3B | Pan | HIST1H3B | 0.439364 | 0.246025 |
| Pan-FGFR2 | Pan | FG FR2 | 0.442069 | 0.412353 |
| Pan-DCAF12L2 | Pan | DCAF12L2 | 0.44296 | 0.107786 |
| Pan-SPOP | Pan | SPOP | 0.445833 | 0.098319 |
| Pan-ABL2 | Pan | ABL2 | 0.446851 | 0.12319 |
| Pan-HSP90AA1 | Pan | HSP90AA1 | 0.448276 | 0.451547 |
| Pan-GRM3 | Pan | GRM3 | 0.448925 | 0.14617 |
| Pan-KAT7 | Pan | KAT7 | 0.449275 | 0.127957 |
| Pan-MACC1 | Pan | MACC1 | 0.449324 | 0.088196 |
| LUAD-LIFR | LUAD | LIFR | 0.45 | 0.314882 |
| Pan-CLTC | Pan | CLTC | 0.451087 | 0.068674 |
| Pan-KAT6B | Pan | KAT6B | 0.451138 | 0.082477 |
| Pan-TMSB4X | Pan | TMSB4X | 0.453393 | 0.099996 |
| Pan-MLLT3 | Pan | MLLT3 | 0.455172 | 0.322774 |
| Pan-MET | Pan | MET | 0.455395 | 0.125347 |
| Pan-RAC1 | Pan | RAC1 | 0.456774 | 0.205262 |
| Pan-SFMBT2 | Pan | SFMBT2 | 0.46106 | 0.129695 |

(continued)

| model | cohort | oncogene | youden.mean | youden.sd |
|---|---|---|---|---|
| Pan-REL | Pan | REL | 0.4625 | 0.221673 |
| Pan-ESR1 | Pan | ESR1 | 0.462568 | 0.287571 |
| Pan-NTRK1 | Pan | NTRK1 | 0.462848 | 0.052058 |
| Pan-PRKCB | Pan | PRKCB | 0.463403 | 0.236887 |
| Pan-JAK3 | Pan | JAK3 | 0.463424 | 0.115631 |
| Pan-ABCB1 | Pan | ABCB1 | 0.465655 | 0.229671 |
| Pan-MYH11 | Pan | MYH11 | 0.467231 | 0.189578 |
| Pan-ERBB2 | Pan | ERBB2 | 0.467766 | 0.048983 |
| Pan-FGD5 | Pan | FGD5 | 0.468718 | 0.226548 |
| Pan-CD79B | Pan | CD79B | 0.473262 | 0.075595 |
| BLCA-CCNE1 | BLCA | CCNE1 | 0.475 | 0.246855 |
| Pan-IDH2 | Pan | IDH2 | 0.477778 | 0.286693 |
| ACC-CDK4 | ACC | CDK4 | 0.478571 | 0.116885 |
| LUSC-PAK2 | LUSC | PAK2 | 0.479167 | 0.101642 |
| Pan-ELF4 | Pan | ELF4 | 0.47948 | 0.053509 |
| Pan-KCNJ5 | Pan | KCNJ5 | 0.47963 | 0.50502 |
| Pan-SMC1A | Pan | SMC1A | 0.482612 | 0.140116 |
| Pan-MDM2 | Pan | MDM2 | 0.48593 | 0.088099 |
| BRCA-ERBB2 | BRCA | ERBB2 | 0.486138 | 0.105516 |
| Pan-BTK | Pan | BTK | 0.486574 | 0.127107 |
| Pan-TRIM24 | Pan | TRIM24 | 0.487713 | 0.257476 |
| Pan-PTPRC | Pan | PTPRC | 0.489255 | 0.124279 |
| Pan-H3F3A | Pan | H3F3A | 0.490268 | 0.132316 |
| STAD-CCND1 | STAD | CCND1 | 0.490909 | 0.286119 |
| Pan-STAT6 | Pan | STAT6 | 0.492857 | 0.091906 |
| UCEC-CACNA1A | UCEC | CACNA1A | 0.497619 | 0.343285 |
| Pan-PIK3R2 | Pan | PIK3R2 | 0.502517 | 0.183271 |
| Pan-PPT2 | Pan | PPT2 | 0.502778 | 0.482905 |
| Pan-SETBP1 | Pan | SETBP1 | 0.50509 | 0.261744 |
| LGG-EGFR | LGG | EGFR | 0.505882 | 0.167159 |
| Pan-BCL2 | Pan | BCL2 | 0.507586 | 0.512898 |
| Pan-UNCX | Pan | UNCX | 0.509487 | 0.126103 |
| Pan-MED12 | Pan | MED12 | 0.512158 | 0.12072 |
| STAD-CCNE1 | STAD | CCNE1 | 0.52 | 0.113597 |
| Pan-NRK | Pan | NRK | 0.526681 | 0.128306 |
| Pan-AR | Pan | AR | 0.527377 | 0.146641 |
| Pan-MTCP1 | Pan | MTCP1 | 0.530438 | 0.080982 |
| Pan-HIP1 | Pan | HIP1 | 0.538956 | 0.257347 |
| Pan-HOXC13 | Pan | HOXC13 | 0.543064 | 0.251318 |

(continued)

| model | cohort | oncogene | youden.mean | youden.sd |
|---|---|---|---|---|
| ESCA-LPP | ESCA | LPP | 0.55 | 0.155655 |
| Pan-GTF2I | Pan | GTF2I | 0.551807 | 0.243009 |
| Pan-HGF | Pan | HGF | 0.565141 | 0.190121 |
| BLCA-MYC | BLCA | MYC | 0.568707 | 0.251654 |
| Pan-BCLAF1 | Pan | BCLAF1 | 0.570696 | 0.185699 |
| ESCA-SOX2 | ESCA | SOX2 | 0.570833 | 0.272201 |
| LUAD-EGFR | LUAD | EGFR | 0.571111 | 0.442874 |
| Pan-PMS1 | Pan | PMS1 | 0.575099 | 0.347238 |
| Pan-BCL7A | Pan | BCL7A | 0.576316 | 0.362409 |
| Pan-PPP2R1A | Pan | PPP2R1A | 0.576389 | 0.342702 |
| Pan-NFE2L2 | Pan | NFE2L2 | 0.57751 | 0.25752 |
| LGG-CDK4 | LGG | CDK4 | 0.581132 | 0.163881 |
| Pan-KIF5B | Pan | KIF5B | 0.587908 | 0.210222 |
| LUAD-ZBTB7B | LUAD | ZBTB7B | 0.588889 | 0.218047 |
| Pan-ERBB3 | Pan | ERBB3 | 0.590164 | 0.154993 |
| GBM-MDM2 | GBM | MDM2 | 0.607875 | 0.144792 |
| Pan-KIFC1 | Pan | KIFC1 | 0.617593 | 0.38207 |
| Pan-UGT2B17 | Pan | UGT2B17 | 0.624074 | 0.312421 |
| Pan-FGFR3 | Pan | FG FR3 | 0.633333 | 0.430092 |
| Pan-P2RY8 | Pan | P2RY8 | 0.634043 | 0.323578 |
| SARC-JUN | SARC | JUN | 0.648649 | 0.265116 |
| Pan-MYCN | Pan | MYCN | 0.67381 | 0.142327 |
| Pan-LTB | Pan | LTB | 0.741503 | 0.153006 |
| *Pan-EWSR1 | Pan | EWSR1 | 0.752941 | 0.060045 |
| *Pan-JUN | Pan | JUN | 0.754054 | 0.220932 |
| *Pan-RBFOX2 | Pan | RBFOX2 | 0.756481 | 0.285991 |
| *Pan-POLD1 | Pan | POLD1 | 0.796552 | 0.309539 |
| *SARC-MDM2 | SARC | MDM2 | 0.950893 | 0.094209 |
| *SARC-CDK4 | SARC | CDK4 | 0.962054 | 0.057103 |

*Prospective assessment of predictor performance*

To further assess performance in a prospective setting, the inventors used data from two different cohorts: i) TRACERx Lung, including 126 patients with lung adenocarcinoma and/or non-small cell lung cancer with paired primary and metastatic samples, and ii) Hartwig Medical Foundation (HMF), an extensive cohort that includes a subset of 227 patients with different tumour types that have biopsies obtained at different time points. To also illustrate how thresholding would perform in these examples, the inventors set the threshold to binarise scores i) such that it would yield the same number of predicted positives as in the training (TCGA) cohort in TRACERx Lung and ii) based on the frequency of amplifications in patients that do not have paired data in the Hartwig Medical Foundation cohort.

Bakir *et al (2023)* previously described in TRACERx Lung the acquisition of new amplifications in the oncogene *HIST1H3B* in metastases that were not present in the matched primary tissue. The inventors posited that the *HIST1H3B* model constructed in TCGA should enable to predict which primary tumours went on to acquire the amplification of this oncogene later on in the matched metastases. *HIST1H3B* model was only available pan-cancer (see **Table 8),** due to the limited number of samples with the amplification in primary lung tumours. This pan-cancer model, when applied to primary tumours in TRACERx Lung, accurately predicted which patients went on to acquire this amplification (Figure 28, a)). Because multiple primary and metastatic samples were available for some patients, the inventors randomly selected only one sample of each type per patient.

Acquisition of an amplification of the oncogene *AR* is a well-established driver of aggressive disease and androgen deprivation therapy in prostate cancer. The inventors posited that the acquisition of *AR* could be forecasted using the model trained in TCGA. Again, only a pan-cancer model was available for AR oncogene **(Table 8)** due to the low number of samples with acquired *AR* in early primary prostate tumours; a frequency that substantially increases upon treatment and metastasis. The inventors applied this pan-cancer model of *AR* amplification to the first sample of 48 pairs of metastatic samples from prostate cancer patients in the Hartwig Medical Foundation cohort. The results showed that patients acquiring *AR* amplification had significantly higher scores according to their model than patients that did not acquire such amplification (Figure 28, b).

Finally, *ERBB2* amplification drives tumorigenesis in multiple tumour types, including breast and oesophageal cancer. Because of its relevance across multiple tumour types, the inventors tested the pan-cancer *ERBB2* model constructed in TCGA **(Table 8)** in 227 pairs from the Hartwig Medical Foundation. Again, the results demonstrated that patients acquiring *ERBB2* amplification had significantly higher scores according to their model than patients that did not acquire such amplification (Figure 28, c).

To further assess performance, the inventors used the Glioma Longitudinal Analysis Consortium (GLASS) cohort consisting of 154 diffuse glioma patients that were biopsied at two time points.

In particular, the inventors downloaded copy number profiles from a total of 504 diffuse glioma samples included in the Glioma Longitudinal Analysis Consortium (GLASS) dataset. After filtering samples with no detectable CIN (<20 CNAs), the dataset included 154 patients with at least one pair of time-separated tumour samples which were used to test the predictor. Only one sample pair per patient was considered in the analysis. If a patient had multiple sample pairs, we prioritised the pair where the early biopsy was obtained from the primary tumour. If this was not feasible, the inventors randomly selected one of the available sample pairs. Copy number profiles were derived from whole genome sequencing data for 36 patients (72 samples, 36 pairs) and from whole exome sequencing data for 118 patients (236 samples, 118 pairs) as described in Barthel et al. 2019. For each patient, the inventors computed amplification scores in the early time point biopsy and observed the presence of a new focal amplification in the later time point biopsy ($\geq$6 copies with respect to the early sample). Wilcoxon two-sided rank test was used to compare amplification scores between patients with and without a new focal amplification in the later relapse biopsy. Spearman's rho was also computed to assess the correlation of amplification scores and the number of amplifications acquired in the relapse.

Thus, the inventors applied the method to the early biopsy and observed the emergence of any amplification in the later relapse biopsy **(Figure 15c).** Tumour pairs with new amplifications at the second time point had significantly higher scores than pairs without amplifications **(Figure 15d).** The amplification scores also correlated with the total number of amplifications acquired **(Figure 17).**

Taken together these results show that the method described herein can predict oncogene amplification across different tumour types.

Example 6 - Training of the oncogene amplification model using paired lonaitudinal datasets

The inventors also demonstrated the potential to use a different framework to train and develop models and test them when pairs of samples from the same patient are available. This procedure consists of i) defining the first and second sample within a pair from a given patient, ii) identifying the mechanisms of CIN causing the amplification of a specific oncogene in the second biopsy (as described above in TCGA, but in this case using the amplifications observed in the second biopsies) and iii) applying the model to the first biopsy and test whether the predictions match with the observed acquisition of amplification (Figure 29).

The inventors used this approach with data from two different cohorts: i) TRACERx Lung, and ii) HMF (as described above in Example 5).

Using TRACERx Lung pairs, the inventors applied this procedure to metastatic samples to train models of HIST1H3B, SETBD1 and MDM2. All of these models, when applied to primary samples in TRACERx Lung, predicted with high accuracy patients that went on to acquire the amplification of these oncogenes (Figure 30a, TRACERx, 126 pairs).

Using Hartwig Medical Foundation pairs from different tumour types, the inventors applied this procedure to the latest biopsy from each patient to train models of AR and ERBB2 amplification. The application of these models to the earliest biopsy from each patient identified with high accuracy patients that went on to acquire the amplification of these oncogenes (Figure 30b, Hartwig, 227 pairs).

Taken together, these results show that training models of acquisition of amplification using the latest biopsy within each of the pairs in a cohort yields accurate results, hence serving as an alternative accurate framework to obtain these models. Thus, in practice a patient for which a reference cohort with longitudinal data exists, where the patient matches the characteristics of an early time point in the longitudinal, models can be used with parameters estimated using later time points for the reference cohort.

Example 7 - Robustness Assessment

*Robustness of the signature-based predictor across technologies*

To test the robustness of the signature-based predictor across sequencing technologies, the inventors computed amplification scores in the SNP6- and sWGS-derived copy number profiles from the 478 samples included in both the TCGA and the PCAWG project (see Example 1, section "Sample-level signature stability across technologies"). They used a Pearson correlation to compare sample pairs and showed a high correlation of scores obtained from different technologies (r = 0.82, p-value < 2.2e-16, **Figure 22).**

*Robustness of using the universal set of signatures*

The use of a signature subset for decoding mutational processes operating in tumours could erroneously attribute alterations caused by unrepresented signatures to the signature set. This presents a fundamental challenge in the mutational signatures field, potentially affecting our ability to predict amplifications driven by these unrepresented signatures. Previous attempts to mitigate this effect have involved *de novo* signature discovery within the cohort of interest and subsequently mapping them back to a compendium, which may identify unmapped signatures. However, this approach is only feasible when there is a sufficiently large cohort for *de novo* discovery. Unfortunately, this condition is not met for many of the cohorts with oncogene amplifications used here. Therefore, in order to strike a balance and facilitate technology-agnostic forecasting, the inventors decided to exclusively use the universal signatures.

To evaluate the extent of copy number events that may be erroneously assigned to the set of universal signatures, the inventors applied the mapping approach described in Example 1 using the whole set of CIN signatures and allocated each copy number event to the signature with the highest associated probability. Subsequently, they estimated the rate of events assigned to a CIN signature not included in the universal set. This analysis revealed a misassignment rate of 22.5% (145,554 out of 647,630 events), with most of them (17%) assigned to a signature linked to subclonal changes with unknown aetiology (CX7). The average misassignment rate per sample was 23.3±14.4%.

To further warrant the use of universal signatures to build the signature-based predictor, the inventors explored the impact of the presence of these misassigned events within copy number profiles on the signature quantification task. They compared activities of universal signatures quantified in TCGA profiles including and excluding copy number events not mapped to the universal signature set using both Pearson's correlation (average Pearson's r across signatures of 0.84; **Table 10)** and cosine similarities (average cosine similarity across signatures of 0.91; **Table 3).**

Table 10. Concordance of sample-level activities after excluding misassigned events

| CIN signature | Pearson's r | Cosine similarity |
|---|---|---|
| CX1 | 0.86 | 0.92 |
| CX2 | 0.73 | 0.90 |
| CX3 | 0.83 | 0.97 |
| CX4 | 0.94 | 0.96 |
| CX5 | 0.80 | 0.88 |
| CX8 | 0.91 | 0.92 |
| CX9 | 0.72 | 0.79 |
| CX10 | 0.85 | 0.92 |
| CX13 | 0.92 | 0.92 |

In addition, the inventors tested whether quantifying the universal set of CIN signatures was sufficient for predicting amplifications. To do this, they performed a Pearson's correlation to compare amplification scores computed by including all 16 signatures (CX6 was excluded since it's not captured with the new feature encoding) and those obtained using only the 9 universal signatures. Results demonstrated a high correlation between the scores obtained with both signature sets in the TCGA dataset (r = 0.86, p-value < 2.2e-16).

Finally, as the mutational processes active in a specific cohort may be different from those captured by the 9 universal signatures, the inventors also conducted a comparison between the amplification scores computed using the universal signature set to those derived from a cohort-specific set of signatures. To undertake this analysis, they leveraged the TCGA-BRCA cohort, which has the largest sample size within the TCGA dataset (n=730), as the de novo signature extraction process requires a substantial cohort. This extraction was performed using non-negative matrix factorisation (NMF) on the input matrix as detailed in Drews et al. 2022. A total of 6 breast-specific signatures were identified, all of which were mapped with one of the signatures included in our compendium of CIN signatures (CX2, CX3, CX4, CX7, CX9, and CX14). Results demonstrated a high correlation between the scores obtained using both signature sets in the TCGA-BRCA dataset (r = 0.91, p-value < 2.2e-16).

Altogether, these findings show that there is low impact of applying a fixed signature repertoire for sample-level quantification and for forecasting focal amplifications.

*Assessing the threshold for defining focal amplifications*

To select the optimal threshold for defining focal amplifications, the inventors conducted an extensive analysis of the predictor's performance across a range of thresholds following two strategies: 1) they used absolute copy number values (ranging from $\geq 6$ to $\geq 10$ copies), and 2) they used copy number values relative to tumour ploidy (ranging from $\geq 4$ to $\geq 8$ copies respect to tumour's ploidy). For each threshold value, the inventors built a pan-cancer, pan-oncogene model for forecasting oncogene amplifications. They then assessed its performance by applying it back to the training cohort using a retrospective strategy, where signature activities were quantified after removing amplified segments harbouring an oncogene. Performance was evaluated by comparing amplification scores between samples with and without oncogene amplifications using Wilcoxon two-sided test, and by computing the area under the receiver operating characteristic curve (AUC) at pan-cancer and pan-oncogene level **(Figure 23a, b)**. Although the inventors observed marginal differences in performance across these different threshold values, higher thresholds tended to show improved performance. However, higher threshold values resulted in fewer amplifications available to train the model, limiting our ability to predict across as many oncogenes. The inventors obtained similar positive performance using the ploidy-aware strategy, which indirectly may take into account whether a whole-genome doubling event has occurred. They therefore chose a threshold of $\geq 8$ (absolute copy number) as it represented a balance between performance and inclusivity.

Example 8 - Demonstrating clinical utility

To demonstrate the clinical utility of forecasting oncogene amplification the inventors applied the method to two use-cases: predicting probable MET-amplification driven resistance to EGFR inhibitor treatment; and identifying low-grade gliomas likely to become aggressive via CDK4 amplification.

*Predicting amplification-driven drug resistance*

Acquired resistance to EGFR tyrosine kinase inhibitors (EGFR-TKI) (i.e. osimertinib, erlotinib, gefitinib or afatinib) via *MET* amplification occurs in approximately 10-30% of patients with NSCLC (Oxnard, G. R. et al., 2018).The inventors applied the signature-based approach to compute the *MET* amplification scores in a cohort of primary *EGFR*-mutant non-small cell lung cancers (NSCLC) treated with Osimertinib, using the *MET*-specific signature weights for computing the predictions (data not shown). The top 30% of samples based on amplification scores were set as likely to acquire MET amplification driven resistance to EGFR-TKI. This threshold is set based on the expected percentage of MET amplification after EGFR-TKI resistance described in the literature.

As relapse biopsies were not available in this cohort to confirm acquisition of *MET* amplification, the inventors carried out long term culture of 4 lung cancer cell lines in the presence of EGFR-TKI and tracked the emergence of *MET* amplifications in resistant clones.

The inventors computed *MET* amplification scores (using the pancancer MET model with weights provided in **Table 8** - row "pancan - MET") in four parental *EGFR*-mutant NSCLC cell lines, including HCC827, HCC4006, PC9 and H1975, and observed the acquisition of *MET* amplification in the resistant clones emerged after long-term culture in the presence of EGFR-TKI (see section "Experimental data: Human cancer cell lines" for further details). Amplification scores were normalized by the total number of CNAs per cell line. Droplet digital PCR was used to detect *MET* amplification in both the parental and resistant cell lines, using the ddPCR™ Copy Number Assay: MET, Human (dHsaCP1000038). We used the ddPCR™ Copy Number Assay: AGO1, Human (dHsaCP2500349) as a reference. Copy number was estimated as the ratio of the concentration of *MET* with respect to *AGO1* using the Bio-Rad QuantaSoft software. *MET* amplification was absent in the parental culture. An acquisition of *MET* amplification-driven resistance was considered when resistant clones showed 2-fold increase of the parental value.

None of the *EGFR*-mutant NSCLC cell lines (HCC827, HCC4006, PC9 and H1975) had initial *MET* amplifications. Canonical mechanisms of EGFR-TKI resistance were analysed in each resistant subclone, confirming that none of them acquired *EGFR* T790M or C797S mutations. *MET* amplification-driven resistance was observed in 11 /12 resistant clones derived from HCC827, while no *MET* amplifications were observed in resistant clones from the other lines. Our signature-based approach assigned the highest score to the HCC827 parental cell line **(Figure 23a).**

Similar results were observed when analysed data from an independent study (Jia et al. 2013), in which 3 out of the 4 *EGFR*-mutant NSCLC cell lines (HCC827, HCC4006 and PC9) were long-term cultured in the presence of erlotinib until the emergence of resistant clones **(Figure 23b).** The highest amplification score was assigned to the two HCC827 parental cell lines, which were in fact the unique ones acquiring *MET* amplification as a resistant mechanism to erlotinib.

The inventors then also explored if the signature-based predictor can dissect heterogenous development of resistance mechanisms. To this, they generated single clones from the HCC827 parental cell line and carried out long term culture in the presence of EGFR-TKI to generate resistant clones. They then evaluated the number of resistant clones that acquired *MET* amplification and correlated them with the amplification scores computed by the signature-based predictor **(Figure 23c).** The parental clone with the lowest number of *MET*-amplified resistant clones (HCC827_MC2) presented the lowest amplification score. This indicates that the signature-based predictor is able to distinguish subclonal heterogeneity even in a cell line that is well-known and fully-described in the literature to resist EGFR-TKIs via *MET* amplification.

*Predicting prognosis*

Oncogene amplification plays a fundamental role in cancer initiation and progression. Therefore, forecasting oncogene amplification linked to aggressiveness can help identify early-stage patients with likely poor outcomes. To test this, we focused on *CDK4* amplification in low-grade glioma (LGG) patients from the TCGA, where amplification is linked to worse outcomes (Forst et al. 2014; Mirchia et al. 2019).

The inventors applied their signature-based approach, and more specifically, the low-grade glioma specific model of CDK4 amplification (model weights provided in **Table 7,** row "LGG - CDK4") to compute the *CDK4* amplification scores in 273 low-grade gliomas (LGG) from the TCGA project. The inventors applied a univariate (Kaplan-Meier estimates) and a multivariate method (Cox proportional hazard model) for comparing overall survival between TCGA-LGG patients with and without *CDK4* amplification, and also between non-*CDK4* amplified patients at high and low risk of acquiring *CDK4* amplification in the future based on the signature-based approach. The inventors used the *CDK4*-specific signature weights for computing amplification scores, and the median value in the *CDK4* amplified group was used as threshold for classifying those patients without *CDK4* amplification but with different risks of acquiring it in the future. For the Cox proportional hazard models, the inventors included as covariates the fraction of genome altered and age at diagnosis. Age at diagnosis was categorised into four groups by dividing cohort distribution into quantiles.

As expected, TCGA-LGG patients with *CDK4* amplification survived shorter regardless of the fraction of genome altered (a genomic feature previously liked to poor prognosis) and age at diagnosis (HR = 5.48, p-value = 4.81e-07; **Figure 24a, Figure 25** - the latter shows that the amplification scores is associated with the largest hazard ratio, which was also the case when testing variables independently). However, the inventors hypothesised that the non-*CDK4* amplified group may in fact include two subsets of patients: one with worse prognosis where *CDK4* amplification will likely emerge in the future, and another with longer survival rates where *CDK4* amplification is unlikely to be acquired. Classification of the 253 TCGA-LGG patients without *CDK4* amplification into high and low-risk according to the signature-based amplification scores yielded a significant difference in overall survival after controlling for the fraction of genome altered and age at diagnosis (HR = 2.56, p-value = 0.017; **Figure 24a, Figure 26).** In fact, the TCGA-LGG patients without *CDK4* amplification that were predicted to likely acquire *CDK4* amplification presented similar overall survival to those who already had the amplification (HR = 1.96, p-value = 0.17; **Figure 27).**

Considering the limited progress in improving survival outcomes in glioma patients, the inventors then proceeded to explore the potential of integrating the signature-based predictor into the brain tumour risk stratification algorithm (Sepúlveda-Sánchez, J. M. et al., 2018; Louis et al. 2016) (i.e. applying both the method described herein and the brain tumour risk stratification algorithm). This algorithm currently classifies patients into three subgroups: i) IDH mutant, ii) IDH wildtype without 1p/19q codeletion and iii) IDH wildtype with 1p/19q codeletion, from lower to higher risk groups. Importantly, this algorithm does not currently include *CDK4* amplification as a molecular feature for classifying patients, probably due to its presence in only a fraction of the rapidly progressing gliomas at time of diagnosis (Richardson et al. 2018). In the analysis the inventors performed in TCGA-LGG, none of the patients included in the subtype with lowest risk (IDH-mutation with 1p/19q codeletion) was classified as having high risk of acquiring *CDK4* amplification based on the signature-based predictor. In the IDH-mutant subtype, which generally exhibits a more favourable prognosis compared to its IDH-wildtype counterparts, the inventors observed substantial differences in survival rates across TCGA-LGG patients without *CDK4* amplification classified as high and low-risk according to our signature-based predictor (HR = 4.62, p-value = 0.058; **Figure 24b).** Similarly, classification of IDH-wildtype TCGA-LGG patients without *CDK4* amplification as likely and unlikely to acquire the oncogene amplification yielded a significant separation in overall survival (HR = 8.80, p-value = 0.021; **Figure 24b).** All these survival analyses were corrected by the fraction of genome altered and age of diagnosis via multivariate Cox proportional hazards model (i.e. adding these variables in the Cox ,odel as independent variables - fraction of genome altered was added as a continuous/numeric variable, and age was included as categorical variable splitting samples in 4 groups according to quantile distribution.) to account for confounding factors. Hence, this predictor-based subclassification provides an opportunity to define tailored follow-up and treatment strategies for glioma patients, even within a particular molecular subtype.

Example 9 - Pan-oncogene model

The inventors also constructed a model capturing the potential for amplification of a tumour, not constrained to any particular oncogene. Following the same rationale as the oncogene-specific tumour-specific models and the pan-cancer oncogene-specific models, the predictive model of amplification is a linear model of copy-number signature activities - i.e. the result of adding up a set of multiplications of copy-number signature activities by matched weights. For this model, the weights are the average vector of all the pan-cancer oncogene-specific model weights $\omega_{OG,m}$ , above defined.

*Retrospective assessment of predictor performance*

The inventors first tested the performance of the signature-based predictor of amplification using retrospective data from two different pan-cancer cohorts (TCGA and PCAWG) with copy number profiles derived from different sequencing platforms (SNP6 arrays and WGS, respectively). They hypothesized that amplification scores should be higher in samples with an oncogene amplification. To avoid the circularity of predicting an event already present in the genome with a metric that depends on its structure, the inventors removed amplified segments covering one of the 271 oncogenes included in the list (see Example 3) and both of its adjacent segments.

Thus, the inventors first applied the predictor back to the training cohort **(Figure 15a)** computing patient CIN signature activities after removing the signal corresponding to the copy number segment harbouring the oncogene. The inventors compared the amplification scores between samples with and without at least an oncogene amplification using Wilcoxon two-sided rank test. In addition, we also assessed the sensitivity and specificity of our signature-based approach to predict the amplification of a specific oncogene via ROC curves and evaluation of the AUC. This analysis was done only for those oncogenes amplified in a minimum of 10 samples, and the average performance across all oncogenes was also computed. The inventors observed significantly higher scores for samples with an amplification than for those without across 254 oncogenes (p-value < 2.2e-16, Wilcoxon two-sided test; Figure 15b). The mean of the area under the receiver operating characteristic curve (AUC) for specific oncogenes was 0.79 [95%CI: 0.78-0.80] **(Figure 16)**.

Performance assessment using the same strategy across an independent cohort of 1,932 tumours from the Pan-Cancer Analysis of Whole Genomes (PCAWG) project also showed significantly higher scores for samples with an amplification across 168 oncogenes (p-value < 2.2e-16, Wilcoxon two-sided rank test; **Figure 15b)** and a mean AUC of 0.74 [95% CI: 0.73-0.75] **(Figure 16)**.

To illustrate the pan-cancer performance of predicting the amplification of a specific oncogene, the inventors computed the distribution of pan-cancer amplification scores (from the pan-cancer, pan-oncogene model) in samples with and without an amplification in 10 key oncogenes (FGFR1, CDK4, EGFR, MYC, ERBB2, CCNE1, MET, CCND1, KRAS and SOX17) both in the TCGA **(Figure 18)** and the PCAWG cohorts (Figure 19). Across both cohorts, they observed significant differences between amplification scores in samples with and without amplification. This supports the accuracy of the model predicting the amplification of a specific oncogene in a pan-cancer manner.

Furthermore, to illustrate that the accuracy of predicting oncogene amplifications was not driven by differences in amplification rates and CIN signatures across tumour types, the inventors also performed a cancer-specific assessment of the signature-based predictor by averaging prediction performance (AUC values) across all oncogenes by tumour type in both the TCGA **(Figure 20a)** and the PCAWG cohorts **(Figure 20c).** Only oncogenes amplified in a minimum of 10 samples per tumour type were included in this analysis. The data show that the area under the ROC curve is consistently well above 0.5 when predicting the amplification of oncogenes in an individual tumour type. This suggests that our method can be applied to predict the amplification of specific oncogenes in a specific tumour type. As an example, MYC ROC curves in specific tumour types in TCGA **(Figure 20b)** and PCAWG **(Figure 20d)** consistently show high sensitivities and specificities.

The presence of large-scale rearrangements in a tumour sample may lead to the amplification of distant genomic regions linked to the amplified oncogene. For that reason, besides removing the amplified segments covering one of the 271 oncogenes and its adjacent segments, the inventors also removed segments connected to them via complex/simple structural variants (SV). These segments connected to the regions with amplification were detected in the PCAWG cohort by Kim et al, Nature Genetics 2020, and available in Supplementary Table S1 of the Kim et al. article. This is to confirm that the performance of the approach in this retrospective scenario is not entirely driven by the amplification of other non-adjacent segments (results on **Figure 21).** Wilcoxon two-sided rank test showed that the amplification scores were significantly higher even after removing segments connected to amplifications via simple/complex SV. This shows that the signal that enables accurate performance in the retrospective scenario comes from copy-number alterations unrelated to the amplifications that are predicted. This analysis was done only in the subset of 980 PCAWG samples with available amplicon annotations.

To assess the performance of the pan-oncogene model, the inventors used the Glioma Longitudinal Analysis Consortium (GLASS) cohort consisting of 154 diffuse glioma patients that were biopsied at two time points. This cohort includes multiple patients that acquire many amplifications but that are not restricted to specific oncogenes, hence making it a suitable cohort for the evaluation of the pan-oncogene model.

In particular, the inventors downloaded copy number profiles from a total of 504 diffuse glioma samples included in the Glioma Longitudinal Analysis Consortium (GLASS) dataset. After filtering samples with no detectable CIN (<20 CNAs), the dataset included 154 patients with at least one pair of time-separated tumour samples which were used to test the predictor. Only one sample pair per patient was considered in the analysis. If a patient had multiple sample pairs, we prioritised the pair where the early biopsy was obtained from the primary tumour. If this was not feasible, the inventors randomly selected one of the available sample pairs. Copy number profiles were derived from whole genome sequencing data for 36 patients (72 samples, 36 pairs) and from whole exome sequencing data for 118 patients (236 samples, 118 pairs) as described in Barthel et al. 2019. For each patient, the inventors computed amplification scores in the early time point biopsy and observed the presence of a new focal amplification in the later time point biopsy ($\geq$6 copies with respect to the early sample). Wilcoxon two-sided rank test was used to compare amplification scores between patients with and without a new focal amplification in the later relapse biopsy. Spearman's rho was also computed to assess the correlation of amplification scores and the number of amplifications acquired in the relapse.

Thus, the inventors applied the method to the early biopsy and observed the emergence of any amplification in the later relapse biopsy (Figure 15c). Tumour pairs with new amplifications at the second time point had significantly higher scores than pairs without amplifications **(Figure 15d).** The amplification scores also correlated with the total number of amplifications acquired (**Figure 17a**).

Example 10 - Discussion

These examples present an approach for forecasting oncogene amplifications using CIN signatures. These results support the hypothesis that active mechanisms of CIN determine how readily a tumour acquires amplifications. The richer the substrate of amplifications, the more likely it is that one of these amplifications eventually confers a fitness advantage through amplification of an oncogene and becomes overrepresented in the bulk of the tumour in the future.

CIN is known to drive substantial genomic evolution contributing to drug resistance (Lukow et al. 2021). For many targeted therapies, a frequent mechanism of resistance is oncogene amplification (Koivisto, P. et al., 1997; Corcoran et al. 2010). Forecasting which patients are at risk of acquiring this kind of genetic alteration has the potential to enable the deployment of adaptive or combination treatment approaches. As *MET* amplification-driven resistant NSCLC following EGFR inhibition can be treated with MET inhibitors, identifying patients at high-risk of *MET* amplification at diagnosis using the approach described herein can enable upfront combination treatment with both EGFR and MET inhibitors. Finally, by identifying low-grade glioma patients likely to acquire *CDK4* amplifications, they may be classified into a higher risk category enabling better clinical management.

Our study presents some limitations. For example, the inventors assumed in this study that the CIN processes identified using our CIN signatures are ongoing and contributing to the future potential of acquiring an amplification. However, some of these processes may be historical and no longer relevant. Improved profiling of tumours at single cell resolution can help distinguish ongoing from historical CIN and potentially improve forecasting performance. Indeed, by identifying events that are unique to single cells and their associated signatures using methods as described herein, it is possible to identify signatures that are currently active (as signatures that are historically active are expected to generate events that have either been selected out or subject to clonal amplifications leading to shared rather than unique events).

This work presents compelling evidence of how chromosomal instability can constrain the evolution of a tumour. This study represents a fundamental step change in modelling the tumour genome and serves as a proof-of-principle for forecasting other types of mutations, which may ultimately enable the creation of a definitive model of tumour evolution.

References

Raine KM, et al. ascatNgs: Identifying Somatically Acquired Copy-Number Alterations from Whole-Genome Sequencing Data. Curr Protoc Bioinformatics. 2016 Dec 8;56:15.9.1-15.9.17.

Van Loo, P. et al. Allele-specific copy number analysis of tumors. Proc. Natl. Acad. Sci. U. S. A. 107, 16910-16915 (2010).

Langmead, B., Trapnell, C., Pop, M. et al. Ultrafast and memory-efficient alignment of short DNA sequences to the human genome. Genome Biol 10, R25 (2009).

Carter, S., Cibulskis, K., Helman, E. et al. Absolute quantification of somatic DNA alterations in human cancer. Nat Biotechnol 30, 413-421 (2012).

Favero F, Joshi T, Marquard AM, Birkbak NJ, Krzystanek M, Li Q, Szallasi Z, Eklund AC. Sequenza: allele-specific copy number and mutation profiles from tumor sequencing data. Ann Oncol. 2015 Jan;26(1):64-70.

Adalsteinsson, V.A., Ha, G., Freeman, S.S. et al. Scalable whole-exome sequencing of cell-free DNA reveals high concordance with metastatic tumors. Nat Commun 8, 1324 (2017).

Drews, R.M., Hernando, B., Tarabichi, M. et al. A pan-cancer compendium of chromosomal instability. Nature 606, 976-983 (2022).

Macintyre, G. et al. Copy number signatures and mutational processes in ovarian carcinoma. Nat. Genet. 50, 1262-1270 (2018).

Christopher Pockrandt and others, GenMap: ultra-fast computation of genome mappability, Bioinformatics, Volume 36, Issue 12, June 2020, Pages 3687-3692.

Mehran Karimzadeh and others, Umap and Bismap: quantifying genome and methylome mappability, Nucleic Acids Research, Volume 46, Issue 20, 16 November 2018, Page e120.

Li H, Durbin R. 2009. Fast and accurate short read alignment with Burrows-Wheeler transform. Bioinformatics 25: 1754-1760.

Li H. (2013) Aligning sequence reads, clone sequences and assembly contigs with BWA-MEM. arXiv:1303.3997v2

Seshan VE, Olshen A (2023). DNAcopy: DNA Copy Number Data Analysis. doi:10.18129/B9.bioc.DNAcopy, R package version 1.76.0, ioconductor.org/packages/DNAcopy.

Scheinin, I. et al. DNA copy number analysis of fresh and formalin-fixed specimens by shallow whole-genome sequencing with identification and exclusion of problematic regions in the genome assembly. Genome Res. 24, 2022-2032 (2014).

Madrid, L. et al. Predicting response to cytotoxic chemotherapy. bioRxiv 2023.01.28.525988 (2023) doi:10.1101/2023.01.28.525988.

Alexandrov, L. B. et al. The repertoire of mutational signatures in human cancer. Nature 578, 94-101 (2020).

Grün, B. & Leisch, F. FlexMix Version 2: Finite Mixtures with Concomitant Variables and Varying and Constant Parameters. J. Stat. Softw. 028, (2008).

Gerstung, M. et al. The evolutionary history of 2,658 cancers. Nature 578, 122-128 (2020). Dentro, S. C. et al. Characterizing genetic intra-tumor heterogeneity across 2,658 human cancer genomes. Cell 184, 2239-2254.e39 (2021).

ICGC/TCGA Pan-Cancer Analysis of Whole Genomes Consortium. Pan-cancer analysis of whole genomes. Nature 578, 82-93 (2020).

Hübschmann, D. et al. Analysis of mutational signatures with yet another package for signature analysis. Genes Chromosomes Cancer 60, 314-331 (2021).

Li, H. et al. The Sequence Alignment/Map format and SAMtools. Bioinformatics 25, 2078-2079 (2009).

McGranahan, N., Burrell, R. A., Endesfelder, D., Novelli, M. R. & Swanton, C. Cancer chromosomal instability: therapeutic and diagnostic challenges. EMBO Rep. 13,528-538 (2012).

Steele, C. D. et al. Signatures of copy number alterations in human cancer. Nature 606, 984-991 (2022).

Therasse, P. et al. New guidelines to evaluate the response to treatment in solid tumors. European Organization for Research and Treatment of Cancer, National Cancer Institute of the United States, National Cancer Institute of Canada. J. Natl. Cancer Inst. 92, 205-216 (2000).

Ramirez, M. et al. Diverse drug-resistance mechanisms can emerge from drug-tolerant cancer persister cells. Nat. Commun. 7, 1-8 (2016).

Mermel, C. H. et al. GISTIC2.0 facilitates sensitive and confident localization of the targets of focal somatic copy-number alteration in human cancers. Genome Biol. 12, R41 (2011).

Lehmann, B. D. et al. Refinement of Triple-Negative Breast Cancer Molecular Subtypes: Implications for Neoadjuvant Chemotherapy Selection. PLoS One 11, e0157368 (2016).

Barthel, F. P. et al. Longitudinal molecular trajectories of diffuse glioma in adults. Nature 576, 112-120 (2019).

Minussi, D. C. et al. Breast tumours maintain a reservoir of subclonal diversity during expansion. Nature 592, 302-308 (2021).

Bailey, M. H. et al. Comprehensive Characterization of Cancer Driver Genes and Mutations. Cell 173, 371-385.e18 (2018).

Jassal, B. et al. The reactome pathway knowledgebase. Nucleic Acids Res. 48, D498-D503 (2020).

Martínez-Jiménez, F. et al. A compendium of mutational cancer driver genes. Nat. Rev. Cancer 20, 555-572 (2020).

Sondka, Z. et al. The COSMIC Cancer Gene Census: describing genetic dysfunction across all human cancers. Nat. Rev. Cancer 18, 696-705 (2018).

Zack, T. I. et al. Pan-cancer patterns of somatic copy number alteration. Nat. Genet. 45, 1134-1140 (2013).

Stratton, M. R., Campbell, P. J. & Futreal, P. A. The cancer genome. Nature 458, 719-724 (2009).

López, S. et al. Interplay between whole-genome doubling and the accumulation of deleterious alterations in cancer evolution. Nat. Genet. 52, 283-293 (2020).

Kim, H. et al. Extrachromosomal DNA is associated with oncogene amplification and poor outcome across multiple cancers. Nat. Genet. 52, 891-897 (2020).

Polak, P. et al. Cell-of-origin chromatin organization shapes the mutational landscape of cancer. Nature 518, 360-364 (2015).

Kaufmann, T. L. et al. MEDICC2: whole-genome doubling aware copy-number phylogenies for cancer evolution. Genome Biol. 23, 241 (2022).

Nik-Zainal, S. et al. The life history of 21 breast cancers. Cell 149, 994-1007 (2012).

Oxnard, G. R. et al. Assessment of Resistance Mechanisms and Clinical Implications in Patients With EGFR T790M-Positive Lung Cancer and Acquired Resistance to Osimertinib. JAMA Oncol 4, 1527-1534 (2018).

Jia, P. et al. Next-generation sequencing of paired tyrosine kinase inhibitor-sensitive and -resistant EGFR mutant lung cancer cell lines identifies spectrum of DNA changes associated with drug resistance. Genome Res. 23, 1434-1445 (2013).

Forst, D. A., Nahed, B. V., Loeffler, J. S. & Batchelor, T. T. Low-grade gliomas. Oncologist 19, 403-413 (2014).

Mirchia, K. et al. Total copy number variation as a prognostic factor in adult astrocytoma subtypes. Acta Neuropathol Commun 7, 92 (2019).

Sepúlveda-Sánchez, J. M. et al. SEOM clinical guideline of diagnosis and management of low-grade glioma (2017). Clin. Transl. Oncol. 20, 3-15 (2018).

Louis, D. N. et al. The 2016 World Health Organization Classification of Tumors of the Central Nervous System: a summary. Acta Neuropathol. 131, 803-820 (2016).

Richardson, T. E. et al. Genetic and Epigenetic Features of Rapidly Progressing IDH-Mutant Astrocytomas. J. Neuropathol. Exp. Neurol. 77, 542-548 (2018).

Lukow, D. A. et al. Chromosomal instability accelerates the evolution of resistance to anti-cancer therapies. Dev. Cell 56, 2427-2439.e4 (2021).

Koivisto, P. et al. Androgen Receptor Gene Amplification: A Possible Molecular Mechanism for Androgen Deprivation Therapy Failure in Prostate Cancer1. Cancer Res. 57, 314-319 (1997).

Corcoran, R. B. et al. BRAF gene amplification can promote acquired resistance to MEK inhibitors in cancer cells harboring the BRAF V600E mutation. Sci. Signal. 3, ra84 (2010).

Al Bakir M, et al. The evolution of non-small cell lung cancer metastases in TRACERx. Nature. 2023 Apr;616(7957):534-542.

The components and signatures in Drews et al. 2022 are reproduced below as **Tables 11 and 12.**

**Table 11.** Definition of the 43 mixture components used in Drews et al. Feature=One of the five fundamental features of copy number profiles quantified in Drews et al; Mean = If SD is present, then this is the mean of the Gaussian component, otherwise mean of the Poisson component; SD = If present, standard deviation of the Gaussian component; Weight = Weight in the mixture identified by the converged mixture model; Number = Number of component used as identifiers.

| Feature | Mean | SD | Weight | Number |
|---|---|---|---|---|
| segsize | 38420.7697 | 15438.8759 | 0.0150893 | 1 |
| segsize | 86319.107 | 30243.0733 | 0.02246956 | 2 |
| segsize | 171234.256 | 55096.0468 | 0.03478786 | 3 |
| segsize | 427454 | 174237 | 0.1237 | 4 |
| segsize | 918939.408 | 260126.652 | 0.07107799 | 5 |
| segsize | 1494667.9 | 404499.004 | 0.07395141 | 6 |
| segsize | 2496039.63 | 608098.846 | 0.09346463 | 7 |
| segsize | 3898217.97 | 583841.597 | 0.0566778 | 8 |
| segsize | 4908317.82 | 398896.482 | 0.01339669 | 9 |
| segsize | 6371179 | 985163 | 0.0659 | 10 |
| segsize | 8780945 | 1086234 | 0.0506 | 11 |
| segsize | 13435586 | 1439442 | 0.0414 | 12 |
| segsize | 18665397 | 1664701 | 0.0331 | 13 |
| segsize | 22913723.2 | 1028090.2 | 0.01095836 | 14 |
| segsize | 30753807 | 2370747.45 | 0.01373617 | 15 |
| segsize | 36812267.4 | 1799660.32 | 0.01285096 | 16 |
| segsize | 44866788 | 4250506.18 | 0.02235963 | 17 |
| segsize | 57831002.6 | 3574456.9 | 0.01970078 | 18 |
| segsize | 68086495.8 | 6023604.95 | 0.01683004 | 19 |

(continued)

| Feature | Mean | SD | Weight | Number |
|---|---|---|---|---|
| segsize | 81015695.9 | 3149494.22 | 0.01317039 | 20 |
| segsize | 93268268 | 10492824.6 | 0.01645642 | 21 |
| segsize | 154120037 | 27901372.7 | 0.02569139 | 22 |
| changepoint | 0.10594582 | 0.06312023 | 0.06094551 | 1 |
| changepoint | 0.34283504 | 0.1572276 | 0.11805556 | 2 |
| changepoint | 1.00818588 | 0.2528096 | 0.37043868 | 3 |
| changepoint | 1.40754134 | 0.3988254 | 0.06601043 | 4 |
| changepoint | 2.20266882 | 0.4656683 | 0.2117235 | 5 |
| changepoint | 3.20155742 | 0.32865165 | 0.02131326 | 6 |
| changepoint | 4.11442601 | 0.49429328 | 0.02397049 | 7 |
| changepoint | 5.13850887 | 1 .16026304 | 0.01390753 | 8 |
| changepoint | 6.93357762 | 1.76527488 | 0.01834703 | 9 |
| changepoint | 9.96341765 | 2.61954362 | 0.01218381 | 10 |
| bp10MB | 3.90E-08 | NA | 0.75713738 | 1 |
| bp10MB | 1.04971801 | NA | 0.23064104 | 2 |
| bp10MB | 5.43989834 | NA | 0.01222158 | 3 |
| bpchrarm | 0.05683922 | NA | 0.47183552 | 1 |
| bpchrarm | 1.9260777 | NA | 0.38270107 | 2 |
| bpchrarm | 7.06398355 | NA | 0.12115462 | 3 |
| bpchrarm | 18.1504596 | NA | 0.02221902 | 4 |
| bpchrarm | 46.6651445 | NA | 0.00208977 | 5 |
| osCN | 0.24540037 | NA | 0.91590854 | 1 |
| osCN | 2.42532595 | NA | 0.08006416 | 2 |
| osCN | 9.36453963 | NA | 0.00402731 | 3 |

Table 12. Signature definitions used in Drews et al. Columns = Mixture components; Rows = Signatures. Cells = Weight.

| | CX1 | CX2 | CX3 | CX4 | CX5 | CX7 | CX8 | CX9 |
|---|---|---|---|---|---|---|---|---|
| segsize1 | 0 | 0 | 0.000161 84 | 0 | 0.002163 51 | 0 | 0 | 0.010485 3 |
| segsize2 | 0 | 0.004842 54 | 0.001439 1 | 1.12E-12 | 1.31E-24 | 0 | 0 | 0.008863 83 |
| segsize3 | 1.80E-42 | 0.024084 67 | 0.001462 89 | 0.019717 36 | 1.10E-17 | 0 | 0 | 0.019546 22 |
| segsize4 | 4.93E-05 | 0.071322 46 | 1.98E-27 | 0.058888 86 | 0.030462 83 | 3.62E-19 | 1.82E-11 | 0.047604 96 |
| segsize5 | 0 | 0.063986 59 | 0 | 0.045371 72 | 0.053767 63 | 0 | 0.011922 21 | 0.045445 27 |
| segsize6 | 1.34E-15 | 0.053289 49 | 5.88E-17 | 0.035112 57 | 0.067033 74 | 0 | 1.71E-10 | 0.020035 37 |
| segsize7 | 0.001246 38 | 0.037081 2 | 0.008746 25 | 0.018896 91 | 0.070958 21 | 0 | 0 | 2.74E-14 |
| segsize8 | 0.000732 04 | 0.015215 3 | 0.012208 94 | 0.006856 98 | 0.043562 22 | 0 | 0 | 0 |
| segsize9 | 0.000267 15 | 0.006612 38 | 0.009996 93 | 0.001071 58 | 0.025702 63 | 4.00E-45 | 0 | 0 |
| segsize10 | 0.000186 58 | 0.006664 03 | 0.019639 67 | 0.002980 85 | 0.034705 04 | 0 | 0 | 0 |
| segsize11 | 0 | 0.005179 94 | 0.026051 92 | 0.004941 72 | 0.030683 29 | 0 | 0 | 0 |
| segsize12 | 0 | 0.002982 02 | 0.029969 57 | 0.006696 32 | 0.020356 81 | 0 | 0 | 0 |
| segsize13 | 0.000596 21 | 0.000838 85 | 0.022901 95 | 0.006631 82 | 0.010099 59 | 0 | 0 | 0.007066 06 |
| segsize14 | 0.000898 38 | 5.19E-24 | 0.015476 21 | 0.005454 47 | 0.003841 32 | 8.62E-43 | 8.28E-32 | 0.008614 75 |
| segsize15 | 0.001892 99 | 0 | 0.023483 16 | 0.013919 8 | 8.81E-09 | 0.007746 5 | 0.008003 51 | 0.012259 05 |
| segsize16 | 0.001792 58 | 0 | 0.010223 32 | 0.005073 05 | 2.84E-35 | 0 | 0.001715 33 | 0.007452 38 |
| segsize17 | 0.002457 39 | 0 | 0.014779 38 | 0.003581 63 | 0 | 0 | 0.009181 82 | 0.019815 39 |
| segsize18 | 0.001911 46 | 0 | 0.007311 91 | 0.001590 02 | 0 | 0 | 0.005144 97 | 0.012515 94 |
| segsize19 | 0.001341 38 | 0 | 0.005831 15 | 0.001173 43 | 0 | 0 | 0.002712 98 | 0.010932 06 |
| segsize20 | 0.001088 47 | 0 | 0.001819 84 | 0.000187 12 | 0 | 0 | 0 | 0.005039 65 |
| segsize21 | 0.002751 01 | 0 | 0.003167 85 | 5.46E-30 | 0 | 0 | 0.002548 37 | 0.011036 19 |
| segsize22 | 0.003206 38 | 0 | 0 | 0 | 0 | 0 | 0 | 0 |
| changepoin t1 | 0.007145 53 | 0 | 0.005705 93 | 0.006444 97 | 0 | 0.371466 04 | 0 | 0 |
| changepoin t2 | 0.001811 6 | 0.002314 29 | 0.020661 05 | 0 | 0.025922 85 | 0.620780 4 | 0 | 0 |
| changepoin t3 | 0.005714 1 | 0.302647 35 | 0.062988 24 | 0 | 0 | 0 | 0 | 0 |
| changepoin t4 | 0.003951 69 | 0.159043 2 | 0.044087 44 | 0.143703 95 | 4.89E-07 | 0 | 0 | 0 |

| | CX1 | CX2 | CX3 | CX4 | CX5 | CX7 | CX8 | CX9 |
|---|---|---|---|---|---|---|---|---|
| changepoin t5 | 0.000818 06 | 0.001303 16 | 0.018847 35 | 0.447355 42 | 2.87E-22 | 0 | 0 | 0 |
| changepoin t6 | 0 | 0 | 0.001134 33 | 0.046267 92 | 0.005720 15 | 0 | 0.257728 04 | 0.080345 99 |
| changepoin t7 | 0 | 0 | 2.48E-07 | 0.013576 | 0.000604 75 | 0 | 0.262413 68 | 0.083380 61 |
| changepoin t8 | 7.51E-07 | 2.87E-05 | 0.000141 51 | 0.013358 23 | 0.001870 02 | 0 | 0.230635 18 | 0.086011 07 |
| changepoin t9 | 5.55E-06 | 0.000213 89 | 6.04E-11 | 0.003975 18 | 0.001563 76 | 0 | 0.161477 58 | 0.084338 21 |
| changepoin t10 | 3.72E-23 | 4.57E-05 | 1.20E-42 | 0.001200 84 | 0.000662 37 | 0 | 0.046516 25 | 0.117254 83 |
| bp10MB1 | 0.602878 1 | 0.017073 79 | 0.207462 46 | 1.65E-11 | 2.13E-07 | 0 | 0 | 4.12E-39 |
| bp10MB2 | 0.234540 46 | 0.071340 7 | 0.236155 67 | 7.28E-09 | 0.081240 33 | 0 | 0 | 0 |
| bp10MB3 | 0.003535 74 | 0.077466 6 | 0.018107 88 | 0.049594 95 | 0.056056 63 | 0 | 0 | 0.011633 67 |
| bpchrarm1 | 0.076709 44 | 0 | 8.65E-12 | 0 | 0 | 0 | 0 | 0 |
| bpchrarm2 | 0.039898 53 | 0 | 0.039072 97 | 0 | 0 | 0 | 0 | 0 |
| bpchrarm3 | 0.001231 7 | 0.023689 54 | 0.030150 67 | 0.008671 43 | 0.007378 51 | 0 | 0 | 0.025643 05 |
| bpchrarm4 | 0 | 0.006978 82 | 0 | 0.001882 51 | 0.014755 45 | 0 | 0 | 0 |
| bpchrarm5 | 0 | 0.001026 92 | 0 | 0 | 0 | 0 | 0 | 3.10E-06 |
| osCN1 | 0.001341 09 | 4.71E-25 | 0.080729 28 | 0 | 0.334153 12 | 0 | 0 | 0.262370 02 |
| osCN2 | 0 | 0.037034 66 | 0.020083 09 | 0.022435 13 | 0.076734 59 | 0 | 0 | 0.002307 12 |
| osCN3 | 0 | 0.007693 25 | 0 | 0.003387 28 | 0 | 7.01E-06 | 0 | 0 |

**Table 12 (continued).** Signature definitions normalised per signature.

| | CX10 | CX11 | CX12 | CX13 | CX14 | CX15 | CX16 | CX17 |
|---|---|---|---|---|---|---|---|---|
| segsize1 | 0.049090 98 | 0.153255 6 | 0.542871 95 | 0 | 0 | 0 | 0.194129 1 | 0.003021 81 |
| segsize2 | 0.062405 38 | 0.133703 65 | 0.295015 78 | 0 | 0 | 1.36E-34 | 0.053132 93 | 0.006445 56 |
| segsize3 | 0.115454 27 | 0.159663 16 | 0.139738 29 | 0 | 0 | 1.17E-37 | 0.014584 14 | 0.011311 12 |
| segsize4 | 0.150823 64 | 0.137439 67 | 0 | 0 | 0 | 1.17E-19 | 0.007637 52 | 0.018338 61 |
| segsize5 | 0.042825 43 | 0.012869 7 | 0 | 0 | 8.89E-14 | 0.006756 61 | 0.007518 93 | 0.025673 3 |
| segsize6 | 5.97E-16 | 0 | 0 | 0 | 0.006578 32 | 0.017889 6 | 0.005815 55 | 0.023978 68 |
| segsize7 | 0 | 0 | 0 | 0 | 7.36E-13 | 0.033679 93 | 0.008717 48 | 0.021186 97 |
| segsize8 | 0 | 0 | 0 | 0 | 0.009435 1 | 0.026109 13 | 0.012459 1 | 0.019626 75 |
| segsize9 | 0 | 0 | 0 | 0 | 0.006031 25 | 0.0174711 | 0.011347 12 | 0.010033 73 |
| segsize10 | 0 | 0 | 0 | 0 | 2.76E-43 | 0.029263 29 | 0.015379 62 | 0.016005 13 |
| segsize11 | 1.14E-22 | 0 | 0 | 0 | 0 | 0.034468 9 | 0.011314 85 | 0.022535 18 |
| segsize12 | 0.001092 33 | 0 | 0 | 0 | 3.72E-26 | 0.033800 73 | 0.009719 38 | 0.024128 89 |
| segsize13 | 7.82E-15 | 0 | 0 | 0 | 0 | 0.022647 92 | 0.005472 9 | 0.026728 35 |
| segsize14 | 4.00 E-44 | 0 | 0 | 0 | 3.08E-27 | 0.016246 92 | 0.004343 68 | 0.016478 04 |
| segsize15 | 1.26E-36 | 0 | 0 | 0 | 0.000322 32 | 0.025077 39 | 0 | 0.017593 23 |
| segsize16 | 0 | 0 | 0 | 0 | 0.038787 36 | 0.007450 51 | 0.000310 91 | 0.009218 88 |
| segsize17 | 0 | 0 | 0 | 0 | 0.036322 37 | 0.012869 8 | 0 | 0.011824 35 |
| segsize18 | 0.000763 24 | 0 | 0 | 0 | 0.033321 87 | 0.002900 87 | 0 | 0.015730 12 |
| segsize19 | 0 | 0 | 0 | 0 | 0.050692 18 | 0.004478 89 | 0 | 0.010589 43 |
| segsize20 | 0 | 0 | 0 | 0 | 0.089617 7 | 2.54E-06 | 0 | 1.24E-35 |
| segsize21 | 0 | 0 | 0 | 0 | 0.070834 35 | 0.0040517 | 0 | 7.90E-11 |
| segsize22 | 0 | 0 | 0 | 0 | 0.245369 63 | 0 | 0 | 0 |
| changepoin t1 | 0.038025 24 | 0.012312 51 | 0 | 0 | 0.199358 79 | 0.050830 28 | 0.002899 8 | 0 |
| changepoin t2 | 0 | 0 | 0 | 0 | 0.213324 53 | 0.101168 92 | 0 | 0 |

| | | | | | | | | |
|---|---|---|---|---|---|---|---|---|
| changepoin t3 | 4.66E-34 | 0 | 0 | 0 | 1.41E-12 | 0.075782 69 | 0 | 0.028231 78 |
| changepoin t4 | 0.054246 71 | 2.27E-31 | 0 | 0 | 1.35E-10 | 0.034761 57 | 0.037978 7 | 0.071093 28 |
| changepoin t5 | 0.171547 5 | 3.48E-10 | 0 | 0 | 9.78E-33 | 3.03E-07 | 0.153292 79 | 0.134141 73 |
| changepoin t6 | 0.027719 87 | 0 | 0 | 0 | 0 | 0 | 0.085675 9 | 0.036897 86 |
| changepoin t7 | 0.011989 16 | 0 | 0 | 0 | 0 | 0 | 0.012790 51 | 0.026261 75 |
| changepoin t8 | 0.009621 78 | 0.000100 44 | 0 | 0 | 0 | 4.96E-05 | 0.014137 91 | 0.022583 89 |
| changepoin t9 | 0.003663 84 | 3.63E-05 | 0 | 0.102813 73 | 8.66E-39 | 0.000145 21 | 0.008377 55 | 0.013634 31 |
| changepoin t10 | 0 | 0.000124 2 | 0 | 0.887093 3 | 2.85E-33 | 0 | 0.003308 37 | 0.013015 23 |
| bp10MB1 | 1.94E-08 | 4.59E-22 | 0 | 0 | 6.09E-23 | 3.17E-10 | 0.058677 45 | 5.26E-08 |
| bp10MB2 | 6.05E-17 | 6.33E-37 | 0 | 0 | 1.03E-28 | 0.180420 5 | 0.063262 3 | 0.100611 76 |
| bp10MB3 | 0.076840 01 | 0.069363 49 | 2.14E-24 | 0 | 0 | 0.031417 76 | 0.067932 4 | 0.037035 85 |
| bpchrarm1 | 0.002008 04 | 0 | 0 | 0 | 1.08E-08 | 0 | 0 | 0 |
| bpchrarm2 | 0 | 0 | 0 | 0 | 3.59E-24 | 0.046830 46 | 0 | 0.018122 08 |
| bpchrarm3 | 0.003259 85 | 0.019247 26 | 1.00E-45 | 0 | 0 | 0.027572 27 | 0.016621 04 | 0.031504 75 |
| bpchrarm4 | 0.017386 71 | 0.006487 27 | 0 | 0 | 0 | 1.29E-12 | 0.013803 78 | 0.000696 97 |
| bpchrarm5 | 0.000976 41 | 0.002387 64 | 0.000591 88 | 0.010092 9 | 0 | 0 | 0.000416 07 | 0 |
| osCN1 | 0.069690 12 | 0.209602 16 | 0 | 0 | 0 | 0.123581 92 | 0.042018 65 | 0.118685 07 |
| osCN2 | 0.084353 72 | 0.080326 54 | 0.016640 69 | 0 | 0 | 0.032272 63 | 0.046685 89 | 0.035918 45 |
| osCN3 | 0.006215 59 | 0.003080 44 | 0.005141 42 | 0 | 4.33E-06 | 0 | 0.010237 75 | 0.001117 11 |

All references cited herein are incorporated herein by reference in their entirety and for all purposes to the same extent as if each individual publication or patent or patent application was specifically and individually indicated to be incorporated by reference in its entirety.

The specific embodiments described herein are offered by way of example, not by way of limitation. Various modifications and variations of the described compositions, methods, and uses of the technology will be apparent to those skilled in the art without departing from the scope and spirit of the technology as described. Any sub-titles herein are included for convenience only and are not to be construed as limiting the disclosure in any way. Unless context dictates otherwise, the descriptions and definitions of the features set out above are not limited to any particular aspect or embodiment of the invention and apply equally to all aspects and embodiments which are described.

The methods of any embodiments described herein may be provided as computer programs or as computer program products or computer readable media carrying a computer program which is arranged, when run on a computer, to perform the method(s) described above.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined, without departing from the scope or spirit of the invention.

It must be noted that, as used In the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

**Claims**

1. A computer-implemented method of predicting whether a tumour is likely to acquire a gene amplification, the method comprising:

   obtaining a tumour copy number profile for the tumour;
   determining, for each of one or more signatures of chromosomal instability, a metric indicative of the presence of the signature in the tumour copy number profile, wherein a signature of chromosomal instability comprises a set of weights for each of a plurality of components each associated with a copy number feature of a set of copy number features quantified for a plurality of segments in the copy number profile, wherein the weights are indicative of the probability that a mutational process associated with the signature generates a segment having characteristics defined by the component; and
   determining whether the tumour is likely to acquire a gene amplification based on the output of a model trained to take as input the values of said metrics and produce as output a score indicative of the likelihood that the tumour will acquire a gene amplification, wherein the model has been trained using copy number profiles for a plurality of tumours comprising tumours that have the gene amplification and tumours that do not have the gene amplification.

2. The method of claim 1, wherein the gene is an oncogene, optionally an oncogene selected from the genes listed in Tables 7 and 8, and/or wherein the model is specific to a single gene, and/or wherein a gene amplification is the presence of a segment encompassing the gene in a tumour copy number profile, where the segment has an absolute copy number >=6 or a copy number >=4 above tumour ploidy, optionally where the segment has an absolute copy number >=8.

3.  The method of any preceding claim, wherein the copy number features comprises or consists of: (a) segment size, copy number change-point, and breakpoint count in one or both flanking regions of a predetermined length, optionally wherein the breakpoint count is a summarised value of the breakpoint count in the upstream and downstream flanking regions of a predetermined length around the segment; or (b) segment size, copy number change-point, breakpoint count per predetermined length of sequence, breakpoint count per chromosome arm, and number of segments with oscillating copy number, optionally wherein the predetermined length of sequence is 10Mb,
    wherein the components define a respective distribution of values of the associated copy number feature, optionally wherein the components are selected from those in Table 1 or those in Table 11, or corresponding distributions obtaining by fitting mixture models to values for the set of copy number features obtained from copy number profiles from a plurality of tumour samples.

4.  The method of any preceding claim, wherein the metric indicative of the presence of a signature in the tumour copy number profile is the activity of the signature in the tumour copy number profile, optionally wherein the activity of one or more signatures in a tumour copy number profile is determined by:

    obtaining, for each component, the sum of probabilities associated with the respective component for each of a plurality of segments in the copy number profile; and
    determining a linear combination of one or more signatures comprising the signature that satisfies the equation: $PbC \approx E \times SbC$ where E is a vector of size n comprising coefficients $E_{1,...,n}$ where $E_i$ is the activity of signature i; PbC is a vector of size c, each element in the vector representing a sum of probabilities associated with a respective component; and SbC is a matrix of size c by n, each value representing the weight of a component in a signature I, and the probability associated with a component for a segment is the probability of a segment with a copy number feature value equal to the value for the segment having been drawn from a distribution associated with the component.

5.  The method of any preceding claims, wherein the signatures are selected from those in Table 3 or those in Table 12, optionally wherein the signatures are selected from CX1, CX2, CX3, CX4, 5, CX8, CX9, CX10, and CX13 defined in Table 3 or Table 12 or corresponding signatures obtained by quantifying the set of copy number features in a plurality of tumour samples, and identifying one or more signatures likely to result in the copy number profiles of the plurality of tumour samples by nonnegative matrix factorisation.

6.  The method of any preceding claim, wherein the trained model is a model that produces a score indicative of the likelihood of acquiring an oncogene amplification as a weighted combination of the values of the metrics indicative of the presence of the one or more signatures in the tumour copy number profile, optionally wherein the combination is a linear combination and/or wherein the model produces a score according to the equation: $score = \sum_{m=1}^{n} CX_m \omega_m$ where $CX_m$ is the activity of signature m in the tumour copy number profile, $\omega_m$ is a signature weight for signature m for the gene, and n is the total number of signatures considered in the model.

7.  The method of claim 6, wherein the weights of the model are determined in a tumour type-specific manner using a reference cohort of samples as, for each signature, a ratio of (i) an averaged signature score of all amplified segments spanning the gene in samples of the specific tumour type in the reference cohort and (ii) an averaged signature scores of all non-diploid segments found in the reference cohort, wherein the signature score for a segment and signature is a metric indicative of association between the signatures of chromosomal instability and the segment, optionally wherein said metric is the sum of the product of probabilities associated with each of the plurality of components of the signature for the segment by the corresponding weight in the signature, the probability for a segment being the probability that a segment with a copy number feature value equal to the value for the segment having been drawn from a distribution associated with the component, or an activity corrected and/or normalised version of said sum, wherein an activity corrected version of said sum is the value of the sum multiplied by the activity of the signature in the copy number profile and a normalised version of said sum or activity corrected sum is a value obtaining by dividing the value by the sum of corresponding values for each of the one or more signatures.

8.  The method of claim 6, wherein the weights of the model are determined in a tumour type-agnostic manner using a reference cohort of samples as, for each signature, a ratio of (i) a weighted average signature score of all amplified segments spanning the gene in samples of a specific tumour type in the reference cohort, wherein the average signature scores in the specific tumour type are weighted by the normalised empirical frequency of the gene amplification in the respective tumour type in the reference cohort and (ii) an averaged signature scores of all non-diploid segments found in the reference cohort, wherein the signature score for a segment and signature is a metric indicative of association between the signatures of chromosomal instability and the segment, optionally wherein said metric is the sum of the product of probabilities associated with each of the plurality of components of the signature for the segment by the corresponding weight in the signature, the probability for a segment being the probability that a segment with a copy number feature value equal to the value for the segment having been drawn from a distribution associated with the component, or an activity corrected and/or normalised version of said sum, wherein an activity corrected version of said sum is the value of the sum multiplied by the activity of the signature in the copy number profile and a normalised version of said sum or activity corrected sum is a value obtaining by dividing the value by the sum of corresponding values for each of the one or more signatures.

9. The method of claim 7 or claim 8, wherein the weights determined in a tumour type-specific manner are used when the reference cohort comprises less than a threshold number of samples of the specific tumour type or the frequency of amplification of the gene in the specific tumour type in the reference cohort is lower than a threshold frequency, optionally wherein the threshold number of sample is 5 and the threshold frequency is 5%, and the weights determined in a tumour type-agnostic manner are used otherwise.

10. The method of any preceding claim, wherein the tumour is a primary and/or untreated tumour, or wherein the tumour is a treated and/or advanced tumour and the weights of the model are determined in a tumour type-agnostic manner using for (i) only tumour types in which the gene is recurrently amplified in the reference cohort, optionally wherein a gene is recurrently amplified in a tumour type if it is amplified in more than 5% of the samples of the tumour type in the reference cohort.

11. The method of any preceding claim, further comprising classifying the tumour between a first class that has a high likelihood of acquiring the gene amplification and a second class that has a low likelihood of acquiring the gene amplification, wherein the tumour is classified in the first class when the output of the model is above a predetermined threshold,

    wherein the predetermined threshold has been determined by obtaining the output of the model for each of a plurality of samples in a reference cohort with a known expected % of samples with the gene amplification, and selecting the threshold that is such that a % of samples corresponding to the expected % of samples are classified in the first class, or

    wherein the predetermined threshold has been determined by obtaining the output of the model for each of a plurality of samples in a reference cohort with known gene amplification status, and selecting the threshold that satisfies one or more predetermined criteria that apply to the sensitivity, selectivity or Youden's index when used to classify the samples between the first and second classes.

12. The method of any preceding claim, wherein the model has been trained using a reference cohort comprising samples that are one or more of: the same tumour type, the tumour grade/stage, undergone the same treatment, have the same driver mutations, have been analysed with the same sequencing technology, as the tumour to be analysed; and/or wherein the tumour is selected from sarcoma, breast cancer, lung adenocarcinoma, ovarian cancer, bladder carcinoma, glioblastoma, head and neck squamous cell carcinoma, renal adenocarcinoma, lung squamous cell carcinoma, adenocarcinoma, stomach adenocarcinoma, esophagal carcinoma, liver hepatocellular carcinoma, Cervical Squamous Cell Carcinoma and Endocervical Adenocarcinoma, Uterine Corpus Endometrial Carcinoma, Colon Adenocarcinoma, Brain Lower Grade Glioma.

13. A computer-implemented method of predicting whether a tumour is likely to respond to a therapy, the method comprising:
predicting whether the tumour is likely to acquire a gene amplification using the method of any preceding claim, wherein the gene amplification is associated with resistance to the therapy, and whether a tumour predicted as likely to acquire the gene amplification is unlikely to respond to the therapy, optionally wherein:

    the gene is MET and the therapy is an EGFR inhibitor, optionally wherein the method further comprises recommending a subject with a tumour that is unlikely to respond to the therapy for upfront treatment with both EGFR and MET inhibitors;
    the gene is BRAF and the therapy is a MEK inhibitor;

the gene is androgen receptor (AR), the tumour is a prostate tumour and the therapy is androgen deprivation therapy.

14. A computer-implemented method of providing a prognosis for a subject with a tumour, the method comprising: predicting whether the tumour is likely to acquire a gene amplification using the method of any of claims 1 to 22, wherein the gene amplification is associated with poor prognosis, optionally wherein the gene is CDK4 and the tumour is a low grade glioma.

15. A computer-implemented of providing a model for predicting whether a tumour is likely to acquire a gene amplification, the method comprising:

obtaining, a plurality of tumour copy number profiles for a reference cohort of samples, the plurality of tumour copy number profiles comprising copy number profiles that comprise the gene amplification and copy number profiles that do not comprise the amplification; and

determining, for each of one or more signatures of chromosomal instability, a weight that is a ratio of (i) an average signature score of all amplified segments spanning the gene in samples of a specific tumour type in the reference cohort, optionally wherein the average signature scores in the specific tumour type are weighted by the normalised empirical frequency of the gene amplification in the respective tumour type in the reference cohort and (ii) an average signature scores of all non-diploid segments found in the reference cohort, wherein the signature score for a segment and signature is a metric indicative of association between the signatures of chromosomal instability and the segment,

optionally wherein said metric is the sum of the product of probabilities associated with each of the plurality of components of the signature for the segment by the corresponding weight in the signature, the probability for a segment being the probability that a segment with a copy number feature value equal to the value for the segment having been drawn from a distribution associated with the component, or an activity corrected and/or normalised version of said sum, wherein an activity corrected version of said sum the value of the sum multiplied by the activity of the signature in the copy number profile and a normalised version of said sum or activity corrected sum is a value obtaining by dividing the value by the sum of corresponding values for each of the one or more signatures.

FIG. 1A

Obtain cohort of samples — 110

Obtain sequence data — 112

Obtain tumour copy number profile for sample — 114

Quantify set of copy number features of non-diploid segments (events) in copy number profile — 116

Determine event-specific associations with signature(s) of chromosomal instability — 118

Determine sample-level exposures to signature(s) of chromosomal instability — 120

Determine sample-normalised event specific associations for events including gene amplification event (if present) — 122

For each sample in cohort

Obtain signature weight for gene amplification — 124

Determine threshold for amplification score — 126

Provide results to user — 128

FIG. 1B

200

Obtain tumour sample

202

Determine whether sample has high/low likelihood of oncogene amplification (see Fig. 1A)

204

Classify subject as responder / non – responder to therapy

205

Classify subject as poor / good prognosis

206

Identify treatment

208

Treat subject with identified therapy

**Fig. 2**

3

2

6

1

103

102

101

**Fig. 3**

FIG. 4

FIG. 5

FIG. 6

FIG. 7

EP 4 557 299 A1

**No driver amplification**

Division rate $\beta(1-\mu)$

Death rate
$\alpha$

Amplification rate $\beta\mu$

**Driver amplification**

Division rate $\beta(1+s)$

Death rate
$\alpha(1-s)$

FIG. 8

FIG. 9

**b)**

**e)**

FIG. 9 (Continued)

FIG. 9 (Continued)

FIG. 10

FIG. 10 (Continued)

**d)** EGFR

EGFR

**e)** ERBB2

ERBB2

FIG. 10 (Continued)

**Factors contributing to oncogene amplification**

FIG. 11

**a)** Rationale

**b)** Signature weights derivation

$$W_{CX} = \frac{\overline{CX}_a \times f_{amps}}{\overline{CX}_{background}}$$

For all 6,335 TCGA samples,
9 CIN signatures, and 271 oncogenes

**c)** Signature-based predictor of focal amplifications

$$\text{Amplification score} = \sum_{s=CX_1}^{CX_n} \text{Sample activities} \times \text{Signature weights } (W_{CX})$$

FIG. 12

Cohort of interest

Is there a reference cohort available?

No → Interested in one of the 271 oncogenes in our list?

No → Apply global model

Yes → Is the oncogene relevant for a specific tumour type?

No → Apply pan-cancer model

Yes → Apply tumour-specific model

Thresholding of scores based on literature

Correlate scores with the number of focal amplifications

Yes → Train the model in the reference cohort → Apply model in the cohort of interest → Thresholding of scores based on the reference cohort (Bayesian approach) → Classify samples as either acquiring or not acquiring an oncogene amplification

FIG. 13

FIG. 14A-1

FIG. 14A-1 (Continued)

FIG. 14A-2

FIG. 14A-2 (Continued)

Youden's index (mean & sd across runs)

FIG. 14B-1

FIG. 14B-2

**a)** Retrospective assessment of performance

**b)**

FIG. 15

**c)** Glioma Longitudinal Analysis Consortium cohort

Apply predictor    Count new amplifications

Time

**d)**

GLASS

p = 0.0012

Amplification scores in early biopsy

No (n=96)    Yes (n=58)

Focal amplification
in later biopsy

FIG. 15 (Continued)

FIG. 16

FIG. 17

FIG. 18

FIG. 19

FIG. 20

FIG. 20 (Continued)

EP 4 557 299 A1

FIG. 21

A

FIG. 22

B Amplification threshold independent to tumour's ploidy

C Amplification threshold based on tumour's ploidy

FIG. 22 (Continued)

NSCLC cell lines

Persister-derived resistant colonies

| | Resistant clones with *MET* amplified | *MET* fold-change by ddPCR | Signature-based *MET* amplification score |
|---|---|---|---|
| HCC827 | 11/12 | 3.41 ± 1.06 | 4.25e-4 |
| HCC4006 | 0/7 | 1.22 ± 0.02 | 3.01e-4 |
| PC9 | 0/4 | 0.95 ± 0.30 | 2.01e-4 |
| H1975 | 0/13 | 0.60 ± 0.07 | 3.43e-4 |

Long-term culture with EGFR tyrosine kinase inhibitor

FIG. 23A

Jai2013

FIG. 23B

$R = 0.92$, $p = 0.26$

Frequency of resistant clones
with MET amplification

FIG. 23C

FIG. 24

## Hazard ratio

FIG. 25

## Hazard ratio

FIG. 26

FIG. 27

FIG. 28

FIG. 29

FIG. 30A

AR
AUC = 0.76

ERBB2
AUC = 0.94

FIG. 30B

Europäisches
Patentamt

European
Patent Office

Office européen
des brevets

## EUROPEAN SEARCH REPORT

**Application Number**

EP 23 38 3180

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2023/057392 A1 (CAMBRIDGE ENTPR LTD [GB] ET AL.) 13 April 2023 (2023-04-13) * abstract * * page 2, line 1 – page 3, line 25 * * page 12, line 25 – page 18, line 16 * | 1-15 | INV. G16B20/10 C12Q1/68 G16B40/20 |
| A | PLADSEN ARNE V. ET AL: "DNA copy number motifs are strong and independent predictors of survival in breast cancer", COMMUNICATIONS BIOLOGY, vol. 3, no. 1, 1 December 2020 (2020-12-01), XP055931148, DOI: 10.1038/s42003-020-0884-6 Retrieved from the Internet: URL:https://www.nature.com/articles/s42003 -020-0884-6.pdf> * abstract * * page 2, column 1, paragraph 5 * * page 7, column 1, paragraph 6 – column 2, paragraph 2 * | 1-15 | |
| A | C. D. GREENMAN ET AL: "PICNIC: an algorithm to predict absolute allelic copy number variation with microarray cancer data", BIOSTATISTICS, vol. 11, no. 1, 1 January 2010 (2010-01-01), pages 164-175, XP055001227, ISSN: 1465-4644, DOI: 10.1093/biostatistics/kxp045 * the whole document * | 1-15 | TECHNICAL FIELDS SEARCHED (IPC)  G16B |

-/--

The present search report has been drawn up for all claims

| Place of search | Date of completion of the search | Examiner |
|---|---|---|
| Munich | 2 May 2024 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 23 38 3180

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STEELE CHRISTOPHER D ET AL: "Signatures of copy number alterations in human cancer", NATURE,, vol. 606, no. 7916, 15 June 2022 (2022-06-15), pages 984-991, XP037898747, DOI: 10.1038/S41586-022-04738-6 [retrieved on 2022-06-15] * abstract * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 2 May 2024 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

page 2 of 2

EP 4 557 299 A1

## ANNEX TO THE EUROPEAN SEARCH REPORT
## ON EUROPEAN PATENT APPLICATION NO.

EP 23 38 3180

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2024

| Patent document cited in search report | Publication date | Patent family member(s) | | Publication date |
|---|---|---|---|---|
| WO 2023057392 A1 | 13-04-2023 | AU | 2022358908 A1 | 11-04-2024 |
| | | CA | 3233198 A1 | 13-04-2023 |
| | | WO | 2023057392 A1 | 13-04-2023 |

For more details about this annex : see Official Journal of the European Patent Office, No. 12/82

EPO FORM P0459

127

**EP 4 557 299 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023057392 A, Drews

**Non-patent literature cited in the description**

- **JIA et al.** Signature-based MET amplification scores were computed in parental cell lines and displayed in the y axis. *Genome Research*, 2013
- **CHAVES-URBANO, B** ; **HERNANDO, B** ; **GARCIA, M. J** ; **MACINTYRE, G**. CNpare: matching DNA copy number profiles. *Bioinformatics*, 2022, vol. 38, 3638-3641
- **WICKHAM, H et al.** Welcome to the tidyverse. *Journal of Open Source Software*, 2019, vol. 4
- **EPSKAMP, S.** ; **CRAMER, A. O. J.** ; **WALDORP, L. J.** ; **SCHMITTMANN, V. D** ; **BORSBOOM, D**. qgraph: Network Visualizations of Relationships in Psychometric Data. *Journal of Statistical Software*, 2012, vol. 48, 1-18
- **GU, Z.** ; **EILS, R.** ; **SCHLESNER, M**. Complex heatmaps reveal patterns and correlations in multidimensional genomic data. *Bioinformatics*, 2016, vol. 32, 2847-2849
- **LAWRENCE, M. et al.** Software for Computing and Annotating Genomic Ranges. *PLoS Computational Biology*, 2013, vol. 9
- **HUBER, W. et al.** Orchestrating high-throughput genomic analysis with Bioconductor. *Nature Methods*, 2015, vol. 12, 115-121
- **KASSAMBARA, A.** ; **KOSINSKI, M** ; **BIECEK, P** ; **FABIAN, S**. *Drawing Survival Curves using 'ggplot2*, 2021
- **THERNEAU**. *T. M. A package for survival analysis in R*, 2023
- **KEILWAGEN, J** ; **GROSSE, I.** ; **GRAU, J**. Area under precision-recall curves for weighted and unweighted data. *PLoS One*, 2014, vol. 9, e92209
- **ROBIN, X. et al.** pROC: an opensource package for R and S+ to analyze and compare ROC curves. *BMC Bioinformatics*, 2011, vol. 12, 77
- **DURINCK, S.** ; **SPELLMAN, P. T.** ; **BIRNEY, E.** ; **HUBER, W**. Mapping identifiers for the integration of genomic datasets with the R/Bioconductor package biomaRt.. *Nat. Protoc*, 2009, vol. 4, 1184-1191
- **GROLEMUND, G** ; **WICKHAM, H**. Dates and Times Made Easy with lubridate. *Journal of Statistical Software*, 2011, vol. 40, 1-25

- **USHEY, K.** ; **ALLAIRE, J. J.** ; **WICKHAM, H.** ; **RITCHIE, G.** rstudioapi: Safely Access the RStudio API. *CRAN.R-project.org/package=rstudioapi*, 2022
- **GU, Z.** ; **GU, L** ; **EILS, R.** ; **SCHLESNER, M.** ; **BRORS, B**. circlize implements and enhances circular visualization in R. *Bioinformatics*, 2014, vol. 30, 2811-2812
- **WICKHAM, H.** Reshaping Data with the reshape Package. *Journal of Statistical Software*, 2007, vol. 21, 1-20
- **WICKHAM, H.** ; **FRANÇOIS, R** ; **HENRY, L** ; **MÜLLER, K** ; **VAUGHAN, D.** A Grammar of Data Manipulation. *CRAN.R-project.org/package=dplyr*, 2023
- **ARORA, S.** ; **MORGAN, M** ; **CARLSON, M** ; **PAGÈS, H**. GenomeInfoDb: Utilities for manipulating chromosome names, including modifying them to follow a particular naming style. *bioconductor.org/packages/-GenomeInfoDb*, 2023
- **SCHEININ, I et al.** DNA copy number analysis of fresh and formalin-fixed specimens by shallow whole-genome sequencing with identification and exclusion of problematic regions in the genome assembly. *Genome Res*, 2014, vol. 24, 2022-2032
- **HUBER, W. et al.** Orchestrating high-throughput genomic analysis with Bioconductor.. *Nature Methods*, 2015, vol. 12, 115-121
- **LÓPEZ, S. et al.** Interplay between whole-genome doubling and the accumulation of deleterious alterations in cancer evolution. *Nat. Genet.*, 2020, vol. 52, 283-293
- **RAINE KM et al.** Identifying Somatically Acquired Copy-Number Alterations from Whole-Genome Sequencing Data.. *Curr Protoc Bioinformatics*, 08 December 2016, vol. 56, 15.9.1-15.9.17
- **VAN LOO, P et al.** Allele-specific copy number analysis of tumors. *Proc. Natl. Acad. Sci. U. S. A.*, 2010, vol. 107, 16910-16915

- **LANGMEAD, B** ; **TRAPNELL, C** ; **POP, M et al.** Ultrafast and memory-efficient alignment of short DNA sequences to the human genome.. *Genome Biol*, 2009, vol. 10, R25

- **CARTER, S** ; **CIBULSKIS, K.** ; **HELMAN, E et al.** Absolute quantification of somatic DNA alterations in human cancer. *Nat Biotechnol*, 2012, vol. 30, 413-421

- **FAVERO F** ; **JOSHI T** ; **MARQUARD AM** ; **BIRKBAK NJ** ; **KRZYSTANEK M** ; **LI Q** ; **SZALLASI Z** ; **EKLUND AC**. Sequenza: allele-specific copy number and mutation profiles from tumor sequencing data. *Ann Oncol.*, January 2015, vol. 26 (1), 64-70

- **ADALSTEINSSON, V.A.** ; **HA, G** ; **FREEMAN, S.S et al.** Scalable whole-exome sequencing of cell-free DNA reveals high concordance with metastatic tumors. *Nat Commun*, 2017, vol. 8, 1324

- **DREWS, R.M.** ; **HERNANDO, B.** ; **TARABICHI, M et al.** A pan-cancer compendium of chromosomal instability. *Nature*, 2022, vol. 606, 976-983

- **MACINTYRE, G. et al.** Copy number signatures and mutational processes in ovarian carcinoma. *Nat. Genet.*, 2018, vol. 50, 1262-1270

- **CHRISTOPHER POCKRANDT**. GenMap: ultra-fast computation of genome mappability. *Bioinformatics*, June 2020, vol. 36 (12), 3687-3692

- **MEHRAN KARIMZADEH**. Umap and Bismap: quantifying genome and methylome mappability. *Nucleic Acids Research*, 16 November 2018, vol. 46 (20), e120

- **LI H** ; **DURBIN R**. Fast and accurate short read alignment with Burrows-Wheeler transform. *Bioinformatics*, 2009, vol. 25, 1754-1760

- **LI H**. Aligning sequence reads, clone sequences and assembly contigs with BWA-MEM. *arXiv:1303.3997v2*, 2013

- **SESHAN VE** ; **OLSHEN A**. *DNAcopy: DNA Copy Number Data Analysis*, 2023

- **SCHEININ, I. et al.** DNA copy number analysis of fresh and formalin-fixed specimens by shallow whole-genome sequencing with identification and exclusion of problematic regions in the genome assembly. *Genome Res*, 2014, vol. 24, 2022-2032

- **MADRID, L. et al.** Predicting response to cytotoxic chemotherapy. *bioRxiv 2023.01.28.525988*, 2023

- **ALEXANDROV, L. B. et al.** The repertoire of mutational signatures in human cancer. *Nature*, 2020, vol. 578, 94-101

- **GRÜN, B.** ; **LEISCH, F.** FlexMix Version 2: Finite Mixtures with Concomitant Variables and Varying and Constant Parameters. *J. Stat. Softw*, 2008, vol. 028

- **GERSTUNG, M. et al.** The evolutionary history of 2,658 cancers. *Nature*, 2020, vol. 578, 122-128

- **DENTRO, S. C. et al.** Characterizing genetic intra-tumor heterogeneity across 2,658 human cancer genomes. *Cell*, 2021, vol. 184, 2239-2254.e39

- ICGC/TCGA Pan-Cancer Analysis of Whole Genomes Consortium. Pan-cancer analysis of whole genomes. *Nature*, 2020, vol. 578, 82-93

- **HÜBSCHMANN, D. et al.** Analysis of mutational signatures with yet another package for signature analysis. *Genes Chromosomes Cancer*, 2021, vol. 60, 314-331

- **LI, H et al.** The Sequence Alignment/Map format and SAMtools. *Bioinformatics*, 2009, vol. 25, 2078-2079

- **MCGRANAHAN, N** ; **BURRELL, R. A** ; **ENDESFELDER, D.** ; **NOVELLI, M. R** ; **SWANTON, C.** Cancer chromosomal instability: therapeutic and diagnostic challenges. *EMBO Rep*, 2012, vol. 13, 528-538

- **STEELE, C. D. et al.** Signatures of copy number alterations in human cancer.. *Nature*, 2022, vol. 606, 984-991

- **THERASSE, P et al.** New guidelines to evaluate the response to treatment in solid tumors. European Organization for Research and Treatment of Cancer, National Cancer Institute of the United States, National Cancer Institute of Canada. *J. Natl. Cancer Inst.*, 2000, vol. 92, 205-216

- **RAMIREZ, M. et al.** Diverse drug-resistance mechanisms can emerge from drug-tolerant cancer persister cells.. *Nat. Commun*, 2016, vol. 7, 1-8

- **MERMEL, C. H. et al.** GISTIC2.0 facilitates sensitive and confident localization of the targets of focal somatic copy-number alteration in human cancers. *Genome Biol.*, 2011, vol. 12, R41

- **LEHMANN, B. D. et al.** Refinement of Triple-Negative Breast Cancer Molecular Subtypes: Implications for Neoadjuvant Chemotherapy Selection. *PLoS One*, 2016, vol. 11, e0157368

- **BARTHEL, F. P. et al.** Longitudinal molecular trajectories of diffuse glioma in adults. *Nature*, 2019, vol. 576, 112-120

- **MINUSSI, D. C. et al.** Breast tumours maintain a reservoir of subclonal diversity during expansion. *Nature*, 2021, vol. 592, 302-308

- **BAILEY, M. H. et al.** Comprehensive Characterization of Cancer Driver Genes and Mutations. *Cell*, 2018, vol. 173, 371-385.e18

- **JASSAL, B. et al.** The reactome pathway knowledgebase. *Nucleic Acids Res.*, 2020, vol. 48, D498-D503

- **MARTÍNEZ-JIMÉNEZ, F. et al.** A compendium of mutational cancer driver genes. *Nat. Rev. Cancer*, 2020, vol. 20, 555-572

- **SONDKA, Z et al.** The COSMIC Cancer Gene Census: describing genetic dysfunction across all human cancers. *Nat. Rev. Cancer*, 2018, vol. 18, 696-705
- **ZACK, T. I. et al.** Pan-cancer patterns of somatic copy number alteration. *Nat. Genet.*, 2013, vol. 45, 1134-1140
- **STRATTON, M. R.** ; **CAMPBELL, P. J.** ; **FUTREAL, P. A**. The cancer genome. *Nature*, 2009, vol. 458, 719-724
- **LÓPEZ, S et al.** Interplay between whole-genome doubling and the accumulation of deleterious alterations in cancer evolution. *Nat. Genet.*, 2020, vol. 52, 283-293
- **KIM, H. et al.** Extrachromosomal DNA is associated with oncogene amplification and poor outcome across multiple cancers. *Nat. Genet.*, 2020, vol. 52, 891-897
- **POLAK, P. et al.** Cell-of-origin chromatin organization shapes the mutational landscape of cancer. *Nature*, 2015, vol. 518, 360-364
- **KAUFMANN, T. L. et al.** MEDICC2: whole-genome doubling aware copy-number phylogenies for cancer evolution. *Genome Biol.*, 2022, vol. 23, 241
- **NIK-ZAINAL, S. et al.** The life history of 21 breast cancers. *Cell*, 2012, vol. 149, 994-1007
- **OXNARD, G. R et al.** Assessment of Resistance Mechanisms and Clinical Implications in Patients With EGFR T790M-Positive Lung Cancer and Acquired Resistance to Osimertinib. *JAMA Oncol*, 2018, vol. 4, 1527-1534
- **JIA, P et al.** Next-generation sequencing of paired tyrosine kinase inhibitor-sensitive and -resistant EGFR mutant lung cancer cell lines identifies spectrum of DNA changes associated with drug resistance. *Genome Res*, 2013, vol. 23, 1434-1445
- **FORST, D. A.** ; **NAHED, B. V.** ; **LOEFFLER, J. S.** ; **BATCHELOR, T. T**. Low-grade gliomas.. *Oncologist*, 2014, vol. 19, 403-413
- **MIRCHIA, K. et al.** Total copy number variation as a prognostic factor in adult astrocytoma subtypes. *Acta Neuropathol Commun*, 2019, vol. 7, 92
- **SEPÚLVEDA-SÁNCHEZ, J. M et al.** SEOM clinical guideline of diagnosis and management of low-grade glioma (2017).. *Clin. Transl. Oncol*, 2018, vol. 20, 3-15
- **LOUIS, D. N et al.** The 2016 World Health Organization Classification of Tumors of the Central Nervous System: a summary. *Acta Neuropathol.*, 2016, vol. 131, 803-820
- **RICHARDSON, T. E et al.** Genetic and Epigenetic Features of Rapidly Progressing IDH-Mutant Astrocytomas. *J. Neuropathol. Exp. Neurol.*, 2018, vol. 77, 542-548
- **LUKOW, D. A. et al.** Chromosomal instability accelerates the evolution of resistance to anti-cancer therapies. *Dev. Cell*, 2021, vol. 56, 2427-2439.e4
- **KOIVISTO, P. et al.** Androgen Receptor Gene Amplification: A Possible Molecular Mechanism for Androgen Deprivation Therapy Failure in Prostate Cancer1. *Cancer Res.*, 1997, vol. 57, 314-319
- **CORCORAN, R. B. et al.** BRAF gene amplification can promote acquired resistance to MEK inhibitors in cancer cells harboring the BRAF V600E mutation. *Sci. Signal.*, 2010, vol. 3, ra84
- **AL BAKIR M et al.** The evolution of non-small cell lung cancer metastases in TRACERx. *Nature*, April 2023, vol. 616 (7957), 534-542